# EUROPEAN PATENT SPECIFICATION

(11) **EP 0 730 590 B1**
(45) Date of publication and mention of the grant of the patent: **17.01.2001**
(21) Application number: 95901915.9
(22) Date of filing: 14.11.1994
(51) Int. Cl.: C07D 261/04, C07D 413/12, A61K 31/42, C07D 498/10, C07D 413/06, C07D 413/04, C07F 9/653, C07F 9/6571

(54) **ISOXAZOLINE AND ISOXAZOLE FIBRINOGEN RECEPTOR ANTAGONISTS**
ISOXAZOLINE UND ISOXAZOLE DERIVATE ALS FIBRINOGEN REZEPTOR ANTAGONISTEN
ANTAGONISTES DE RECEPTEUR DE FIBRINOGENE, A BASE D'ISOXAZOLINE ET D'ISOXAZOLE

(30) Priority: 24.11.1993 US 157598; 22.04.1994 US 232961; 10.11.1994 US 337920
(43) Date of publication of application: 11.09.1996
(60) Divisional application: 99119541.3
(73) Proprietor: Dupont Pharmaceuticals Company, Wilmington, DE 19805 (US)
(72) Inventor: WITYAK, John, West Grove, PA 19390 (US); XUE, Chu-Biao, Hockessin, DE 19707-2400 (US); SIELECKI-DZURDZ, Thais, Motria, Newark, DE 19711-3450 (US); OLSON, Richard, Eric, Wilmington, DE 19809-1320 (US); DeGRADO, William, Frank, Moylan, PA 19063-4207 (US); CAIN, Gary, Avonn, Wilmington, DE 19809 (US); BATT, Douglas Guy, Wilmington, DE 19803 (US); PINTO, Donald, Newark, DE 19702 (US)
(74) Representative: von Kreisler, Alek, Dipl.-Chem.
(86) International application number: US9413155
(87) International publication number: WO9514683

(56) References cited:
- EP-A- 0 478 328
- EP-A- 0 512 831
- EP-A- 0 525 629
- WO-A-93/16697
- JOURNAL OF MEDICINAL CHEMISTRY, vol.35, no.23, 13 November 1992, WASHINGTON US pages 4393 - 4407 LEO ALLIG ET AL 'Low molecular weight,non-peptide fibrinogen receptor antagonists'

## Description

This invention relates to novel isoxazolines and isoxazoles which are useful as antagonists of the platelet glycoprotein IIb/IIIa fibrinogen receptor complex, to pharmaceutical compositions containing such compounds, processes for preparing such compounds, and to the use of these compounds, alone or in combination with other therapeutic agents, for the preparation of a medicament for the inhibition of platelet aggregation, for the treatment of thrombosis and/or for the treatment of thromboembolic disorders.

### BACKGROUND OF THE INVENTION

Hemostasis is the normal physiological process in which bleeding from an injured blood vessel is arrested. It is a dynamic and complex process in which platelets play a key role. Within seconds of vessel injury, resting platelets become activated and are bound to the exposed matrix of the injured area by a phenomenon called platelet adhesion. Activated platelets also bind to each other in a process called platelet aggregation to form a platelet plug. The platelet plug can stop bleeding quickly, but it must be reinforced by fibrin for long-term effectiveness, until the vessel injury can be permanently repaired.

Thrombosis may be regarded as the pathological condition wherein improper activity of the hemostatic mechanism results in intravascular thrombus formation. Activation of platelets and the resulting platelet aggregation and platelet factor secretion has been associated with a variety of pathophysiological conditions including cardiovascular and cerebrovascular thromboembolic disorders, for example, the thromboembolic disorders associated with unstable angina, myocardial infarction, transient ischemic attack, stroke, atherosclerosis and diabetes. The contribution of platelets to these disease processes stems from their ability to form aggregates, or platelet thrombi, especially in the arterial wall following injury.

Platelets are activated by a wide variety of agonists resulting in platelet shape change, secretion of granular contents and aggregation. Aggregation of platelets serves to further focus clot formation by concentrating activated clotting factors at the site of injury. Several endogenous agonists including adenosine diphosphate (ADP), serotonin, arachidonic acid, thrombin, and collagen, have been identified. Because of the involvement of several endogenous agonists in activating platelet function and aggregation, an inhibitor which acts against all agonists would represent a more efficacious antiplatelet agent than currently available antiplatelet drugs, which are agonist-specific.

Current antiplatelet drugs are effective against only one type of agonist; these include aspirin, which acts against arachidonic acid; ticlopidine, which acts against ADP; thromboxane A₂ synthetase inhibitors or receptor antagonists, which act against thromboxane A₂; and hirudin, which acts against thrombin.

Recently, a common pathway for all known agonists has been identified, namely platelet glycoprotein IIb/IIIa complex (GPIIb/IIIa), which is the membrane protein mediating platelet aggregation. A recent review of GPIIb/IIIa is provided by Phillips et al. *Cell* (1991) 65: 359-362. The development of a GPIIb/IIIa antagonist represents a promising new approach for antiplatelet therapy.

GPIIb/IIIa does not bind soluble proteins on unstimulated platelets, but GPIIb/IIIa in activated platelets is known to bind four soluble adhesive proteins, namely fibrinogen, von Willebrand factor, fibronectin, and vitronectin. The binding of fibrinogen and von Willebrand factor to GPIIb/IIIa causes platelets to aggregate. The binding of fibrinogen is mediated in part by the Arg-Gly-Asp (RGD) recognition sequence which is common to the adhesive proteins that bind GPIIb/IIIa.

In addition to GPIIb/IIIa, increasing numbers of other cell surface receptors have been identified which bind to extracellular matrix ligands or other cell adhesion ligands thereby mediating cell-cell and cell-matrix adhesion processes. These receptors belong to a gene superfamily called integrins and are composed of heterodimeric transmembrane glycoproteins containing α- and β-subunits. Integrin subfamilies contain a common β-subunit combined with different α-subunits to form adhesion receptors with unique specificity. The genes for eight distinct β-subunits have been cloned and sequenced to date.

Two members of the β1 subfamily, α4/β1 and α5/β1 have been implicated in various inflammatory processes. Antibodies to a4 prevent adhesion of lymphocytes to synovial endothelial cells *in vitro,* a process which may be of importance in rheumatoid arthritis (VanDinther-Janssen et al., J. Immunol., 1991, 147:4207). Additional studies with monoclonal anti-α4 antibodies provide evidence that α4/β1 may additionally have a role in allergy, asthma, and autoimmune disorders (Walsh et al., J. Immunol., 1991, 146:3419; Bochner et al., J. Exp. Med., 1991 173:1553; Yednock et al., Nature, 1992, 356:63). Anti-α4 antibodies also block the migration of leukocytes to the site of inflammation (Issedutz et al., J. Immunol., 1991, 147:4178).

The αᵥ/β₃ heterodimer, commonly referred to as the vitronectin receptor, is another member of the β₃ integrin subfamily and has been described in platelets, endothelial cells, melanoma, smooth muscle cells and on the surface of osteoclasts (Horton and Davies, J. Bone Min. Res. 1989, 4:803-808; Davies et al., J. Cell. Biol. 1989, 109:1817-1826; Horton, Int. J. Exp. Pathol., 1990, 71:741-759). Like GPIIb/IIIa, the vitronectin receptor binds a variety of RGD-containing adhesive proteins such as vitronectin, fibronectin, VWF, fibrinogen, osteopontin, bone sialo protein II and thrombosponden in a manner mediated by the RGD sequence. Possible roles for αᵥ/β₃ in angiogenesis, tumor progression, and neovascularization have been proposed (Brooks et al., Science, 1994, 264:569-571). A key event in bone resorption is the adhesion of osteoclasts to the matrix of bone. Studies with monoclonal antibodies have implicated the αᵥ/β₃ receptor in this process and suggest that a selective αᵥ/β₃ antagonist would have utility in blocking bone resorption (Horton et al., J. Bone Miner. Res., 1993, 8:239-247; Helfrich et al., J. Bone Miner. Res., 1992, 7:335-343).

Several RGD-peptidomimetic compounds have been reported which block fibrinogen binding and prevent the formation of platelet thrombi.
EP-A-0478363 relates to compounds having the general formula: 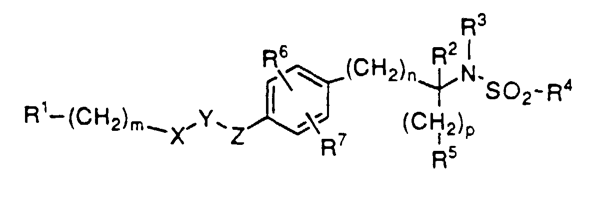
EP-A-0478328 relates to compounds having the general formula: 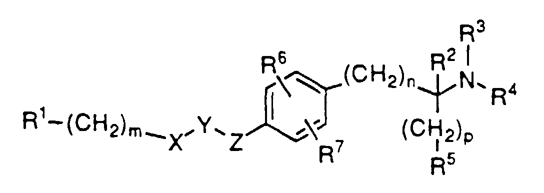
EP-A-0525629 (corresponds to
   CA-A- 2,074,685) discloses compounds having the general formula: 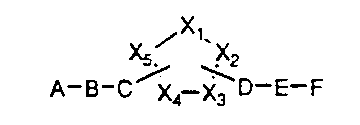 WO 9307867 relates to compounds having the general formula: 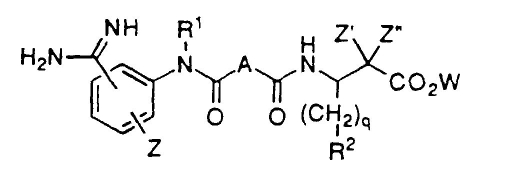
EP-A-04512831 relates to compounds having the general formula:

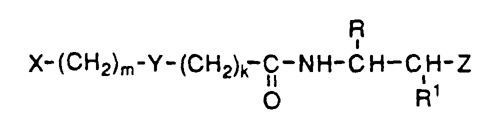

None of the above references teaches or suggests the compounds of the present invention which are described in detail below.

### SUMMARY OF THE INVENTION

The present invention provides nonpeptide compounds of the Formula I (described below) which bind to integrin receptors thereby altering cell-matrix and cell-cell adhesion processes. The compounds of the present invention are useful for the treatment of inflammation, bone degradation, tumors, metastases, thrombosis, cell aggregation-related conditions in a mammal. The compounds of Formula I are useful as antagonists of the platelet glycoprotein IIb/IIIa complex. The compounds of Formula I inhibit the binding of fibrinogen to platelet glycoprotein IIb/IIIa complex and inhibit the aggregation of platelets.

The present invention also provides pharmaceutical compositions containing such compounds of Formula I and a pharmaceutically acceptable carrier, and the use of compounds of Formula I for preparing medicaments using such compounds for the inhibition of platelet aggregation, for the treatment of thrombosis and/or for the treatment of thromboembolic disorders.

The above medicament may be utilized for treating cardiovascular disease, thrombosis or harmful thrombolysis, reperfusion injury, or restenosis. The medicament may contain the compound of Formula I alone or in combination with one or more additional therapeutic agents selected from: anti-coagulants such as warfarin or heparin; anti-platelet agents such as aspirin, piroxicam or ticlopidine; thrombin inhibitors such as boroarginine derivatives, hirudin or argatroban; or thrombolytic agents such as tissue plasminogen activator, anistreplase, urokinase or streptokinase; or combinations thereof.

The above medicament may also be utilized for the treatment or prevention of diseases which involve cell adhesion processes, including rheumatoid arthritis, asthma, allergies, adult respiratory distress syndrome, graft versus host disease, organ transplantation, septic shock, psoriasis, eczema, contact dermatitis, osteoporosis, osteoarthritis, atherosclerosis, metastasis, wound healing, diabetic retinopathy, inflammatory bowel disease and other autoimmune diseases.

### DETAILED DESCRIPTION OF THE INVENTION

The present invention provides:
(1) compounds of Formula I: 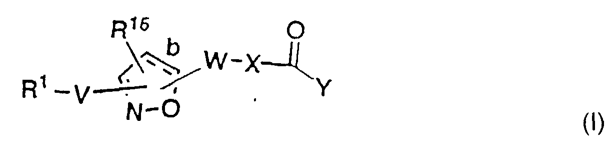 or a pharmaceutically acceptable salt or prodrug forms thereof wherein:
   - b: is a single or double bond;
   - R¹: is selected from R^{2a}(R³)N-, R²(R³)N(R²N=)C-, R^{2a}(R³)N(CH₂)_{p'}Z-, R²(R³)N(R²N=)C(CH₂)_{p"}Z-, R²(R³)N(R²N=)CN(R²)-, 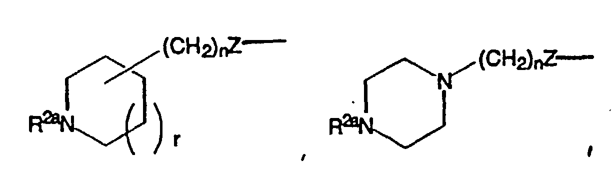 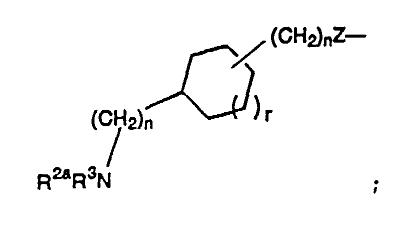
   - Z: is selected from a bond (i.e., is absent), O, S, S(=O), and S(=O)₂;
   - R² and R³: are independently selected from H, C₁-C₁₀ alkyl, C₃-C₆ alkenyl, C₃-C₁₁ cycloalkyl, C₄-C₁₁ cycloalkyl-alkyl, C₆-C₁₀ aryl, C₇-C₁₁ arylalkyl, C₂-C₇ alkyl-carbonyl,C₇-C₁₁ arylcarbonyl, C₂-C₁₀ alkoxycarbonyl, C₄-C₁₁ cycloalkoxycarbonyl, C₇-C₁₁ bicycloalkoxycarbonyl, C₇-C₁₁ aryloxycarbonyl, aryl(C₁-C₁₀ alkoxy)-carbonyl, (C₁-C₆ alkyl)carbonyloxy(C₁-C₄ alkoxy)car-bonyl, arylcarbonyloxy(C₁-C₄ alkoxy)carbonyl, and (C₄-C₁₁ cycloyalkylcarbonyl)oxy(C₁-C₄ alkoxy)carbonyl;
   - R^{2a}: is R² or R²(R³)N(R²N=)C-;
   - V: is selected from a single bond (i.e., V is not present), -(phenyl)-Q-, -(pyridyl)-Q- and -(pyridazinyl)-Q-, said phenyl, pyridyl and pyridazinyl residues being substituted with 0-2 groups independently selected from H, C₁-C₁₀ alkyl, hydroxy, C₁-C₁₀ alkoxy, nitro, (C₁-C₁₀ alkyl)-carbonyl, -N(R¹²)R¹³, cyano, and halo;
   - Q: is selected from a single bond (i.e., Q is not present), -(CH₂)ₘ-, -CH₂O-, and -OCH₂-, provided that when b is a single bond, and R¹-V- is a substituent on C5 of the central 5-membered ring of Formula I, then Q is not -CH₂O-;
   - W: is -(C(R⁴)₂)_{n'}-C(=O)-N(R^{5a})- or -C(=O)-N(R^{5a})-(C(R⁴)₂)_{n'}-;
   - X: is -(C(R⁴)₂)_{n'}-C(R⁴)(R⁸)-C(R⁴)(R^{4a})-, with the proviso that when n' is 0 or 1, then at least one of R^{4a} or R⁸ is other than H or methyl;
   - Y: is selected from hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxy-carbonyloxyalkyloxy, C₂-C₁₀ alkoxycarbonylalkyloxy, C₅-C₁₀ cycloalkylcarbonyloxyalkyloxy, C₅-C₁₀ cyclo-alkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyl-oxyalkyloxy, C₅-C₁₀ alkoxyalkylcarbonyloxyalkyloxy, C₅-C₁₀ (5-alkyl-1,3-dioxa-cyclopenten-2-one-yl)methyloxy, C₁₀-C₁₄ (5-aryl-1,3-dioxa-cyclopenten-2-one-yl)methyloxy, and (R²)(R³)N-(C₁-C₁₀ alkoxy)-;
   - R⁴: is selected from H, C₁-C₁₀ alkyl, C₁-C₁₀ alkycarbonyl, aryl, arylalkyl, cycloalkyl, and cycloalkylalkyl;
   alternatively, two R⁴ groups on adjacent carbon atoms may join to form a bond thereby to form a carbon-carbon double or triple bond between such adjacent carbon atoms;
   - R^{4a}: is selected from H, hydroxy, C₁-C₁₀ alkoxy, nitro, -N(R⁵)R^{5a}, -N(R¹²)R¹³, -N(R¹⁶)R¹⁷, C₁-C₁₀ alkyl substituted with 0-3 R⁶, aryl substituted with 0-3 R⁶, heteroaryl substituted with 0-3 R⁶, and C₁-C₁₀ alkylcarbonyl;
   - R^{4b}: is selected from H, C₁-C₆ alkyl, C₂-C₆ alkenyl, C₂-C₆ alkynyl, C₃-C₇ cycloalkyl, C₇-C₁₄ bicycloalkyl, hydroxy, C₁-C₆ alkoxy, C₁-C₆ alkylthio, C₁-C₆ alkylsulfinyl, C₁-C₆ alkylsulfonyl, nitro, C₁-C₆ alkylcarbonyl, C₆-C₁₀ aryl, -N(R¹²)R¹³, halo, CF₃, CN, (C₁-C₆ alkoxy)carbonyl, carboxy, piperidinyl, morpholinyl, and pyridinyl;
   - R⁵: is selected from H, C₁-C₈ alkyl, C₃-C₆ alkenyl, C₃-C₁₁ cycloalkyl, C₄-C₁₁ cycloalkylmethyl, C₆-C₁₀ aryl, C₇-C₁₁ arylalkyl, C₁-C₁₀ alkyl substituted with 0-6 R^{4b};
   - R^{5a}: is selected from H, hydroxy, C₁-C₈ alkyl, C₃-C₆ alkenyl, C₃-C₁₁ cycloalkyl, C₄-C₁₁ cycloalkylmethyl, C₁-C₆ alkoxy, benzyloxy, C₆-C₁₀ aryl, heteroaryl, heteroarylalkyl, C₇-C₁₁ arylalkyl, adamantylmethyl, and C₁-C₁₀ alkyl substituted with 0-2 R^{4b};
   alternatively, R⁵ and R^{5a} when both are substituents on the same nitrogen atom (as in -N(R⁵)R^{5a}) can be taken together with the nitrogen atom to which they are attached to form 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl or 1-piperazinyl, each being optionally substituted with C₁-C₆ alkyl, C₆-C₁₀ aryl, heteroaryl, C₇-C₁₁ arylalkyl,
   (C₁-C₆ alkyl)carbonyl, (C₃-C₇ cycloalkyl)carbonyl,
   (C₁-C₆ alkoxy)carbonyl, (C₇-C₁₁ arylalkoxy)carbonyl,
   C₁-C₆ alkylsulfonyl or C₆-C₁₀ arylsulfonyl;
   - R^{5b}: is selected from C₁-C₈ alkyl, C₂-C₆ alkenyl, C₃-C₁₁ cycloalkyl, C₄-C₁₁ cycloalkylmethyl, C₆-C₁₀ aryl, C₇-C₁₁ arylalkyl, and C₁-C₁₀ alkyl substituted with 0-2 R^{4b};
   - R⁶: is selected from H, C₁-C₁₀ alkyl, hydroxy, C₁-C₁₀ alkoxy, nitro, (C₁-C₁₀ alkyl)carbonyl, -N(R¹²)R¹³, cyano, halo, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, -SiMe₃, C₂-C₆ alkenyl, C₃-C₁₁ cycloalkyl, C₄-C₁₁ cycloalkylmethyl,
   -N(R⁵)R^{5a}, -S(O)ₘR⁵, -OR⁵;
   C₆-C₁₀ aryl optionally substituted with 1-3 groups selected from halogen, C₁-C₆ alkoxy, C₁-C₆ alkyl, CF₃, -S(O)ₘMe, and -NMe₂;
   C₇-C₁₁ arylalkyl, said aryl being optionally substituted with 1-3 groups selected from halogen,
   C₁-C₆ alkoxy, C₁-C₆ alkyl, CF₃, S(O)ₘMe, and -NMe₂;
   methylenedioxy when R⁶ is a substituent on aryl;
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁷;
   - R⁷: is selected from H, C₁-C₁₀ alkyl, hydroxy, C₁-C₁₀ alkoxy, nitro, (C₁-C₁₀ alkyl)carbonyl, -N(R¹²)R¹³, cyano, halo, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, C₂-C₆ alkenyl, C₃-C₁₁ cycloalkyl, C₄-C₁₁ cycloalkylmethyl, C₆-C₁₀ aryl, and C₇-C₁₁ arylalkyl;
   - R⁸: is selected from R⁶, C₁-C₁₀ alkyl substituted with 0-3 R⁶, C₂-C₁₀ alkenyl substituted with 0-3 R⁶, C₂-C₁₀ alkynyl substituted with 0-3 R⁶, C₃-C₈ cycloalkyl substituted with 0-3 R⁶, C₅-C₆ cycloalkenyl substituted with 0-3 R⁶, aryl substituted with 0-3 R⁶;
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁶;
   - R¹² and R¹³: are independently H, C₁-C₁₀ alkyl, (C₁-C₁₀ alkoxy)carbonyl, (C₁-C₁₀ alkyl)carbonyl, C₁-C₁₀ alkylsulfonyl, aryl(C₁-C₁₀ alkyl)sulfonyl, arylsulfonyl, aryl(C₂-C₁₀ alkenyl)sulfonyl, heteroarylsulfonyl, aryl, C₂-C₆ alkenyl, C₃-C₁₁ cycloalkyl, C₄-C₁₁ cycloalkylalkyl, C₇-C₁₁ arylalkyl, C₇-C₁₁ arylcarbonyl, C₄-C₁₁ cycloalkoxycarbonyl, C₇-C₁₁ bicycloalkoxycarbonyl, C₇-C₁₁ aryloxycarbonyl, heteroarylcarbonyl, heteroarylalkylcarbonyl, or aryl(C₁-C₁₀ alkoxy)carbonyl, wherein said aryls are substituted with 0-3 substituents selected from C₁-C₄ alkyl, C₁-C₄ alkoxy, halo, CF₃, and NO₂;
   - R¹⁵: is selected from H, hydroxy, C₁-C₁₀ alkyl, C₂-C₁₀ alkenyl, C₂-C₁₀ alkynyl, C₁-C₁₀ alkoxy, (C₁-C₁₀ alkoxy)carbonyl, -CO₂R⁵, and -C(=O)N(R⁵)R^{5a};
   - R¹⁶: is selected from -C(=O)-O-R^{18a}, -C(=O)-R^{18b}, -C(=O)N(R^{18b})₂, -C(=O)NHSO₂R^{18a}, -C(=O)NHC(=O)R^{18b}, -C(=O)NHC(=O)OR^{18a}, -C(=O)NHSO₂NHR^{18b}, -C(=S)-NH-R^{18b}, -NH-C(=O)-O-R^{18a}, -NH-C(=O)-R^{18b}, -NH-C(=O)-NH-R^{18b}, -SO₂-O-R^{18a}, -SO₂-R^{18a}, -SO₂-N(R^{18b})₂, -SO₂-NHC(=O)OR^{18b}, -P(=S)(OR^{18a})₂, -P(=O)(OR^{18a})₂, -P(=S)(R^{18a})₂, -P(=O)(R^{18a})₂, and 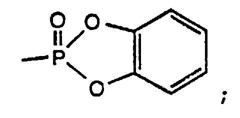
   - R¹⁷: is selected from H, C₁-C₁₀ alkyl, C₂-C₆ alkenyl, C₃-C₁₁ cycloalkyl, C₄-C₁₅ cycloalkyalkyl, aryl, and aryl(C₁-C₁₀ alkyl)-;
   - R^{18a}: is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹;
   a 5-10 membered heterocyclic ring system having 1-3 heteroatoms selected independently from O, S, and N, said heterocyclic ring being substituted with 0-4 R¹⁹;
   C₁-C₆ alkyl substituted with a 5-10 membered heterocyclic ring system having 1-3 heteroatoms selected independently from O, S, and N, said heterocyclic ring being substituted with 0-4 R¹⁹;
   - R^{18b}: is R^{18a} or H;
   - R¹⁹: is selected from H, halogen, CF₃, CN, NO₂, NR¹²R¹³, C₁-C₈ alkyl, C₂-C₆ alkenyl, C₂-C₆ alkynyl, C₃-C₁₁ cycloalkyl, C₄-C₁₁ cycloalkylalkyl, aryl, aryl(C₁-C₆ alkyl)-, C₁-C₆ alkoxy, and C₁-C₄ alkoxycarbonyl;

   m is 0-2,
   n is 0-4,
   n' is 0-4,
   p' is 1-7,
   p" is 1-7, and
   r is 0-3;
   provided that n' is chosen such that the number of in-chain atoms connecting R¹ and Y is in the range of 8-18.
(2) Preferred compounds are those compounds of Formula I as defined in (1) above, wherein:
   - Z: is a bond, O, or S; and/or
   - R²: is H, aryl(C₁-C₁₀ alkoxy)carbonyl or C₂-C₁₀ alkoxycarbonyl; and/or
   - W: is -(CH₂)_{n'}-C(=O)-N(R^{5a})-; and/or
   - X: is -(C(R⁴)₂)_{n'}-C(R⁴)(R⁸)-CH(R⁴)-, with the proviso that when n' is 0 or 1, then R⁸ is other than H or methyl; and/or
   - R⁵: is H or C₁-C₁₀ alkyl substituted with 0-6 R^{4b}; and/or
   - R⁶: is selected from H, C₁-C₁₀ alkyl, hydroxy, C₁-C₁₀ alkoxy, nitro, (C₁-C₁₀ alkyl)carbonyl, -N(R¹²)R¹³, -N(R⁵)R^{5a}, -CO₂R⁵, -S(O)ₘR⁵, -OR⁵, cyano, halo;
   C₆-C₁₀ aryl optionally substituted with 1-3 groups selected from halogen, C₁-C₆ alkoxy, C₁-C₆ alkyl, CF₃, -S(O)ₘMe, and -NMe₂;
   C₇-C₁₁ arylalkyl, said aryl being optionally substituted with 1-3 groups selected from halogen,
   C₁-C₆ alkoxy, C₁-C₆ alkyl, CF₃, -S(O)ₘMe, and -NMe₂;
   methylenedioxy when R⁶ is a substituent on aryl;
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁷; and/or
   - R⁷: is selected from H, C₁-C₁₀ alkyl, hydroxy, C₁-C₁₀ alkoxy, nitro, (C₁-C₁₀ alkyl)carbonyl, -N(R¹²)R¹³, cyano, and halo; and/or
   - R⁸: is selected from -CON(R⁵)NR^{5a}, -CO₂R⁵, C₁-C₁₀ alkyl substituted with 0-3 R⁶, C₂-C₁₀ alkenyl substituted with 0-3 R⁶, C₂-C₁₀ alkynyl substituted with 0-3 R⁶, C₃-C₈ cycloalkyl substituted with 0-3 R⁶, C₅-C₆ cycloalkenyl substituted with 0-3 R⁶, aryl substituted with 0-2 R⁶;
   a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁶; and/or
   - R¹² and R¹³: are each independently selected from: H, C₁-C₁₀ alkyl, (C₁-C₁₀ alkoxy)carbonyl, (C₁-C₁₀ alkyl)carbonyl, C₁-C₁₀ alkylsulfonyl, aryl(C₁-C₁₀ alkyl)sulfonyl, arylsulfonyl, aryl, heteroarylcarbonyl and heteroarylalkylcarbonyl, wherein said aryls are substituted with 0-3 substituents selected from: C₁-C₄ alkyl, C₁-C₄ alkoxy, halo, CF₃, and NO₂; and/or
   - R¹⁵: is selected from H, C₁-C₁₀ alkyl, C₂-C₁₀ alkenyl, C₂-C₁₀ alkynyl, C₁-C₁₀ alkoxy, (C₁-C₁₀ alkoxy)carbonyl, -CO₂R⁵, and -C(=O)N(R⁵)R^{5a}.
(3) Further preferred compounds are those compounds of Formula I as defined in (2) above, wherein:
   - Z: is a bond or O; and/or
   - W: is -(CH₂)_{n'}-C(=O)-N(R^{5a})-; and/or
   - X: is -C(R⁴)(R⁸)-CH(R⁴)-.
(4) Further preferred compounds are those compounds of Formula I as defined in (2) above, wherein:
   - R¹: is R²NHC(=NR²)- or R²NHC(=NR²)NH-, and V is phenylene or pyridylene; or
   - R¹: is 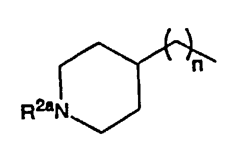
   - V: is a single bond, and n is 1 or 2;
   - X: is -CHR⁸CH₂-;
   - Y: is selected from hydroxy, C₁-C₁₀ alkoxy, methylcarbonyloxymethoxy-, ethylcarbonyloxymethoxy-, t-butylcarbonyloxymethoxy-, cyclohexylcarbonyl-oxymethoxy-, 1-(methylcarbonyloxy)ethoxy-, 1-(ethyl-carbonyloxy)ethoxy-, 1-(t-butylcarbonyloxy)ethoxy-, 1-(cyclohexylcarbonyloxy)ethoxy-, i-propyloxy-carbonyloxymethoxy-, t-butyloxycarbonyloxymethoxy-, 1-(i-propyloxycarbonyloxy)ethoxy-, 1-(cyclohexyloxycarbonyloxy)ethoxy-, 1-(t-butyl-oxycarbonyloxy)ethoxy-, dimethylaminoethoxy-, diethylaminoethoxy-, (5-methyl-1,3-dioxacyclopenten-2-on-4-yl)methoxy-, (5-(t-butyl)-1,3-dioxacyclopenten-2-on-4-yl)methoxy-, (1,3-dioxa-5-phenyl-cyclopenten-2-on-4-yl)methoxy-, and 1-(2-(2-methoxy-propyl)carbonyloxy)ethoxy-;
   - R⁶: is selected from H, C₁-C₄ alkyl, hydroxy, C₁-C₄ alkoxy, nitro, C₁-C₁₀ alkylcarbonyl, -N(R¹²)R¹³, -N(R⁵)R^{5a}, -CO₂R⁵, -S(O)ₘR⁵, -OR⁵, cyano, halo;
   C₆-C₁₀ aryl optionally substituted with 1-3 groups selected from halogen, C₁-C₆ alkoxy, C₁-C₆ alkyl, CF₃, -S(O)ₘMe, and -NMe₂;
   methylenedioxy when R⁶ is a substituent on aryl;
   a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, benzimidazolyl, piperidinyl, tetrahydrofuranyl, pyranyl, pyridinyl, 3H-indolyl, pyrrolidinyl, piperidinyl, indolinyl, isoxazolinyl and morpholinyl;
   - R⁸: is selected from -CON(R⁵)NR^{5a}, -CO₂R⁵, C₁-C₁₀ alkyl substituted with 0-3 R⁶, C₂-C₁₀ alkenyl, substituted with 0-3 R⁶, C₂-C₁₀ alkynyl substituted with 0-3 R⁶; C₃-C₈ cycloalkyl substituted with 0-3 R⁶, aryl substituted with 0-2 R⁶;
   a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyridinyl, 3H-indolyl, pyrrolidinyl, piperidinyl, indolinyl and morpholinyl, said heterocyclic ring being substituted with 0-2 R⁶;
   - R¹²: is selected from H, C₁-C₆ alkyl, (C₁-C₄ alkoxy)carbonyl, (C₁-C₆ alkyl)carbonyl, C₁-C₆ alkylsulfonyl, aryl(C₁-C₄ alkyl)sulfonyl, arylsulfonyl, aryl, pyridylcarbonyl and pyridylmethylcarbonyl, wherein said aryls are substituted with 0-3 substituents selected from C₁-C₄ alkyl, C₁-C₄ alkoxy, halo, CF₃, and NO₂; and
   - R¹³: is H.
(5) Specifically preferred compounds are compounds, or pharmaceutically acceptable salt or prodrug forms thereof, selected from:
   3(*R*, *S*)-{5(*R*, *S*)-N-[3-(4-amidinophenyl)isoxazolin-5-ylacetyl]amino}-3-phenylpropanoic acid;
   3(*R*, *S*)-{5(*R*, *S*)-N-[3-(4-amidinophenyl)isoxazolin-5-ylacetyl]amino}-pentanoic acid;
   3(*R*)-{5(*R*, *S*)-N-[3-(4-amidinophenyl)isoxazolin-5-ylacetyl]amino}heptanoic acid;
   3(*R*, *S*)-{5(*R*, *S*)-N-[3-(4-amidinophenyl)isoxazolin-5-ylacetyl]amino}-4-(phenylthio)butanoic acid;
   3(*R,S*)-{5(*R*,*S*)-N-[3-(4-amidinophenyl)isoxazolin-5-ylacetyl]amino}-4-(phenylsulfonamido)butanoic acid;
   3(*R,S*)-{5(*R*,*S*)-N-[3-(4-amidinophenyl)isoxazolin-5-ylacetyl]amino}-4-(n-butylsulfonamido)butanoic acid;
   3(*S*)-{5(*R,S*)-N-[3-(4-amidinophenyl)isoxazolin-5-ylacetyl]amino}-3-(adamantylmethylaminocarbonyl)-propanoic acid;
   3(*S*)-{5(*R*,*S*)-N-[3-(4-amidinophenyl)isoxazolin-5-ylacetyl]amino}-3-(1-azabicyclo[3.2.2]nonyl-carbonyl)propanoic acid;
   3(*S*)-{5(*R*,*S*)-N-[3-(4-amidinophenyl)isoxazolin-5-ylacetyl]amino}-3-(phenethylaminocarbonyl)propanoic acid;
   3(*R*)-{5(*R*,*S*)-N-[3-(4-amidinophenyl)isoxazolin-5-ylacetyl]amino}-3-(3-pyridylethyl)propanoic acid;
   3(*R*)-{5(*R*,*S*)-N-[3-(4-amidinophenyl)isoxazolin-5-ylacetyl]amino}-3-(2-pyridylethyl)propanoic acid; and
   3(*R*)-{5(*R*,*S*)-N-[3-(4-amidinophenyl)isoxazolin-5-ylacetyl]amino}-3-(phenylpropyl)propanoic acid.
(6) Also preferred compounds are those compounds of the Formula I as defined in (1) above, wherein:
   Z is selected from a bond (i.e., is absent), O, or S;
   - R² and R³: are independently selected from H, aryl(C₁-C₁₀ alkoxy)carbonyl, and (C₁-C₁₀ alkoxy)carbonyl;
   - R^{2a}: is R² or R²(R³)N(R²N=)C;
   - W: is -C(R⁴)₂-C(=O)-N(R^{5a})- or -C(=O)-N(R^{5a})-C(R⁴)₂-;
   - X: is -C(R⁴)(R⁸)-CHR^{4a}-;
   - R⁴: is selected from H, C₁-C₁₀ alkyl, C₁-C₁₀ alkycarbonyl, aryl, arylalkyl, cycloalkyl, and cycloalkylalkyl;
   - R^{4a}: is selected from hydroxy, C₁-C₁₀ alkoxy, nitro, -N(R⁵)R^{5a}, -N(R¹²)R¹³, -N(R¹⁶)R¹⁷, C₁-C₁₀ alkyl substituted with 0-3 R⁶, aryl substituted with 0-3 R⁶, and C₁-C₁₀ alkylcarbonyl;
   - R^{4b}: is selected from H, C₁-C₆ alkyl, C₂-C₆ alkenyl, C₂-C₆ alkynyl, hydroxy, C₁-C₆ alkoxy, C₁-C₆ alkylthio, C₁-C₆ alkylsulfinyl, C₁-C₆ alkylsulfonyl, nitro, (C₁-C₆ alkyl)carbonyl, C₆-C₁₀ aryl, -N(R¹²)R¹³, halo, CF₃, CN, (C₁-C₆ alkoxy)carbonyl, carboxy, piperidinyl, morpholinyl, and pyridinyl;
   - R⁵: is C₁-C₁₀ alkyl substituted with 0-2 R^{4b};
   - R^{5a}: is selected from H, hydroxy, C₁-C₈ alkyl, C₃-C₆ alkenyl, C₃-C₁₁ cycloalkyl, C₄-C₁₁ cycloalkylmethyl, C₁-C₆ alkoxy, benzyloxy, C₆-C₁₀ aryl, heteroaryl, heteroarylalkyl, C₇-C₁₁ arylalkyl, adamantylmethyl, and C₁-C₁₀ alkyl substituted with 0-2 R^{4b};
   alternatively, R⁵ and R^{5a} can be taken together to be
   3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl or 1-piperazinyl, each being optionally substituted with C₁-C₆ alkyl, C₆-C₁₀ aryl, heteroaryl, C₇-C₁₁ arylalkyl, (C₁-C₆ alkyl)carbonyl, (C₃-C₇ cycloalkyl)carbonyl, (C₁-C₆ alkoxy)carbonyl, or (C₇-C₁₁ arylalkoxy)carbonyl;
   - Y: is selected from hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxy-carbonyloxyalkyloxy, C₂-C₁₀ alkoxycarbonylalkyloxy, C₅-C₁₀ cycloalkylcarbonyloxyalkyloxy, C₅-C₁₀ cyclo-alkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyl-oxyalkyloxy, C₅-C₁₀ alkoxyalkylcarbonyloxyalkyloxy, C₅-C₁₀ (5-alkyl-1,3-dioxa-cyclopenten-2-one-yl)methyloxy, and C₁₀-C₁₄ (5-aryl-1,3-dioxa-cyclopenten-2-one-yl)methyloxy;
   - R⁶ and R⁷: are each independently selected from H, C₁-C₁₀ alkyl, hydroxy, C₁-C₁₀ alkoxy, nitro, (C₁-C₁₀ alkyl)carbonyl, -N(R¹²)R¹³, cyano, and halo;
   - R¹² and R¹³: are each independently selected from H, C₁-C₁₀ alkyl, (C₁-C₁₀alkoxy)carbonyl, (C₁-C₁₀ alkyl)carbonyl, C₁-C₁₀ alkylsulfonyl, aryl(C₁-C₁₀ alkyl)sulfonyl, arylsulfonyl, heteroarylcarbonyl, heteroarylalkyl-carbonyl and aryl, wherein said aryls are substituted with 0-3 substituents selected from C₁-C₄ alkyl, C₁-C₄ alkoxy, halo, CF₃, and NO₂;
   - R¹⁵: is selected from: H, C₁-C₁₀ alkyl, C₂-C₁₀ alkenyl, C₂-C₁₀ alkynyl, C₁-C₁₀ alkoxy, (C₁-C₁₀ alkoxy)carbonyl,
   -CO₂R⁵, and -C(=O)N(R⁵)R^{5a};
   - R¹⁶: is selected from -C(=O)-O-R^{18a}, -C(=O)-R^{18b}, -C(=O)N(R^{18b})₂, -SO₂-R^{18a}, and -SO₂-N(R^{18b})₂;
   - R¹⁷: is H or C₁-C₄ alkyl;
   - R^{18a}: is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹;
   a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyrimidinyl, 3H-indolyl, pyrrolidinyl, piperidinyl, indolinyl and morpholinyl, said heterocyclic ring being substituted with 0-4 R¹⁹;
   C₁-C₆ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, or morpholinyl, said heterocyclic ring being substituted with 0-4 R¹⁹; and
   the variables V, Q, R¹, R^{5b}, R^{18b}, R¹⁹, b, n, p', p" and r are as defined in (1) above.
(7) Further preferred compounds are compounds as defined in (6) above of Formula Ib 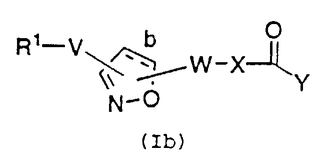 wherein:
   - R¹: is selected from R^{2a}(R³)N-, R²NH(R²N=)C-, R²NH(R²N=)CNH-, R^{2a}(R³)N(CH₂)_{p'}Z-, R²NH(R²N=)C(CH₂)_{p"}Z-,

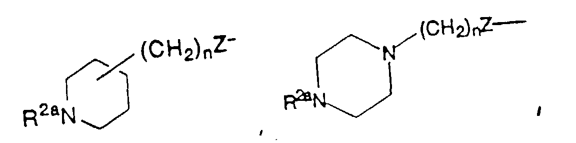

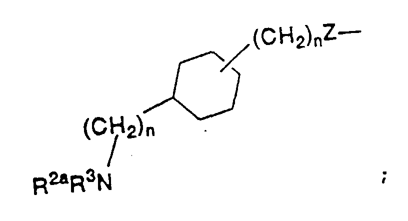

   n is 0-1;
   p' is 4-6;
   p" is 2-4;

   - Z: is a bond (i.e., is absent) or O;
   - V: is a single bond (i.e., V is not present), -(phenyl)- or -(pyridinyl)-;
   - W: is -C(R⁴)₂-C(=O)-N(R^{5a})- or -C(=O)-N(R^{5a})-CH₂-;
   - X: is -CH₂-CH(N(R¹⁶)R¹⁷)-, or -CH₂-CH(N(R⁵)R^{5a})-;
   - Y: is selected from hydroxy, C₁-C₁₀ alkoxy, methylcarbonyloxymethoxy-, t-butylcarbonyloxymethoxy-, cyclohexylcarbonyloxymethoxy-, 1-(methylcarbonyloxy)ethoxy-, 1-(ethylcarbonyloxy)ethoxy-, 1-(t-butylcarbonyl-oxy)ethoxy-, 1-(cyclohexylcarbonyloxy)ethoxy-, i-propyloxycarbonyloxymethoxy-, t-butyloxycarbonyloxymethoxy-, 1-(i-propyloxycarbonyloxy)ethoxy-, 1-(cyclohexyloxycarbonyloxy)ethoxy-, 1-(t-butyl-oxycarbonyloxy)ethoxy-, dimethylaminoethoxy-, diethylaminoethoxy-, (5-methyl-1,3-dioxacyclopenten-2-on-4-yl)methoxy-, (5-(t-butyl)-1,3-dioxacyclopenten-2-on-4-yl)methoxy-, (1,3-dioxa-5-phenyl-cyclopenten-2-on-4-yl)methoxy-, and 1-(2-(2-methoxy-propyl)carbonyloxy)ethoxy-;
   - R¹⁶: is selected from -C(=O)-O-R^{18a}, -C(=O)-R^{18b}, -S(=O)₂-R^{18a}, and -SO₂-N(R^{18b})₂;
   - R¹⁷: is H or C₁-C₅ alkyl;
   - R^{18a}: is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹,
   a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyridinyl, 3H-indolyl, carbazole, pyrrolidinyl, piperidinyl, indolinyl and morpholinyl, said heterocyclic ring being substituted with 0-4 R¹⁹;
   C₁-C₆ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, and morpholinyl, said heterocyclic ring being substituted with 0-4 R¹⁹.
(8) Further preferred compounds are those compounds of formula Ib as defined in (7) above, wherein:
   - R¹: is R²NH(R²N=)C- or R²HN(R²N=)CNH-, and V is phenylene or pyridylene; or
   - R¹: is 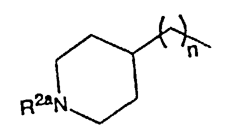
   - V: is a single bond, and n is 1 or 2; and
   - R^{18a}: is selected from C₁-C₄ alkyl substituted with 0-2 R¹⁹, C₂-C₄ alkenyl substituted with 0-2 R¹⁹, C₂-C₄ alkynyl substituted with 0-2 R¹⁹, C₃-C₇ cycloalkyl substituted with 0-2 R¹⁹, aryl substituted with 0-4 R¹⁹, aryl(C₁-C₄ alkyl)- substituted with 0-4 R¹⁹,
   a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyrimidinyl, 3H-indolyl, pyrrolidinyl, piperidinyl, indolinyl, isoxazolinyl and morpholinyl, said heterocyclic ring being substituted with 0-4 R¹⁹;
   C₁-C₄ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, isoxazolinyl and morpholinyl, said heterocyclic ring being substituted with 0-4 R¹⁹.
(9) Specifically preferred compounds are compounds, or pharmaceutically acceptable salt forms thereof, selected from:
   N³-[2-{3-(4-formamidinophenyl)-isoxazolin-5(*R*,*S*)-yl}-acetyl]-N2-(phenylsulfonyl)-2,3-(S)-diaminopropanoic acid;
   N³-[2-{3-(4-formamidinophenyl)-isoxazolin-5(*R*,*S*)-yl}-acetyl]-N2-(4-methyl-phenyl-sulfonyl)-2,3-(S)-diaminopropanoic acid;
   N³-[2-{3-(4-formamidinophenyl)-isoxazolin-5(*R,S*)-yl}-acetyl]-N2-(butanesulfonyl)-2,3-(S)-diaminopropanoic acid;
   N³-[2-{3-(4-formamidinophenyl)-isoxazolin-5(*R*,*S*)-yl}-acetyl]-N2-(propanesulfonyl)-2,3-(S)-diaminopropanoic acid;
   N³-[2-{3-(4-formamidinophenyl)-isoxazolin-5(*R,S*)-yl}-acetyl]-N2-(ethanesulfonyl)-2,3-(S)-diaminopropanoic acid;
   N³-[2-{3-(4-formamidinophenyl)-isoxazolin-5(*R,S*)-yl}-acetyl]-N2-(methyloxycarbonyl)-2,3-(S)-diaminopropanoic acid;
   N³-[2-{3-(4-formamidinophenyl)-isoxazolin-5(*R*,*S*)-yl}-acetyl]-N2-(ethyloxycarbonyl)-2,3-(S)-diaminopropanoic acid;
   N³-[2-{3-(4-formamidinophenyl)-isoxazolin-5(*R,S*)-yl}-acetyl]-N2-(1-propyloxycarbonyl)-2,3-(S)-diaminopropanoic acid;
   N³-[2-{3-(4-formamidinophenyl)-isoxazolin-5(*R,S*)-yl}-acetyl]-N2-(2-propyloxycarbonyl)-2,3-(S)-diaminopropanoic acid;
   N³-[2-{3-(4-formamidinophenyl)-isoxazolin-5(*R,S*)-yl}-acetyl]-N2-(n-butyloxycarbonyl)-2,3-(S)-diaminopropanoic acid;
   N³-[2-{3-(4-formamidinophenyl)-isoxazolin-5(*R*)-yl}-acetyl]-N2-(n-butyloxycarbonyl)-2,3-(S)-diaminopropanoic acid;
   N³-[2-{3-(4-formamidinophenyl)-isoxazolin-5(*S*)-yl}-acetyl]-N2-(n-butyloxycarbonyl)-2,3-(S)-diaminopropanoic acid;
   N³-[2-{3-(4-formamidinophenyl)-isoxazolin-5(*R*)-yl}-acetyl]-N2-(n-butyloxycarbonyl)-2,3-(R)-diaminopropanoic acid;
   N³-[2-{3-(4-formamidinophenyl)-isoxazolin-5(*S*)-yl}-acetyl]-N2-(n-butyloxycarbonyl)-2,3-(R)-diaminopropanoic acid;
   N³-[2-{3-(4-formamidinophenyl)-isoxazolin-5(*R,S*)-yl}-acetyl]-N2-(2-butyloxycarbonyl)-2,3-(S)-diaminopropanoic acid;
   N³-[2-{3-(4-formamidinophenyl)-isoxazolin-5(*R,S*)-yl}-acetyl]-N2-(1-(2-methyl)-propyloxycarbonyl)-2,3-(S)-diaminopropanoic acid;
   N³-[2-{3-(4-formamidinophenyl)-isoxazolin-5(*R*,*S*)-yl}-acetyl]-N2-(2-(2-methyl)-propyloxycarbonyl)-2,3-(S)-diaminopropanoic acid;
   N³-[2-{3-(4-formamidinophenyl)-isoxazolin-5(*R,S*)-yl}-acetyl]-N2-(benzyloxycarbonyl)-2,3-(S)-diaminopropanoic acid;
   N³-[2-{3-(4-formamidinophenyl)-isoxazolin-5(*R*)-yl}-acetyl]-N2-(benzyloxycarbonyl)-2,3-(S)-diaminopropanoic acid;
   N³-[2-{3-(4-formamidinophenyl)-isoxazolin-5(*S*)-yl}-acetyl]-N2-(benzyloxycarbonyl)-2,3-(S)-diaminopropanoic acid;
   N³-[2-{3-(4-formamidinophenyl)-isoxazolin-5(*R,S*)-yl}-acetyl]-N2-(4-methylbenzyloxycarbonyl)-2,3-(S)-diaminopropanoic acid;
   N³-[2-{3-(4-formamidinophenyl)-isoxazolin-5(*R,S*)-yl}-acetyl]-N2-(4-methoxybenzyloxycarbonyl)-2,3-(S)-diaminopropanoic acid;
   N³-[2-{3-(4-formamidinophenyl)-isoxazolin-5(*R,S*)-yl}-acetyl]-N2-(4-chlorobenzyloxycarbonyl)-2,3-(S)-diaminopropanoic acid;
   N³-[2-{3-(4-formamidinophenyl)-isoxazolin-5(*R,S*)-yl}-acetyl]-N2-(4-bromobenzyloxycarbonyl)-2,3-(S)-diaminopropanoic acid;
   N³-[2-{3-(4-formamidinophenyl)-isoxazolin-5(*R,S*)-yl}-acetyl]-N2-(4-fluorobenzyloxycarbonyl)-2,3-(S)-diaminopropanoic acid;
   N³-[2-{3-(4-formamidinophenyl)-isoxazolin-5(*R,S*)-yl}-acetyl]-N2-(4-phenoxybenzyloxycarbonyl)-2,3-(S)-diaminopropanoic acid;
   N³-[2-{3-(4-formamidinophenyl)-isoxazolin-5(*R,S*)-yl}-acetyl]-N2-(2-(methyloxyethyl)-oxycarbonyl)-2,3-(S)-diaminopropanoic acid;
   N³-[2-{3-(4-formamidinophenyl)-isoxazolin-5(*R,S*)-yl}-acetyl]-N2-(2-pyridinylcarbonyl)-2,3-(S)-diaminopropanoic acid;
   N³-[2-{3-(4-formamidinophenyl)-isoxazolin-5(*R,S*)-yl}-acetyl]-N2- (3-pyridinylcarbonyl)-2,3-(S)-diaminopropanoic acid;
   N³-[2-{3-(4-formamidinophenyl)-isoxazolin-5(*R,S*)-yl}-acetyl]-N2-(4-pyridinyl-carbonyl)-2,3-(S)-diaminopropanoic acid;
   N³-[2-{3-(4-formamidinophenyl)-isoxazolin-5(*R,S*)-yl}-acetyl]-N2-(2-(2-pyridinyl)-acetyl)-2,3-(S)-diaminopropanoic acid;
   N³-[2-{3-(4-formamidinophenyl)-isoxazolin-5(*R,S*)-yl}-acetyl]-N2-(2-(3-pyridinyl)-acetyl)-2,3-(S)-diaminopropanoic acid;
   N³-[2-{3-(4-formamidinophenyl)-isoxazolin-5(*R,S*)-yl}-acetyl]-N2-(2-(4-pyridinyl)-acetyl)-2,3-(S)-diaminopropanoic acid;
   N³-[2-{3-(4-formamidinophenyl)-isoxazolin-5(*R,S*)-yl}-acetyl]-N2-(2-pyridyl-methyloxycarbonyl)-2,3-(S)-diaminopropanoic acid;
   N³-[2-{3-(4-formamidinophenyl)-isoxazolin-5(*R,S*)-yl}-acetyl]-N2-(3-pyridyl-methyloxycarbonyl)-2,3-(S)-diaminopropanoic acid;
   N³-[2-{3-(4-formamidinophenyl)-isoxazolin-5(*R,S*)-yl}-acetyl]-N2-(4-pyridyl-methyloxycarbonyl)-2,3-(S)-diaminopropanoic acid.
   N³-[2-{3-(4-formamidinophenyl)-isoxazolin-5(*R,S*)-yl}-acetyl]-N2-(4-butyloxyphenylsulfonyl)-2,3-(S)-diaminopropanoic acid;
   N³-[2-{3-(4-formamidinophenyl)-isoxazolin-5(*R,S*)-yl}-acetyl]-N2-(2-thienylsulfonyl)-2,3-(S)-diaminopropanoic acid;
   N³-[2-{3-(4-formamidinophenyl)-isoxazolin-5(*R,S*)-yl}-acetyl]-N2-(3-methylphenylsulfonyl)-2,3-(R,S)-diaminopropanoic acid;
   N³-[2-{3-(4-formamidinophenyl)-isoxazolin-5(*R,S*)-yl}-acetyl]-N2-(3-methylphenylsulfonyl)-2,3-(S)-diaminopropanoic acid;
   N³-[2-{3-(4-formamidinophenyl) -isoxazolin-5(*R,S*)-yl}-acetyl]-N2-(3-methylphenylsulfonyl)-2,3-(R)-diaminopropanoic acid;
   N³-[2-{3-(4-formamidinophenyl)-isoxazolin-5(*R*)-yl}-acetyl]-N2-(3-methylphenylsulfonyl)-2,3-(S)-diaminopropanoic acid;
   N³-[2-{3-(4-formamidinophenyl)-isoxazolin-5(*S*)-yl}-acetyl]-N2-(3-methylphenylsulfonyl)-2,3-(S)-diaminopropanoic acid;
   N³-[2-{3-(4-formamidinophenyl) -isoxazolin-5(*S*)-yl}-acetyl]-N2-(3-methylphenylsulfonyl)-2,3-(R)-diaminopropanoic acid;
   N³-[2-{3-(4-formamidinophenyl)-isoxazolin-5(*R*)-yl}-acetyl]-N2-(3-methylphenylsulfonyl)-2,3-(R)-diaminopropanoic acid;
   N³-[2-{3-(4-formamidinophenyl)-isoxazolin-5(*R,S*)-yl}-acetyl]-N2-(4-iodophenylsulfonyl)-2,3-(S)-diaminopropanoic acid;
   N³-[2-{3-(4-formamidinophenyl)-isoxazolin-5(*R,S*)-yl]-acetyl]-N2-(3-trifluoromethylphenylsulfonyl)-2,3-(S)-diaminopropanoic acid;
   N³-[2-{3-(4-formamidinophenyl)-isoxazolin-5(*R,S*)-yl}-acetyl]-N2-(3-chlorophenylsulfonyl)-2,3-(S)-diaminopropanoic acid;
   N³-[2-{3-(4-formamidinophenyl)-isoxazolin-5(*R,S*)-yl}-acetyl]-N2-(3-2-methoxycarbonylphenylsulfonyl)-2,3-(S)-diaminopropanoic acid;
   N³-[2-{3-(4-formamidinophenyl)-isoxazolin-5(*R,S*)-yl}-acetyl]-N2-(2,4,6-trimethylphenylsulfonyl)-2,3-(S)-diaminopropanoic acid;
   N³-[2-{3-(4-formamidinophenyl)-isoxazolin-5(*R,S*)-yl}-acetyl]-N2-(2-chlorophenylsulfonyl)-2,3-(S)-diaminopropanoic acid;
   N³-[2-{3-(4-formamidinophenyl)-isoxazolin-5(*R,S*)-yl}-acetyl]-N2- (4-trifluoromethylphenylsulfonyl)-2,3-(S)-diaminopropanoic acid;
   N³-[2-{3-(4-formamidinophenyl)-isoxazolin-5(*R,S*)-yl}-acetyl]-N2-(2-trifluoromethylphenylsulfonyl)-2,3-(S)-diaminopropanoic acid;
   N³-[2-{3-(4-formamidinophenyl)-isoxazolin-5(*R*,*S*)-yl}-acetyl]-N2-(2-fluorophenylsulfonyl)-2,3-(S)-diaminopropanoic acid;
   N³-[2-{3-(4-formamidinophenyl)-isoxazolin-5(*R,S*)-yl}-acetyl]-N2-(4-fluorophenylsulfonyl)-2,3-(S)-diaminopropanoic acid;
   N³-[2-{3-(4-formamidinophenyl)-isoxazolin-5(*R,S*)-yl}-acetyl]-N2-(4-methoxyphenylsulfonyl)-2,3-(S)-diaminopropanoic acid;
   N³-[2-{3-(4-formamidinophenyl)-isoxazolin-5(*R,S*)-yl}-acetyl]-N2-(2,3,5,6-tetramethylphenylsulfonyl)-2,3-(S)-diaminopropanoic acid;
   N³-[2-{3-(4-formamidinophenyl)-isoxazolin-5(*R,S*)-yl}-acetyl]-N2-(4-cyanophenylsulfonyl)-2,3-(S)-diaminopropanoic acid;
   N³-[2-{3-(4-formamidinophenyl)-isoxazolin-5(*R,S*)-yl}-acetyl]-N2-(4-chlorophenylsulfonyl)-2,3-(S)-diaminopropanoic acid;
   N³-[2-{3-(4-formamidinophenyl)-isoxazolin-5(*R,S*)-yl}-acetyl]-N2-(4-propylphenylsulfonyl)-2,3-(S)-diaminopropanoic acid;
   N³-[2-{3-(4-formamidinophenyl) -isoxazolin-5(*R,S*)-yl}-acetyl] -N2- (2-phenylethylsulfonyl) -2,3-(S)-diaminopropanoic acid;
   N³-[2-{3-(4-formamidinophenyl)-isoxazolin-5(*R,S*)-yl}-acetyl]-N2-(4-isopropylphenylsulfonyl)-2,3-(S)-diaminopropanoic acid;
   N³-[2-{3-(4-formamidinophenyl)-isoxazolin-5(*R,S*)-yl}-acetyl]-N2-(3-phenylpropylsulfonyl)-2,3-(S)-diaminopropanoic acid;
   N³-[2-{3-(4-formamidinophenyl) -isoxazolin-5 (*R,S*)-yl}-acetyl]-N2-(3-pyridylsulfonyl)-2,3-(S)-diaminopropanoic acid;
   N³-[2-{3-(4-formamidinophenyl) -isoxazolin-5 (*R,S*)-yl}-acetyl]-N2-(phenylaminosulfonyl)-2,3-(S)-diaminopropanoic acid;
   N³-[2-{3-(4-formamidinophenyl) -isoxazolin-5 (*R,S*)-yl}-acetyl]-N2-(benzylaminosulfonyl)-2,3-(S)-diaminopropanoic acid;
   N³-[2-{3-(4-formamidinophenyl) -isoxazolin-5 (*R,S*)-yl}-acetyl]-N2-(dimethylaminosulfonyl)-2,3-(S)-diaminopropanoic acid,
   N³-[2-{3-(2-fluoro-4-formamidinophenyl)-isoxazolin-5(*R,S*)-yl}-acetyl]-N2-(3-methylphenylsulfonyl)-2,3-(S)-diaminopropanoic acid,
   N³-[2-{3-(2-formamidino-5-pyridinyl)-isoxazolin-5(*R,S*)-yl}-acetyl]-N2-(n-butyloxycarbonyl)-2,3-(S)-diaminopropanoic acid,
   N³-[2-{3-(2-formamidino-5-pyridinyl)-isoxazolin-5(*R,S*)-yl}-acetyl]-N2-(3-methylphenylsulfonyl)-2,3-(S)-diaminopropanoic acid,
   N³-[2-{3-(3-formamidino-6-pyridinyl)-isoxazolin-5(*R,S*)-yl}-acetyl]-N2-(n-butyloxycarbonyl)-2,3-(S)-diaminopropanoic acid,
   N³-[2-{3-(3-formamidino-6-pyridinyl)-isoxazolin-5(*R,S*)-yl}-acetyl]-N2-(3-methylphenylsulfonyl)-2,3-(S)-diaminopropanoic acid,
   N³-[2-{3-(4-formamidinophenyl)-isoxazolin-5(*R,S*)-yl}-acetyl]-N2-(phenylaminocarbonyl)-2,3-(S)-diaminopropanoic acid;
   N³-[2-{3-(4-formamidinophenyl)-isoxazolin-5(*R,S*)-yl}-acetyl]-N2-(4-fluorophenylaminocarbonyl)-2,3-(S)-diaminopropanoic acid;
   N³-[2-{3-(4-formamidinophenyl)-isoxazolin-5(*R,S*)-yl}-acetyl]-N2-(1-naphthylaminocarbonyl)-2,3-(S)-diaminopropanoic acid;
   N³-[2-{3-(4-formamidinophenyl)-isoxazolin-5(*R,S*)-yl}-acetyl]-N2-(benzylaminocarbonyl)-2,3-(S)-diaminopropanoic acid;
   N³-[2-{3-(4-formamidinophenyl)-isoxazolin-5(*R,S*)-yl}-acetyl]-N2-(3-bromo-2-thienylsulfonyl)-2,3-(S)-diaminopropanoic acid;
   N³-[2-{3-(4-formamidinophenyl)-isoxazolin-5(*R,S*)-yl}-acetyl]-N2-(3-methyl-2-benzothienylsulfonyl)-2,3-(S)-diaminopropanoic acid;
   N³-[2-{3-(4-formamidinophenyl)-isoxazolin-5(*R,S*)-yl}-acetyl]-N2-(isobutyloxycarbonyl)-2,3-(S)-diaminopropanoic acid;
   N³-[2-{3-(4-formamidinophenyl)-isoxazolin-5(*R*)-yl}-acetyl]-N2-(isobutyloxycarbonyl)-2,3-(S)-diaminopropanoic acid;
   N³-[2-{3-(4-formamidinophenyl)-isoxazolin-5(*S*)-yl}-acetyl]-N2-(isobutyloxycarbonyl)-2,3-(S)-diaminopropanoic acid;
   N³-[2-{3-(4-formamidinophenyl)-isoxazolin-5(*R,S*)-yl}-acetyl]-N2-(2-cyclopropylethoxycarbonyl)-2,3-(S)-diaminopropanoic acid;
   N³-[2-{3-(4-formamidinophenyl)-isoxazolin-5(*R*)-yl}-acetyl]-N2-(2-cyclopropylethoxycarbonyl)-2,3-(S)-diaminopropanoic acid;
   N³-[2-{3-(4-formamidinophenyl)-isoxazolin-5(*S*)-yl}-acetyl]-N2-(2-cyclopropylethoxycarbonyl)-2,3-(S)-diaminopropanoic acid;
   N³-[2-{3-(4-guanidinophenyl)-isoxazolin-5(*R,S*)-yl}-acetyl]-N2-(n-butyloxycarbonyl)-2,3-(S)-diaminopropanoic acid;
   N³-[2-{3-(4-guanidinophenyl)-isoxazolin-5(*R*)-yl}-acetyl]-N2-(n-butyloxycarbonyl)-2,3-(S)-diaminopropanoic acid;
   N³-[2-{3-(4-guanidinophenyl)-isoxazolin-5(*R*)-yl}-acetyl] -N2-(3-methylphenylsulfonyl)-2,3-(S)-diaminopropanoic acid; and
   N³-[2-{5-(4-formamidinophenyl)isoxazolin-3(*R,S*)-yl}-acetyl]-N2-(n-butyloxycarbonyl)-2,3-(S)-diaminopropanoic acid.
(10) Also specifically preferred are prodrug esters of the specifically preferred compounds as defined in (9) above said esters being selected from:
   methyl, ethyl, isopropyl, methylcarbonyloxymethyl-, ethylcarbonyloxymethyl-, t-butylcarbonyloxymethyl-, cyclohexylcarbonyloxymethyl-, 1-(methylcarbonyloxy)-ethyl-, 1-(ethylcarbonyloxy)ethyl-, 1-(t-butylcarbonyloxy)ethyl-, 1-(cyclohexylcarbonyloxy)ethyl-, i-propyloxycarbonyloxymethyl-, cyclohexylcarbonyloxymethyl-, t-butyloxycarbonyloxymethyl-, 1-(i-propyloxycarbonyloxy)ethyl-, 1-(cyclohexyloxycarbonyloxy)ethyl, 1-(t-butyloxycarbonyloxy)ethyl-, dimethylaminoethyl-, diethylaminoethyl-, (5-methyl-1,3-dioxacyclopenten-2-on-4-yl)methyl-, (5-(t-butyl)-1,3-dioxacyclopenten-2-on-4-yl)methyl-, (1,3-dioxa-5-phenylcyclopenten-2-on-4-yl)methyl-, and 1-(2-(2-methoxypropyl)carbonyloxy)ethyl-.

In the present invention it has been found that the compounds of Formula I above are useful as inhibitors of cell-matrix and cell-cell adhesion processes. The compounds of Formula I are suitable for the prevention or treatment of diseases resulting from abnormal cell adhesion to the extracellular matrix.

In the present invention it has also been found that the compounds of Formula I above are useful as inhibitors of glycoprotein IIb/IIIa (GPIIb/IIIa). The compounds of the present invention inhibit the activation and aggregation of platelets induced by all known endogenous platelet agonists.

The present invention also provides pharmaceutical compositions comprising a compound of Formula I and a pharmaceutically acceptable carrier.

The compounds of Formula I of the present invention are useful for the treatment (including prevention) of thromboembolic disorders. The term "thromboembolic disorders" as used herein includes conditions involving platelet activation and aggregation, such as arterial or venous cardiovascular or cerebrovascular thromboembolic disorders, including, for example, thrombosis, unstable angina, first or recurrent myocardial infarction, ischemic sudden death, transient ischemic attack, stroke, atherosclerosis, venous thrombosis, deep vein thrombosis, thrombophlebitis, arterial embolism, coronary and cerebral arterial thrombosis, myocardial infarction, cerebral embolism, kidney embolisms, pulmonary embolisms, or such disorders associated with diabetes.

The compounds of Formula I of the present invention are useful for the treatment or prevention of other diseases which involve cell adhesion processes, including infammation, bone degradation, rheumatoid arthritis, asthma, allergies, adult respiratory distress syndrome, graft versus host disease, organ transplantation rejection, septic shock, psoriasis, eczema, contact dermatitis, osteoporosis, osteoarthritis, atherosclerosis, tumors, metastasis, diabetic retinopathy, inflammatory bowel disease and other autoimmune diseases. The compounds of Formula I of the present invention are also useful for wound healing.

The compounds of the present invention are useful for inhibiting the binding of fibrinogen to blood platelets, inhibiting aggregation of blood platelets, treating thrombus formation or embolus formation, or preventing thrombus or embolus formation in a mammal. The compounds of the invention may be used as a medicament for blocking fibrinogen from acting at its receptor site in a mammal.

Compounds of the invention are useful in surgery on peripheral arteries (arterial grafts, carotid endarterectomy) and in cardiovascular surgery where manipulation of arteries and organs, and/or the interaction of platelets with artificial surfaces, leads to platelet aggregation and consumption, and where the aggregated platelets may form thrombi and thromboemboli. The compounds of the present invention may be administered to these surgical patients to prevent the formation of thrombi and thromboemboli.

Extracorporeal circulation is routinely used during cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the extracorporeal circuit. Platelets released from artificial surfaces show impaired homeostatic function. The compounds of the invention may be administered to prevent such *ex vivo* adhesion.

The compounds of the present invention may be used for other *ex vivo* applications to prevent cellular adhesion in biological samples.

Other applications of these compounds include prevention of platelet thrombosis, thromboembolism, and reocclusion during and after thrombolytic therapy and prevention of platelet thrombosis, thromboembolism and reocclusion after angioplasty of coronary and other arteries and after coronary artery bypass procedures. The compounds of the present invention may also be used to prevent myocardial infarction. The compounds of the present invention are useful as thrombolytics for the treatment of thromboembolic disorders.

The compounds of the present invention can also be utilized in combination with one or more additional therapeutic agents select from: anti-coagulant or coagulation inhibitory agents, such as heparin or warfarin; anti-platelet or platelet inhibitory agents, such as aspirin, piroxicam, or ticlopidine; thrombin inhibitors such as boropeptides, hirudin or argatroban; or thrombolytic or fibrinolytic agents, such as plasminogen activators, anistreplase, urokinase, or streptokinase.

The compounds of Formula I of the present invention can be utilized in combination with one or more of the foregoing additional therapeutic agents, thereby to reduce the doses of each drug required to achieve the desired therapeutic effect. Thus, the combination treatment permits the use of lower doses of each component, with reduced adverse, toxic effects of each component. A lower dosage minimizes the potential of side effects of the compounds, thereby providing an increased margin of safety relative to the margin of safety for each component when used as a single agent. Such combination therapies may be employed to achieve synergistic or additive therapeutic effects for the treatment of thromboembolic disorders.

The term anti-coagulant agents (or coagulation inhibitory agents), as used herein, denotes agents that inhibit blood coagulation. Such agents include warfarin (available as Coumadin™) and heparin.

The term anti-platelet agents (or platelet inhibitory agents), as used herein, denotes agents that inhibit platelet function such as by inhibiting the aggregation, adhesion or granular secretion of platelets. Such agents include the various known non-steroidal anti-inflammatory drugs (NSAIDS) such as aspirin, ibuprofen, naproxen, sulindac, indomethacin, mefenamate, droxicam, diclofenac, sulfinpyrazone, and piroxicam, including pharmaceutically acceptable salts or prodrugs thereof. Of the NSAIDS, aspirin (acetylsalicyclic acid or ASA), and piroxicam. Piroxicam is commercially available from Pfizer Inc. (New York, NY), as Feldane™. Other suitable anti-platelet agents include ticlopidine, including pharmaceutically acceptable salts or prodrugs thereof. Ticlopidine is also a preferred compound since it is known to be gentle on the gastro-intestinal tract in use. Still other suitable platelet inhibitory agents include thromboxane-A2-receptor antagonists and thromboxane-A2-synthetase inhibitors, as well as pharmaceutically acceptable salts or prodrugs thereof.

The phrase thrombin inhibitors (or anti-thrombin agents), as used herein, denotes inhibitors of the serine protease thrombin. By inhibiting thrombin, various thrombin-mediated processes, such as thrombin-mediated platelet activation (that is, for example, the aggregation of platelets, and/or the granular secretion of plasminogen activator inhibitor-1 and/or serotonin) and/or fibrin formation are disrupted. Such inhibitors include boroarginine derivatives and boropeptides, hirudin and argatroban, including pharmaceutically acceptable salts and prodrugs thereof. Boroarginine derivatives and boropeptides include N-acetyl and peptide derivatives of boronic acid, such as C-terminal α-aminoboronic acid derivatives of lysine, ornithine, arginine, homoarginine and corresponding isothiouronium analogs thereof. The term hirudin, as used herein, includes suitable derivatives or analogs of hirudin, referred to herein as hirulogs, such as disulfatohirudin. Boropeptide thrombin inhibitors include compounds described in Kettner et al., U.S. Patent No. 5,187,157 and EP-A-0 293 881.

Other suitable boroarginine derivatives and boropeptide thrombin inhibitors include those disclosed in WO 92/07869 and EP-A-0 471 651.

The phrase thrombolytics (or fibrinolytic) agents (or thrombolytics or fibrinolytics), as used herein, denotes agents that lyse blood clots (thrombi). Such agents include tissue plasminogen activator, anistreplase, urokinase or streptokinase, including pharmaceutically acceptable salts or prodrugs thereof. Tissue plasminogen activator (tPA) is commercially available from Genentech Inc., South San Francisco, California. The term anistreplase, as used herein, refers to anisoylated plasminogen streptokinase activator complex, as described, for example, in EP-A-0 028 489.

Anistreplase is commercially available as Eminase™. The term urokinase, as used herein, is intended to denote both dual and single chain urokinase, the latter also being referred to herein as prourokinase.

Administration of the compounds of Formula I of the invention in combination with such additional therapeutic agent, may afford an efficacy advantage over the compounds and agents alone, and may do so while permitting the use of lower doses of each. A lower dosage minimizes the potential of side effects, thereby providing an increased margin of safety.

GPIIb/IIIa is known to be overexpressed in metastatic tumor cells. The compounds or combination products of the present invention may also be useful for the treatment, including prevention, of metastatic cancer.

The compounds of the present invention are also useful as standard or reference compounds, for example as a quality standard or control, in tests or assays involving the binding of fibrinogen to platelet GPIIb/IIIa. Such compounds may be provided in a commercial kit, for example, for use in pharmaceutical research involving GPIIb/IIIa. The compounds of the present invention may also be used in diagnostic assays involving platelet GPIIb/IIIa.

The compounds herein described may have asymmetric centers. Unless otherwise indicated, all chiral, diastereomeric and racemic forms are included in the present invention. Many geometric isomers of olefins, C=N double bonds, and the like can also be present in the compounds described herein, and all such stable isomers are contemplated in the present invention. It will be appreciated that compounds of the present invention that contain asymmetrically substituted carbon atoms may be isolated in optically active or racemic forms. It is well known in the art how to prepare optically active forms, such as by resolution of racemic forms or by synthesis, from optically active starting materials. All chiral, diastereomeric, racemic forms and all geometric isomeric forms of a structure are intended, unless the specific stereochemistry or isomer form is specifically indicated.

When any variable (for example but not limited to, R², R⁴, R⁶, R⁷, R⁸, R¹², and n) occurs more than one time in any constituent or in any formula, its definition on each occurrence is independent of its definition at every other occurrence. Thus, for example, if a group is shown to be substituted with 0-2 R⁴, then said group may optionally be substituted with up to two R⁴ and R⁴ at each occurrence is selected independently from the defined list of possible R⁴. Also, by way of example, for the group -N(R^{5a})₂, each of the two R^{5a} substituents on N is independently selected from the defined list of possible R^{5a}. Similarly, by way of example, for the group -C(R⁷)₂-, each of the two R⁷ substituents on C is independently selected from the defined list of possible R⁷.

When a bond to a substituent is shown to cross the bond connecting two atoms in a ring, then such substituent may be bonded to any atom on the ring. When a bond joining a substituent to another group is not specifically shown or the atom in such other group to which the bond joins is not specifically shown, then such substituent may form a bond with any atom on such other group.

When a substituent is listed without indicating the atom via which such substituent is bonded to the rest of the compound of Formula I, then such substituent may be bonded via any atom in such substituent. For example, when the substituent is piperazinyl, piperidinyl, or tetrazolyl, unless specified otherwise, said piperazinyl, piperidinyl, tetrazolyl group may be bonded to the rest of the compound of Formula I via any atom in such piperazinyl, piperidinyl, tetrazolyl group.

Combinations of substituents and/or variables are permissible only if such combinations result in stable compounds. By stable compound or stable structure it is meant herein a compound that is sufficiently robust to survive isolation to a useful degree of purity from a reaction mixture, and formulation into an efficacious therapeutic agent.

The term "substituted", as used herein, means that any one or more hydrogen on the designated atom is replaced with a selection from the indicated group, provided that the designated atom's normal valency is not exceeded, and that the substitution results in a stable compound. When a substitent is keto (i.e., =O), then 2 hydrogens on the atom are replaced.

As used herein, "alkyl" is intended to include both branched and straight-chain saturated aliphatic hydrocarbon groups having the specified number of carbon atoms (for example, "C₁-C₁₀" denotes alkyl having 1 to 10 carbon atoms); "haloalkyl" is intended to include both branched and straight-chain saturated aliphatic hydrocarbon groups having the specified number of carbon atoms, substituted with 1 or more halogen (for example -CᵥF_{w} where v = 1 to 3 and w = 1 to (2v+1)); "alkoxy" represents an alkyl group of indicated number of carbon atoms attached through an oxygen bridge; "cycloalkyl" is intended to include saturated ring groups, including mono-,bi- or poly-cyclic ring systems, such as cyclopropyl, cyclobutyl, cyclopentyl, cyclohexyl, cycloheptyl, cyclooctyl, and adamantyl; and "biycloalkyl" is intended to include saturated bicyclic ring groups such as [3.3.0]bicyclooctane, [4.3.0]bicyclononane, [4.4.0]bicyclodecane (decalin) and [2.2.2]bicyclooctane. "Alkenyl" is intended to include hydrocarbon chains of either a straight or branched configuration and one or more unsaturated carbon-carbon bonds which may occur in any stable point along the chain, such as ethenyl and propenyl; and "alkynyl" is intended to include hydrocarbon chains of either a straight or branched configuration and one or more triple carbon-carbon bonds which may occur in any stable point along the chain, such as ethynyl and propynyl.

The terms "alkylene", "alkenylene", "phenylene", and the like, refer to alkyl, alkenyl, and phenyl groups, respectively, which are connected by two bonds to the rest of the structure of Formula I. Such "alkylene", "alkenylene" and "phenylene" may alternatively and equivalently be denoted herein as "-(alkyl)-", "-(alkyenyl)-" and "-(phenyl)-".

"Halo" or "halogen" as used herein refers to fluoro, chloro, bromo and iodo; and "counterion" is used to represent a small, negatively charged species such as chloride, bromide, hydroxide, acetate, sulfate and the like.

As used herein, "aryl" or "aromatic residue" is intended to mean phenyl or naphthyl; the term "arylalkyl" represents an aryl group attached through an alkyl bridge.

As used herein, "carbocycle" or "carbocyclic residue" is intended to mean any stable 3- to 7-membered monocyclic or bicyclic or 7- to 14-membered bicyclic or tricyclic or an up to 26-membered polycyclic carbon ring, any of which may be saturated, partially unsaturated, or aromatic. Examples of such carbocyles include cyclopropyl, cyclopentyl, cyclohexyl, phenyl, biphenyl, naphthyl, indanyl, adamantyl, or tetrahydronaphthyl (tetralin).

As used herein, the term "heterocycle" or "heterocyclic" is intended to mean a stable 5- to 7-membered monocyclic or bicyclic or 7- to 10-membered bicyclic heterocyclic ring which may be saturated, partially unsaturated, or aromatic, and which consists of carbon atoms and from 1 to 4 heteroatoms independently selected from the group consisting of N, O and S and wherein the nitrogen and sulfur heteroatoms may optionally be oxidized, and the nitrogen may optionally be quaternized, and including any bicyclic group in which any of the above-defined heterocyclic rings is fused to a benzene ring. The heterocyclic ring may be attached to its pendant group at any heteroatom or carbon atom which results in a stable structure. The heterocyclic rings described herein may be substituted on carbon or on a nitrogen atom if the resulting compound is stable. Examples of such heterocycles include pyridyl (pyridinyl), pyrimidinyl, furanyl (furyl), thiazolyl, thienyl, pyrrolyl, pyrazolyl, imidazolyl, tetrazolyl, benzofuranyl, benzothiophenyl, indolyl, indolenyl, isoxazolinyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl or octahydroisoquinolinyl, azocinyl, triazinyl, 6*H*-1,2,5-thiadiazinyl, 2*H*,6*H*-1,5,2-dithiazinyl, thianthrenyl, pyranyl, isobenzofuranyl, chromenyl, xanthenyl, phenoxathiinyl, 2*H*-pyrrolyl, pyrrolyl, imidazolyl, pyrazolyl, isothiazolyl, isoxazolyl, oxazolyl, pyridinyl, pyrazinyl, pyrimidinyl, pyridazinyl, indolizinyl, isoindolyl, 3*H*-indolyl, indolyl, 1*H*-indazolyl, purinyl, 4*H*-quinolizinyl, isoquinolinyl, quinolinyl, phthalazinyl, naphthyridinyl, quinoxalinyl, quinazolinyl, cinnolinyl, pteridinyl, 4a*H*-carbazole, carbazole, β-carbolinyl, phenanthridinyl, acridinyl, perimidinyl, phenanthrolinyl, phenazinyl, phenarsazinyl, phenothiazinyl, furazanyl, phenoxazinyl, isochromanyl, chromanyl, pyrrolidinyl, pyrrolinyl, imidazolidinyl, imidazolinyl, pyrazolidinyl, pyrazolinyl, piperidinyl, piperazinyl, indolinyl, isoindolinyl, quinuclidinyl, morpholinyl or oxazolidinyl. Also included are fused ring and spiro compounds containing, for example, the above heterocycles.

At used herein, the term "heteroaryl" refers to aromatic heterocyclic groups. Such heteroaryl groups are preferably 5-6 membered monocylic groups or 8-10 membered fused bicyclic groups. Examples of such heteroaryl groups include pyridyl (pyridinyl), pyrimidinyl, furanyl (furyl), thiazolyl, thienyl, pyrrolyl, pyrazolyl, imidazolyl, indolyl, isoxazolyl, oxazolyl, pyrazinyl, pyrimidinyl, pyridazinyl, benzofuranyl, benzothienyl, benzimidazolyl, quinolinyl, or isoquinolinyl.

As used herein, "pharmaceutically acceptable salts" refer to derivatives of the disclosed compounds wherein the parent compound of Formula I is modified by making acid or base salts of the compound of Formula I.

Examples of pharmaceutically acceptable salts include mineral or organic acid salts of basic residues such as amines; and alkali or organic salts of acidic residues such as carboxylic acids.

"Prodrugs" are considered to be any covalently bonded carriers which release the active parent drug according to Formula I *in vivo* when such prodrug is administered to a mammalian subject. Prodrugs of the compounds of Formula I are prepared by modifying functional groups present in the compounds in such a way that the modifications are cleaved, either in routine manipulation or *in vivo,* to the parent compounds. Prodrugs include compounds of Formula I wherein hydroxyl, amino, sulfhydryl, or carboxyl groups are bonded to any group that, when administered to a mammalian subject, cleaves to form a free hydroxyl, amino, sulfhydryl, or carboxyl group respectively. Examples of prodrugs include, but are not limited to, acetate, formate and benzoate derivatives of alcohol and amine functional groups in the compounds of Formula I.

Examples of representative carboxyl and amino prodrugs are included under the definition of R², R³, and Y.

The pharmaceutically acceptable salts of the compounds of Formula I include the conventional non-toxic salts or the quaternary ammonium salts of the compounds of Formula I formed, for example, from non-toxic inorganic or organic acids. For example, such conventional non-toxic salts include those derived from inorganic acids such as hydrochloric, hydrobromic, sulfuric, sulfamic, phosphoric and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic and isothionic.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt-forming inorganic or organic acid or base in a suitable solvent or various combinations of solvents.

The pharmaceutically acceptable salts of the acids of Formula I with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine and benzylamine or a quaternary ammonium hydroxide such as tetramethylammoinum hydroxide.

As discussed above, pharmaceutically acceptable salts of the compounds of the invention can be prepared by reacting the free acid or base forms of these compounds with a stoichiometric amount of the appropriate base or acid, respectively, in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

### Synthesis

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include those described below.

The following abbreviations are used herein:
- β-Ala: 3-aminopropionic acid
- Boc: tert-butyloxycarbonyl
- Boc₂O: di-tert-butyl dicarbonate
- BSTFA: *N,O*-bis(trimethylsilyl)trifluoromethylacetamide
- Cbz: benzyloxycarbonyl
- DCC: 1,3-dicyclohexylcarbodiimide
- DEAD: diethyl azodicarboxylate
- DEC: 1-(3-dimethylaminopropyl)-3-ethylcarbodiimide hydrochloride
- DIEA: diisopropylethylamine
- DCHA: dicyclohexylamine
- DCM: dichloromethane
- DMAP: 4-dimethylaminopyridine
- DMF: *N,N*-dimethylformamide
- EtOAc: ethyl acetate
- EtOH: ethyl alcohol
- HOBt: 1-hydroxybenzotriazole
- IBCF: iso-butyl chloroformate
- LAH: lithium aluminum hydride
- NCS: *N*-chlorosuccinimide
- NMM: *N*-methylmorpholine
- PPh3: triphenylphosphine
- pyr: pyridine
- TBTU: 2-(1H-Benzotriazol-1-yl)-1,1,3,3-tetramethyluronium tetrafluoroborate
- TFA: trifluoroacetic acid
- THF: tetrahydrofuran

A convenient method for the synthesis of the compounds of this invention utilizes a dipolar cycloaddition of nitrile oxides with appropriate dipolarophiles to prepare the isoxazoline rings present in compounds of Formula I (for reviews of 1,3-dipolar cycloaddition chemistry, see 1,3-Dipolar Cycloaddition Chemistry (Padwa, ed.), Wiley, New York, 1984; Kanemasa and Tsuge, Heterocycles 1990, 30, 719).

Scheme I describes one synthetic sequence to the compounds of the second embodiment of this invention. An appropriately substituted hydroxylamine is treated with NCS in DMF according to the method of Liu, et al. (J. Org. Chem. 1980, 45**,** 3916). The resulting hydroximinoyl chloride is then dehydrohalogenated in situ using TEA to give a nitrile oxide, which undergoes a 1,3-dipolar cycloaddition to a suitably substituted alkene to afford the isoxazoline. Alternatively, the oxime may be oxidatively chlorinated, dehydrochlorinated and the resulting nitrile oxide trapped by a suitable alkene under phase transfer conditions according to the method of Lee (Synthesis 1982, 508). Hydrolysis of the ester using conventional methods known to one skilled in the art of organic synthesis gives the desired acids. Intermediates containing alkali-sensitive functionality, such as nitrile, may be deesterified with excellent chemoselectivity using sodium trimethylsilanolate according to the procedure of Laganis and Ehenard (Tetrahedron Lett, 1984, 25, 5831). Coupling of the resulting acids to an appropriately substituted α- or β-amino ester using standard coupling reagents, such as DCC/HOBt, affords a nitrile-amide. The nitrile is then converted to the amidine *via* the imidate or thioimidate under standard conditions followed by ester saponification (LiOH, THF/H₂O).

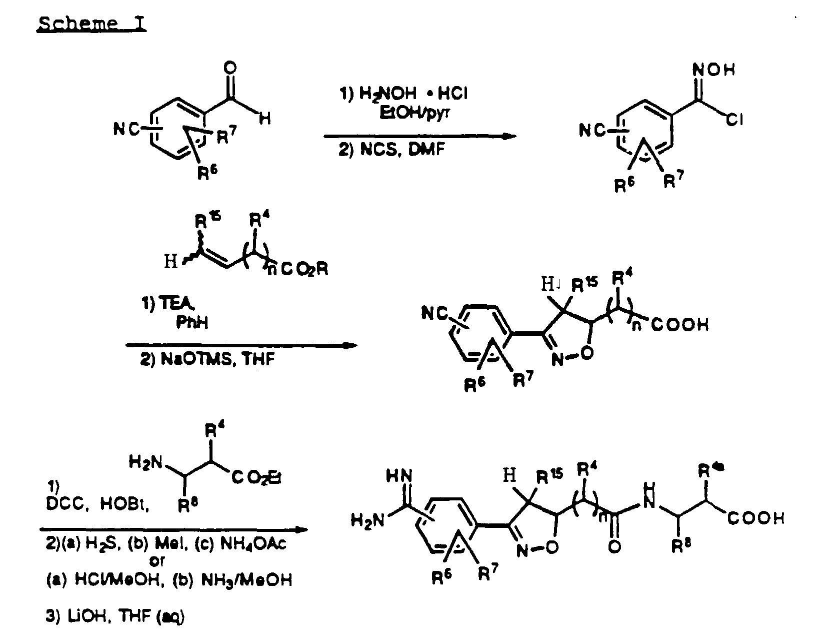

An example of a related method of preparation for compounds of the present invention is illustrated in Scheme Ia. Conversion of 3-(4-Cyanophenyl)-isoxazolin-5-ylacetic acid to the corresponding amidine, followed by protection as the Boc-derivative and saponification provides 3-(4-Boc-amidinophenyl)isoxazolin-5-ylacetic acid which is coupled with β-amino acid esters as shown. Deprotection provides the desired isoxazolinylacetyl-β-aminoalaninyl esters. Saponification as described above gives the free acids.

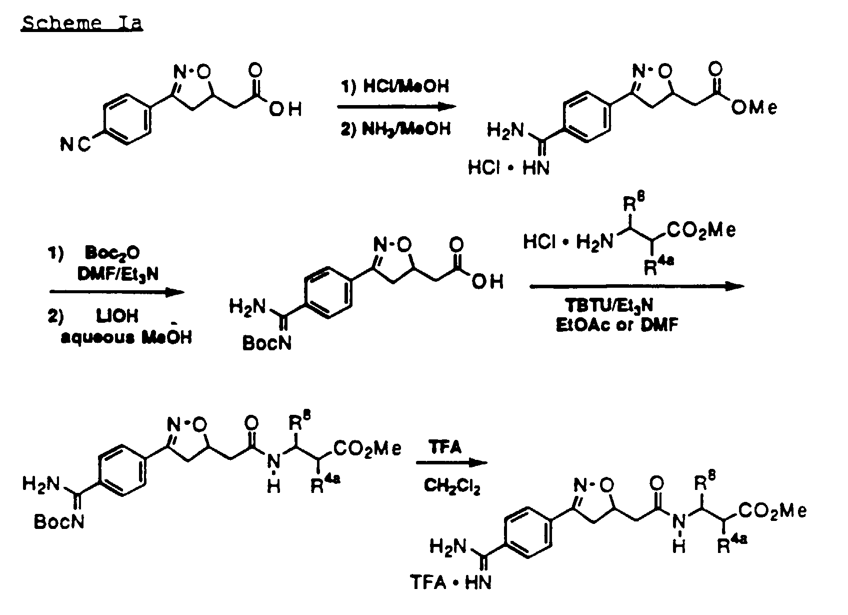

A further example of the synthesis of compounds of the present invention is shown in Scheme Ib. Cycloaddition of commerically available 4-cyanostyrene and t-butylformyloxime using the method described by Gree et al. (Bioorganic and Med. Chem. Lett., 1994, 253) provides t-butyl [5-(4-cyanophenyl)isoxazolin-3-yl]acetate. Using the procedures described above, this intermediate is converted to compounds of formula I wherein the isoxazoline ring is in the reverse orientation with respect to the compounds prepared via Schemes I and Ia.

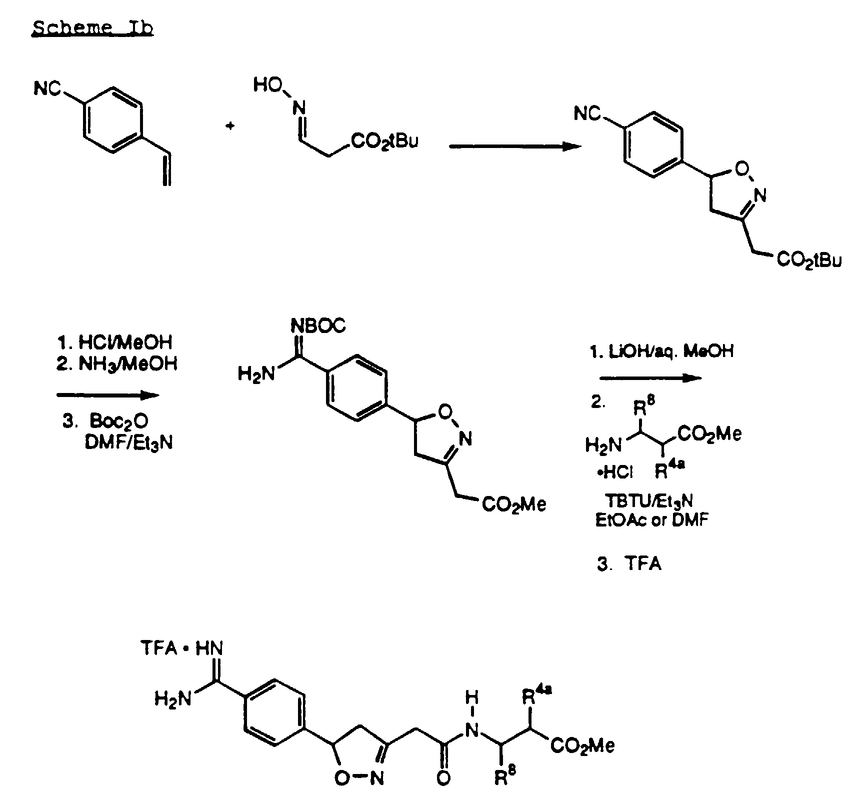

Additional isoxazolinyl acetates useful as starting materials for the preparation of compounds of Formula I, wherein V is -(phenyl)-Q- and Q is other than a single bond, can be prepared by cycloaddition of a suitably substituted chloro or bromooxime with an ester of vinyl acetic acid as shown in Scheme Ic using literature methods or modifications thereof. (D. P. Curran & J. Chao, J. Org. Chem., 1988, 53, 5369-71; J. N. Kim & E. K. Ryu, Heterocycles, 1990, 31, 1693-97).

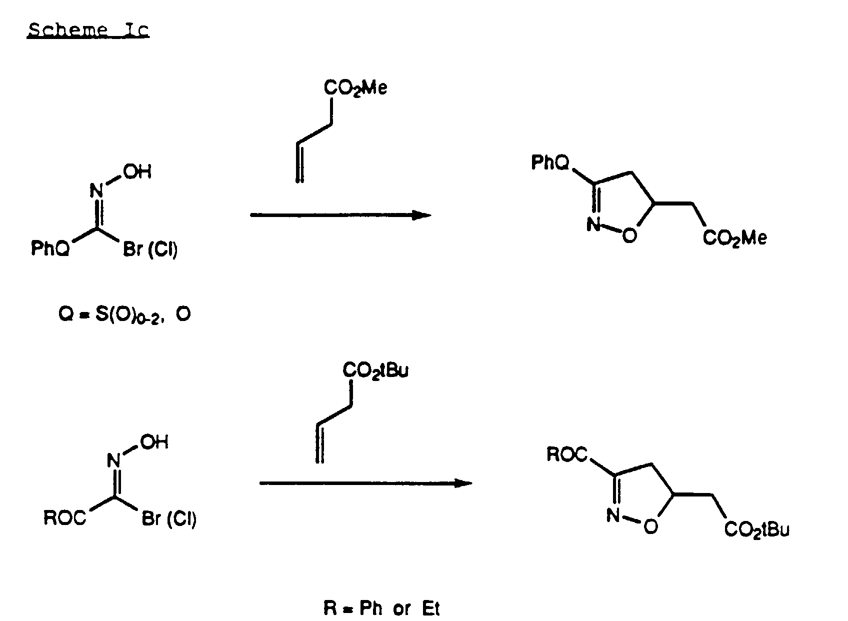

The compounds of the present invention where R² or R³ is e.g. alkoxycarbonyl may be prepared by reacting the free amidines, amines or guanidines with an activated carbonyl derivative, such as an alkyl chloroformate. In compounds of the second embodiment, the conversion of the free amines, amidines and guanidines to such acyl-nitrogen groups may optionally be performed prior to coupling an isoxazoline acetic acid with e.g β-amino acids, as illustrated in Scheme Ia.

The compounds of the present invention wherein Y is an oxyalkoxy group, e.g. alkoxycarbonyloxyalkoxy, may be prepared by reacting a suitably protected carboxylic acid of Formula I with an e.g. an alkoxycarbonyloxyalkyl chloride in the presence of an iodide source, such as tetrabutylammonium iodide or potassium iodide, and an acid scavenger, such as triethylamine or potassium carbonate, using procedures known to those skilled in the art.

The appropriately substituted racemic β-amino acids may be purchased commercially or, as is shown in Scheme II, Method 1, prepared from the appropriate aldehyde, malonic acid and ammonium acetate according to the procedure of Johnson and Livak (J. Am. Chem. Soc. 1936, 5B, 299). Racemic β-substituted-β-amino esters may be prepared through the reaction of dialkylcuprates or alkyllithiums with 4-benzoyloxy-2-azetidinone followed by treatment with anhydrous ethanol (Scheme I, Method 2) or by reductive amination of β-keto esters as is described in W09316038. (Also see Rico et al., J. Org. Chem. 1993, 58, 7948-51.) Enantiomerically pure β-substituted-β-amino acids can be obtained through the optical resolution of the racemic mixture or can be prepared using numerous methods, including: Arndt-Eistert homologation of the corresponding α-amino acids as shown in Scheme II, Method 3 (see Meier, and Zeller, Angew. Chem. Int. Ed. Engl. 1975, 14, 32; Rodriguez, et al. Tetrahedron Lett. 1990, 31, 5153; Greenlee, J. Med. Chem. 1985, 28, 434 and references cited within); and through an enantioselective hydrogenation of a dehydroamino acid as is shown in Scheme II, Method 4 (see Asymmetric Synthesis, Vol. 5, (Morrison, ed.) Academic Press, New York, 1985). A comprehensive treatise on the preparation of β-amino acid derivatives may be found in WO 9307867.

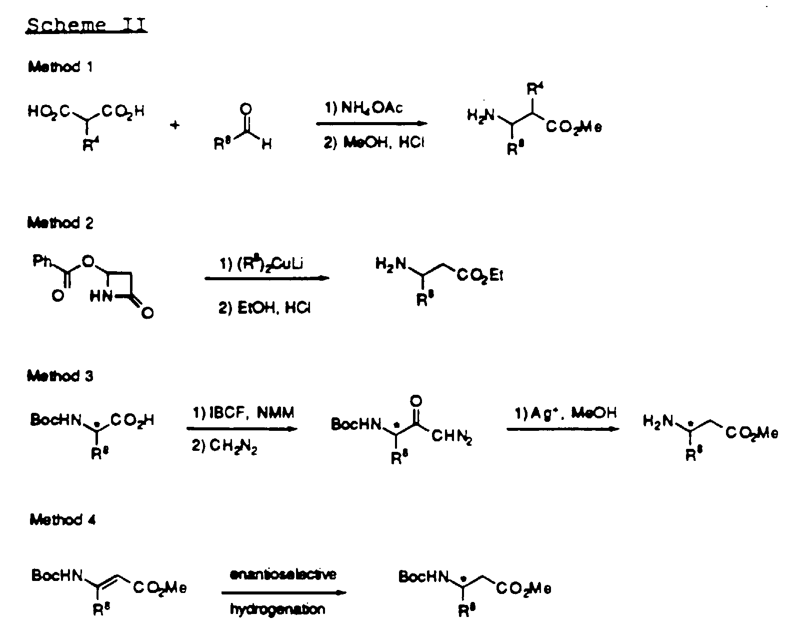

The synthesis of N²-substituted diaminopropionic acid derivatives can be carried out via Hoffman rearrangement of a wide variety of asparagine derivatives as described in Synthesis, 266-267, (1981).

The appropriately substituted pyrrolidine-, piperidine- and hexahydroazepineacetic acids may be prepared using a number of methods. The pyrrolidines are conveniently prepared using an Arndt-Eistert homologation of the corresponding proline as shown in Scheme III, Method 1 (see Meier, and Zeller, Angew, Chem. Int. Ed. Engl, 1975, 14, 32; Rodriguez, et al. Tetrahedron Lett. 1990, 31, 5153; Greenlee, J. Med. Chem. 1985, 28, 434 and references cited within). The piperidines can be prepared by reduction of the corresponding pyridine as shown in Scheme III, Method 2. The hexahydroazepines are prepared by reduction of the corresponding vinylogous amide using sodium cyanoborohydride as depicted in Scheme III, Method 3.

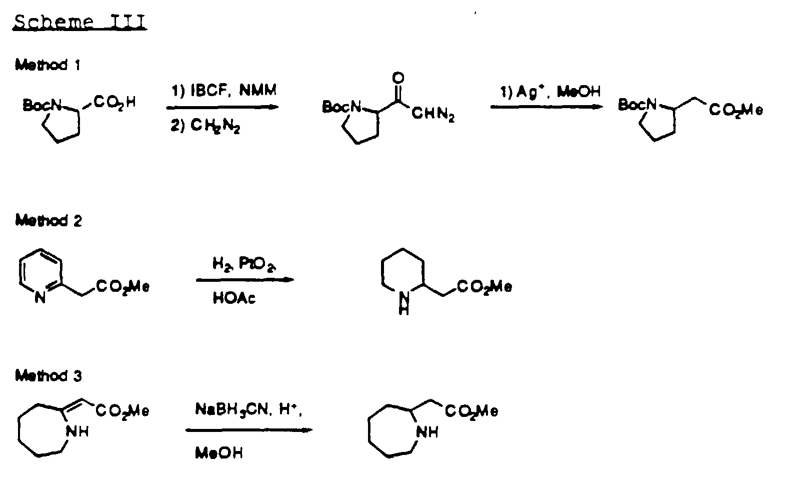

Many additional appropriately substituted heterocycles are available commercially or can be readily modified by procedures known by one skilled in the art. Appropriately substituted morpholines can be prepared from amino acids via the sequence of steps depicted in Scheme IIIa, method 1 (see Brown, et. al. *J*. *Chem. Soc. Perkin Trans I,* **1987**, 547.; Bettoni, et. al. *Tetrahedron* **1980**, *36*, 409. Clarke, F.H. *J. Org. Chem.* **1962**, 27, 3251 and references therein.) N-ethoxycarbonylmethyl-1,2-diazaheterocyles are prepared by condensation of suitably substituted dibromides with benzylhydrazine followed by Mitsunobu reaction with ethyl hydroxyacetate and deprotection as shown in Scheme IIIa, method 2 (see Kornet, et. al. J. Pharm. Sci. 1979, 68, 377.; Barcza, et. al. J. Org. Chem. 1976, 41, 1244 and references therein.)

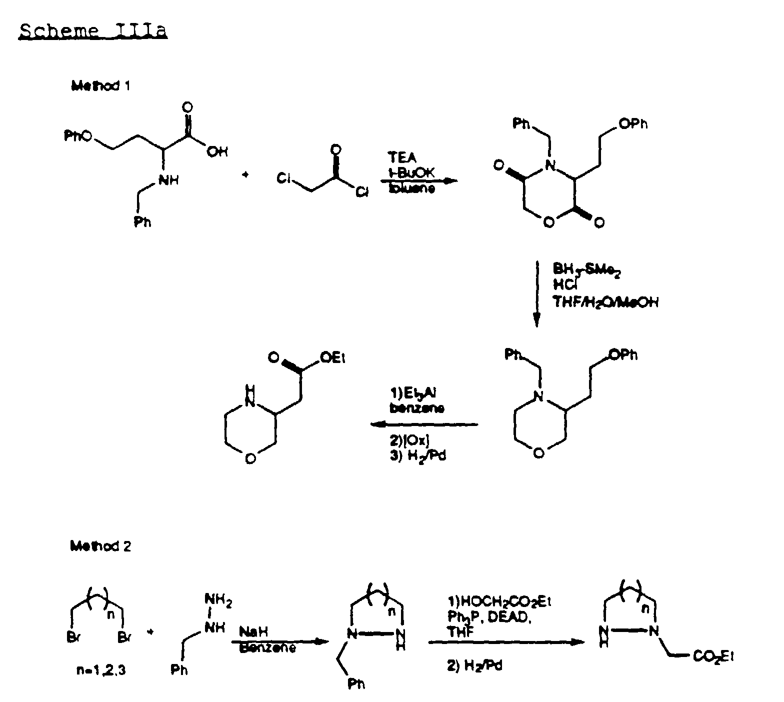

A general synthetic protocol to the compounds of the first embodiment of this invention is depicted in Scheme IV. Coupling of a suitable Boc-protected amino alcohol to an appropriately substituted phenol under Mitsunobu conditions (see Mitsunobu, Synthesis 1981, 1) is followed by oximation using hydroxylamine hydrochloride in 1:1 ethanol/pyridine. Isoxazoline formation, ester saponification and Boc-deprotection (33% TFA/DCM) then affords the compounds of this invention in good overall yield.

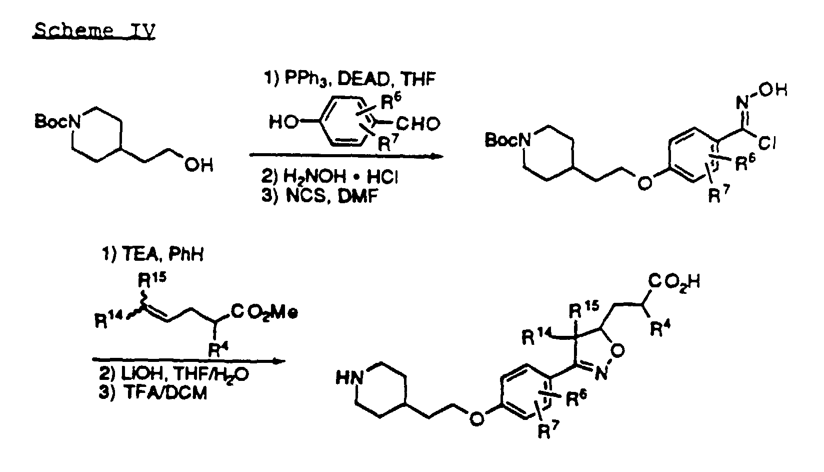

Compounds of formula I wherein b is a double bond can be prepared using one of the routes depicted in Scheme V. Bromination followed by subsequent dehydrobromination of a suitably substituted methyl 3-{cyanophenyl)isoxazolin-5-ylacetate, prepared as described above, using the method of Elkasaby & Salem (Indian J. Chem., **1980**, *19B,* 571-575) provides the corresponding isoxazole intermediate. Alternately, this intermediate can be obtained by 1,3-dipolar cycloaddition of a cyanophenylnitrile oxide (prepared from the corresponding chlorooxime as described in Scheme I) with an appropriate alkyne to give the isoxazole directly. Hydrolysis of the ester using conventional methods known to one skilled in the art of organic synthesis gives the acetic acids. Coupling of the resulting acids to an appropriately substituted α-or β-amino ester using standard coupling reagents, such as TBTU, affords a nitrile-amide. The nitrile is then converted to the amidine *via* the imidate or thioimidate under standard conditions to give the prodrug esters. Saponification gives the acids.

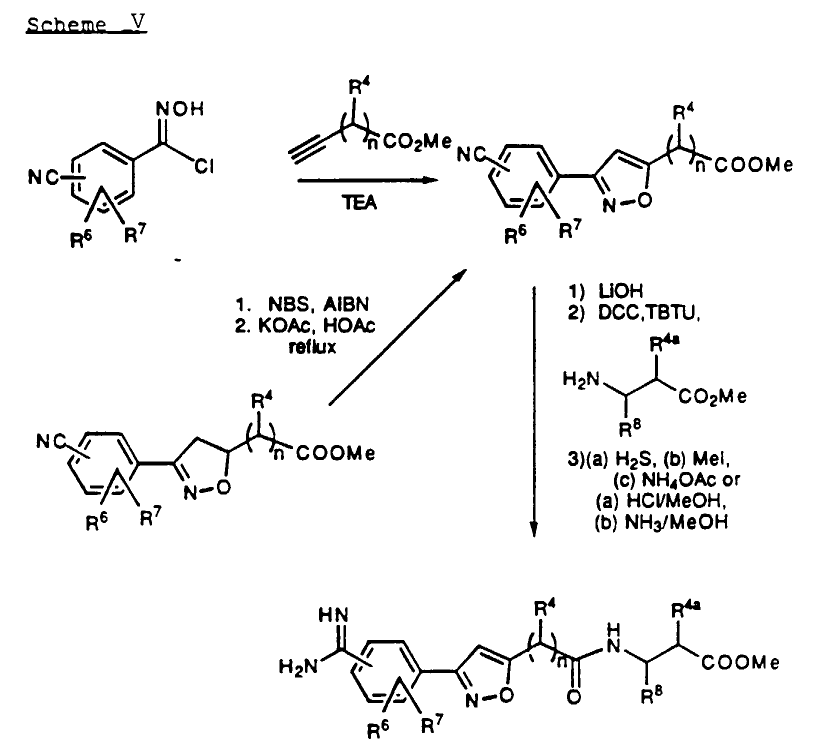

Compounds of Formula I wherein R¹ is (R²) (R³)N(R²N=)CN(R²)- and V is phenylene are prepared as illustrated in Scheme VI. Cycloaddition of an appropriately N-protected aminophenylaldoxime with vinyl acetic acid, t-butyl ester, using the conditions described above provides t-butyl [3-(4-t-butyloxycarbonylaminophenyl)isoxazolin-5-yl]acetate. Hydrolysis of the ester with lithium hydroxide provides the free acid which can be coupled with a suitably substituted methyl 3-aminopropionate as previously described. After deprotection, the aniline is converted to the corresponding guanidine using the method described by Kim et al. (Tetrahedron Lett., 1993, 48, 7677). A final deprotection step to remove the BOC groups provides guanidino compounds of Formula I.

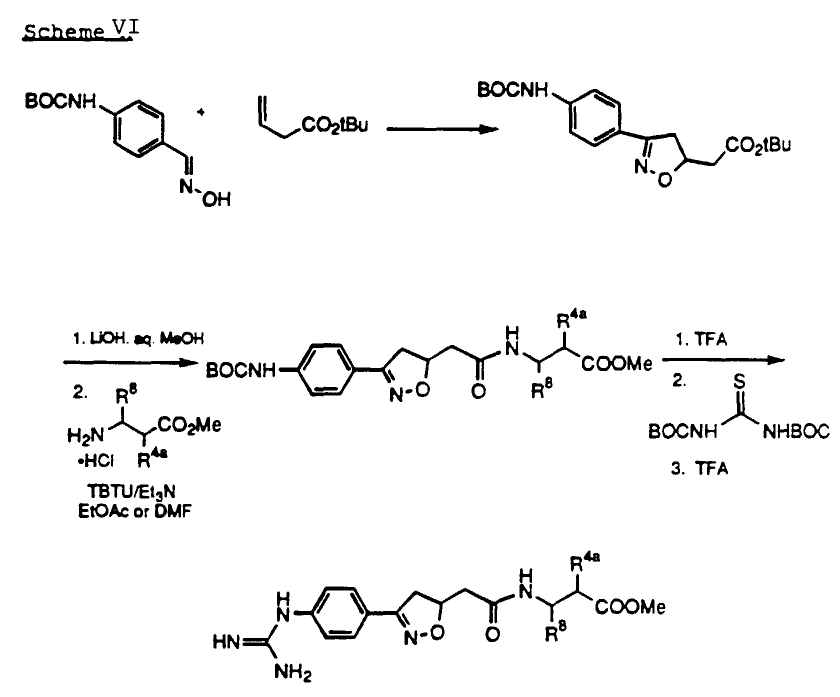

The compounds of this invention and their preparation can be further understood by the following procedures and examples.

### Example 1

### 3-[4-(2-Piperidin-4-yl)ethoxyphenyl]-(5R,S)-isoxazolin-5-ylacetic Acid, Trifluoroacetic Acid Salt

### Part A. Preparation of 2-(4-N-t-Butyloxycarbonylpiperidinyl)ethanol

This material was prepared from 4-piperidine-2-ethanol according to EP-A-0 478 363.

### Part B. 4-[(2-N-t-Butyloxycarbonylpiperidin-4-yl)ethoxy]benzaldehyde

To a solution of 2-(4-*N-t*-Butyloxycarbonylpiperidinyl)ethanol (7.71 g, 33.6 mmol), 4-hydroxybenzaldehyde (4.11 g, 33.6 mmol) and PPh₃ (8.82 g, 33.6 mmol) in THF (60 ml) at -20 °C was added a solution of DEAD (5.3 ml, 33.7 mmol) in THF (30 ml) over 2 hours. During the addition, a deep red solution resulted, which changed to a golden color upon warming to room temperature overnight (18 hours). At this time the solution was concentrated and redissolved in EtOAc. It was then washed with water, 0.1*M* HCl, 1*M* NaOH, sat. NaCl and dried (MgSO₄). Concentration gave a solid (∼20 g), which was purified using flash chromatography (10-20-30-40-50% EtOAc/hexanes step gradient), affording 7.82 g (70%) of the desired ether after pumping to constant weight; mp 76.4-79.7 °C; ¹H NMR (300 MHz, CDCl₃) δ 9.88 (s 1H), 7.83 (d, J = 8.4 Hz, 2H), 6.98 (d, J = 8.4 Hz, 2H), 4.10 (bd, J = 12.8 Hz, 2H), 4.04 (t, J = 6.6 Hz 2H), 2.69 (bt, 2H), 1.84 (m, 2H), 1.70 (bd J = 14.3 Hz, 2H), 1.46 (s, 9H, overlapped with m, 2H), 1.10 (m, 2H).

### Part C. 4-[(2-N-t-Butyloxycarbonylpiperidin-4-yl)ethoxy]benzaldoxime

To a solution of 4-[(2-*N-t*-butyloxycarbonylpiperidin-4-yl)ethoxy]benzaldehyde (3.16 g, 9.48 mmol) in MeOH (20 ml) was added hydroxylamine hydrochloride (1.27 g, 18.3 mmol) and 2*M* NaOH (7 ml, 14 mmol). The resulting suspension was stirred overnight at room temperature (18 hours). The mixture was brought to pH 4 using 1*M* HCl, followed by filtration and water wash. The crystals were dried under vacuum over P₂O₅, affording 2.88 g (87%); mp: 114.4-116.1 °C; ¹H NMR (300 MHz, CDCl₃) δ 8.09 (s, 2H), 7.51 (d, J = 8.8 Hz, 2H), 6.89 (d, J = 8.8 Hz, 2H), 4.10 (b, 2H), 4.03 (t, J = 6.2 Hz 2H), 2.71 (bt, 2H), 1.73 (m, 4H), 1.46 (s, 9H), 1.19 (m, 2H).

### Part D. 4-[(2-N-t-Butyloxycarbonylpiperidin-4-yl)ethoxy]benzaldoximinoyl Chloride

To a solution of 4-[(2-*N*-*t*-butyloxycarbonylpiperidin-4-yl)ethoxy]benzaldoxime (955 mg, 2.74 mmol) in DMF (5 ml) was added NCS (366 mg, 2.74 mmol) in 3 portions. After 3 hours, the solution was diluted with EtOAc and washed with water, sat. NaCl, dried (MgSO₄) and concentrated. The resulting solid was crystallized from ether/hexanes to give 548 mg (52%) of the oximinoyl chloride; mp: 119.3-119.9 °C; ¹H NMR (300 MHz, CDCl₃) δ 8.37 (bs 1H), 7.77 (d, J = 8.8 Hz, 2H), 6.88 (d, J = 8.8 Hz, 2H), 4.12 (bd, J = 13.2 Hz, 2H), 4.04 (t, J = 6.2 Hz 2H), 2.72 (bt, J = 12.1 Hz, 2H), 1.70 (m, 5H), 1.46 (s, 9H), 1.10 (m, 2H).

### Part E. Methyl 3-[4-{(2-N-t-Butyloxycarbonylpiperidin-4-yl)ethoxy)phenyl]-(5R,S)-isoxazolin-5-ylacetate

To a solution of 4-[(2-*N*-*t*-butyloxycarbonylpiperidin-4-yl)ethoxy]benzaldoximinoyl chloride (400 mg, 1.045 mmol) and methyl 3-butenoate (200 mg, 2.00 mmol) was added TEA (0.15 ml, 1.1 mmol). The resulting suspension was heated at reflux for 5 hours, cooled to room temperature and diluted with EtOAc. It was then washed with 0.1*M* HCl, water, sat. NaCl, dried (MgSO₄) and concentrated. The resulting solid was crystallized from DCM/hexanes to give 357 mg (77%) of the isoxazoline; mp: 139.1-140.9 °C; ¹H NMR (300 MHz, CDCl₃) δ 7.59 (d, J = 8.8 Hz, 2H), 6.90 (d, J = 8.8 Hz, 2H), 5.08 (m, 1H), 4.10 (bd, J = 13.2 Hz, 2H), 4.04 (t, J = 5.9 Hz 2H), 3.73 (s, 3H), 3.53 (dd, J = 16.5, 10.1 Hz, 1H), 3.10 (dd, J = 16.8, 7.1 Hz, 1H), 2.88 (dd, J = 16.1, 5.9 Hz, 1H), 2.71 (bt, J = 12.8 Hz, 2H), 2.64 (dd, J = 15.8, 7.7 Hz, 1H), 1.72 (m, 5H), 1.46 (s, 9H), 1.08 (m, 2H).

### Part F. 3-[4-{(2-N-t-Butyloxycarbonylpiperidin-4-yl)ethoxy}phenyl]-(5R,S)-isoxazolin-5-ylacetic Acid

To a solution of methyl 3-[4-{(2-*N-t*-butyloxycarbonylpiperidin-4-yl)ethoxy}phenyl]-(5*R*,*S*)-isoxazolin-5-ylacetate (47 mg, 0.105 mmol) in THF (2 ml) was added 0.5*M* LiOH (1 ml, 0.5 mmol). The reaction was stirred at room temperature for 5 hours, then was acidified to pH 3 using 0.1*M* HCl. The mixture was washed with DCM and the combined organic fraction dried (MgSO₄) and concentrated. The resulting solid was crystallized from EtOAc/hexanes to give 34 mg (74%) of the carboxylic acid; mp: 169.1-170.6 °C; ¹H NMR (300 MHz, CDCl₃) δ 7.60 (d, J = 8.8 Hz, 2H), 6.91 (d, J = 8.8 Hz, 2H), 5.10 (m, 1H), 4.08 (bd, 2H, overlapped with t, J = 5.9 Hz 2H), 3.55 (dd, J = 16.5, 10.2 Hz, 1H), 3.11 (dd, J = 16.8, 7.0 Hz, 1H), 2.93 (dd, J = 16.1, 6.2 Hz, 1H), 2.71 (m, 3H), 2.00 (m, 2H), 1.72 (m, 5H), 1.46 (s, 9H).

### Part G. 3-[4-(2-Piperidin-4-yl)ethoxyphenyl]-(5R,S)-isoxazolin-5-ylacetic Acid, Trifluoroacetic Acid Salt

To a solution of 3-[4-{(2-*N-t*-Butyloxycarbonylpiperidin-4-yl)ethoxy}phenyl]-(5*R*,*S*)-isoxazolin-5-ylacetic acid (53 mg, 0.12 mmol) in DCM (2 ml) was added TFA (1 ml, 13 mmol). After 1.5 hours, the product was crystallized by the addition of ether, affording 33 mg (60%) of the amino acid; mp: 142.4-143.1 °C; ¹H NMR (300 MHz, CDCl₃) δ 7.59 (dd, J = 8.8, 2.6 Hz, 2H), 6.96 (dd, J = 8.8, 2.6 Hz, 2H), 5.03 (m, 1H), 4.10 (m, 2H), 3.55 (ddd, J = 16.8, 10.3, 2.2 Hz, 1H), 3.38 (bd, J = 12.4 Hz, 2H), 3.16 (ddd, J = 17.2, 7.7, 2.2 Hz, 1H), 2.98 (bt, J = 13.2 Hz, 2H), 2.69 (m, 2H), 2.01 (bd, J = 14.3 Hz, 2H), 1.91 (m, 1H), 1.80 (m, 2H), 1.46 (m, 2H).

### Example 4

### (2S)-(5R,S)-[3-[4-{(2-Piperidin-4-yl)ethoxy}phenyl]isoxazolin-5-yl{[(benzyloxy)carbonyl]amino}]acetate, Trifluoroacetic Acid Salt

### Part A. Benzyl L-2-[[Benzyloxy)carbonyl]amino]-3-butenoate

This material was prepared from *N*-Cbz-L-glutamic acid α-benzyl ester according to Krol, et al. (J. Org. Chem. 1991, 728).

### Part B. Benzyl (2S)-(5R,S)-[3-[4-{(2-N-t-Butyloxycarbonylpiperidin-4-yl)ethoxy}phenyl]isoxazolin-5-yl{[(benzyloxy)carbonyl]amino}]acetate

To a solution of 4-[(2-*N*-*t*-butyloxycarbonylpiperidin-4-yl)ethoxy]benzaldoxime (852 mg, 2.44 mmol) and benzyl L-2-[[(benzyloxy)carbonyl]amino]-3-butenoate (612 mg, 1.88 mmol) in DCM (10 ml) was added 5% NaOCl (common household bleach, 4 ml, 2.8 mmol). The mixture was rapidly stirred at room temperature for 22 hours, after which time it was diluted with water and DCM. After separation of the layers, the aqueous was washed with DCM (3x). The combined organic extracts were dried (MgSO₄) and concentrated *in vacuo,* giving 1.4 g. Purification using flash chromatography (10% EtOAc/hexanes - 30% EtOAc/hexanes) then afforded 886 mg (70%) of an oily product as a 2.5 : 1 mixture of the *erythro* and *threo* isomers; ¹H NMR (400 MHz, CDCl₃) δ 7.50 (m, 2H), 7.34 (m, 5H), 7.23 (m, 5H), 6.87 (d, J = 8.8 Hz, 2H), 5.47 (bd, 1H), 5.12 (m, 5H), 4.60 (m, 1H), 4.07 (m, overlapped with 4.03 (t, J = 6.1 Hz, 4H)), 3.36 (m, 2H), 2.71 (bt, J = 12.7 Hz, 2H), 1.70 (m, 5H), 1.45 (s, 9H), 1.18 (m, 2H); Anal. Calc. for C₃₈H₄₅N₃O₈: C, 67.93; H, 6.76; N, 6.26. Found: C, 67.95; H, 6.77; N, 6.17.

### Part C. (2S)-(5R,S)-[3-[4-{(2-N-t-Butyloxycarbonylpiperidin-4-yl)ethoxy}phenyl]isoxazolin-5-yl{[(benzyloxy)carbonyl]amino}]acetic Acid

A solution of benzyl (2*S*)-(5*R,S*)-[3-[4-{(2-*N-t*-butyloxycarbonylpiperidin-4-yl)ethoxy)phenyl]isoxazolin-5-yl{[(benzyl-oxy)carbonyl]amino)]acetate (875 mg, 1.302 mmol) in THF (5 ml) was saponified over 5 hours using 0.5M LiOH (3.5 ml) according to Example 1, Part F. To the crude product was added methanol, causing crystallization of one of the diastereomers. Filtration and pumping to constant weight gave 295 mg (39%); mp: 216.1 °C; ¹H NMR (400 MHz, DMSO-d₆, 80 °C) δ 7.50 (d, J = 8.9 Hz, 2H), 7.23 (s, 5H), 6.96 (d, J = 8.9 Hz, 2H), 6.17 (bs, 1H), 4.99 (m, 3H), 4.07 (t, J = 6.1 Hz, 2H), 3.90 (m, 3H), 3.35 (d, J = 9.3 Hz, 2H), 2.72 (bt, J = 12.4 Hz, 2H), 1.67 (m, 5H), 1.39 (s, 9H), 1.08 (m, 2H). The filtrate was concentrated in vacuo and pumped until constant weight was achieved, giving 200 mg (26%) of the carboxylic acids as a mixture of *erythro-* and *threo*-isomers; TLC (silica gel 60, 20% MeOH/CHCl₃) R_{f} = 0.23, Mass Spectrum (ESI, e/z, relative abundance) 582 (M + H)⁺, 32%; 526 (M - C₄H₉ + H₂)⁺, 100%; 482 (M - Boc + H₂)⁺, 91%).

### Part D. (2S)-(5R,S)-[3-[4-{(2-Piperidin-4-yl)ethoxy}phenyl]isoxazolin-5-yl{[(benzyloxy)carbonyl]amino}]acetic Acid (isomer A)

(2*S*)-(5*R*,*S*)-[3-[4-{(2-*N-t*-Butyloxycarbonylpiperidin-4-yl)ethoxy}phenyl]isoxazolin-5-yl{[(benzyloxy)carbonyl]amino}]acetic acid (23 mg, 0.039 mmol) was Boc-deprotected using 33% TFA/DCM according to Example 1, Part G, giving 15 mg (79%); mp: 302 °C (dec); ¹H NMR (400 MHz, DMSO-d₆, 60 °C) δ 7.57 (d, J = 8.8 Hz, 2H), 7.30 (s, 5H), 6.99 (d, J = 8.8 Hz, 2H), 5.05 (s, 2H, coincident with m, 1H), 4.35 (d, J = 4.9 Hz, 1H), 4.09 (t, J = 6.1 Hz, 2H), 3.52 (dd, J = 17.3, 10.7 Hz, 1H), 3.26 (m, 3H), 2.88 (dt, J = 12.7, 2.7 Hz, 2H), 1.88 (bd, J = 14.4 Hz, 2H), 1.80 (m, 1H), 1.72 (m, 2H), 1.38 (m, 2H).

### Part D'. (2S)-(5R,S)-[3-[4-{(2-Piperidin-4-yl)ethoxy}phenyl]isoxazolin-5-yl{[(benzyloxy)carbonyl]amino}]acetic Acid, Trifluoroacetic Acid Salt (isomer B)

(2*S*)-(5*R,S*)-[3-[4-{(2-*N-t*-Butyloxycarbonylpiperidin-4-yl)ethoxy)phenyl]isoxazolin-5-yl{[(benzyloxy)carbonyl]amino}]acetic acid (177 mg, 0.304 mmol) was Boc-deprotected using 33% TFA/DCM according to Example 1, Part G, giving 3 mg (2%) of the TFA salt; mp: >400 °C; ¹H NMR (400 MHz, DMSO-d₆, 60 °C) δ 8.48 (bs, 0.5H), 8.15 (bs, 0.5H), 7.55 (d, J = 8.9 Hz, 2H), 7.30 (m, 5H), 6.97 (d, J = 8.9 Hz, 2H), 5.05 (s, 2H), 4.96 (m, 1H), 4.33 (m, 1H), 4.07 (t, J = 6.3 Hz, 2H), 3.38 (m, 2H), 3.26 (bd, J = 12.0 Hz, 2H), 2.87 (m, 2H), 1.86 (bd, J = 14.2 Hz, 2H), 1.78 (m, 1H), 1.70 (apparent q, J - 6.3 Hz, 2H), 1.36 (bq, J = 13.2 Hz, 2H).

### Example 6

### 3-(3-[4-(Piperidin-4-ylmethoxy)phenyl]-(5R,S)-isoxazolin-5-yl)propionic Acid, Trifluoroacetic Acid Salt

### Part A. Ethyl N-t-Butyloxycarbonylpiperidine-4-carboxylate

To a stirred solution of ethyl isonipecotate (20.01 g, 0.1273 mol) in EtOAc (100 ml) at 0 °C was added dropwise a solution of Boc₂O (27.76 g, 0.1272 mol) in EtOAc (50 ml). The mixture was allowed to warm to room temperature overnight. After 20 hours, the mixture was washed with water, 0.1*M* HCl, sat. NaHCO₃, sat. NaCl and dried (MgSO₄). Concentration and pumping under vacuum to constant weight gave 32.54 g (99%) of the desired carbamate as a mobile oil; ¹H NMR (300 MHz, CDCl₃) δ 4.13 (q, J = 7.0 Hz, 2H), 4.03 (dm, J = 13.6 Hz 2H), 2.81 (m, 2H), 2.41 (m, 1H), 1.86 (dm, J = 13.6 Hz, 2H), 1.62 (m, 2H), 1.44 (s, 9H), 1.24 (t, J = 7.0 Hz, 3H).

### Part B. N-t-Butyloxycarbonylpiperidin-4-ylmethanol

To a solution of ethyl *N-t*-butyloxycarbonylpiperidine-4-carboxylate (32.34 g, 0.1257 mol) in THF (100 ml) at 0 °C was added dropwise 1*M* LAH in THF (87.9 ml, 0.0879 mol). After 2 hours, excess hydride was quenched by the addition of water (3.2 ml), 2*M* NaOH (3.2 ml) and water (10 ml). The mixture was filtered, washed with EtOAc and the filtrate washed with water, sat. NaCl, dried (MgSO₄) and concentrated. Pumping to constant weight gave 22.72 g (84%); mp: 79.2-81.1 °C; ¹H NMR (300 MHz, CDCl₃) δ 4,12 (bd, J = 12.8 Hz 2H), 3.49 (d, J = 6.2 Hz, 2H), 2.68 (dt, J = 13.2, 1.8 Hz, 2H), 1.69 (m, 3H), 1.44 (s, 9H, overlapped with m, 1H), 1.14 (m, 2H).

### Part C. 4-(N-t-Butyloxycarbonylpiperidin-4-ylmethoxy)-benzaldehyde

To *N*-*t*-butyloxycarbonylpiperidin-4-ylmethanol (7.87 g, 36.5 mmol), p-hydroxybenzaldehyde (4.46 g, 36.5 mmol) and PPh₃ (9.59 g, 36.5 mmol) in THF (100 ml) at -20 °C was added DEAD (5.75 ml, 36.5 mmol) in THF (50 ml) according to Example 1, Part B, affording 8.14 g (70%); mp: 115.6-116.8 °C; ¹H NMR (300 MHz, CDCl₃) δ 9.86 (s, 1H), 7.81 (d, J = 8.8 Hz, 2H), 6.96 (d, J = 8.8 Hz, 2H), 4.15 (bd, J = 13.2 Hz 2H), 3.87 (d, J = 6.6 Hz, 2H), 2.74 (dt, J = 12.4, 1.8 Hz, 2H), 1.97 (m, 1H), 1.81 (bd, J = 12.8 Hz, 2H), 1.45 (s, 9H), 1.27 (dq, J = 12.1, 4.0 Hz, 2H).

### Part D. 4-(N-t-Butyloxycarbonylpiperidin-4-ylmethoxy)-benzaldoxime

A mixture of 4-(*N-t*-butyloxycarbonylpiperidin-4-ylmethoxy)benzaldehyde (3.16 g, 9.89 mmol) and hydroxylamine hydrochloride (1.27 g, 18.3 mmol) in 9:1 MeOH/pyridine (30 ml) was heated at reflux for 18 hours. The mixture was cooled to room temperature and concentrated to dryness. The residue was dissolved in EtOAc and washed with 0.1*M* HCl (3x), water, sat. CuSO₄ (2x), water, sat. NaCl, dried (MgSO₄) and concentrated, giving 3.19 g (96%) of the oxime; mp: 140.1-141.8 °C; ¹H NMR (300 MHz, CDCl₃) δ 8.07 (s, 1H), 7.48 (d, J = 8.8 Hz, 2H), 6.86 (d, J = 8.8 Hz, 2H), 4.14 (bs, 2H), 3.80 (d, J = 6.2 Hz, 2H), 2.71 (bt, J = 12.4 Hz, 2H), 1.95 (m, 1H), 1.80 (bd, J = 12.4 Hz, 2H), 1.45 (s, 9H), 1.26 (m, 2H).

### Part E. 4-(N-t-Butyloxycarbonylpiperidin-4-ylmethoxybenzaldoximinoyl Chloride

4-(*N-t*-Butyloxycarbonylpiperidin-4-ylmethoxy)-benzaldoxime (3.19 g, 9.54 mmol) in DMF (10 ml) was reacted with NCS (1.27 g, 9.51 mmol) for 18 hours according to Example 1, Part D to afford the hydroximinoyl chloride (1.17 g, 33%); mp: 178.0-179.8 °C; ¹H NMR (300 MHz, CDCl₃) δ 7.75 (d, J = 9.0 Hz, 2H), 6.86 (d, J = 9.0 Hz, 2H), 4.17 (bd, J = 12.4 Hz, 2H), 3.80 (d, J = 6.2 Hz, 2H), 2.74 (dt, J = 12.8, 1.8 Hz, 2H), 1.95 (m, 1H), 1.81 (bd, J = 12.1 Hz, 2H), 1.46 (s, 9H), 1.27 (dq, J = 12.5, 4.0 Hz, 2H).

### Part F. Methyl 3-(3-[4-(N-t-Butyloxycarbonylpiperidin-4-ylmethoxy)phenyl]-(5R,S)-isoxazolin-5-yl)propionate

4-(*N-t*-Butyloxycarbonylpiperidin-4-ylmethoxy)benzaldoximinoyl chloride (738 mg, 2.00 mmol), methyl 4-pentenoate (230 mg, 2.02 mmol) and TEA (0.28 ml, 2.0 mmol) were heated at reflux for 1 hour according to Example 1, Part E. Crystallization from ether/hexanes afforded 537 mg (60%). mp: 97.9-99.9 °C; ¹H NMR (300 MHz, CDCl₃) δ 7.57 (d, J = 9.0 Hz, 2H), 6.87 (d, J = 9.0 Hz,2H), 4.74 (m, 1H), 4.15 (bd, J = 13.2 Hz, 2H), 3.81 (d, J = 6.2 Hz, 2H), 3.67 (s, 3H), 3.40 (dd, J = 16.5, 10.2 Hz, 1H), 2.95 (dd, J = 16.5, 7.3 Hz, 1H), 2.73 (dt, J = 13.2, 1.1 Hz, 2H), 2.52 (t, J = 7.3 Hz, 2H), 1.98 (q, J = 7.0 Hz, 2H, overlapping m, 1H), 1.81 (bd, J = 12.8 Hz, 2H), 1.45 (s, 9H), 1.26 (dq, J = 12.4, 3.7 Hz, 2H).

### Part G. 3-(3-[4-(N-t-Butyloxycarbonylpiperidin-4-ylmethoxy)phenyl]-(5R,S)-isoxazolin-5-yl)propionic Acid

Methyl 3-(3-[4-(*N-t*-butyloxycarbonylpiperidin-4-ylmethoxy)phenyl]-(5*R,S*)-isoxazolin-5-yl)propionate (250 mg, 0.560 mmol) was saponified using 0.5*M* LiOH (2 ml) 1 mmol) in THF (2 ml). The reaction was stirred at room temperature for 3 hours, according to Example 1, Part F. The resulting solid was crystallized from DCM/hexanes to give 163 mg (67%) of the carboxylic acid; mp: 146.5-147.7 °C; ¹H NMR (300 MHz, CDCl₃) δ 7.57 (d, J = 8.8 Hz, 2H), 6.88 (d, J = 8.8 Hz, 2H), 4.75 (m, 1H), 3.81 (d, J = 6.2 Hz, 2H), 3.41 (dd, J = 16.5, 10.3 Hz, 1H), 2.95 (dd, J = 16.5, 7.3 Hz, 1H), 2.75 (bt, J = 12.4 Hz, 2H), 2.57 (t, J = 7.3 Hz, 2H), 1.97 (m, 3H), 1.81 (bd, J = 12.1 Hz, 2H), 1.45 (s, 9H), 1.24 (m, 2H).

### Part H. 3-(3-[4-(Piperidin-4-ylmethoxy)phenyl]-(5R,S)-isoxazolin-5-yl)propionic Acid, Trifluoroacetic Acid Salt

3-(3-[4-(*N-t*-Butyloxycarbonylpiperidin-4-ylmethoxy)phenyl]-(5*R,S*)-isoxazolin-5-yl)propionic acid (103 mg, 0.238 mmol) was Boc-deprotected using 33% TFA/DCM according to Example 1, Part G, giving 88 mg (83%) of the TFA salt; mp: 179.1-181.8 °C; ¹H NMR (400 MHz, MeOHd₄) δ 7.60 (d, J = 9.0 Hz, 2H), 6.97 (d, J = 9.0 Hz, 2H), 4.73 (m, 1H), 3.94 (d, J = 6.1 Hz, 2H), 3.46 (m, 3H), 3.06 (m, 3H), 2.45 (dt, J = 7.3, 1.2 Hz, 2H), 2.16 (m, 1H), 2.08 (bd, J = 15.4 Hz, 2H), 1.94 (q, J = 6.6 Hz, 1H), 1.64 (dq, J = 14.2, 4.2 Hz, 2H).

### Example 7

### 3-[4-(Piperidin-4-ylmethoxy)phenyl]-(5R,S)-isoxazolin-5-ylacetic Acid, Trifluoroacetic Acid Salt

### Part A. Methyl 3-[4-(N-t-Butyloxycarbonylpiperidin-4-ylmethoxy)phenyl]-(5R,S)-isoxazolin-5-ylacetate

4-(*N-t*-Butyloxycarbonylpiperidin-4-ylmethoxy)benzaldoximinoyl chloride (412 mg, 1.12 mmol), methyl 3-butenoate (200 mg, 2.00 mmol) and TEA (0.18 ml, 1.3 mmol) were heated at reflux for 2 hours according to Example 1, Part E. Crystallization from chloroform/cyclohexane afforded 329 mg (68%), mp: 97.9-99.9 °C; ¹H NMR (300 MHz, CDCl₃) δ 7.58 (d, J = 8.8 Hz, 2H), 6.88 (d, J = 8.8 Hz,2H), 5.04 (m, 1H), 4.15 (bd, J = 13.2 Hz, 2H), 3.81 (d, J = 6.2 Hz, 2H), 3.71 (s, 3H), 3.54 (dd, J = 16.8, 10.3 Hz, 1H), 3.08 (dd, J = 16.8, 7.3 Hz, 1H), 2.86 (dd, J = 16.1, 5.9 Hz, 1H), 2.73 (dt, J = 12.8, 1.8 Hz, 2H), 2.62 (dd, J = 15.8, 7.7 Hz, 1H), 1.95 (m, 1H), 1.81 (bd, J = 13.2 Hz, 2H), 1.45 (s, 9H), 1.25 (dq, J = 12.8, 4.4 Hz, 2H).

### Part B. 3-[4-(N-t-Butyloxycarbonylpiperidin-4-ylmethoxy)phenyl]-(5R,S)-isoxazolin-5-ylacetic Acid

Methyl 3-[4-(*N-t*-butyloxycarbonylpiperidin-4-ylmethoxy)phenyl]-(5*R,S*)-isoxazolin-5-ylacetate (329 mg, 0.762 mmol) was saponified using 0.5*M* LiOH (3 ml, 1.5 mmol) in THF (5 ml). The reaction was stirred at reflux for 4 hours, according to Example 1, Part F to give 72 mg (22%) of the carboxylic acid; mp: 164.0-164.8 °C; ¹H NMR (300 MHz, CDCl₃) δ 7.58 (d, J = 8.8 Hz, 2H), 6.88 (d, J = 8.8 Hz, 2H), 5.07 (m, 1H), 4.15 (bd, J = 13.6 Hz, 2H), 3.82 (d, J = 6.2 Hz, 2H), 3.53 (dd, J = 16.8, 10.3 Hz, 1H), 3.10 (dd, J = 16.8, 7.0 Hz, 1H), 2.91 (dd, J = 16.1, 5.9 Hz, 1H), 2.73 (dt, J = 14.6, 1.8 Hz, 2H), 2.68 (dd, J = 16.1, 7.3 Hz, 1H), 1.97 (m, 1H), 1.81 (bd, J = 13.2 Hz, 2H), 1.45 (s, 9H), 1.26 (dq, J = 12.8, 4.4 Hz, 2H).

### Part C. 3-[4-(Piperidin-4-ylmethoxy)phenyl]-(5R,S)-isoxazolin-5-ylacetic Acid, Trifluoroacetic Acid Salt

3-[4-(*N-t*-Butyloxycarbonylpiperidin-4-ylmethoxy)phenyl]-(5*R,S*)-isoxazolin-5-ylacetic acid (72 mg, 0.172 mmol) was Boc-deprotected using 33% TFA/DCM according to Example 1, Part G, giving 64 mg (94%) of the TFA salt; mp: 220 °C (dec); ¹H NMR (300 MHz, MeOHd₄) δ 7.61 (d, J = 9.2 Hz, 2H), 6.97 (d, J = 9.2 Hz, 2H), 5.04 (m, 1H), 3.95 (d, J = 5.9 Hz, 2H), 3.56 (dd, J = 17.2, 10.2 Hz, 1H), 3.45 (bd, J = 12.8 Hz, 2H), 3.18 (dd, J = 17.2, 7.3 Hz, 1H), 3.04 (dt, J = 10.2, 2.9 Hz, 2H), 2.69 (m, 2H), 2.18 (m, 1H), 2.08 (bd, J = 14.6 Hz, 2H) 1.63 (bq, 2H).

### Example 8

### 3-[4-(2-Piperidin-4-yl)ethoxyphenyl]-(5R,S)-isoxazolin-5-ylpropionic Acid, Trifluoroacetic Acid Salt

This material was prepared analogously to Example 1, giving the desired material; mp: 114.8-115.7 °C; ¹H NMR (300 MHz, CD₃OD) δ 7.59 (d, J = 8.4 Hz, 2H), 6.95 (d, J = 8.4 Hz, 2H), 4.72 (m, 1H), 4.07 (t, J = 5.9 Hz, 2H), 3.47 (dd, J = 16.8. 10.2 Hz, 1H), 3.37 (dd, J = 16.8, 7.7 Hz, 1H), 2.98 (m, 2H), 2.44 (t, J = 7.3 Hz, 2H), 2.01 (bd, J = 15.0 Hz, 2H), 1.93 (m, 3H), 1.80 (m, 2H), 1.44 (m, 2H).

### Example 9

### erythro- and-threo-3-[3-[4-[(piperidin-4-yl)methoxy]phenyl]isoxazolin-5-yl{[butanesulfonyl]amino]propionate, Trifluoroacetic Acid Salt

### Part A. Dicyclohexylammonium D,L-2-[(Butanesulfonyl)-amino]-4-pentenoic acid

To a suspension of D,L-2-amino-4-pentenoic acid (2.54 g, 22.06 mmol) in acetonitrile (35 ml) was added BSTFA (7.3 ml, 27.5 mmol). The suspension was heated at 55 °C for 2 hours, after which time a golden yellow solution resulted. To this solution was added pyridine (2.2 ml, 27.2 mmol) and *n*-butanesulfonyl chloride (3.0 ml, 23.1 mmol). The mixture was heated at 70 °C for 20 hours, then cooled to room temperature. Concentration *in vacuo* afforded a brown oil, to which was added 15% KHSO₄ (5 ml). The mixture was stirred for 1 hour and shaken with EtOAc (3x). The combined organic extracts were washed with sat. NaCl, dried (MgSO₄), concentrated and the resulting oil dissolved in ether (5 ml). To this solution was added DCHA (4.38 ml, 22.0 mmol), causing immediate precipitation of the dicyclohexylammonium salt. The solid was collected by filtration and pumped to constant weight, giving 8.42 g (92%); mp: 207.1-208.6 °C; ¹H NMR (400 MHz, MeOH-d₄) δ 5.84 (m, 1H), 5.09 (dm, J = 17.1 Hz, 1H), 5.04 (dm, J = 10.2 Hz, 1H), 3.80 (dd, J = 7.1, 5.1 Hz, 1H), 3.18 (m, 2H), 3.02 (m, 2H), 2.49 (m, 2H), 2.06 (m, 4H), 1.78 (m, 8H), 1.55 (m, 12H), 0.94 (t, J = 7.3 Hz).

### Part B. Methyl D,L-2-[(Butanesulfonyl)amino]-4-pentenoate

To a solution of dicyclohexylammonium D,L-2-[(butanesulfonyl)amino]-4-pentenoate (8.36 g, 20.07 mmol) in MeOH (50 ml) was added HCl-saturated MeOH (50 ml). The resulting suspension was stirred at room temperature for 18 hours, diluted with ether, and filtered. Concentration of the filtrate in vacuo was followed by the addition of ether, a second filtration, and washing of the filtrate with 0.1*M* HCl, sat. NaHCO₃, sat. NaCl. The solution was dried over anhydrous MgSO₄, concentrated and placed under vacuum until constant weight to give 4.49 g (90%) of the desired ester as a light brown oil; ¹H NMR (300 MHz, CDCl₃) δ 5.68 (m, 1H), 5.19 (bd, J = 1.5 Hz, 1H), 5.15 (m, 1H), 4.78 (bd, J = 8.4 Hz, 1H), 4.20 (dt, J = 8.8, 5.8 Hz, 1H), 3.77 (s, 3H), 2.99 (m, 2H), 2.54 (t, J = 6.6 Hz, 2H), 1.76 (m, 2H), 1.42 (sextuplet, J = 7.3 Hz, 2H), 0.93 (t, J = 7.3 Hz, 3H).

### Part C. Methyl erythro- and-threo-3-(3-[4-{(Butyloxycarbonylpiperidin-4-yl)methoxy}phenyl]isoxazolin-5-yl{[butanesulfonyl]amino})propionate

To a solution of 4-[(*N-t*-butyloxycarbonylpiperidin-4-yl)methoxy]benzaldoxime (2.680 g, 8.01 mmol), methyl D,L-2-[(butanesulfonyl)amino]-4-pentenoate (2.000 g, 8.02 mmol) and TEA (0.11 ml, 0.79 mmol) in THF (10 ml) was added a 5% solution of NaOCl (common household bleach, 15 ml, 10.5 mmol). The resulting mixture was rapidly stirred at room temperature for 20 hours. The mixture was diluted with EtOAc and water and the layers were separated. The aqueous portion was washed with EtOAc, and the combined organic fraction washed with sat. NaCl and dried over MgSO₄.Concentration in vacuo afforded a light brown oil (4.8 g), which was purified using flash chromatography (0-50% EtOAc/hexanes in 5 steps), giving four components. The least polar of these materials (fractions 8-11) was determined by ¹H NMR to be the starting olefin (1.520 g, 76%). The next component isolated in order of increasing polarity (fractions 12-15) was determined by ¹H NMR to be the starting oxime (1.423 g, 53%). The next component off of the column (fraction 20) was determined to be the faster of the two diastereomers (317 mg). This material had co-eluted with an impurity having a ¹H NMR profile similar to the starting oxime and appeared to be approximately 50% pure. The most polar component isolated (fractions 22-25) was assigned as the second diastereomer (395 mg, 8%); mp: 127.5-129.3 °C; ¹H NMR (300 MHz, CDCl₃) δ 7.56 (d, J = 8.6 Hz, 2H), 6.87 (d, J = 8.6 Hz, 2H), 5.25 (d, J = 9.5 Hz, 1H), 4.87 (m, 1H), 4.35 (dt, J = 9.2, 3.7 Hz, 1H), 4.15 (bs, 2H), 3.81, (d, J = 6.2 Hz, 2H), 3.78 (s, 3H), 3.49 (dd, J = 16.5, 10.3 Hz, 1H), 3.05 (t, J = 7.7 Hz, 2H), 2.97 (dd, J = 16.5, 7.0 Hz, 1H), 2.73 (bt, J = 12.1 Hz, 2H), 2.21 (m, 1H), 1.94 (m, 2H), 1.82 (m, 4H), 1.45 (s, 9H), 1.24 (m, 3H), 0.92 (t, J = 7.3 Hz, 3H).

### Part D. 3-(3-[4-{(Butyloxycarbonylpiperidin-4-yl)methoxy)phenyl]isoxazolin-5-yl{[butanesulfonyl]amino})-propionic Acid (More Polar Diastereomer)

A solution of methyl 3-(3-[4-{(butyloxycarbonylpiperidin-4-yl)methoxy)phenyl]isoxazolin-5-yl{[butanesulfonyl]amino))propionate more polar diastereomer (200 mg, 0.344 mmol) in THF (1 ml) was saponified using 0.5*M* LiOH (1 ml, 0.5 mmol) over 4 hours as per Example 1, Part F. The crude carboxylic acid was crystallized from EtOAc/hexanes, affording 77 mg (39%) of the desired material; mp: 137.3-139.0 °C; ¹H NMR (300 MHz, CDCl₃) δ 7.55 (d, J = 8.8 Hz, 2H), 6.87 (d, J = 8.8 Hz, 2H), 5.45 (d, J = 9.5 Hz, 1H), 4.92 (m, 1H), 4.37 (m, 1H), 4.15 (b, 2H), 3.81, (d, J = 6.2 Hz, 2H), 3.47 (dd, J = 16.5, 9.9 Hz, 1H), 3.08 (t, J = 8.1 Hz, 2H), 3.01 (dd, J = 16.5, 7.0 Hz, 1H), 2.74 (bt, J = 12.1 Hz, 2H), 2.26 (m, 1H), 2.01 (m, 2H), 1.81 (m, 4H), 1.45 (s, 9H, overlapped with m, 1H), 1.24 (m, 3H), 0.91 (t, J = 7.3 Hz, 3H).

### Part D'. 3-(3-[4-{(Butyloxycarbonylpiperidin-4-yl)methoxy}phenyl]isoxazolin-5-yl{[butanesulfonyl]amino})-propionie Acid (Less Polar Diastereomer)

A solution of the impure methyl 3-(3-[4-{(butyloxycarbonylpiperidin-4-yl)methoxy)phenyl]isoxazolin-5-yl{[butanesulfonyl]amino))propionate less polar diastereomer (309 mg) in THF (5 ml) was saponified using 0.5*M* LiOH (2 ml, 1 mmol) over 6 hours as per Example 1, Part F. The crude carboxylic acid was purified using flash chromatography (CHCl₃ - 5-15% MeOH/CHCl₃ step gradient) followed by crystallization from EtOAc/hexanes, affording 169 mg of the desired material; mp: 155 °C (dec); ¹H NMR (400 MHz, DMSO-d₆, 80 °C) δ 7.56 (d, J = 8.8 Hz, 2H), 6.98 (d, J = 8.8 Hz, 2H), 4.80 (m, 1H), 3.96 (bd, J = 13.2 Hz, 2H), 3.90 (d, J = 6.3 Hz, 2H), 3.77 (bs, 3H), 3.52 (t, J = 7.8 Hz, 1H), 3.38 (dd, J = 14.4, 10.0 Hz, 1H), 2.98 (t, J = 7.8 Hz, 2H), 2.76 (dt, J = 12.2, 1.7 Hz, 2H), 1.95 (m, 2H), 1.75 (m, 4H), 1.41 (s, 9H), 1.38 (d, J = 7.6 Hz, 1H), 1.25 (m, 4H), 0.88 (t, J = 7.3 Hz, 3H).

### Part E. 3-(3-[4-{(Piperidin-4-yl)methoxy}phenyl]isoxazolin-5-yl{[butanesulfonyl]amino})propionic Acid, Trifluoroaretic Acid Salt (More Polar Diastereomer)

3-(3-[4-{(Butyloxycarbonylpiperidin-4-yl)methoxy}phenyl]isoxazolin-5-yl{[butanesulfonyl]amino})propionic acid more polar diastereomer(40 mg, 0.070 mmol) was Boc-deprotected using 33% TFA/DCM according to Example 1, Part G. Recrystallization from methanol then afforded 4 mg (10%) of the TFA salt; mp: 263.5 °C (dec).

### Part E'. 3-(3-[4-{(Piperidin-4-yl)methoxy}phenyl]isoxazolin-5-yl{[butanesulfonyl]amino})propionic Acid, Trifluoroacetic Acid Salt (Less Polar Diastereomer)

3-(3-[4-{(Butyloxycarbonylpiperidin-4-yl)methoxy}phenyl]isoxazolin-5-yl{[butanesulfonyl]amino})propionic acid less polar diastereomer(98 mg, 0.173 mmol) was Boc-deprotected using 33% TFA/DCM according to Example 1, Part G, giving 40 mg of the TFA salt. Recrystallization from methanol then afforded 28 mg (29%) of the pure amino acid; mp: 239.4-240.7 °C.

### Example 43

### 3(R,S)-{5(R,S)-N-[3-(4-Amidinophenyl)isoxazolin-5-ylacetyl]amino}-3-phenylpropanoic Acid

### Part A. 4-Cyanobenzaldoxime

This material was prepared from 4-cyanobenzaldehyde according to Kawase and Kikugawa (J. Chem. Soc., Perkin Trans I 1979, 643).

### Part B. Methyl 3-(3-Butenoyl)amino-3-phenylpropionate

To a solution of vinylacetic acid (861 mg, 10.0 mmol), methyl 3-amino-3-phenylpropionate hydrochloride (2.37 g, 11.0 mmol) and TEA (1,6 ml, 12 mmol) in DCM (20 ml) at -10 °C was added DEC (2.11 g, 11.0 mmol). The resulting mixture was stirred at -10 °C for 15 hours. The mixture was then washed with water, 0.1 *M* HCl, sat. NaHCO₃, sat. NaCl and dried over anhydrous MgSO₄. Concentration *in vacuo* followed by pumping until constant weight was achieved gave 2.36 g (95%) of the desired amide as a golden oil of suitable purity for further reaction; ¹H NMR (300 MHz, CDCl₃) δ 7.28 (m, 5H), 6.78 (bd, J = 7.7 Hz, 1H), 5.95 (m, 1H), 5.43 (dt, J = 8.4, 5.9 Hz, 1H), 5.25 (m, 2H), 3.61 (s, 3H), 3.04 (d, J = 7.0 Hz, 2H), 2.88 (dq, J = 15.0, 5.9 Hz, 2H).

### Part C. Methyl 3(R,S)-{5(R,S)-N-[3-(4-Cyanophenyl)isoxazolin-5-ylacetyl]amino}-3-phenylpropanoate

This material was prepared from methyl 3-(3-Butenoyl)amino-3-phenylpropionate (816 mg, 3.30 mmol) and 4-cyanobenzaldoxime (438 mg, 3.00 mmol) according to Example 4, Part B. The crude product was then purified using flash chromatography (70% EtOAc/hexanes), affording 731 mg (62%) of the desired isoxazolines as a 1:1 mixture of diastereomers; ¹H NMR (300 MHz, CDCl₃) δ 7.74 (m, 8H), 7.29 (m, 10H), 6.92 (bm, 2H), 5.42 (m, 2H), 5.16 (m, 2H), 3.64 (s, 3H), 3.60 (s, 3H), 3.48 (m, 2H), 3.26 (dd, J = 17.3, 7.7 Hz, 1H), 3.15 (dd, J = 16.8, 8.1 Hz, 1H), 2.85 (m, 2H), 2.69 (m, 2H).

### Part D. Methyl 3(R,S)-{5(R,S)-N-[3-(4-Amidinophenyl)isoxazolin-5-ylacetyl]amino}-3-phenylpropanoate

Into a solution of methyl 3(*R,S*)-{5(*R,S*)-N-[3-(4-cyanophenyl)isoxazolin-5-ylacetyl]amino)-3-phenylpropanoate (587 mg, 1.50 mmol) in 10% DCM/methanol (55 ml) was bubbled dry HCl gas for 2 hours. The mixture was stirred for 18 hours, then concentrated *in vacuo.* The crude imidate was dissolved in methanol (20 ml) and ammonium carbonate added. The resulting mixture was stirred for 18 hours, then filtered. The filtrate was concentrated *in vacuo* and the residue purified using flash chromatography (CHCl₃ - 20% methanol/CHCl₃). Concentration of the appropriate fractions *in vacuo* followed by placing the residue under vacuum until constant weight was achieved afforded 193 mg (32%) of the desired amidines; Mass Spectrum (NH₃-DCI, e/z, relative abundance) 409 (M + H)⁺, 100%.

### Part E. 3(R,S)-{5(R,S)-N-[3-(4-Amidinophenyl)isoxazolin-5-ylacetyl]amino}-3-phenylpropanoic Acid, Trifluoroacetic Acid Salt

Methyl 3(*R,S*)-{5(*R,S*)-N-[3-(4-amidinophen-yl)isoxazolin-5-ylacetyl]amino)-3-phenylpropanoate (45 mg, 0.113 mmol) was saponified using 0.5 *M* LiOH (0.6 ml, 0.3 mmol) according to Example 1, Part F, affording 28 mg (49%); Mass Spectrum (NH₃-DCI, e/z, relative abundance) 412 (M + H)⁺, 100%.

### Example 120a

### Methyl 3(R)-{5(R,S)-N-[3-(4-amidinophenyl)isoxazolin-5-ylacetyl]amino}-3-phenethylpropanoate

### Part A. (E)-Methyl-5-phenyl-2-pentenoate

A solution of hydrocinnamaldehyde (13.42 g, 0.1 mol) and methyl(triphenylphosphoranylidene)acetate (33.44 g, 0.1 mol) in THF was stirred at reflux for 20 hours. The reaction mixture was concentrated under vacuum and the residue was purified by flash chromatography using hexane:EtOAc::9:1. The desired product was obtained as a clear, pale yellow oil (8.0 g, 0.042 mol, 42%); ¹H NMR (300 MHz, CDCl₃) δ 7.3-7.2 (m, 2H), 7.2-7.1 (m, 3H), 7.1-6.9 (m, 1H), 5.85 (d, 1H, *J* = 5.8 Hz), 3.75 (s, 3H), 2.8 (t, 2H, J = 7.7 Hz), 2.55 (q, 2H, *J* = 7.4 Hz); MS (NH₃-DCI) 191 (M+H)⁺.

### Part B. Methyl 3-(R)-[N-(1-(R)-1-phenylethyl)amino]-5-phenylpentanoate

A mixture of (*E*)-methyl-5-phenyl-2-pentenoate (5.70 g, 0.03 mol) and R-methylbenzylamine (14.54 g, 0.12 mol) was heated at 110°C over 94 hours. The cooled reaction mixture was purified by flash chromatography using hexane:EtOAc::8:2 to afford 1.18 g (0.0038 mol, 12%) of the desired product as a clear liquid; ¹H NMR (300 MHz, CDCl₃) δ 7.4-7.0 (m, 11H), 3.9 (q, 1H, *J* = 6.5 Hz), 3.65 (s, 3H), 2.9-2.65 (m, 2H), 2.6-2.35 (m, 3H), 1.75-1.6 {m, 2H), 1.35 (d, 3H, *J* = 6.2 Hz); MS (NH₃-DCI) 312 (M+H)⁺.

### Part C. Methyl 3-(R)-amino-5-phenylpentanoate • acetic acid salt

A mixture of methyl 3-(*R*)-[*N*-(1-(*R*)-1-phenylethyl)amino]-5-phenylpentanoate (0.72 g, 2.3 mmol), 20% Pd(OH)2/C (0.38 g), cyclohexene (8.2 ml), glacial HOAc (0.13 ml, 2.3 mmol), and MeOH (15 ml) was heated at reflux under N₂ for 20 hours. After cooling, the catalyst was removed by filtration through a Celite plug, rinsed with MeOH, and the solution concentrated under vacuum. The residue was triturated with hexane to afford 0.46 g (96%) of a white solid, mp = 73-75°C ; ¹H NMR (300 MHz, DMSO) δ 8.3 (bs, 2H), 7.35-7.15 (m, 5H), 3.65 (s, 3H), 3.45-3.35 (m, 1H), 2.8-2.6 (m, 4H), 2.0-1.7 (m, 2H); [α]_{D}²⁵ -12.50° (c=0.0032, MeOH).

### Part D. Methyl 3 (R)-{5(R,S)-N-[3-(4-cyanophenyl)isoxazolin-5-ylacetyl]amino}heptanoate

To a suspension of 3-(4-cyanophenyl)isoxazolin-5-ylacetic acid (460 mg, 2.0 mmol) in EtOAc (15 ml) was added methyl 3-(*R*)-amino-5-phenylpentanoate acetic acid salt (410 mg, 2.0 mmol), TBTU (640 mg, 2.0 mmol), and Et₃N (0.56 ml, 400 mg, 4.0 mmol). After stirring at room temp for 16 hours, the reaction mixture was concentrated under vacuum then purified by flash chromatography using EtOAc to afford 690 mg (83%) of a colorless oil. ¹H NMR (300 MHz, DMSO) δ 8.05 (brs, 1H), 7.95-7.9 (m, 2H), 7.85-7.8 (m, 2H), 7.3-7.25 (m, 2H), 7.2-7.1 (m, 2H), 5.15-5.0 (m, 1H), 4.15-4.0 (m, 1H), 3.6 (d, 3H, *J =* 9.9 Hz), 3.3 (d, 2H, *J* = 6.9 Hz), 3.25-3.15 (m, 1H), 2.75-2.35 (m, 6H), 1.8-1.6 (m, 2H); MS (NH₃-DCI) 420 (M+H)⁺.

### Part E Methyl 3(R)-{5(R,S)-N-[3-(4-amidinophenyl)isoxazolin-5-ylacetyl]amino}-3-phenethylpropanoate

This material was prepared from methyl 3(*R*)-{5(*R,S*)-*N*-[3-(4-cyanophenyl)isoxazolin-5-ylacetyl]amino)-3-phenethylpropanoate (670 mg, 1.6 mmol) according to Example 43, Part D. The crude product was triturated with cold ether to afford 272 mg (39%) of a white solid of the title compound as a 1:1 mixture of diastereomers, mp - 76-78°C; ¹H NMR (300 MHz, DMSO) δ 8.1-8.0 (m, 1H), 8.0-7.8 (m, 4H), 7.95-7.85 (m, 5H), 7.35-7.2 (m, 5H), 5.1-5.0 (m, 1H), 4.1-4.0 (m, 1H), 3.6 (s, 3H), 3.3-3.15 (m, 2H), 2.7-2.4 (m, 6H), 1.8-1.7 (m, 2H), 1.1-1.0 (m, 2H); Mass Spectrum (NH₃-ESI,) 437 (M + H)⁺.

### Example 120b

### Methyl 3(S)-{5(R,S)-N-[3-(4-amidinophenyl}isoxazolin-5-ylacetyl]amino}-3-phenethylpropanoate

### Part A. Methyl 3-(S)-[N-(1-(R)-1-phenylethyl)amino]-5-phenylpentanoate

A mixture of (*E*)-methyl-5-phenyl-2-pentenoate (5.70 g, 0.03 mol) and R-methylbenzylamine (14.54 g, 0.12 mol) was heated at 110°C over 94 hours. The cooled reaction mixture was purified by flash chromatography using hexane:EtOAc::8:2 to afford 1.20 g (0.0039 mol, 13%) of the desired product as a clear liquid; ¹H NMR (300 MHz, CDCl₃) δ 7.35-7.0 (m, 11H), 3.9 (q, 1H, *J* = 6.6 Hz), 3.65 (s, 3H), 2.95-2.8 (m, 1H), 2.75-2.5 (m, 2H), 2.45-2.35 (m, 2H), 1.9-1.65 (m, 2H), 1.3 (d, 3H, *J* = 6.6 Hz); MS (NH₃-DCI) 312 (M+H)⁺.

### Part B. Methyl 3-(S)-amino-5-phenylpentanoate • acetic acid salt

Methyl 3-(*S*)-[*N*-benzyl-*N*-(1-(*R*)-1-phenylethyl)amino]heptanoate (0.93 g, 2.9 mmol), 20% Pd(OH)₂/C (0.47 g), cyclohexene (10.1 ml), glacial HOAc (0.17 ml, 2.9 mmol), and MeOH (20 ml) were heated at reflux under N₂ for 48 hours. After cooling, the catalyst was removed by filtration through a Celite plug, rinsed with MeOH, and the solution concentrated under vacuum. The residue was triturated with hexane to afford 0.65 g (80%) of a white solid, mp = 86-88°C; ¹H NMR (300 MHz, CDCl₃) δ 7.35-7.15 (m, 5H), 5.3 (brs, 2H), 3.65 (s, 3H), 3.35-3.2 (m, 1H), 2.8-2.55 (m, 3H), 2.5-2.4 (m, 1H), 2.0 (s, 3H), 1.8 (q, 2H, *J* = 7.4 Hz); [α]_{D}²⁵ +9.55° (c-0.220, MeOH).

### Part C. Methyl 3(S-{5(R,S)-N-[3-(4-cyanophenyl)isoxazolin-5-ylacetyl]amino}heptanoate

To a suspension of 3-(4-cyanophenyl)isoxazolin-5-ylacetic acid (700 mg, 2.6 mmol) in EtOAc (15 ml) was added methyl 3-(*S*)-amino-5-phenylpentanoate acetic acid salt (600 mg, 2.6 mmol), TBTU (830 mg, 2.6 mmol), and Et₃N (1.09 ml, 790 mg, 7.8 mmol). After stirring at room temp 16 hours, the reaction mixture was concentrated under vacuum then purified by flash chromatography using EtOAc to afford 420 mg (38%) of a colorless oil. ¹H NMR (300 MHz, CDCl₃) δ 8.05-8.0 (m, 1H), 7.95-7.9 (m, 2H), 7.85-7.8 (m, 2H), 7.3-7.2 (m, 2H), 7.2-7.1 (m, 3H), 5.15-5.0 (m, 1H), 4.15-4.0 (m, 1H), 3.6-3.55 (m, 3H), 3.3-3.1 (m, 1H), 2.7-2.4 (m, 6H), 1.8-1.6 (m, 2H); MS (NH₃-DCI) 420 (M+H)⁺.

### Part D. Methyl 3(S)-{5(R,S)-N-[3-(4-amidinophenyl)isoxazolin-5-ylacetyl]amino}-3-phenethylpropanoate

This material was prepared from methyl 3(*S*)-{5(*R*,*S*)-*N*-[3-(4-cyanophenyl)isoxazolin-5-ylacetyl]amino)-3-phenethylpropanoate (360 mg, 0.86 mmol) according to Example 43, Part D. The crude product was triturated with cold ether to afford 230 mg (62%) of an amorphous solid of the title compound as a 1:1 mixture of diastereomers, mp = 84-86°C; ¹H NMR (300 MHz, DMSO) δ 8.1-8.0 (m, 1H), 8.0-7.8 (m, 4H), 7.75-7.7 (m, 1H), 7.3-7.1 (m, 6H), 5.1-5.0 (m, 1H), 4.15-4.0 (m, 1H), 3.65 (s, 3H), 3.3-3.1 (m, 1H), 2.7-2.6 (m, 3H), 2.5-2.4 (m, 3H), 1.8-1.65 (m, 2H), 1.1-1.0 (m, 2H); Mass Spectrum (NH₃-ESI) 437 (M + H)⁺.

### Example 275

### N³-[3-(4-amidinophenyl)isoxazolin-5(R,S)-ylacetyl]-L-2,3-diaminopropinoic acid TFA salt

### Part A. 3-(4-cyanophenyl)isoxazolin-5(R,S)-ylacetic acid.

To a solution of 4-cyanobenzaldoxime (see Ex 43, Part A) (312 g, 2.13 mol) in tetrahydrofuran (3000 ml) at room temperature was added vinyl acetic acid (552g, 6.41 mol). The yellow solution was cooled in an ice bath and sodium hypochlorite solution (5200 ml)was added in a dropwise fashion over 2h. After stirring overnight at room temperature the reaction was quenched with a 5% citric acid solution and diluted with 200ml ether. The layers were separated and the aqueous acidified to pH 4 using citric acid. The acid layer was washed twice with 200 ml ether, the ether layers combined and extracted with saturated sodium bicarbonate solution. After acidifying the basic layer with citric acid, the product was extracted into 400 ml ether. The organic phase was washed three times with 150 ml water, once with brine, dried (MgSO₄) and concentrated to give 220g of 3-(4-cyanophenyl)isoxazolin-5-ylacetic acid as a white solid. Recrystallization from 25% water/ethanol yielded 165g of analytically pure material. Anal. Calcd for C₁₂H₁₀N₂O₃: C,62.61; H,4.38; N, 12.17. Found: C, 62.37; H 4.47; N, 11.71, ¹H NMR(300MHz, CDCl₃): δ 7.77-7.76 (d, 2H, J=1.8Hz); 7.72-7.71 (d, 2H, J=1.8Hz); 5.22-5.14 (m, 1H); 3.63-3.54 (dd, 1H, J=10.6Hz, 16.8Hz); 3.19-3.11 (dd, 1H, J=7.3Hz, 16.8Hz); 3.00-2.93 (dd, 1H, J=6.2Hz, 16.5Hz); 2.79-2.72 (dd, 1H, J=7.3Hz, 16.5Hz). IR(KBr pellet): 3202, 2244, 1736, 1610, 1432, 1416, 1194, 1152, 928, 840, 562 cm⁻¹.

### Part B. Methyl N²-Cbz-L-2,3-diaminopropionate HCl salt.

N₂-Cbz-*L*-2,3-diaminopropionic acid (10 mmol, 2.39 g) was dissolved in 20 ml methanol and 20 ml, 4 N HCl in dioxane and the solution was stirred for 4 hours and then concentrated to give a solid. The solid was washed with ether several times to give 2.50 g (87%) product. NMR (DMSO-d₆): δ 8.38 (b, 3H); 7.96 (d, 1H); 7.38 (m, 5H); 5.05 (s, 2H); 4.44 (m, 1H); 3.66 (s, 3H); 3.14 (m, 2H).

### Part C. Methyl N²-Cbz-N³-[3-(4-cyanophenyl)isoxazolin-5(R,S)-ylacetyl]-L-2,3-diaminopropionate.

To a solution of 3-(4-cyanophenyl)isoxazolin-5(*R, S*)-ylacetic acid. (19 mmol, 4.37 g), methyl N²-Cbz-*L*-2,3-diaminopropionate HCl salt (20 mmol, 5.76 g) and triethylamine (60 mmol, 8.36 ml) was added TBTU (20 mmol, 6.42 g) and the solution was stirred for 2 hours. Ethyl acetate was added and the solution was washed with dilute citric acid, brine, NaHCO₃ and brine, dried (MgSO₄), and concentrated. Crystallization from ethyl acetate/ether gave 6.85 g (78%) product. NMR (DMSO-d₆) : δ 8.16 (t, 1H); 7.92 (d, 2H); 7.82 (d, 2H); 7.68 (d, 1H); 7.36 (m, 5H); 5.04 (m, 3H); 4.20 (m, 1H); 3.64 (s, 3H); 3.50 (m, 2H); 3.26 (m, 2H);2.50 (m, 2H).

### Part D. Methyl N³-[3-(4-amidinophenyl)isoxazolin-5(R, S)-ylacetyl]-L-2,3-diaminopropionate HCl salt.

HCl gas was bubbled into a solution of methyl N²-Cbz-N³-[3-(4-cyanophenyl)isoxazolin-5(*R,S*)-ylacetyl]-*L*-2,3-diaminopropionate (2.1 mmol, 1.0 g) for 1 hour and the solution was stirred overnight and concentrated. The residue was dissolved in 30 ml 2 M ammonia in methanol and the solution was stirred overnight, and concentrated to give 1.2 g crude product.

### E. N³-[3-(4-amidinophenyl)isoxazolin-5(R,S)-ylacetyl]-L-2,3-diaminopropionic acid TFA salt.

Methyl N³-[3-(4-amidinophenyl)isoxazclin-5(*R,S*)-ylacetyl]-*L*-2,3-diaminopropionate HCl salt (200 mg) was saponified with 1 ml methanol and 1 ml 1 N NaOH for 1 hour and acidified with acetic acid. Purification on reversed phase HPLC gave 40 mg product. ESI (M+H)⁺: Calcd 334.2; Found 334.2.

### Example 276

### N²-Cbz-N3-[3-(4-amidinophenyl)isoxazolin-5(R,S)-ylacetyl]-L-2,3-diaminopropionic acid TFA salt

### Part A. Methyl N²-Cbz-N³-[3-(4-amidinophenyl)isoxazolin-5(R,S)-ylacetyl]-L-2,3-diaminopropionate TFA salt.

To a solution of the compound of Ex. 275, part E (1.0 mmol, 385 mg) and sodium bicarbonate (5.0 mmol, 400 mg) in 2 ml water, 2 ml acetonitrile and 1 ml DMF was added benzyl chloroformate (1 mmol, 143 µl) and the mixture was stirred for 2 hours at room temperature. The solution was filtered, acidified with TFA and purified on reversed phase HPLC to give 150 mg (25%) product. NMR (DMSO-d₆): δ 9.40 (s, 2H); 9.20 (s, 2H); 8.18 (t, 1H); 7.86 (m, 4H); 7.68 (d, 1H); 7.35 (m, 5H); 5.02 (m, 3H); 4.20 (m, 1H); 3.64 (s, 3H); 3.52 (m, 2H); 3.26 (m, 2H); 2.50 (m, 2H).

### Part B. N²-Cbz-N³-[3-(4-amidinophenyl)isoxazolin-5(R, S)-ylacetyl]-L-2,3-diaminopropionic acid TFA salt .

Methyl N²-Cbz-N³-[3-(4-amidinophenyl)isoxazolin-5(*R, S*)-ylacetyl]-*L*-2,3-diaminopropionate TFA salt ( 0.12 mmol, 70 mg) was dissolved in 2 ml methanol and 1 ml 1 N NaOH and after 1 hour, the solution was acidified with acetic acid. Purification on reversed phase HPLC gave 50 mg (74%) product. ESI (M+H)⁺: Calcd 468.2; Found 468.2.

### Example 278

### N²-n-butyloxycarbonyl-N³-[3-(4-amidinophenyl)isoxazolin-5(R,S)-ylacetyl]-L-2,3-diaminopropionic acid TFA salt

### Part A. Methyl N2-n-butyloxycarbonyl-N3-[3-(4-amidinophenyl)isoxazolin-5(R,S)-ylacetyl]-L-2,3-diaminopropionate TFA salt.

To a solution of the compound of Ex. 275, part E (1.0 mmol, 385 mg) and sodium bicarbonate (2.5 mmol, 200 mg) in 2 ml water, 2 ml acetonitrile and 1 ml DMF cooled in an ice bath was added n-butyl chloroformate (1 mmol, 127 µl). After stirring for 1 hour, the solution was acidified with acetic acid and purified on reversed phase HPLC to give 150 mg (27%) product. NMR (DMSO-d₆): δ 9.40 (s, 2H); 9.20 (s, 2H); 8.16 (t, 1H); 7.86 (m, 4H); 7.47 (d, 1H); 5.02 (m, 1H); 4.16 (m, 1H); 3.94 (t, 2H); 3.62 (s, 3H); 3.50 (m, 2H); 3.26 (m, 2H); 2.50 (m, 2H); 1.52 (m, 2H); 1.32 (m, 2H); 0.88 (t, 3H). ESI (M+H)⁺: Calcd 448.3; Found 448.3.

### Part B. N2-n-butyloxycarbonyl-N3-[3-(4-amidinophenyl)isoxazolin-5(R, S)-ylacetyl]-(S)-2,3-diaminopropionic acid TFA salt.

Methyl N2-n-butyloxycarbonyl-N3-[3-(4-amidinophenyl)isoxazolin-5(*R,S*)-ylacetyl)-(S)-2,3-diaminopropionate TFA salt (0.107 mmol, 60 mg) was dissolved in 2 ml methanol and 2 ml 1 N NaOH and after 1 hour, the solution was acidified with acetic acid. Purification on reversed phase HPLC gave 53 mg (89%) product. ESI (M+H)⁺: Calcd 434.3; Found 434.3.

### Example 314A

### Methyl N²-n-butyloxycarbonyl-N³-[3-(4-amidinophenyl)isoxazolin-5(S)-ylacetyl]-(S)-2,3-diaminopropionate TFA salt

### Part A: Methyl N²-Cbz-N³-Boc-L-2,3-diaminopropionate.

To a solution of methyl N²-Cbz-(S)-2,3-diaminopropionate HCl salt (16.3 mmol, 4.7 g) and di-tert-butyl dicarbonate (16.3 mmol, 3.56 g) in 30 ml chloroform cooled in an ice bath was added triethylamine (34 mmol, 4.7 ml) and the solution was stirred in the ice bath for 1 hour and at room temperature for 3 hours and concentrated. The residue was taken up in ethyl acetate and the solution was washed with dilute citric acid, brine, NaHCO₃ and brine, dried (MgSO₄), and concentrated. Crystallization from ether/petroleum ether gave 5.2 g (92%) product. NMR (DMSO-d₆): δ 7.60 (d, 1H); 7.35 (m, 5H); 6.88 (t, 1H); 5.02 (s, 2H); 4.14 (m, 1H); 3.60 (s, 3H); 3.28 (m, 2H); 1.37 (s, 9H).

### Part B: Methyl N³-Boc-(S)-2,3-diaminopropionate HCO₂H salt.

A mixture of methyl N²-Cbz-N³-Boc-(S)-2,3-diaminopropionate. (14 mmol, 5.0 g), formic acid (42 mmol, 1.6 ml) and 10% Pd/C (500 mg) in 40 ml methanol was stirred at room temperature for 1 hour and filtered through a celite. The filtrate was concentrated and the residue was triturated with ether-petroleum ether to give 3.7 g (100%) solid product. NMR (DMSO-d₆): δ 8.20(s, 1H); 6.90 (t, 1H); 5.36 (b, 3H); 3.61 9s, 3H); 3.51 (t, 1H); 3.18 (t, 2H); 1.38 (s, 9H).

### Part C: Methyl N²-n-butyloxycarbonyl-N³-Boc-(S)-2,3-diaminopropionate.

To a mixture of methyl N₃-Boc-(S)-2,3-diaminopropionate HCO₂H salt (14 mmol, 3.7 g) and NaHCO₃ (40 mmol, 3.4 g) in 10 ml water and 10 ml THF cooled in an ice bath was added slowly butyl chloroformate (16 mmol, 2 ml) over 15 min. After stirring for 1 hour, ethyl acetate was added and the solution was washed with dilute citric acid, brine, NaHCO₃ and brine, dried (MgSO₄), and concentrated to give 4.4 g (100%) oily product. NMR (DMSO-d₆): δ 7.37 (d, 1H); 6.84 (t, 1H); 4.10 (m, 1H); 3.96 (t, 2H); 3.60 (s, 3H); 3.26 (m, 2H); 1.52 (m, 2H); 1.38 (s, 9H); 1.36 (m, 2H); 0.88 (t, 3H).

### Part D: Methyl N²-butyloxycarbonyl-(S)-2,3-diaminopropionate TFA salt.

Methyl N²-n-butyloxycarbonyl-N³-Boc-(S)-2,3-diaminopropionate (13.9 mmol, 4.4 g) was dissolved in 25 ml methylene chloride and 35 ml TFA and after 1 hour, the solution was concentrated to give an oily product. Yield 4.8 g (100%). NMR (DMSO-d₆): δ 8.02 (b, 3H); 7.68 (d, 2H); 4.38 (m, 1H); 3.99 (t, 2H); 3.68 (s, 3H); 3.22 (m, 1H); 3.06 (m, 1H); 1.55 (m, 2H); 1.34 (m, 2H); 0.89 (t, 3H).

### Part E: Methyl -N²-n-butyloxycarbonyl-N³-[3-(4-cyanophenyl)isoxazolin-5(S)-ylacetyl]-(S)-2,3-diaminopropionate

To a solution of 3-(4-cyanophenyl)isoxazolin-5(S)-ylacetic acid (5.2 mmol, 1.2 g) [Chiral starting material was prepared from the racemic compound of Ex. 275, Part A by resolution on a 50 X 2 cm Chiralpak AD column using 0.1% TFA/EtOH at 10° C to give isomer A (faster eluting) and isomer B (slower eluting). Alternately, the isomers were resolved by crystallization of the chinconidine salt of the 5-S isomer of the isoxazolines from acetone, leaving the 5(R) isomer in the mother liquor. The absolute stereochemistry of the crystalline salt was determined by X-ray crystallography to be the 5(S) isoxazoline.] and methyl N²-butyloxycarbonyl-(S)-2,3-diaminopropionate TFA salt (6 mmol, 1.53 g) in 20 ml DMF cooled in an ice bath was added diisopropylethylamine (20 mmol, 3.5 ml) followed by BOP (5.5 mmol, 2.43 g). After stirring at room temperature for 3 hours, ethyl acetate was added and the solution was washed with 0.5 N HCl, brine, NaHCO₃ and brine, dried (MgSO₄), and concentrated to give 1.9 g (87%) product. NMR IDMSO-d₆): δ 8.12 (t, 1H); 7.94 (d, 2H); 7.83 (d, 2H); 7.46 (d, 1H); 5.04 (m, 1H); 4.16 (m, 1H); 3.96 (t, 2H); 3.64 (s, 3H); 3.58 (dd, 1H); 3.40 (m, 2H); 3.20 (dd, 1H); 2.56 (dd, 1H); 2.43 (dd, 1H); 1.52 (m, 2H); 1.32 (m, 2H); 0.88 (t, 3H).

### Part F: Methyl-N²-n-butyloxycarbonyl-N³-[3-(4-amidinophenyl)isoxazolin-5(S)-ylacetyl]-(S)-2,3-diaminopropionate TFA salt.

To a solution of methyl-N²-n-butyloxycarbonyl-N³-[3-(4-cyanophenyl)isoxazolin-5(S)-ylacetyl]-(S)-2,3-diaminopropionate (4.4 mmol, 1.9 g) in 50 ml methanol was bubbled with HCl gas at 0°C for 1 hour and the solution was stirred at room temperature for 5 hours and concentrated. The residue was taken up in 20 ml methanol and ammonium carbonate (11 mmol, 1.1 g) was added. The mixture was stirred at room temperature overnight and concentrated. The solid was dissolved in methanol/water/TFA and purification on reversed phase HPLC gave 1.0 g (40%) product. ESI (M+H)⁺: Calcd 448.3; Found 448.3.

### Example 314B

### Methyl-N²-n-butyloxycarbonyl-N³-[3-(4-amidinophenyl)isoxazolin-5(R)-ylacetyl]-(S)-2,3-diaminopropionate TFA salt

### Part A: 3-(4-cyanophenyl)isoxazolin-5(R)-ylacetic acid

This material was resolved from 3-(4-cyanophenyl)isoxazolin-5(*R,S*)-ylacetic acid as described above in the proceudure for Example 314A, Part E.

### Part B: Methyl-N²-n-butyloxycarbonyl-N³-[3-(4-cyanophenyl)isoxazolin-5(R)-ylacetyl]-(S)-2,3-diaminopropionate.

This material was synthesized from 3-(4-cyanophenyl)-5(R)-ylacetic acid (4.3 mmol, 1.0 g), Methyl N²-butyloxycarbonyl-(S)-2,3-diaminopropionate TFA salt (5 mmol, 1.27 g), BOP (4.5 mmol, 2 g) and diisopropylethylamine (16 mmol, 2.8 ml) using the same procedure as for Example 314A, Part E. Yield 1.75 g (95%). NMR (DMSO-d₆): δ 8.12 (t, 1H); 7.94 (d, 2H); 7.83 (d, 2H); 7.46 (d, 1H); 5.04 (m, 1H); 4.16 (m, 1H); 3.96 (t, 2H); 3.64 (s, 3H); 3.58 (dd, 1H); 3.40 (m, 2H); 3.20 (dd, 1H); 2.56 (dd, 1H); 2.43 (dd, 1H); 1.52 (m, 2H); 1.32 (m, 2H); 0.88 (t, 3H).

### Part C: Methyl-N²-n-butyloxycarbonyl-N³-[3-(4-amidinophenyl)isoxazolin-5(R)-ylacetyl]-(S)-2,3-diaminopropionate TFA salt.

This compound was synthesized from Methyl-N²-n-butyloxycarbonyl-N³-[3-(4-cyanophenyl)isoxazolin-5(R)-ylacetyl]-(S)-2,3-diaminopropionate (4.0 mmol, 1.7 g) using the same procedure as for Example 314A, Part G. Yield 1.0 g (45%). ESI (M+H)⁺: Calcd 448.3; Found 448.3.

### Example 344

### Methyl 3(R)-{5(R,S)-N-[3-(4-Amidinophenyl)isoxazolin-5-ylacetyl]amino)heptanoate

### Part A. (E)-Methyl 2-heptenoate

To a solution of diethyl methylphosphonoacetate (19 ml, 104 mmol) in dry THF (800 ml) at -4 °C was added 64 ml of n-BuLi (1.6 M in hexane, 102 mmol) dropwise over 45 min. The resulting solution was stirred 1 h at room temp. Valeraldehyle (10.0 ml, 94 mmol) was added and stirred 3.5 h at room temp. The reaction was quenched with 25 ml sat. NH₄Cl. Solvents were distilled at atmospheric pressure, and the resulting solids were taken up in EtOAc, extracted with water and brine, and dried with Na₂SO₄. The solvents were again distilled at atmospheric pressure, and the resulting yellow liquid was distilled under house vacuum to yield 7.2 g clear liquid, boiling range under house vacuum 90-125 °C; HRMS, e/z Calc. for (M+H)⁺: 143.1072. Found: 143.1070; IR(film) 1728, 1658 cm⁻¹.

### Part B. N-(1-(R)-1-Phenylethyl)benzamide

A solution of benzoyl chloride (22.5 ml, 0.19 mole) in dichloromethane (10 ml) was added dropwise over 1.5 h to a 0 °C solution of (*R*)-(+)-α-methylbenzylamine (25 ml, 0.19 mole), triethylamine (31 ml, 0.22 mole), and 4-DMAP (100 mg), in dichloromethane (1 1). After 1.75 h at 0 °C the mixture was concentrated *in vacuo,* then diluted with EtOAc. This mixture was extracted with water, 1 *M* HCl, water, and brine, then dried (MgSO₄) and concentrated to yield 43.4 g of a colorless crystalline solid; mp 121.0-121.5 °C; IR(KBr) 3332, 1636 cm⁻¹; [α]_{D}²⁵ -2.30° (c=1.002, CH₂Cl₂); Anal. Calc. for C₁₅H₁₅NO: C, 79.97; H, 6.71; N, 6.22. Found: C, 79.88; H, 6.65; N, 6.17.

### Part C. N-(1-(R)-1-Phenylethyl)-N-benzylamine

BH₃/THF (1 *M* in THF, 220 ml, 220 mmol) was added dropwise over 1 h to a 0 °C solution of the above benzamide (20 g, 89 mmol) in dry THF (200 ml). The ice bath was removed, and the mixture was heated to reflux for 40 h. A TLC analysis indicated incomplete reaction, so more BH₃/THF (1 *M* in THF, 30 ml, 30 mmol) was added, and heating resumed for 22.5 h. After cooling, MeOH (250 ml) was added dropwise cautiously over 5 h. The resulting mixture was boiled for 2 h, then cooled and concentrated *in vacuo*. Reconcentration from MeOH (2 x 500 ml) and drying under high vacuum gave 19.3 g of an oil containing a small amount of a precipitate. This crude product was stirred with hot 2 *M* HCl (140 ml) to generate a clear solution, then slowly cooled to RT, and ultimately in an ice bath to yield a crystalline solid, as described by Simpkins (Tetrahedron **1990**, *46*(2), 523). The solid was collected by filtration and rinsed with a small amount of water. After air drying for 3 d, 16.35 g of the hydrochloride salt was obtained; mp 178.5-179.5 °C; [α]_{D}²¹ +18.9° (c=4.0, EtOH). The salt was converted to the free base by extraction with Et₂O and aq. KOH, then Kugelrohr distilled, oven temp. 120-140 °C (147 Pa (1.1 mm Hg)) to give 12.5 g of an oil; [α]_{D}²¹ +61.2° (c=3.98, EtOH); Anal. Calc. for C₁₅H₁₇N: C, 85.26; H, 8.11; N, 6.63. Found: C, 84.93; H, 7.75; N, 6.58.

### Part D. Methyl 3-(R)-[N-benzyl-N-(1-(R)-1-phenylethyl)amino]heptanoate

Following the asymmetric Michael addition method of Davies (*Tetrahedron:Asymmetry* **1991**, 2(3), 183), *n*-butyllithium (1.6 *M* in hexanes, 4.4 ml, 7.0 mmol) was added dropwise over 3 min to a 0 °C solution of *N*-(1-(*R*)-1-phenylethyl)-*N*-benzylamine (1.5 g, 7.0 mmol) in dry THF (35 ml), After 30 min, the resulting dark pinkish-red solution was cooled to -78 °C, and a solution of methyl 2-heptenoate (0.50 g, 3.5 mmol) *in* THF (10 ml) was added dropwise over 10 min. After 13 min, the cold reaction was quenched with saturated NH₄Cl (7 ml). After warming to RT, the mixture was extracted with Et₂O and brine, dried (MgSO₄), and concentrated in *vacuo.* The product was purified by chromatography over silica gel, eluting with 0% to 50% EtOAc in hexane. The cleanest major product fractions (apart from a few mixed fractions) were concentrated *in vacuo* to give 0.91 g of a pale yellow oil which by NMR is a single diastereomer, with the newly generated asymmetric center assigned as 3(*R*) by analogy with the Davies reference above; ¹³C NMR (300 MHz, CDCl₃) δ 173.31, 143.40, 141.78, 128.40, 128.27, 128.11, 128.00, 126.91, 126.67, 57.90, 54.22, 51.32, 50.05, 36.83, 33.28, 29.32, 22.72, 19.40, 14.12; [α]_{D}²⁵ +12.96° (c=0.602, MeOH).

### Part E. Methyl 3-(R)-aminoheptanoate • acetic acid salt

Methyl 3-(*R*)-[*N*-benzyl-*N*-(1-(*R*)-1-phenylethyl)amino]heptanoate (0.70 g, 2.0 mmol), 20% Pd(OH)₂/C (0.35 g), cyclohexene (7 ml), glacial HOAc (0.12 ml, 2.1 mmol), and MeOH (14 ml) were heated at reflux under N₂ for 20.5 h. After cooling, the catalyst was removed by filtration thru a Celite plug, rinsed with MeOH, and the solution concentrated *in vacuo.* Drying overnight under high vacuum yielded 0.43 g of a viscous oil; ¹³C NMR (300 MHz, CDCl₃) δ 177.64, 171.52, 51.97, 48.22, 37.24, 33.08, 27.50, 23.31, 22.29, 13.76; [α]_{D}²⁵ -10.6° (c=0.602, MeOH).

### Part F. Methyl 3(R)-{5(R,S)-N-[3-(4-cyanopheny])isoxazolin-5-ylacetyl]amino}heptanoate

To a suspension of 3-(4-cyanophenyl)isoxazolin-5-ylacetic acid (300 mg, 1.3 mmol) in EtOAc (10 ml) was added methyl 3-(*R*)-aminoheptanoate acetic acid salt (287 mg, 1.3 mmol), TBTU (420 mg, 1.3 mmol), and Et₃N (600 µl, 4.3 mmol). After stirring at room temp 2.5 h, the reaction mixture was extracted with 5% KHSO₄, sat NaHCO₃, and brine, then dried with Na₂SO₄. Evaporation, followed by chromatography over silica gel in 50-100% EtOAc/hexanes yielded 245 mg colorless glass. MS (NH₃-DCI) Calc. for (M+H)⁺: 372, (M+NH₄)⁺: 389. Found: 372, 389.

### Part G. Methyl 3(R)-{5(R,S)-N-[3-(4-Amidinophenyl)isoxazolin-5-ylacetyl]amino}heptanoate

To a solution of methyl 3 (*R*)-{5(*R,S*)-*N*-[3-(4-cyanophenyl)isoxazolin-5-ylacetyl]amino)heptanoate (179 mg, .48 mmol) in 15 ml dry MeOH at 0 °C, was added a stream of HCl gas generated from dropping two 20 ml portions of H₂SO₄ into solid NaCl over 35 min. After stirring 20 h at room temp, the solvent was removed with a rapid stream of N₂. Et₂O was added and removed with a rapid stream of N2. The resulting gummy oil was taken up in 15 ml dry MeOH, to which was added (NH₄)₂CO₃ (1.1g, 11.4 mmol). After stirring 19.5 h at room temp, the solvent was removed with a rapid stream of N₂, and the resulting white solid was purified by chromatography over silica gel, eluting with 0-20% MeOH/CHCl₃. Purified product was taken up in 5% MeOH/CHCl₃ and filtered. Concentration of the filtrate yielded 100 mg white solid. IR(KBr) 3600-2800, 1734, 1676, 1640 cm⁻¹; HRMS, e/z Calc. for (M+H)⁺: 389.2189. Found: 389.2192.

### Example 348

### Ethyl 3(R)-{5(R,S)-N-[3-(4-amidinophenyl)isoxazolin-5-ylacetyl]amino)-5-methylhexanoate • trifluoroacetic acid salt

### Part A. (E)-Ethyl 5-methyl-2-hexenoate

Prepared in analogous fashion to methyl 2-heptenoate, using triethyl phosphonoacetate, stirring 17 h at room temp upon addition of isovaleraldehyde. Distillation under house vacuum yielded 72% clear oil, boiling range under house vacuum 80-130 °C; IR(film) 1724, 1656 cm⁻¹.

### Part B. Ethyl 3-(R)-[N-benzyl-N-(1-(R)-1-phenylethyl)amino]-5-methylhexanoate

Prepared in analogous fashion via the asymmetric Michael addition of Ex. 344, part D above. Yield a viscous pale yellow oil (65%); ¹³C NMR (300 MHz, CDCl₃) δ 172.83, 143.56, 142.17, 128.27, 128.21, 128.15, 128.03, 126.96, 126.60, 60.10, 58.56, 52.43, 50.09, 43.23, 36.72, 24.76, 23.48, 22.13, 20.20, 14.21; [α]_{D}²⁵ +5.12° (c=0.606, EtOH).

### Part C. Ethyl 3-(R)-amino-5-methylhexanoate • acetic acid salt

Prepared as previously described except EtOH was used as solvent. Yield a waxy solid (94%); mp 57-61 °C; HRMS, e/z Calc. for (M+H)⁺: 174.1494. Found: 174.1485.

### Part D. Ethyl 3-(R)-amino-5-methylhexanoate • hydrochloric acid salt

The above acetic acid salt (1.1 g, 4.7 mmol) was stirred 4 min in 4 *M* HCl/dioxane (5.0 ml). The resulting solution was triturated with Et₂O, cooled, and the clear liquid decanted, leaving an orange oil which solidified to 960 mg waxy solid on high vacuum; ¹H NMR (300 MHz, CDCl₃) ∂ 8.49 (br, 3H), 4.20 (q, J = 7.3, 2H), 3.70-3.65 (m, 1H), 2.86-2.80 (m, 2H), 1.83-1.80 (m, 2H), 1.58-1.54 (m, 1H), 1.30-1.26 (t, J = 7.3, 3H), 0.99-0.91 (m, 6H).

### Part E. Ethyl 3(R)-{5(R,S)-N-[3-(4-(N-t-butoxycarbonylamidino)phenyl)isoxazolin-5-ylacetyl]amino}-5-methylhexanoate

To a suspension of 3-[4-(*N-t*-butoxycarbonylamidino)phenyl]isoxazolin-5-ylacetic acid (78 mg, 0.22 mmol) in EtOAc (5 ml) was added ethyl 3-(*R*)-amino-5-methylhexanoate hydrochloride salt (47 mg, 0.22 mmol), TBTU (72 mg, 0.22 mmol), and Et₃N (100 µl, 0.72 mmol). After stirring 6 h at room temp, the reaction mixture was extracted with pH 4 buffer (potassium hydrogen phthalate), sat NaHCO₃, and brine, then dried with Na₂SO₄. Evaporation, followed by chromatography over silica gel in 100% EtOAc yielded 33 mg colorless glass; ¹H NMR (300 MHz, CDCl₃) ∂ 7.90 (d, J = 8.4, 2H), 7.70 (dd, J = 8.5, J' = 1.9, 2H), 6.32-6.28 (m, 1H), 5.13-5.11 (m, 1H), 4.34-4.33 (m, 1H), 4.17-4.09 (m, 2H), 3.56-3.47 (m, 1H), 3.25-3.17 (m, 1H), 2.71-2.46 (m, 4H), 1.66-1.47 (m, 2H), 1.56 (s, 9H), 1.31-1.23 (m, 4H), 0.92 (dd, J = 6.6, J' = 1.8, 3H), 0.84 (d, J = 6.6, 3H).

### Part F. Ethyl 3(R)-{5(R,S)-N-[3-(4-amidinophenyl)isoxazolin-5-ylacetyl]amino}-5-methyl hexanoate • trifluoroacetic acid salt

The product from Part E above (29 mg, 0.058 mmol) was dissolved in DCM (300 µl), to which was added TFA (100 µl). The resulting solution was stirred at room temp under a CaSO₄ drying tube for 3.5 h, and triturated with Et₂O. 24 mg white solid were collected by filtration; ¹H NMR (300 MHz, CDCl₃) ∂ 9.4 (br, 1H), 9.0 (br, 1H), 7.8 (s, 4H), 5.0 (m, 1H), 4.2 (m, 1H), 4.0 (q, 2H), 3.6 (m, 1H), 3.3 (m, 2H), 2.4 (m, 3H), 1.6 (m, 1H), 1.4 (m, 1H), 1.2 (m, 4H), 0.8 (m, 6H); HRMS, e/z Calc. for (M+H)⁺: 403.2345. Found: 403.2363.

### Example 350

### Methyl 3(R,S)-{5(R,S)-N-[3-(4-amidinophenyl)isoxazolin-5-ylacetyl]amino}-4-(phenylthio)butanoate • hydrochloric acid salt:

### Part A. Methyl phenylthioacetoacetate

To a solution of thiophenol (5.00 ml, 48.6 mmol) in DMF (20 ml), K₂CO₃ (10.09 g, 73 mmol) and methyl chloroacetoacetate (5.93ml, 48.6 mmol) were added. The reaction mixture was stirred 6 h at 50 °C, diluted with EtOAc, and extracted with saturated Na₂SO₄, water, and brine, then dried (Na₂SO₄) and concentrated. The resulting oil was chromatographed with 20% EtOAc in Hexane to yield 9.40 g yellow oil; MS (CH4-DCI) Calc. for (M+H)⁺: 224. Found: 224; IR(KBr) 2954,1656,1438, 626 cm⁻¹.

### Part B. Methyl-3(R,S)-amino-4-phenylthiobutanoate

To a solution of methyl phenylthioacetoacetate (1.00 g, 4.5 mmol) in MeOH (20 ml), ammonium formate (4.26 g, 6.75 mmol) and sodium cyanoborohydride (0.42 g, 6.7 mmol) were added. The reaction mixture was stirred at room temperature for 18 h, then diluted with EtOAc and partitioned into 1 *M* HCl. The aqueous layer was then basified to pH = 8.0 with NaOH. The desired product was extracted out with EtOAc, washed with water and brine, dried over Na₂SO₄ and concentrated to yield 0.61 g yellow oil; MS (NH3-CI/DDIP) Calc. for (M+H)⁺: 226. Found: 226; ¹H NMR (300 MHz, CDCl₃) δ 7.39 (d, J = 7, 2H), 7.32-7.26 (m, 3H), 7.22 (d, J = 10, 1H), 3.74 (s, 3H), 3.39-3.31 (m, 1H), 3.13-3.07 (dd, J = 13, J' = 9, 1H), 2.91-2.83 (dd, J = 12, J' = 6, 1H), 2.65-2.58 (dd, J = 12, J' = 6, 1H), 2.46-2.38 (dd, J = 16, J' = 8, 1H).

### Part C. Methyl-3(R,S)-{5(R,S)-N-[3-(4-cyanophenyl) isoxazolin-5-ylacetyl]amino}-4-(phenylthio)butanoate

To a suspension of 3-(4-cyanophenyl)isoxazolin-5-ylacetic acid (0.50 g, 2 mmol) in EtOAc (10 ml), methyl-3(*R,S*)amino-4-(phenylthio)butanoate (0.51 g, 2 mmol), TBTU (0.71 g, 2 mmol), and Et₃N (1.24 ml, 8.9 mmol) were added. The reaction mixture was stirred 2 h at room temperature, diluted with EtOAc, washed with 5% citric acid, saturated NaHCO₃, and brine, dried over Na₂SO₄, concentrated, and the resulting oil was chromatographed over silica gel in 100% EtOAc to yield 0.61 g of a yellow glass: MS (NH₃-CI/DDIP) Calc. for (M+H)⁺: 438.1. Found: 438.1; Anal. Calc. for C₃₂H₂₃N₃O₄S₁: C, 63.31; H, 5.30; N, 9.60; S, 7.33. Found: C, 62.99; H, 5.22; N, 9.53; S, 7.30.

### Part D. Methyl 3(R,S)-{5(R,S)-N-[3-(4-amidinophenyl)isoxazolin-5-ylacetyl]amino)-4-(phenylthio)butanoate • hydrochloric acid salt

The product from Part C above (0.30 g,0.68 mmol) was dissolved in dry MeOH (20 ml) at 0 °C. To the resulting solution, HCl gas was bubbled in from a generator as described in Example 344, Part G, over a period of 2 h. The generator was removed and the reaction mixture stirred at 0 °C for 18 h, then concentrated and triturated with CHCl₃. The resulting precipitate was collected by filtration and redissolved in dry MeOH (20 ml). To this solution, ammonium carbonate (0.99 g, 10 mmol) was added and the mixture stirred at room temperature for 18 h. The solution was concentrated and recrystallized from DCM/MeOH to yield 0.14 g white solid; HRMS, e/z Calc. for (M+H)⁺: 455.1753. Found: 455.175; ¹H NMR (300 MHz, *d*₆-DMSO) δ 9.44 (br s,1H), 9.18 (br s, 1H), 8.22 (d, J = 10, 1H), 7.86 (m, 4H), 7.41-7.25 (m, 4H), 7.2 (m, 1H), 5.03 (m, 1H), 4.2 (m, 1H), 3.59 (s, 3H), 3.29-3.05 (m, 4H), 2.8-2.39 (m, 4H).

### Example 359

### Methyl 3(R,S)-{5(R,S)-N-[3-(4-amidinophenyl)isoxazolin-5-ylacetyl]amino}-4-(phenylsulfonamido)butanoate • trifluoroacetic acid salt;

### Part A. Methyl 3-(R,S)-hydroxy-4-aminobutanoate • hydrochloric acid salt

Chlorotrimethylsilane (100 ml, 0.79 mol) was added dropwise over 1.5 h to a stirred 0 °C suspension of 4amino-3-(*R,S*)-hydroxybutyric acid (25 g, 0.21 mol) in MeOH (1 1). The resulting clear solution was allowed to slowly warm to room temperature overnight. The solvent was evaporated *in vacuo,* and the resulting residue was reconcentrated from more MeOH (2 x 500 ml). Drying under high vacuum produced 37 g of a viscous oil; ¹³C NMR (300 MHz, d₆-DMSO) δ 171.42, 90.14, 64.67, 51.89, 44.39; Anal. Calc. for C₅H₁₆ClNO₃: C, 35.41; H, 7.13; N, 8.26; Cl, 20.90. Found: C, 35.18; H, 7.09; N, 8.18; Cl, 20.77.

### Part B. Methyl 3-(R,S)-hydroxy-4-(phenylsulfonamido)butanoate

A solution of benzenesulfonyl chloride (7.5 ml, 59 mmol) in dichloromethane (10 ml) was added dropwise over 55 min to a 0 °C solution of the Part A amine salt (10 g, 50 mmol), and Et₃N (17 ml, 120 mmol) in dichloromethane (110 ml). The mixture was allowed to slowly warm to room temperature, and stirring was continued over the weekend. After solvent removal *in vacuo*, the mixture was diluted with EtOAc and extracted with H₂O, 0.1 *M* HCl, and brine. Drying (MgSO₄) and solvent removal *in vacuo* yielded 14.6 g of a viscous oil; ¹³C NMR (300 MHz, CDCl₃) δ 172.67, 139.79, 132.78, 129.22, 127.02, 66.77, 52.01, 47.72, 38.31; Anal. Calc. for C₁₁H₁₅NO₅S: C, 48.34; H, 5.53; N, 5.13; S, 11.73. Found: C, 48.44; H, 5.61; N, 4.90; S, 11.34.

### Part C. Methyl 3-oxo-4-(phenylsulfonamido)butanoate

The Part B alcohol (2.8 g, 10 mmol) was oxidized with Jones reagent under standard conditions. The ketone was purified by chromatography on silica gel, eluting with 0% to 100% EtOAc in hexane, to yield 1.11 g of a waxy solid; mp 94.5-95.5 °C; ¹³C NMR (300 MHz, CDCl₃) δ 197.08, 166.80, 139.17, 133.08, 129.29, 127.17, 52.71, 51.91, 46.15; Anal. Calc. for C₁₁H₁₃NO₅S: C, 48.70; H, 4.83; N, 5.16; S, 11.82. Found: C, 48.77; H, 4.69; N, 5.08; S, 11.88.

### Part D. Methyl 3-(R,S)-3-amino-4-(phenylsulfonamido)butanoate

To a room temperature solution of the Part C ketone (0.71 g, 2.6 mmol) in MeOH (7 ml) and THF (3 ml) was added ammonium formate (2.5 g, 39 mmol) and sodium cyanoborohydride (0.25 g, 3.9 mmol). After 45.5 h, solvent was evaporated, and the residue was diluted with EtOAc (70 ml). This solution was extracted with 1.0 M NaOH, H₂O, and brine. After concentration, the product was purified by chromatography on silica gel, eluting with 0% to 100% EtOAc in hexane, then 1% to 20% MeOH in EtOAc to yield 0.16 g of a viscous oil, which eventually solidified; ¹H NMR (300 MHz, CDCl₃) ∂ 9.79 (br, 2H), 7.84 (d, 2H, J = 8 Hz), 7.81 (br, 1H), 7.68-7.53 (m, 3H), 4.05-3.92 (m, 1H), 3.75 (s, 3H), 3.33-3.17 (m, 2H), 2.89-2.72 (m, 2H); HRMS, e/z Calc. for (M+H)⁺: 273.0909. Found: 273.0916.

### Part E. Methyl 3-(R,S)-{5(R,S)-N-[3-(4-(N-t-butoxycarbonylamidino) phenyl)isoxazolin-5-ylacetyl]amino}-4-(phenylsulfonamido)butanoate

This compound was prepared analogous to Example 348, Part E, stirring 24 h in 5 ml EtOAc and 1 ml DMF. Chromatography in 5% MeOH/CHCl₃ yielded 80% of an orange solid; IR(KBr) 3296, 2338, 1736, 1660, 1618 cm⁻¹; HRMS, e/z Calc. for (M+H)⁺: 602.2285. Found: 602.2270.

### Part F. Methyl 3(R,S)-{5(R,S)-N-[3-(4-amidinophenyl)isoxazolin-5-ylacetyl]amino}-4-(phenylsulfonamido)butanoate • trifluoroacetic acid salt

The product from Part E was deprotected analogously to Example 348, Part F, yielding 86% pink solid; IR(KBr) 3312, 3104, 1734, 1670; HRMS, e/z Calc. for (M+H)⁺: 502.1760. Found: 502.1761. The more active diastereomer (based on PRP assay) was isolated from the above mixture by SFC HPLC, Chiralpak AD - 2X25 cm, eluted with 0.1% TFA/25% MeOH/75% CO₂. Under these conditions, the more active diastereomer eluted last.

### Example 362

### Methyl 3(R,S)-{5(R,S)-N-[3-(4-amidinophenyl)isoxazolin-5-ylacetyl]amino}-4-(n-butylsulfonamido)butanoate • trifluoroacetic acid salt;

### Part A. Methyl 3-(R,S)-hydroxy-4-(n-butylsulfonamido)butanoate

This compound was prepared entirely analogously to Ex.359, Part B, using *n*-butylsulfonylchloride instead. A colorless, waxy solid of excellent purity was obtained in 65% yield without purification; mp 46-50 °C; ¹³C NMR (300 MHz, CDCl₃) δ 172.64, 67.29, 52.56, 51.99, 47.83, 38.40, 25.57, 21.52, 13.55; Anal. Calc. for C₉H₁₉NO₅S: C, 42.67; H, 7.56; N, 5.53; S, 12.66. Found: C, 42.69; H, 7.59; N, 5.36; S, 12.78.

### Part B. Methyl 3-oxo-4-(n-butylsulfonamido)butanoate

The immediately preceeding alcohol was oxidized as described for Example 359, Part C, to give a 57% yield of a colorless solid; mp 53-55 °C; Anal. Calc. for C₉H₁₇NO₅S: C, 43.02; H, 6.82; N, 5.57; S, 12.76. Found: C, 42.68; H, 7.03; N, 5.74; S, 13.06.

### Part C. Methyl 3(R,S)-3-amino-4-(n-butylsulfonamido)butanoate

This compound was prepared analogous to Example 350, Part B, using the product from Part B above (1.20 g, 4.8 mmol) yielding 0.26 g yellow oil; 1H NMR (300 MHz, CDCl₃) δ 3.70 (s, 3H), 3.38 (m, 1H), 3.24-3.13 (m, 1H), 3.02 (m, 4H), 2.58-2.52 (dd, J = 16, J' = 11, 1H), 1.79 (m, 2H), 1.24 (m, 2H), 0.95 (t, 3H); MS (NH4-DCI) Calc. for (M+H)⁺: 271. Found: 271.

### Part D. Methyl-3(R,S)-{5(R,S)-N-[3-(4-(N-t-butoxycarbonylamidine) phenyl)isoxazolin-5-ylacetyl]amino}-4-(n-butylsulfonylamido)butanoate

To a solution 3-[4-(*N*-*t*-butoxycarbonylamidine)phenyl]isoxazolin-5-ylacetic acid (0.24 g, 0.83 mmol) in DMF (20 ml), the product from Part C above (0.29 gr, 0.83 mmol), TBTU (0.27 g, 0.83 mmol), and Et₃N (0.46 ml, 3.3 mmol) was added. After stirring 4 h at room temperature, the reaction mixture was diluted with EtOAc, extracted with pH 4 buffer (potassium hydrogen phthalate), saturated NaHCO₃, brine, then dried (NaSO₄). Concentration, followed by chromatography over silica gel in 100% EtOAc, yielded 1.17 g of a white foam; MS (NH3-DCI) Calc. for (M+H)⁺: 582.3. Found: 582; IR(KBr) 3312, 2338, 1620, 1144 cm⁻¹.

### Part E. Methyl 3(R,S)-{5(R,S)-N-[3-(4-amidinophenyl)isoxazolin-5-ylacetyl]amino}-4-(n-butylsulfonylamido)butanoate • trifluoroacetic acid

To a solution of the product from Part D above (0.22 g, 0.37 mmol) in DCM (10 ml), trifluoroacetic acid (2.2 ml) was added. The reaction mixture was stirred 2 h at room temperature, triturated with Et₂O, and the resulting precipitate was chromatographed over silica gel in 20% MeOH in CHCl₃ to yield 0.20 g white solid; HRMS, e/z Calc. for (M+H)⁺: 482.2073. Found: 482.2090; mp = 178-184 °C.

### Example 365

### Methyl {5(R,S)-N-[3-(4-amidinophenyl)isoxazolin-5-ylacetyl]amino}-4-(methoxycarbonyl)butanoate • trifluoroacetic acid salt;

### Part A. Dimethyl 3-aminoglutarate • hydrochloric acid salt

This product was prepared similarly to Example 359, Part A, from β-glutamic acid to yield the diester as a colorless gum in quantitative yield; HRMS, e/z Calc. for (M+H)⁺: 176.0923. Found: 176.0933.

### Part B. Methyl {5(R,S)-N-[3-(4-(N-t-butoxycarbonylamidino) phenyl)isoxazolin-5-ylacetyl]amino}-4-(methoxycarbonyl)butanoate

Prepared analogous to Example 359, Part E, to yield 32% of a white solid; IR(KBr) 3306, 2338, 1738, 1656, 1620 cm⁻¹; HRMS, e/z Calc. for (M+H)⁺: 505.2298. Found: 505.2283.

### Part C. Methyl {5(R,S)-N-[3-(4-amidinophenyl)isoxazolin-5-ylacetyl]amino}-4-(methoxycarbonyl)butanoate • trifluoroacetic acid salt

Prepared analogous to Example 348, Part F, yielding 83% white solid; IR(KBr) 3316, 3102, 2340, 1736, 1670 cm⁻¹; HRMS, e/z Calc. for (M+H)⁺: 405.1774. Found: 405.1775.

### Example 368

### Methyl 3(R,S)-{5(R,S)-N-[3-(4-amidinophenyl)isoxazolin-5-ylacetyl]amino}-4-(methoxycarbonyl)pentanoate • trifluoroacetic acid salt;

### Part A. Dimethyl 3-(R,S)-aminoadipate • hydrochloric acid salt

This product was prepared as in Example 359, Part A, from β-aminoadipic acid to yield a colorless gum in quantitative yield; HRMS, e/z Calc. for (M+H)⁺: 190.1079. Found: 190.1080.

### Part B. Methyl-3(R,S)-{5(R,S)-N-[3-(4-(N-t-butoxycarbonylamidine) phenyl)isoxzalin-5-ylacetyl]amino}-4-(methoxycarbonyl) pentanoate

This product was prepared similarly as in Example 362, Part D, using the product from Part B above (0.70 g, 3.1 mmol) instead to yield 1.17 g of a white foam; HRMS, e/z Calc. for (M+H)⁺: 519.2454. Found: 519.2459; Anal. Calc. for C₂₅H₃₄N₄O₈: C, 57.90; H, 6.61; N,10.80. Found: C, 57.73; H, 6.51; N, 10.86.

### Part C. Methyl-3(R,S)-{5(R,S)-N-[3-(4-amidinophenyl)isoxazolin-5-ylacetyl]amine}-4-(methoxyacarbonyl)pentanoate • trifluoroacetic acid salt

This product was prepared as in Example 362, Part E, using the product from Part C above (1.00 g, 1.9 mmol) to yield 0.9 g white solid; HRMS, e/z Calc. for (M+H)⁺: 419.1930. Found: 419.1921; mp = 214-215 °C (decomposes).

### Examples 473A and 473B

### Resolution of Methyl N²-3-methylphenylsulfonyl-N³-[3-(4-amidinophenyl)-5S-ylacetyl]-S-2,3-diaminopropionate trifluoroacetic and Methyl N²-3-methylphenylsulfonyl-N³-[3-(4-amidinophenyl)-5R-ylacetyl]-S-2,3-diaminopropionate hydrochloride

The mixture was initially purified on a Pirkle DNBPG column using 10%HOAc/20%EtOH/70% hexane as the eluting solvent. The column temperature was maintained at 45°C, the flow rate at 1.5ml/min, and the detector set at 280nm. The diastereomers were then separated on a chiralcel OD-25 X 2cm column using an eluting solvent of 0.1%TFA/20%MeOH/80%CO₂. The column temperature was maintained at 30°C, the flow rate at 13ml/min, the pressure at 175 atm, and the detector was set at 280nm. Injections were made on 23mg of sample. Over the two columns a total of 300mg was injected giving 59mg of the R isomer, Ex. 473A (HRMS calc'd for C₂₃H₂₇N₅O₆S 502.176031 Found: 502.175508) and 85mg of the S isomer, Ex. 473B (HRMS calc'd for C₂₃H₂₇N₅O₆S 502.176031 Found: 502.176358).

### Example 473C

### N²-3-methylphenylsulfonyl-N³-[3-(4-amidinophenyl)-5S-ylacetyl]-S-2,3-diaminopropionic acid

### Part A: Methyl-N²-3-methylphenylsulfonyl-N³-[3-(4-cyanophenyl)-5S-ylacetyl]-S-2,3-diaminopropionate

Into a solution of 3-(4-cyanophenyl)isoxazolin-5-S-ylacetic acid (1.82g, 7.90mmol, obtained as described in Es. 314A, part F) in DMF (50ml) was added methyl-N²-3-methylphenylsulfonyl-L-2,3-diaminopropionate HCl salt (2.77g, 7.90mmol), TBTU (2.53g, 7.90mmol), and Hünigs base (2.75ml, 15.8mmol). After stirring at room temperature for 16 hours, the reaction mixture was diluted with EtOAc (500ml) and washed one time with water (200ml), one time with sat'd NaHCO₃ (200ml), one time with 0.1N HCl (200ml), dried (MgSO₄), filtered, and concentrated. Column chromatography on silica gel using 10% EtOAc/hexane as the eluting solvent gave 1.99g (52%) of the desired material as an off-white foam. ¹H NMR: (CDCl₃): δ 7.81-7.78 (d, 2H, J=8.4Hz); 7.16-7.67 (d, 2H, J=8.8Hz); 7.61-7.58 (m, 2H); 7.39-7.37 (d, 2H, J=5.1Hz); 6.35-6.30 (m, 1H); 5.54-5.52 (d, 1H, J=7.7Hz); 5.18-5.17 (m, 1H); 4.00-3.96 (m, 1H); 3.62-3.50 (m, 3H); 3.57 (s, 3H); 3.27-3.19 (dd, 1H, J-7.7, 17.0Hz); 2.78-2.70 (dd, 1H, J=5.9,14.8Hz); 2.64-2.57 (dd, 1H, J=6.6, 14.6Hz); 2.42 (s, 3H).

### Part B: Methyl-N²-3-methylphenylsulfonyl-N³-[3-(4-amidinophenyl)-5S-ylacetyl]-S-2,3-diaminopropionate hydrochloride

Methyl-N²-3-methylphenylsulfonyl-N³-[3-(4-cyanophenyl)-5S-ylacetyl]-S-2,3-diaminopropionate was dissolved in 100ml absolute ethanol at 0°c and a stream of HCl gas was bubbled through the solution for two hours. The reaction vessel was sealed and after sitting at room temperature for 16 hours the volatiles were removed *in vacuo.* The residue was then diluted with 100ml of absolute ethanol, ammonium carbonate (9.6g, 0.123mol) was added and after stirring for 16 hours the reaction mixture was filtered and concentrated *in vacuo.* Column chromatography on silica using a gradient elution from 5%MeOH/CH₂Cl₂ to 20%MeOH/CH₂Cl₂ gave 0.762g (37%) of the desired amidine as a white solid. ¹H NMR (CDCl₃) : δ 8.23-8.20 (m, 1H); 7.91-7.85 (m, 4H); 7.57-7.54 (m, 2H); 7.49-7.46 (m, 2H); 5.00-4.94 (m, 1H); 4.08-3.86 (m, 1H); 3.59-3.49 (m, 1H); 3.39 (s, 3H); 3.38-3.29 (m, 3H); 2.49 (s, 3H); 2.50-2.45 (m, 2H). HRMS: calc'd for C₂₃H₂₇N₅O₆S 502.176031 found 502.175992. [α]_{D} = +48.88° (c=0.180, MeOH).

### Part C: N²-3-methylphenylsulfonyl-N³-[3-(4-amidinophenyl)5(S)-yl]acetyl-S-2,3-diaminopropionic acid

The compound of Ex 473C, part B (0.077 g., 0.14 mmol) was dissolved in MeOH (4ml). To the resulting solution was added a solution of lithium hydroxide (0.0066 g., 0.158 mmol) in water (4 ml) and the mixture was stirred overnight at room temperature. The methanol was removed by evaporation in vacuo, and the product precipitated from the aqueous as a white solid (0.026 g., 35%). HRMS calcd for C₂₂H₂₅N₅O₆S: 488.160381; found: 488.160827.

### Example 473D

### Methyl-N²-3-methylphenylsulfonyl-N³-[3-(4-amidinophenyl)-5R-ylacetyl]-S-2,3-diaminopropionate hydrochloride

### Part A: Methyl-N²-3-methylphenylsulfonyl-N³-[3-(4-cyanophenyl)-5R-ylacetyl]-S-2,3-diaminopropionate

This compound was synthesized from 3-(4-cyanophenyl)isoxazolin-5-(R)-ylacetic acid (3.07g, 0.011mol, obtained as described in Ex. 314B, part B) using the same procedure as for example 473C, part A. Yield 41%. Theory: C 57.02, H 4.99, N 11.56 Found: C 56.83, H 4.87, N 11.45.

### Part B: Methyl-N²-3-methylphenylsulfonyl-N³-[3-(4-amidinophenyl)-5R-ylacetyl]-S-2,3-diaminopropionate hydrochloride

This compound was synthesized from Methyl-N²-3-methylphenylsulfonyl-N³-[3-(4-cyanophenyl)-5R-ylacetyl]-S-2,3-diaminopropionate using the same procedure as for example 473C, part B. Yield 49%. HRMS Calc'd for C₂₃H₂₇N₅O₆S 502.176031 Found: 502.174103.

### Example 496

### Methyl N²-(2,2-diphenyl-1-ethenesulfonyl)-N³-[3-(4-amidinophenyl)isoxazolin-5-(R,S)-ylacetyl]-(S)-2,3-diaminopropionate, trifluoroacetic acid salt

### Part A: Methyl N²-(2,2-diphenyl-1-ethenesulfonyl)-N³-Boc-(S)-2,3-diaminopropionate.

To a mixture of methyl N³-Boc-(S)-2,3-diaminopropionate (255 mg, 1.17 mmol) and 2,2-diphenylethylenesulfonyl chloride (Hasegawa and Hirooka, J. Chem. Soc. Japan 48,1513-1518 (1975); 391 mg, 1.40 mmol) in methylene chloride (10 ml) cooled in an ice bath was added triethylamine (0.25 ml, 1.76 mmol). After 22 h, the mixture was concentrated and flash chromatographed (6:4 toluene/ethyl acetate) to provide 240 mg (46%) of product. NMR (CDCl₃) δ 7.42-7.20 (10H), 6.81 (s,1H), 5.24 (bd,1H), 4.87 (bs,1H), 3.95 (q,1H), 3.72 (s,3H), 3.50-3.42 (2H), 1.44 (s,9H); mass spec (NH₃-CI) m/z 466.54 (M+NH4⁺, 100%).

### Part B: Methyl N²-(2,2-diphenyl-1-ethenesulfonyl)-(S)-2,3-diaminopropionate TFA salt.

The product of Part A (210 mg, 0.468 mmol) was dissolved in 5 ml of methylene chloride and 3 ml TFA. After 1 hour, the solution was concentrated to give an oily product. (222 mg, 100%). NMR (DMSO-d₆) δ 8.02 (bs, 3H),7.40 (m, 5H),7.23 (m, 4H), 7.00 (s, 1H), 4.26 (m, 1H), 3.71 (s, 3H), 3.20 (m, 1H), 2.98 (m, 1H).

### Part C: Methyl N²-(2,2-diphenyl-1-ethenesulfonyl)-N³-[3-(4-N-Boc-amidinophenyl)isoxazolin-5-(R,S)-ylacetyl]-(S)-2,3-diaminopropionate.

The product of part B (220 mg, 0.46 mmol) was reacted with 3-(4-N-Boc-amidinophenyl)-isoxazolin-5-ylacetic acid ( 160 mg, 0.46 mmol) to provide the title product (215 mg, 68%). NMR (CDCl₃) δ 7.84 (m,2H), 7.64 (m,2H), 7.40-7.18 (10H), 6.75 (s,1H), 6.30 (m,1H), 5.30 (m,1H), 5.04 (m,1H), 4.00 (1H), 3.78 (s,3H), 3.62-3.40 (4H), 3.10 (m,1H), 2.70-2.50 (2H), 2.04 (s,1H), 1.58 (s,9H); mass spec (ESI) m/z 690.2 (M+H⁺, 100%).

### Part D: Methyl N²-(2,2-diphenyl-1-ethenesulfonyl)-N³-[3-(4-amidinophenyl)isoxazolin-5-(R,S)-ylacetyl]-(S)-2,3-diaminopropionate, trifluoroacetic acid salt

The product of part C (210 mg, 0.30 mmol) was dissolved in methylene chloride (3 ml) and treated with trifluoroacetic acid (1 ml) to provide the title product (150 mg, 80%). NMR (DMSO-d₆) δ 9.39 (bs,2H), 9.05 (bs,2H), 8.22 (m,1H), 8.00 (m,1H), 7.85 (s,4H), 7.40 (m,6H), 7.20 (m,4H), 6.89 (s,1H), 5.00 (m,1H), 4.00 (m,1H), 3.70-3.18 (5H), 3.62 (2s,3H); mass spec (ESI) m/z 590.2 (M+H⁺, 100%)

### Example 511.

### Methyl N²-(N,N-dimethylsulfamoyl)-N³-[3-(4-amidinophenyl) isoxazolin-5-(R,S)-ylacetyl]-(S)-2,3-diaminopropionate, trifluoroacetic acid salt

### Part A: Methyl N²-(N,N-dimethyl sulfamoyl)-N³-Boc-(S)-2,3-diaminopropionate.

To a mixture of methyl N³-Boc-(S)-2,3-diaminopropionate (400 mg, 1.80 mmol) and Dimethylsulfamoyl chloride (0.24 ml, 2.20 mmol) in methylene chloride (10 ml) cooled in an ice bath was added triethylamine (0.38 ml, 2.20 mmol). After 18 h, the mixture was concentrated and flash chromatographed (6:4 toluene/ethyl acetate) to provide 283 mg (49%) of product. NMR (CDCl₃) δ 5.23 (bd,1H), 4.90(m,1H), 4.06 (m,1H), 3.80 (s,3H), 3.52 (bt,2H), 2.80 (s,6H), 1.42 (s,9H); mass spec (NH₃-CI) m/z 343.0 (M+NH₄⁺, 100%).

### Part B: Methyl N²-(N,N-dimethyl sulfamoyl)-(S)-2,3-diaminopropionate TFA salt.

The Product of Part A was dissolved in 5 ml of methylene chloride and 3 ml TFA. After 1 hour, the solution was concentrated to give an oily product (294 mg, 100%). NMR (DMSO-d₆) δ 6.52 (bs,2H), 4.4-3.9 (2H), 3.8 (bs,3H), 2.93 (bs,6H).

### Part C: Methyl N²-(N,N-dimethyl sulfamoyl)-N³-[3-(4-N-Boc-amidinophenyl)isoxazolin-5-(R,S)-ylacetyl]-L-2,3-diaminopropionate.

The product of part B (200 mg, 0.61 mmol) was reacted with 3-(4-N-Boc-amidinophenyl)isoxazolin-5-ylacetic acid (212 mg, 0.61 mmol) to provide the title product (203 mg, 61%). NMR (CDCl₃) δ 7.78 (m,2H), 7.42 (bt,2H), 7.00 (m,1H), 5.92 (m,1H), 5.04 (m,1H), 3.80 (2s,3H), 3.64 (m,2H), 3.40 (m,1H), 3.05 (m,1H), 2.80 (2s,6H), 2.74 (m,1H), 2.60 (m,1H), 2.02 (s,3H), 1.60 (s,9H); mass spec (ESI) m/z 555.1 (M+H⁺, 100%).

### Part D: Methyl N²-(N,N-dimethyl sulfamoyl)-N³-[3-(4-amidinophenyl)isoxazolin-5-(R,S)-ylacetyl]-L-2,3-diaminopropionate, trifluoroacetic acid salt

The product of part C (183 mg, 0.329 mmol) was dissolved in methylene chloride (3 ml) and treated with trifluoroacetic acid (1 ml) to provide the title product (159 mg, 85%). NMR (DMSO-d₆) δ 9.40 (bs,2H), 9.00 (bs,2H), 8.22 (m,lH), 7.82 (s,4H), 5.00 (m,lH), 3.95 (m,lH), 3.68 (2s,3H), 3.60 (m,2H), 3.20 (m,4H), 2.80 (s,6H); mass spec (ESI) m/z 455.1 (M+H⁺, 100%).

### Example 512

### Methyl N²-(m-toluenesulfonyl)-N³-[3-(4-amidino-2-fluorophenyl)isoxazolin-5-ylacetyl]-S-2,3-diaminopropionate hydrochloric acid salt

### Part A: 3-Fluoro-4-methylbenzamide

3-Fluoro-4-methylbenzoic acid (10 g, 65 mmol) was boiled in thionyl chloride (100 ml) under a drying tube for 2.5 h. The excess SOCl₂ was removed by distillation. The oily acid chloride product was diluted with CH₂Cl₂ (100 ml) and cooled in an ice bath. Conc. aq. NH₃ (20 ml) was added dropwise, and stirring continued at 0 °C for 0.5 h. The CH₂Cl₂ was removed in *vacuo,* then the residue was diluted with EtOAc. The mixture was extracted with sat. aq. Na₂CO₃ (2x), H₂O, and brine, dried (MgSO₄), and concentrated to yield 9.9 g of a pale yellow solid; mp 161-163 °C; IR(KBr) 3382, 1654 cm⁻¹; Anal. Calc. for C₈H₈FNO: C, 62.74; H, 5.27; N, 9.15; F, 12.40. Found: C, 62.66; H, 5.17; N, 9.12; F, 12.28.

### Part B: 3-Fluoro-4-methylbenzonitrile

A solution of trichloroacetyl chloride (7.3 ml, 65 mmol) in CH₂Cl₂ (20 ml) was added dropwise over 0.5 h to a solution/suspension of the Part A amide (9.0 g, 59 mmol) and Et₃N (17 ml, 120 mmol) in CH₂Cl₂ (80 ml) at 0 °C. After 40 min, the mixture was concentrated *in vacuo,* then diluted with Et₂O. This solution was extracted with 1 M HCl, sat. aq. NaHCO₃, H₂O, and brine, then dried (MgSO₄), and concentrated to yield 7.8 g of a tan solid; mp 45-47 °C; IR(KBr) 2232 cm⁻¹; HRMS, e/z Calc. for (M+H)⁺: 135.0484. Found: 135.0482.

### Part C: 2-Fluoro-4-cyanobenzylbromide

N-Bromosuccinimide (9.6 g, 54 mmol) and the part B substrate (7.3 g, 54 mmol) were heated under reflux in CCl₄ (100 ml) under N₂ with irradiation with a high intensity visible lamp for 2 h. After cooling to ambient temp., the mixture was filtered through a Celite pad and concentrated *in vacuo*. The crude product was recrystallized from hot cyclohexane (4x) to yield 4.5 g of off-white needles; mp 75-77 °C; IR(KBr) 2236 cm⁻¹; HRMS, e/z Calc. for (M+H)⁺: 213.9668. Found: 213.9660.

### Part D: 2-Fluoro-4-cyanobenzaldehyde

The part C benzyl bromide (3.68 g, 17 mmol), trimethylamine N-oxide dihydrate (7.6 g, 68 mmol), CH₂Cl₂ (15 ml), and DMSO (30 ml) were stirred at 0 °C for a few h, slowly warming to ambient T overnight. The mixture was diluted with water (30 ml) and brine (30 ml), and extracted with Et₂O (4x). The combined organics were washed with brine, dried (MgSO₄), and concentrated to yield 1.1 g of a yellow solid; IR(KBr) 2238, 1706 cm⁻¹; HRMS, e/z Calc. for (M+H)⁺: 150.0355. Found: 150.0341.

### Part E: 2-Fluoro-4-cyanobenzaldoxime

The part D aldehyde (1.1 g, 7.4 mmol), hydroxylamine hydrochloride (1.0 g, 15 mmol), K₂CO₃ (1.0 g, 7.4 mmol), water (1 ml), and MeOH (10 ml) were heated under reflux for 2.25 h. After brief cooling, the mixture was diluted with water, and the insoluble product was collected by filtration, then rinsed with more water. Drying under high vacuum provided 0.94 g of a pale yellow amorphous solid; mp 179-182 °C; IR(KBr) 3256, 2236, 1556 cm⁻¹; HRMS, e/z Calc. for (M+H)⁺: 165.0464. Found: 165.0455.

### Part F: Methyl 3-(4-cyano-2-fluorophenyl)isoxazolin-5-ylacetate

The part E oxime was allowed to react with Clorox and methyl vinylacetate in the usual way to afford the isoxazoline as a yellow solid in 32% yield; mp 92-94 °C; IR(KBr) 2240, 1746 cm⁻¹; HRMS, e/z Calc. for (M+H)⁺: 263.0832. Found: 263.0818. Anal. Calc. for C₁₃H₁₁FN₂O₃: C, 59.54; H, 4.23; N, 10.68; F, 7.24. Found: C, 59.84; H, 4.31; N, 10.53; F, 7.26.

### Part G: Methyl N²-(m-toluenesulfonyl)-N³-[3-(4-amidino-2-fluorophenyl) isoxazolin-5-ylacetyl]-S-2,3-diaminopropionate hydrochloric acid salt

The part F intermediate was converted to the title compound via the usual sequence of steps: Pinner amidine synthesis, amidine BOC protection, ester saponification, condensation with the 2,3-diaminopropionate sulfonamide ester, and BOC deprotection to provide a yellow gum; HRMS, e/z Calc. for (M+H)⁺: 520.1666. Found: 520.1675.

### Example 513

### Methyl N²-(n-butyloxycarbonyl)-N³-[3-(3-amidinopyrid-6-yl) isoxazolin-5-ylacetyl]-S-2,3-diaminopropionate bis hydrochloric acid salt

Prepared using methods described in Ex. 514 to provide a pale yellow powder; mp 90-110 °C (dec); HRMS, e/z Calc. for (M+H)⁺: 449.2149. Found: 449.2140.

### Example 514

### Methyl N²-(m-toluenesulfonyl)-N³-[3-(3-amidinopyrid-6-yl)isoxazolin-5-ylacetyl]-S-2,3-diaminopropionate bis hydrochloric acid salt

### Part A: 3-cyano-6-pyridaldoxime

5-Cyano-2-picoline (25 g, 0.21 mol) and I₂ were heated under reflux in DMSO (200 ml) for 1 h. After cooling to RT, hydroxylamine hydrochloride (16 g, 0.23 mol), K₂CO₃ (29 g, 0.21 mol), and water (21 ml) were added. The resulting mixture was heated to 80 °C for 2.5 h, cooled, diluted with water (100 ml) and much acetone, and absorbed onto silica gel by concentration. Chromatography on silica gel, eluting with 0% to 50% EtOAc in hexane, afforded 12.2 g of a tan solid; mp 204-207 °C (dec); HRMS, e/z Calc. for (M+H)⁺: 148.0511. Found: 148.0516.

### Part B: Methyl 3-(3-cyanopyrid-6-yl)isoxazolin-5-ylacetate

The oxime of Ex. 514, part A was converted to the isoxazoline as described in Ex. 516, part B in 76% yield as a yellow solid; mp 97-98 °C; HRMS, e/z Calc. for (M+H)⁺: 246.0879. Found: 246.0881. Anal. Calc. for C₁₂H₁₁N₃O₃: C, 58.77; H, 4.52; N, 17.13. Found: C, 58.74; H, 4.51; N, 17.11.

### Part C: Methyl 3-(3-t-butyloxycarbonylamidinopyrid-6-yl)isoxazolin-5-ylacetate

The nitrile of Ex. 514, part B was converted to the amidine as described in the method of Ex. 516, parts D & E (except that 0.6 eq. NaOMe was required), and BOC protected in standard fashion to afford, after purification, a yellow solid; mp 143 °C (gas evolves); HRMS, e/z Calc. for (M+H)⁺: 363.1668. Found: 363.1675. Anal. Calc. for C₁₇H₂₂N₄O₅: C, 56.35; H, 6.12; N, 15.46. Found: C, 56.35; H, 6.10; N, 15.39.

### Part D: Lithium 3-(3-t-butyloxycarbonylamidinopyrid-6-yl) isoxazolin-5-ylacetate

The ester of Ex. 514, part C was saponified and lyophilized as described in the method of Ex. 516, part F to give a colorless amorphous solid quantitatively; mp >230 °C; HRMS, e/z Calc. for conjugate acid (M+H)⁺: 349.1512. Found: 349.1527.

### Part E: Methyl N²-(m-toluenesulfonyl)-N³-[3-(3-amidinopyrid-6-yl) isoxazolin-5-ylacetyl]-S-2,3-diaminopropionate bis hydrochloric acid salt

The part D lithium carboxylate was converted to the title compound by treatment with HCl in MeOH to provide a yellow solid; mp 90 °C (dec); HRMS, e/z Calc. for (M+H)⁺: 503.1713. Found: 507.1718.

### Example 515

### Methyl N²-(n-butyloxycarbonyl)-N³-[3-(2-amidinopyrid-5-yl) isoxazolin-5-ylacetyl]-S-2,3-diaminopropionate bis hydrochloric acid salt

In similar fashion to the method described in Ex. 516, the compound of Ex. 514, part E was coupled with methyl N²-(*n*-butyloxycarbonyl)-2,3-diaminopropionate hydrochloride using conditions described above, followed by BOC deprotection with 4 M HCl/dioxane to yield a pale yellow powder; HRMS, e/z Calc. for (M+H)⁺: 449.2149. Found: 449.2154.

### Example 516

### Methyl N²-(m-toluenesulfonyl)-N³-[3-(2-amidinopyrid-5-yl)isoxazolin-5-ylacetyl]-S-2,3-diaminopropionate bis hydrochloric acid salt

### Part A: 2-Chloro-5-pyridaldoxime

2-Chloro-5-formylpyridine (2.1 g, 15 mmol) was condensed with hydroxylamine hydrochloride in the usual way to give the oxime, 1.5 g, as a yellow crystalline solid; mp 171-175 °C (dec); HRMS, e/z Calc. for (M+H)⁺: 157.0169. Found: 157.0175.

### Part B: Methyl 3-(2-chloropyrid-5-yl)isoxazolin-5-ylacetate

Clorox (20 ml) was added dropwise over 1.75 h to a mixture of the part A oxime (1.13 g, 7.2 mmol), methyl vinylacetate (70% purity, 3.0 g, 21 mmol), CH₂Cl₂ (40 ml), and DMF (4 ml) with stirring at ambient temperature. The CH₂Cl₂ was evaporated, and the mixture was diluted with EtOAc, extracted with water (5x) and brine, then dried (MgSO₄), filtered, and concentrated. Chromatography on silica gel, eluting with 0% to 70% EtOAc in hexane, afforded 1.4 g of a solid; mp 94-96 °C; HRMS, e/z Calc. for (M+H)⁺: 255.0536. Found: 255.0531.

### Part C: Methyl 3-(2-cyanopyrid-5-yl)isoxazolin-5-ylacetate

The part B chloropyridine (0.51 g, 2.0 mmol), zinc cyanide (0.23 g, 2.0 mmol), Pd(PPh₃)₄ (0.12 g, 0.10 mmol), and DMF (2 ml) were heated to 80 °C under N₂ for 3 days. After cooling and concentration, the mixture was preabsorbed onto silica gel by concentration from CHCl₃. Chromatography on silica gel, eluting with 0% to 90% EtOAc in hexane afforded 0.28 g of a pale yellow solid; mp 115-116 °C; HRMS, e/z Calc. for (M+H)⁺: 246.0879. Found: 246.0880. Anal. Calc. for C₁₂H₁₁N₃O₃: C, 58.77; H, 4.52; N, 17.13. Found: C, 58.68; H, 4.48; N, 16.90.

### Part D: Methyl 3-(2-amidinopyrid-5-yl)isoxazolin-5-ylacetate formic acid salt

The part C cyanopyridine (0.47 g, 1.9 mmol) and sodium methoxide (prepared *in situ* from Na metal, 4 mg, 0.2 mmol were stirred in dry MeOH (6 m ) at ambient temperature for 16 h, after which ¹H NMR analysis of a reaction aliquot indicated complete formation of methyl imidate [note 9.25 (s, 1H) and 3.92 (s, 3H)]. Ammonium formate (0.60 g, 9.5 mmol) was added to the reaction mixture, and stirring continued for 7 h. The mixture was absorbed onto silica gel by concentration in vacuo. Chromatography on silica gel, eluting with 0% to 20% MeOH in CHCl₃, and concentration afforded 0.61 g of the amidine as an off-white solid; mp 180-182 °C (dec); HRMS, e/z Calc. for (M+H)⁺: 263.1144. Found: 263.1148.

### Part E: Methyl 3-(2-t-butyloxycarbonylamidinopyrid-5-yl)isoxazolin-5-ylacetate

The part D amidine was BOC protected in standard fashion to afford, after silica gel chromatographic purification, a 41% yield of a colorless foam; HRMS, e/z Calc. for (M+H)⁺: 363.1668. Found: 363.1682.

### Part F: Lithium 3-(2-t-butyloxycarbonylamidinopyrid-5-yl)isoxazolin-5-ylacetate

The part E methyl ester (0.37 g, 1.0 mmol) was saponified by stirring with 0.5 M LiOH in MeOH at RT. The MeOH was removed *in vacuo,* then the aqueous mixture was frozen and lyophilized to produce a pale yellow solid quantitatively; HRMS, e/z Calc. for conjugate acid (M+H)⁺: 349.1512. Found: 349.1531.

### Part G: Methyl N²-(m-toluenesulfonyl)-N³-[3-(2-amidinopyrid-5-yl)isoxazolin-5-ylacetyl]-S-2,3-diaminopropionate bis hydrochloric acid salt

The part F lithium carboxylate was condensed with methyl N²-(*m*-toluenesulfonyl)-2,3-diaminopropionate hydrochloride using conditions described above, followed by standard BOC deprotection with 4 M HCl/dioxane to yield a yellow amorphous solid; HRMS, e/z Calc. for (M+H)⁺: 503.1713. Found: 503.1707.

### Example 548

### Preparation of 3-bromothiophene-2-sulfonyl chloride

A solution of chlorosulfonic acid (14.3 g, 0.12 mol) in 35 mL of 1,2-dichloroethane was chilled to -10°C and protected from moisture, . Phosphorus pentachloride (20.8 g, 0.1 mol) was added in small portions while maintaining the temperature between -5° and -10°C. The resulting slurry was stirred at -10°C for 30 minutes. Then, 3-bromothiophene (16.3 g, 0.1 mol) was added dropwise over a period of 45 minutes, maintaining the temperature between -5° and +5°C. During the addition of the 3-bromothiophene, hydrogen chloride gas was evolved; the reaction mixture became thick and pasty, and difficult to stir. Upon complete addition of the 3-bromothiophene, the reaction temperature was held at 0°C for two hours. The reaction was then heated to 80°C and kept there for one hour; during which the solids dissolved, and hydrogen chloride gas was evolved once more . The reaction mixture was chilled in an ice bath, poured over 250 g crushed ice, and stirred for one hour as the ice melted. The resulting two phase system was separated and the aqueous layer washed three times with 125 mL of chloroform. The combined organic phases were dried over anhydrous MgSO₄, filtered, and concentrated *in vacuo* to give 24.1 g (92%) of crude product as a dark amber oil; ¹H NMR (300 MHz, CDCl₃) δ 7.22 (d, J = 5.3, 1H), 7.73 (d, J = 5.3, 1H); Mass Spectrum (CH₄-DCI / GC-MS, e/z, relative abundance) 262.8, (M+H)⁺, 100%; 226.9, (M+H-HCl)⁺, 89.7%.

### Example 587A

### N²-3-methylphenylsulfonyl-N³-[3-(4-amidinophenyl)-5S-ylacetyl]-S-2,3-diaminopropionic acid

The compound of Example 473C, Part B (0.077g, 0.14mmol) was dissolved in MeOH (4ml), LiOH (0.0066g, 0.158mmol) in H₂O (4ml) was added and the reaction mixture left to stir overnight. After evaporation of methanol the product precipitated from the aqueous as a white solid (0.027g, 35% yield). HRMS calc'd for C₂₂H₂₅N₅O₆S: 488.160381 found: 488.160827.

### Example 602

### Methyl N²-n-butyloxycarbonyl-N³-[3-(4-guanidinophenyl)isoxazolin-5-(R,S)-ylacetyl]-(S)-2,3-diaminopropionate, trifluoroacetic acid salt

### Part A: [3-[(4-t-butyloxycarbonylamino)phenyl]-isoxazolin-5-yl]acetic acid:

This compound was prepared in 49% yield from 4-t-butyloxycarbonylaminobenzaldoxime and t-butyl vinyl acetate using the procedure described above for Ex. 275, Part A. ¹HNMR(CDCl₃) δ 0.99 (t, 3H), 1.35 (m, 2H), 1.50 (s, 9H), 1.61 (m, 2H), 2.60 (dd, J = 7.7 and 16.5 Hz, 1H) 2.84 (dd, J = 5.9 & 16Hz, 1H), 3.06 (dd, J = 7.4 & 16.9 Hz, 1H), 3.48 (dd, J = 10.3 & 16.5Hz, 1H), 4.10 (t, 2H), 5.03 (m, 1H), 6.60 (broad s, 1H), 7.38 (d, J = 8.4 Hz, 2H), 7.58 (J = 8.3Hz, 2H); IR(KBr): 2966, 1734, 1740, 1610, 1578, 1528, 1508, 1458, 1442, 1412, 1392, 1368 1234, 1160, 1058, 916, 878, 828, 772, 612 cm⁻¹; HRMS calcd. for C₂₀H₂₈N₂O₅: 377.207647, Found 377.207278. Standard LiOH saponification conditions then afforded the corresponding carboxylic acid compound as colorless crystals in 88% yield. mp 178-180°C; ¹HNMR(CDCl₃) δ 1.52 (s, 9H), 2.67 (dd, J = 7.8 and 16 Hz, 1H), 2.89 (dd, J = 8.3 & 16Hz, 1H), 3.06 (dd, J = 9.5 & 16.9 Hz, 1H), 3.48 (dd, J = 10.3 & 16.5z, 1H), 5.03(m, 1H).

### Part B: Methyl N²-n-butyloxycarbonyl-N³-[3-[(4-t-butyloxycarbonylamino)phenyl]isoxazolin-5-yl acetyl]-(S)-2,3-diaminopropionate:

The compound of Example 602, Part A was condensed with methyl N²-tBoc-(S)-2,3-diaminopropionate using the procedure described for Ex. 275, Part C above to provide the desired product. mp 80-82°C; ¹HNMR(CDCl₃) δ 1.88 (t,3H), 1.30 (m,2H), 1.47 (sm,20H), 2.50 (dd,1H), 2.61(dd, 1H), 3.07 (dd,1H), 3.40 (dd,1H), 3.63 (t,2H), 3.74 (s,3H), 4.00 (m,2H), 4.38 (m,1H), 5.00 (m,1H), 5.88 (dd, 1H), 6.77 (t,1H), 7.58 (d,2H), 7.84 (d,2H), 10.4 (s, 1H), 11.6 (s, 1H); IR(KBr):3286, 2964, 1722, 1646, 1546, 1414, 1368, 1340, 1312, 1294, 1240, 1156, 1122, 1100, 1058, 1030, 844, 776 cm⁻¹. Mass spectrum (CI/NH₄) 663(M+H,20),563(7), 549(78), 506(81),463(100).

### Part C: Methyl N²-n-butyloxycarbonyl-N³-(3-(4-quanidinophenyl)isoxazolin-5-yl acetyl]-(S)-2,3-diaminopropionate:

The compound of Ex 602, part B was treated with TFA in dichloromethane to afford the corresponding aniline as its TFA salt. This intermediate was converted to the corresponding bis-BOC protected quanidino compound in 59% yield using the method of Kim et al. (Tet. Lett. 1993, 48, 7677). Deprotection under standard conditions (TFA/CH₂Cl₂) provided the title compound as its TFA salt (90%). ¹HNMR(DMSO-d₆) δ 1.89 (t,3H), 1.34 (m,2H), 1.57(m,2H), 2.44 (dd,1H), 2.58 (t,2H), 2.64 (m,1H), 3.17 (m, 1H), 3.40 (m,2H), 3.65 (m,1H), 3.70 (s,3H), 4.00 (t,2H), 4.31 (m,1H), 5.02 (m,1H), 6.80 (m,1H), 7.28 (d,2H), 7.64 (broads,3H), 7.68 (d,2H), 7.84(broad, 1H) ; Mass spectrum(ES) m/z 463 (M+H, 100).

### Example 651

### Methyl N²-benzyloxycarbonyl-N³-methyl-N³-[3-(4-amidinophenyl)isoxazolin-5-(R,S)-ylacetyl]-(S)-2,3-diaminopropionate, trifluoroacetic acid salt

### Part A. Preparation of methyl N²-benzyloxycarbonyl-N³-methyl-[3-(4-N-Boc-amidinophenyl)isoxazolin-5-(R,S)-ylacetyl]-(S)-2,3-diaminopropionate.

To a mixture of 3-(4-N-Boc-amidinophenyl)-isoxazolin-5-ylacetic acid (prepared according to the procedure of Example 434, part F; 189 mg, 0.54 mmol), methyl N³-methyl-N²-Cbz-L-2,3-diaminopropionate (prepared according to Sakai and Ohfune, J. Am. Chem. Soc. 114, 998 (1992); 145 mg, 0.54 mmol) and TBTU (175 mg, 0.54 mmol) in ethyl acetate (10 ml) was added triethylamine (0.15 ml, 1.09 mmol). After stirring for 26 h, the mixture was diluted with ethyl acetate, washed with pH 4 buffer, then with saturated aqueous sodium bicarbonate, then with saturated brine. The organic phase was dried (MgSO₄) and concentrated. The residue was flash chromatographed (ethyl acetate) to provide the product as a colorless glass (279 mg, 86%): NMR (CDCl₃) δ 7.88 (m, 2H), 7.69 (m, 2H), 5.79 (bd, 1H), 5.09 (m, 3H), 4.58 (m, 1H), 3.86 (m, 1H), 3.77 (2s, 3H), 3.63 (m, 2H), 3.14 (dd, 1H), 3.01 (2s, 3H), 2.9 (m, 1H), 2.53 (m, 1H), 1.66 (b, 2H), 1.56 (s, 9H); mass spec (ESI) m/z 596.2 (M+H⁺, 100%).

### Part B. Preparation of Methyl N²-benzyloxycarbonyl-N³-methyl-N³-[3-(4-amidinophenyl)isoxazolin-5-(R,S)-ylacetyl]-(S)-2,3-diaminopropionate, trifluoroacetic acid salt

The product of part A (226 mg, 0.38 mmol) was dissolved in dichloromethane (3 ml) and treated with trifluoroacetic acid (1 ml). After stirring at room temperature for 4 h, the mixture was diluted with ether and stirred. The resulting white solid was collected by filtration to provide the title product as a white solid (201 mg, 87%): NMR (DMSO-d₆) δ 9.39 (bs, 2H), 9.19 (bs, 2H), 7.87 (s, 4H), 7.79 (t, 1H), 7.32 (m, 5H), 5.03 (3H), 4.40 (m, 2H), 3.90 (m, 1H), 3.65 (2s, 3H), 2.95 and 2.82 (4s, 3H), 3.6-2.8 (4H); mass spec (ESI) m/z 496.3 (M+H⁺, 100%).

### Example 701

Methyl N²-n-butyloxycarbonyl-N³-[3-(4-amidinophenyl)isoxazol-5-yl acetyl]-*L*-2,3-diaminopropionate TFA salt.

### Part A. Preparation of Methyl 3-(4-cyanophenyl)isoxazo-5-yl acetate

To a suspension of methyl 3-(4-cyanophenyl)-(5R,S)-isoxazolin-5-yl acetate (5.28 g, 21.62 mmol) in chloroform (150 ml) were added *N*-bromosuccinimide (4.23 g, 23.78 mmol) and AIBN (100 mg) and the mixture was refluxed. Small amounts of AIBN (100 mg - 200 mg) were added at one hour intervals until TLC showed a complete reaction. Potassium acetate (17.3 g) and acetic acid (6.5 ml) were added and the reaction mixture was refluxed for 1 hour, cooled, then poured into 1N NaOH (325 ml). The organic layer was separated and the aqueous layer was extracted with EtOAc (3 x 100 ml). The organic layers were combined and washed with sat. NaCl, dried over Na₂SO₄, filtered, and concentrated in vacuo. The residue was chromatographed on silica gel (15% to 35% EtOAc in Hexane) to yield 2.2 g (42%) of an off-white solid as product; ¹H NMR (300 MHz, CDCl₃) δ 7.93 (dd, 2H), 7.76 (dd, 2H), 6.67 (s, 1H), 3.92 (s, 2H), 3.8 (s, 3H).

### Part B. Preparation of Methyl 3-(4-methoxyiminophenyl)isoxazo-5-yl acetate HCl salt.

A suspension of methyl 3-(4-cyanophenyl)isoxazo-5-yl acetate (2.19 g, 9.04 mmol) in 100 ml of anhydrous methanol was chilled in an ice bath and dry HCl gas was bubbled through the reaction mixture until a solution was obtained. The total addition time was two hours. The reaction flask was sealed and the reaction mixture was allowed to warm to room temperature, with stirring, over a period of about 24 hrs. At this point, the methanolic solution was poured into 500 ml of anhydrous ether, precipitating the product, and the resulting slurry was chilled to -25°C for 3 hours. The precipitate was filtered, washed with two 100 ml portions of chilled anhydrous ether, and suction dried under nitrogen to afford 2.3 g (82%) of the hydrochloride salt; ¹H NMR (300 MHz, suspension in CDCl₃) δ 8.52 (d, J = 8.06 Hz, 2H), 8.03 (d, J = 8.4 Hz, 2H), 6.67 (s, 1H), 4.6 (s, 3H), 3.93 (s, 2H), 3.8 (s, 3H).

### Part C. Preparation of Methyl 3-(4-amidinophenyl)isoxazo-5-yl acetate HCl salt.

A solution of methyl 3-(4-methoxyiminophenyl)isoxazo-5-yl acetate HCl salt (2.3 g, 7.4 mmol) in 50 ml of anhydrous methanol was chilled in an ice bath and 2M ammonia in methanol (18.5 ml, 37 mmol) was added. The reaction flask was sealed and the reaction mixture was allowed to warm to room temperature, with stirring, over a period of 24 h. The amber solution was then concentrated *in vacuo* to give 2.2 g (quant. yield) of a yellow foam; ¹H NMR (300 MHz, d₆-DMSO) δ 9.6-9.2 (b), 8.12 (d, J = 8.4 Hz, 2H), 7.97 (d, J = 8.4 Hz, 2H), 7.14 (s, 1H), 4.15 (s, 2H), 3.7 (s, 3H).

### Part D. Preparation of Methyl 3-(4-N-Boc-amidinophenyl)isoxazo-5-yl acetate.

To a solution of methyl 3-(4-amidinophenyl)isoxazo-5-yl acetate HCl salt (2.2 g, 7.4 mmol) in 30 ml DMF cooled with an ice bath was added triethylamine (2.06 ml, 14.8 mmol) and di-tert-butyl dicarbonate (1.78 g, 8.14 mmol). The reaction mixture was warmed to room temperature and stirred for 64 hrs. The reaction mixture was then partitioned between EtOAc and water. The aqueous layer was washed with EtOAc. The organic layers were combined and washed with water, sat. NaCl, dried over Na₂SO₄, filtered, and concentrated *in vacuo*. The residue was chromatographed on silica gel (15% to 25% EtOAc in Hexane) to afford 1.45 g (54%) of product; ¹H NMR (300 MHz, CDCl₃) δ 7.96 (d, J = 8.4 Hz, 2H), 7.87 (d, J = 8.4 Hz, 2H), 6.65 (s, 1H), 3.91 (s, 2H), 3.8 (s, 3H), 1.56 (s, 9H) .

### Part E. Preparation of 3-(4-N-Boc-amidinophenyl)isoxazo-5-yl acetic acid.

To a solution of methyl 3-(4-*N*-Boc-amidinophenyl)isoxazo-5-yl acetate (1.45 g, 4.03 mmol) in 30 ml of methanol was added a solution of lithium hydroxide monohydrate (0.195 g, 4.64 mmol) in water (5 ml). The mixture was stirred at room temperature for 16 hours. The reaction mixture was then concentrated *in vacuo* and the residue was diluted with water and the resulting mixture was cooled using an ice bath. 1N HCl was slowly added to a pH of 3 - 4 and the resulting acidic aqueous mixture was extracted repeatedly with EtOAc. The organic layers were combined and washed with sat. NaCl, dried over Na₂SO₄, filtered and concentrated *in vacuo* to yield 0.97 g (70%) of an off-white powdery solid as product; ¹H NMR (300 MHz, d₆-DMSO) δ 8.07 (d, J = 8.79 Hz, 2H), 7.97 (d, J = 8.4 Hz, 2H), 7.03 (s, 1H), 3.99 (s, 2H), 1.45 (s, 9H).

### Part F. Preparation of Methyl N²-n-butyloxycarbonyl-N³-[3-(4-N-Boc-amidinophenyl)isoxazo-5-yl acetyl]-L-2,3-diaminopropionate.

To a solution of 3-(4-*N*-Boc-amidinophenyl)isoxazo-5-yl acetic acid (0.262 g, 0.76 mmol), methyl N²-carboxy-*n*-butyl-*L*-2,3-diaminopropionate HCl salt (0.193 g, 0.76 mmol), and TBTU (0.256 g, 0.8 mmol) in DMF (15 ml) was added triethylamine (0.45 ml, 3.23 mmol) and the resulting reaction mixture was allowed to stir at room temperature for 16 hours. The reaction mixture was partitioned between EtOAc and water. The water layer was washed twice with EtOAc. The organic layers were combined and washed with water, pH 4 buffer, 5% NaHCO₃, and sat. NaCl, dried over Na₂SO₄, filtered, and evaporated *in vacuo.* The residue was chromatographed on silica gel (100% EtOAc) to yield 0.315 g (76%) of a slightly amber foam; ¹H NMR (300 MHz, CDCl₃) δ 7.93 (d, J = 8.42 Hz, 2H), 7.83 (d, J = 8.42 Hz, 2H), 6.6 (s, 1H), 6.57 (bm, 1H), 5.66 (bm, 1H), 4.45 (bm, 1H), 4.05 (m, 2H), 3.77 (s, 5H), 3.7 (m, 2H), 1.57 (s, 9H), 1.56 (m, 2H), 1.35 (m, 2H), 0.9 (t, J = 7.32 Hz, 3H). Part G. Preparation of Methyl N²-n-butyloxycarbonyl-N³-[3-(4-amidinophenyl)isoxazo-5-yl acetyl]-*L*-2,3-diaminopropionate TFA salt.

A solution of methyl N²-carboxy-n-butyl-N³-[3-(4-*N*-Boc-amidinophenyl)isoxazo-5-yl acetyl]-*L*-2,3-diaminopropionate (0.215 g, 0.39 mmol) in 1:1 methylene chloride / trifluoroacetic acid (20 ml total) was stirred at room temperature for 16 hours. The reaction mixture was then concentrated *in vacuo* and the residue chromatographed on silica gel (10% to 30% methanol in chloroform) to yield 0.11 g (50%) of a white solid; ¹H NMR (300 MHz, d₆-DMSO) δ 9.4 (bs, 2H), 9.15 (bs, 2H), 8.45 (t, 1H), 8.11 (d, J = 8.42 Hz, 2H), 7.94 (d, J = 8.42 Hz, 2H), 7.53 (d, J = 8.06 Hz, 1H), 7.01 (s, 1H), 4.21 (m, 1H), 3.95 (t, 2H), 3.81 (s, 2H), 3.62 (s, 3H), 3.55 (m, 1H), 3.34 (m, 1H), 1.5 (m, 2H), 1.3 (m, 2H), 0.87 (t, J = 7.32 Hz, 3H).; Mass Spectrum (ESI, e/z, relative abundance) 446.3, (M+H)⁺, 100%.

### Example 829

### Methyl N²-n-butyloxycarbonyl-N³-[5-(4-formamidinophenyl)isoxazolin-3-yl acetyl]-(2S)-2,3-diaminopropionate

### Part A: t-Butyl [5-(4-cyanophenyl)isoxazolin-3-yl]acetate:

Cycloaddition of 4-cyanophenylethylene (MP&D chemical Co.) and tert-butylformyl oxime was carried out following the procedure of Gree et. al. (Bioorganic & Medicinal Chemistry letters 1994, 253) to provide the desired isoxazoline in 72% yield. ¹HNMR(CDCl₃) δ: 1.40 (s, 9H), 3.00 (dd, J = 8.3 and 17Hz, 1H), 3.35 (dd(AB) J = 18 and 8.3 Hz, 2H),3.48 (m, 1H), 5.60 (dd, J = 9 and 4.5 Hz, 1H), 7.47 (d, J = 8Hz, 2H), 7.65 (d, J = 8 Hz, 2H); IR 2235, 1718, 1610 cm⁻¹. Mass spectrum m/z 287 (M+H, 100) .

### Part B: [5-(4-cyanophenyl)isoxazolin-3-yl] acetic acid:

Hydrolysis of the compound of Ex.829, Part A with excess TFA in dichloromethane afforded the acid in 90% yield. ¹HNMR (CDCl₃) δ 3.00 (dd, J = 8 and 17.2 Hz, 1H), 3.55 (s, 2H), 3.59 (m, 1H), 5.66 (dd, J = 8 and 11Hz, 1H), 7.45 (d, J = 8.4 Hz, 2H), 7.66 (d, J = 8.0 Hz, 2H); IR(KBr) 3325, 2235, 1718, 1605 cm⁻¹; Mass spectrum m/z 231 (M+H, 100).

### Part C: Methyl (5-(4-Boc-amidinophenyl)isoxazolin-3-yl] acetate:

The compound of Ex. 829, Part B compound was then subjected to the standard Pinner reaction conditions described in Ex. 275, Part D to afford an amidino compound, which, without purification, was subjected to treatment with di-tert-butyldicarbonate in dioxane/water (9:1) and excess triethylamine to afford the desired compound in 28% yield. ¹HNMR(CDCl₃) δ 1.54 (s,9H), 2.98 (dd, J = 8 and 17 HZ, 1H), 3.49 (s, 2H), 3.53 (m, 1H), 3.71 (s, 3H), 5.63 (dd, J = 8 & 11.4Hz, 1H), 7.38 (d, 8.2Hz, 2H), 7.82 ((d, 8.2Hz, 2H); Mass spectrum m/z 362(M+H, 8), 306(18), 262(M+H-Boc, 100).

### Part D: [5-(4-Boc-amidinophenyl)isoxazolin-3-yl]acetic acid:

Hydrolysis of the ester using standard LiOH conditions afforded the desired acid in 5% yield. ¹HNMR(CDCl₃) δ 1.54 (s,9H), 3.00 (dd, J = 8 and 17 HZ, 1H), 3.51 (s, 2H), 3.53 (m, 1H), 5.63 (dd, J = 8 & 11.4Hz, 1H), 7.38 (d, 8.2Hz, 2H), 7.82 ((d, 8.2Hz, 2H); Mass spectrum m/z 348(M+H,12),248(M+H-Boc, 100).

### Part E: Methyl N²-n-butyloxycarbonyl-N³-[5-(4-amidino phenyl)isoxazolin-3-yl-acetyl](S)-2,3-diaminopropionate, trifluoroacetate:

The compound of Ex. 829, Part D was coupled with methyl-(S)-N²-n-butyloxycarbonyl-2,3-diaminopropionate following the procedure described in Ex. 275, Part C to give the Boc protected intermediate in 80% yield. ¹HNMR(CDCl₃) δ 0.89 (t, 3H), 1.32 (m, 2H), 1.53 (s, 9H), 1.17 (m, 2H), 2.95 (dd, J = 8 and 17 HZ, 1H), 3.33 (s, 2H), 3.46 (m, 1H), 3.60 (m, 2H), 3.73 (s, 3H), 4.00 (m, 2H), 4.31 (m, 1H), 5.60 (dd, J = 8 & 11.4Hz, 1H), 5.70 (bd, 1H), 6.70 (broad, 1H), 7.33 (d, 8.2Hz, 2H), 7.89 ((d, 8.2Hz, 2H); Mass spectrum m/z 534 (M+H, 30), 434 (M+H-Boc, 100). Deprotection by treatment of the above Boc-amidine with excess TFA in dichloromethane provided the title compound as the TFA salt. ¹HNMR(CDCl₃/DMSO-d₆) δ 1.88 (t, 3H), 1.30 (m, 2H), 1.53 (m, 2H), 3.00 (dd, J = 8 and 17 HZ, 1H), 3.32 (s, 2H), 3.40-3.63 (m, 3H), 3.63 (d, 3H), 3.98 (t, 2H), 4.29 (m, 1H), 5.60 (dd, J = 8 & 11Hz, 1H), 6.80 (d, 1H), 7.50 (d, J = 8Hz, 2H), 7.80 (d, J = 8.2Hz, 2H), 8.03 (broad s, 1H), 9.05 (broad s, 2H); IR(KBr): 3388, 1718, 1664, 1620, 1528, 1456, 1436, 1384, 1366, 1280, 1254, 1168, 1144, 1074, 980, 882, 778 cm⁻¹; Mass spectrum(ES) m/z 448 (M+H,100)

Using the above methods and variations thereof known in the art of organic synthesis, the additional examples in Tables 1-2 and 2A-2E can be prepared.

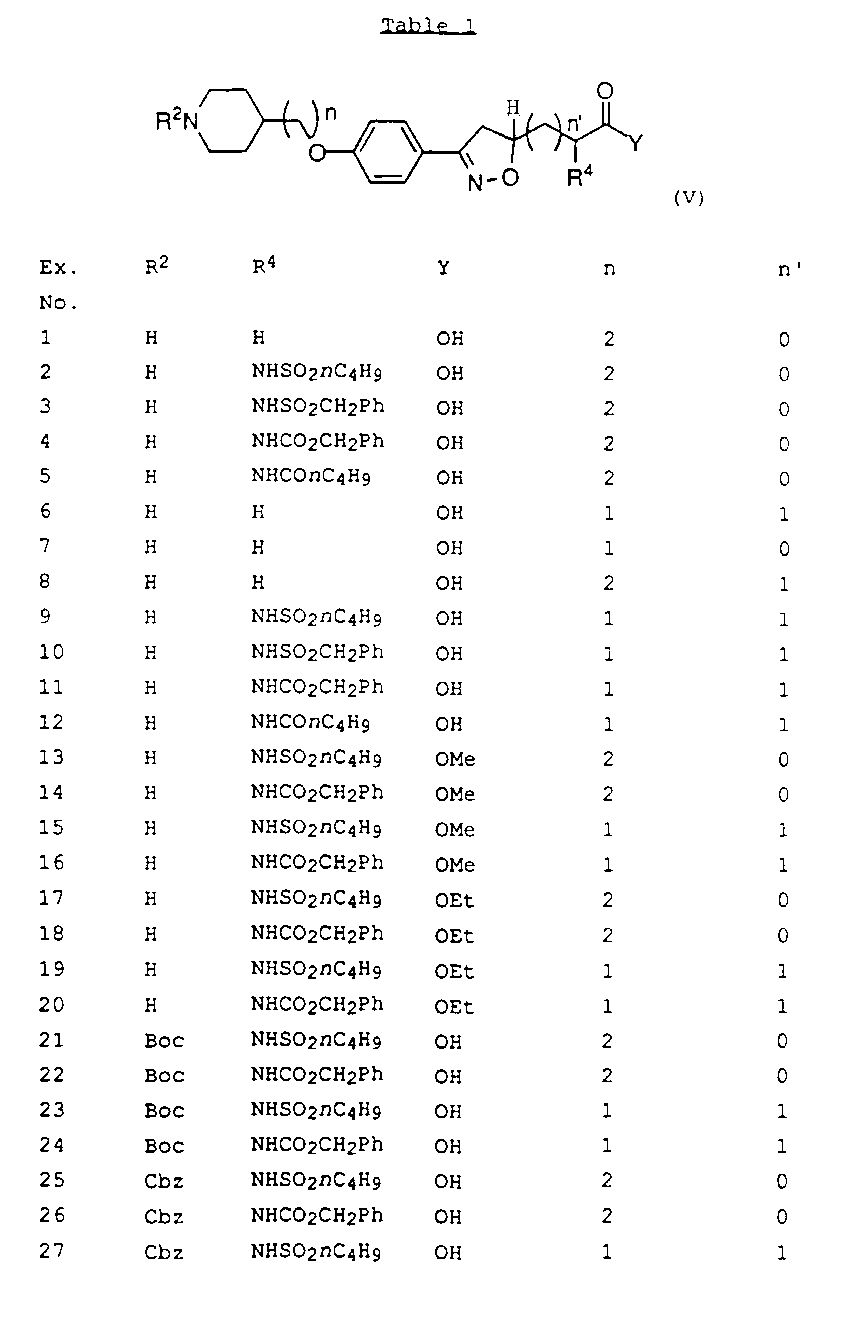

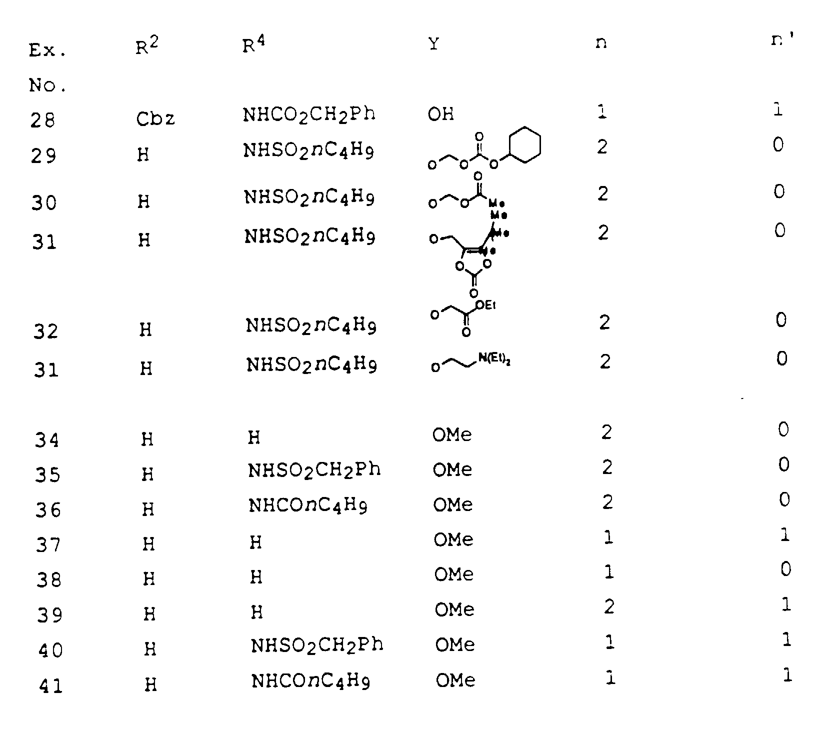

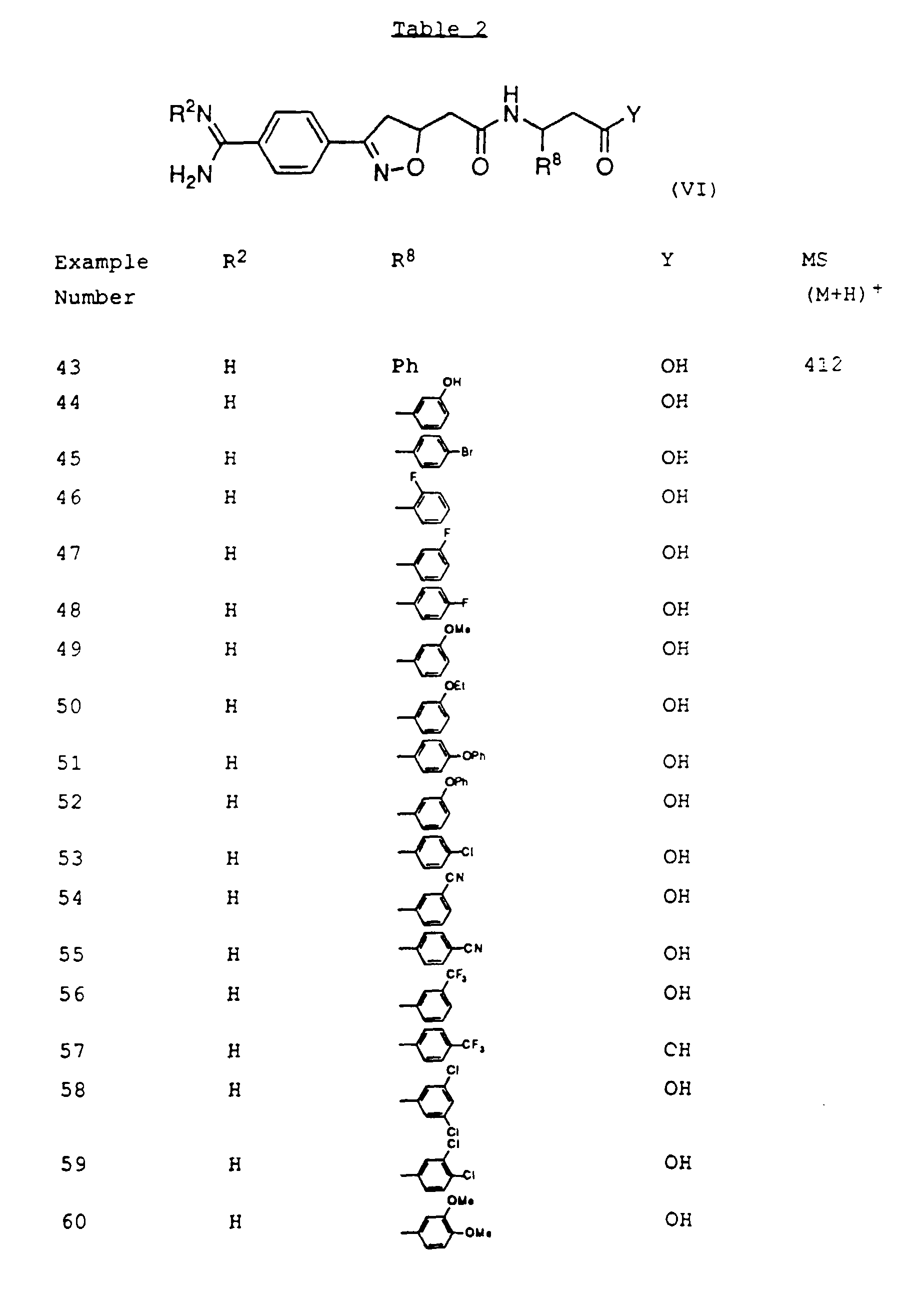

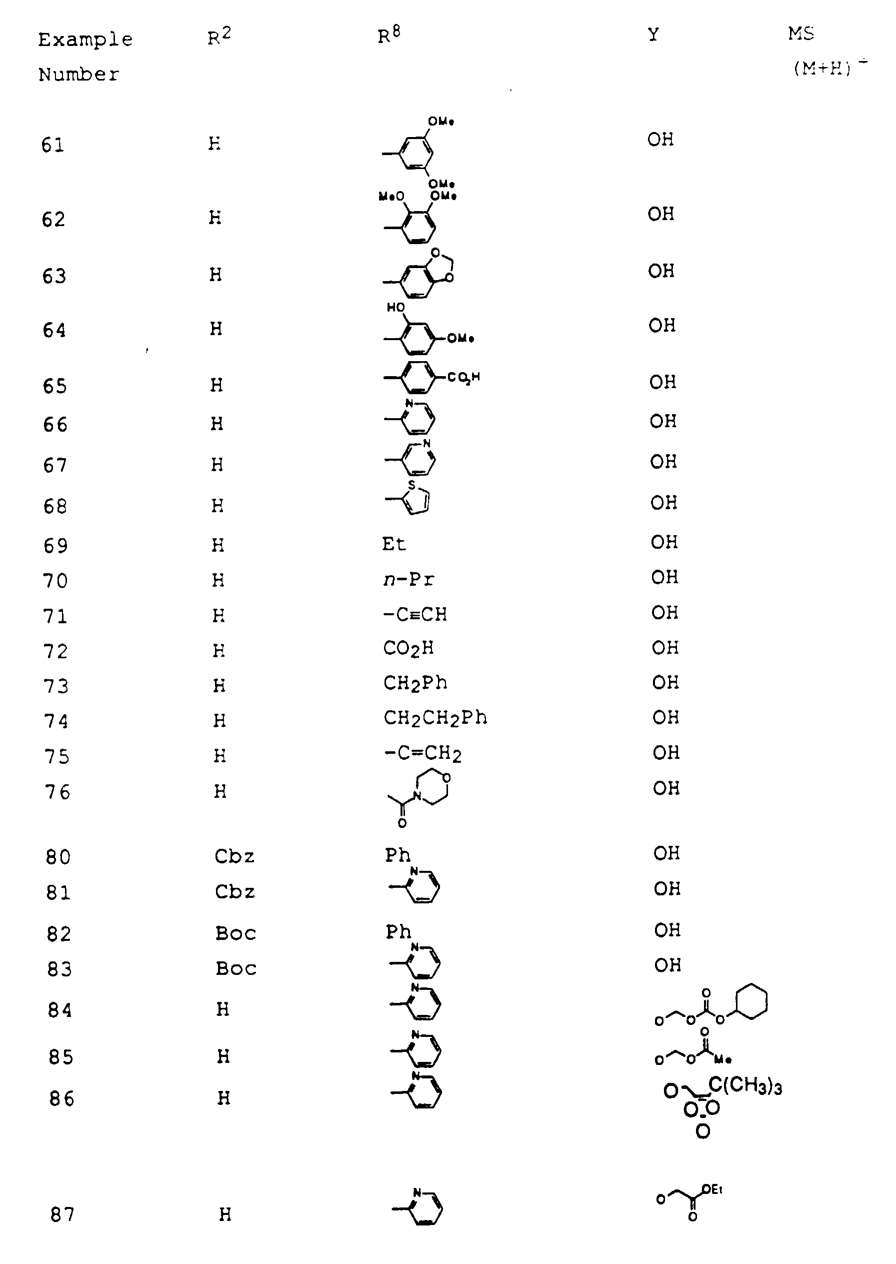

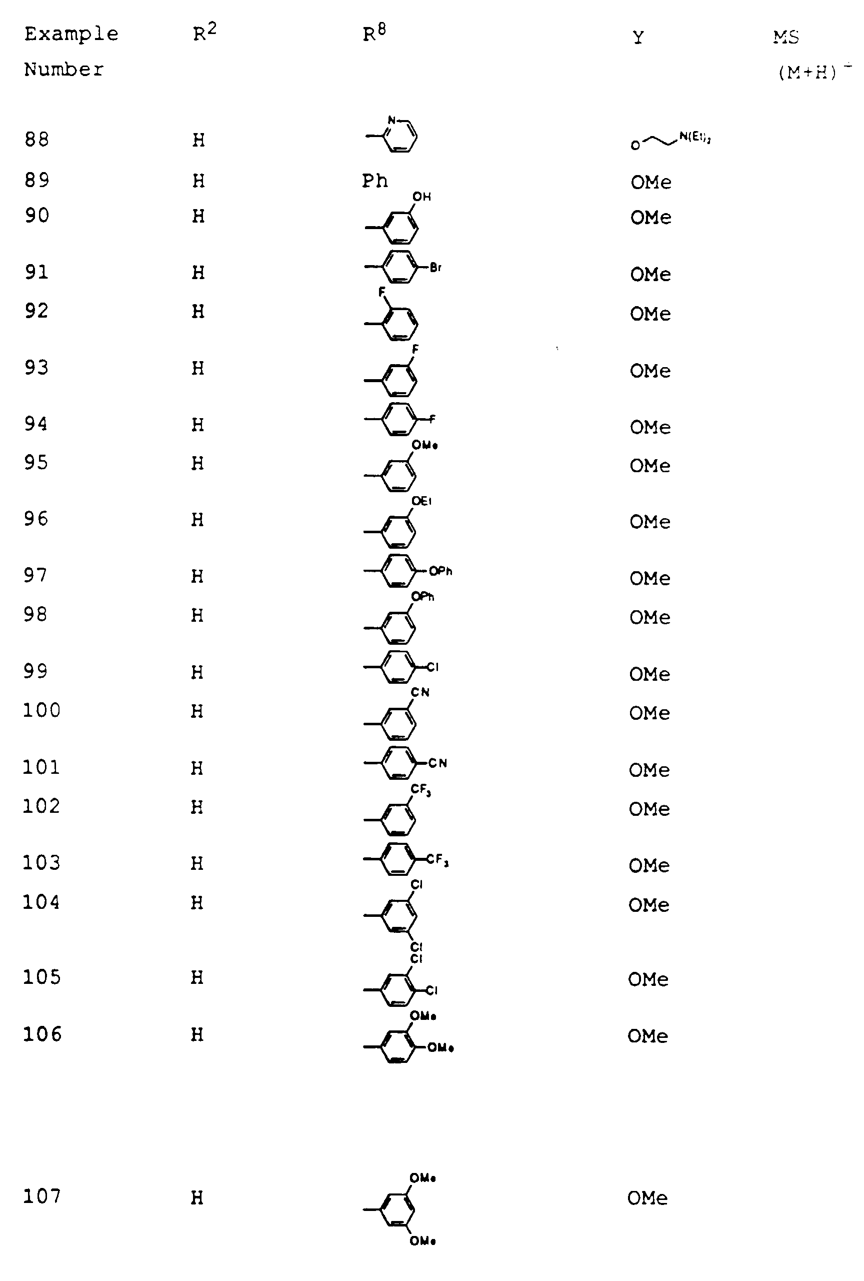

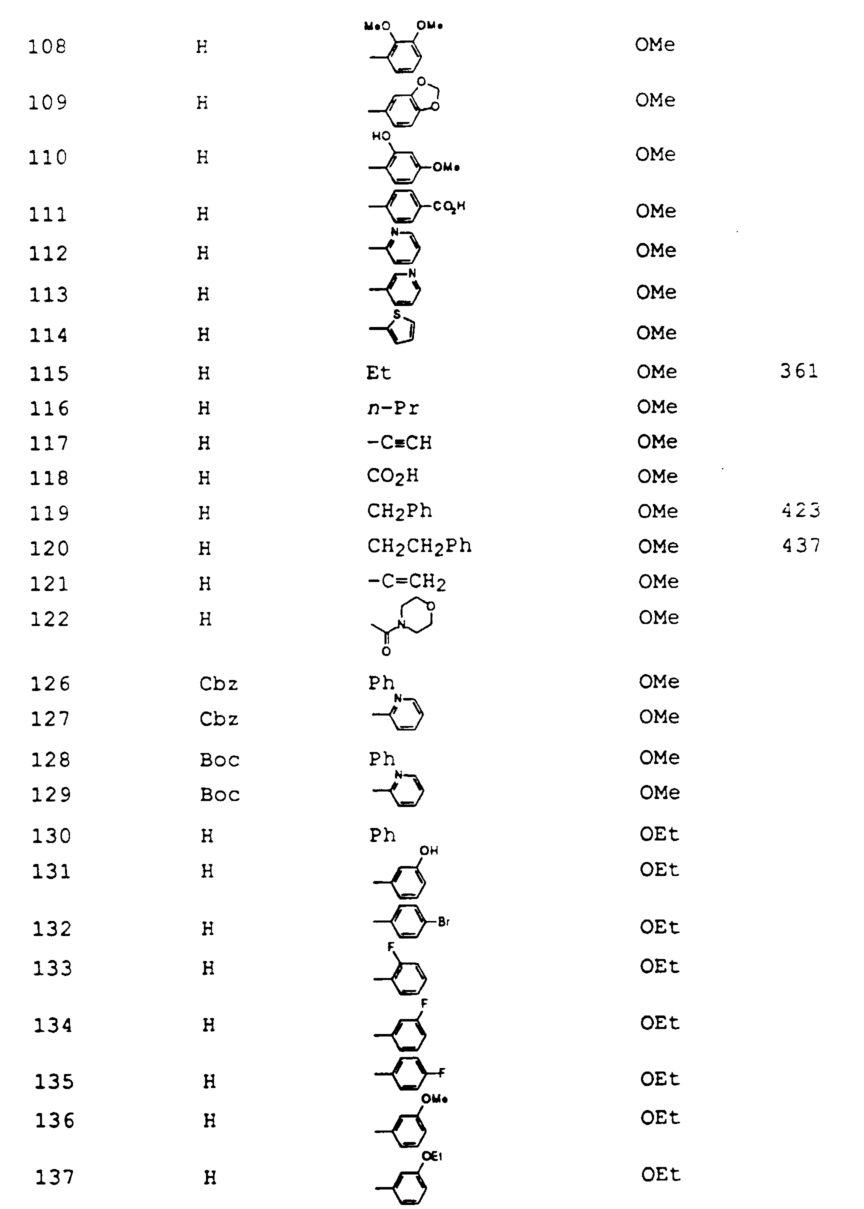

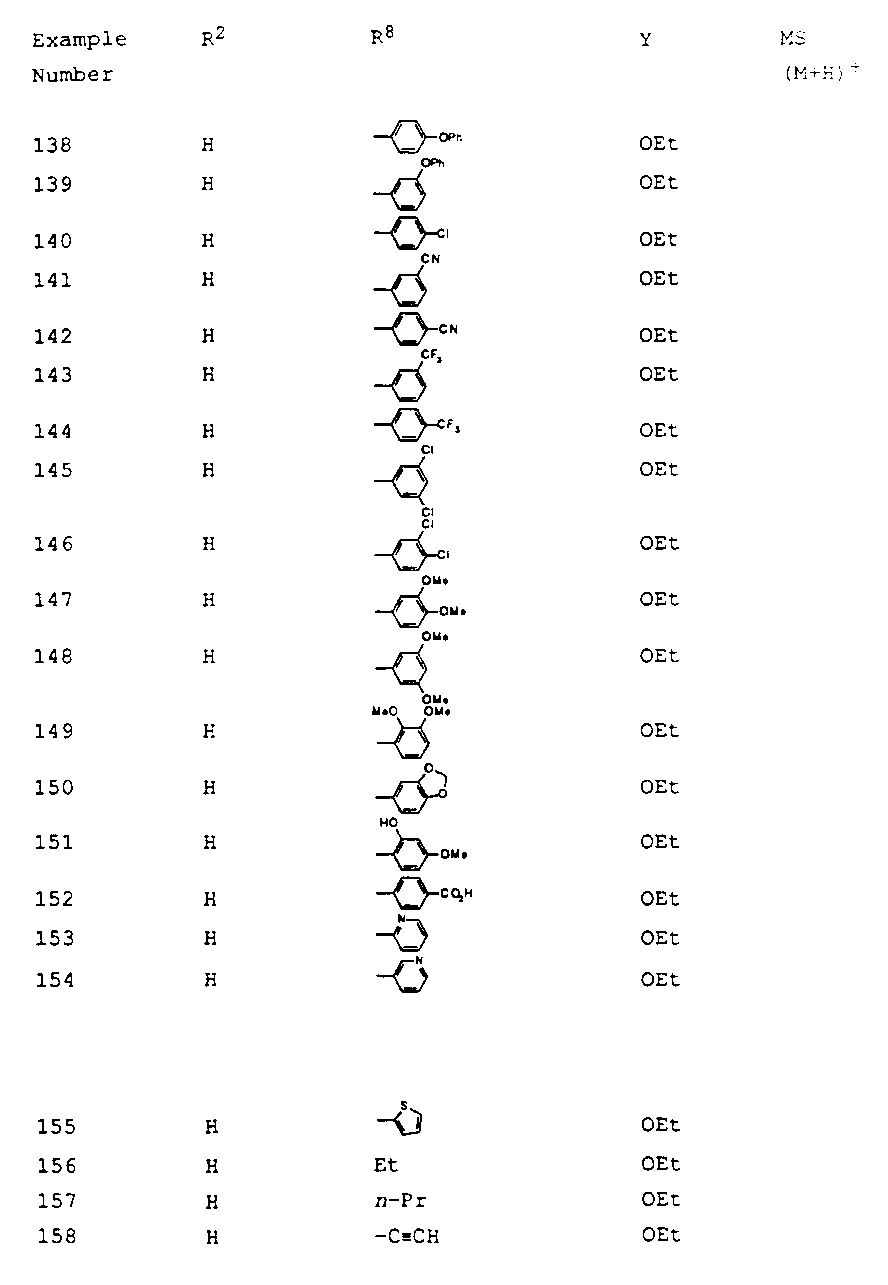

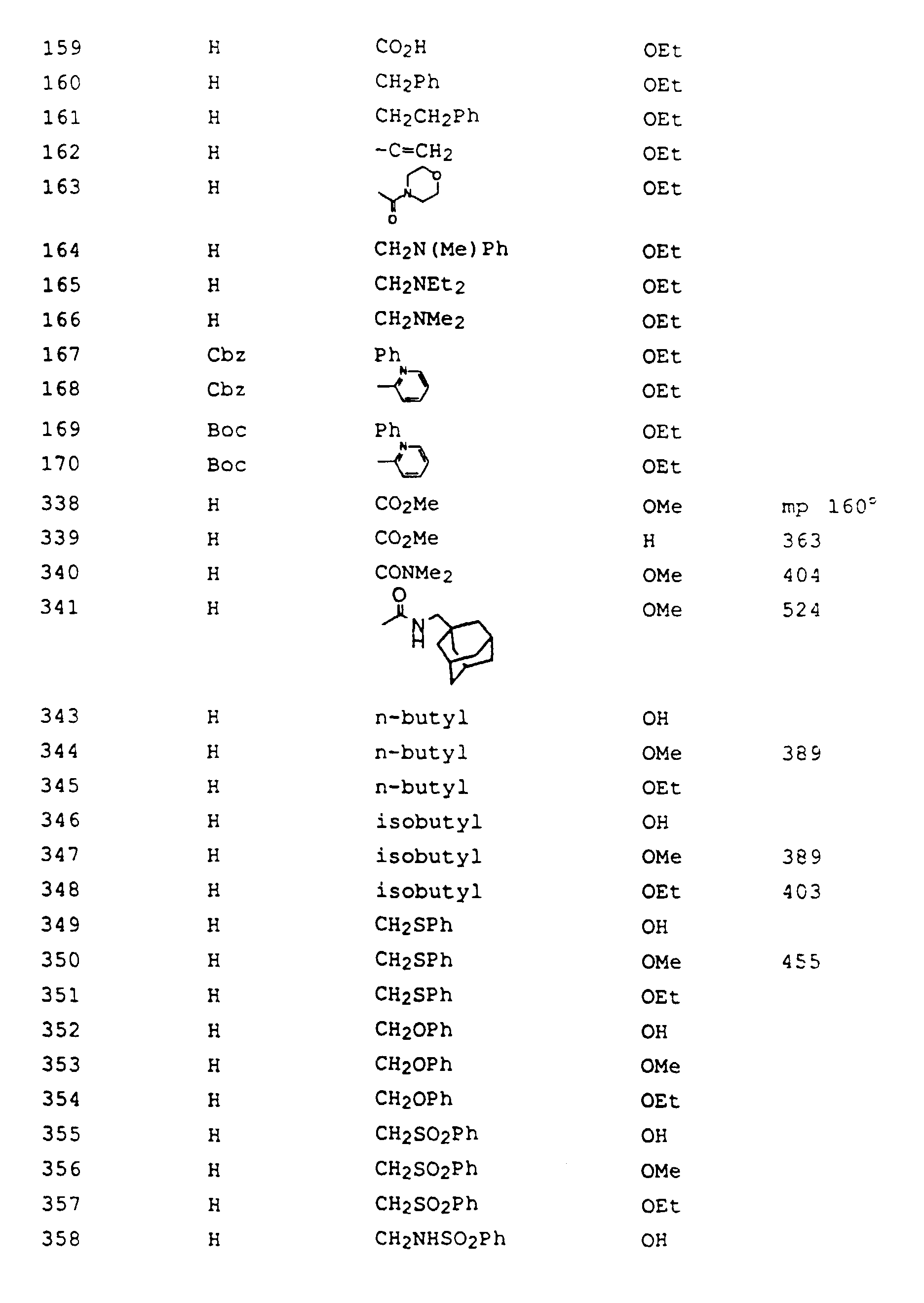

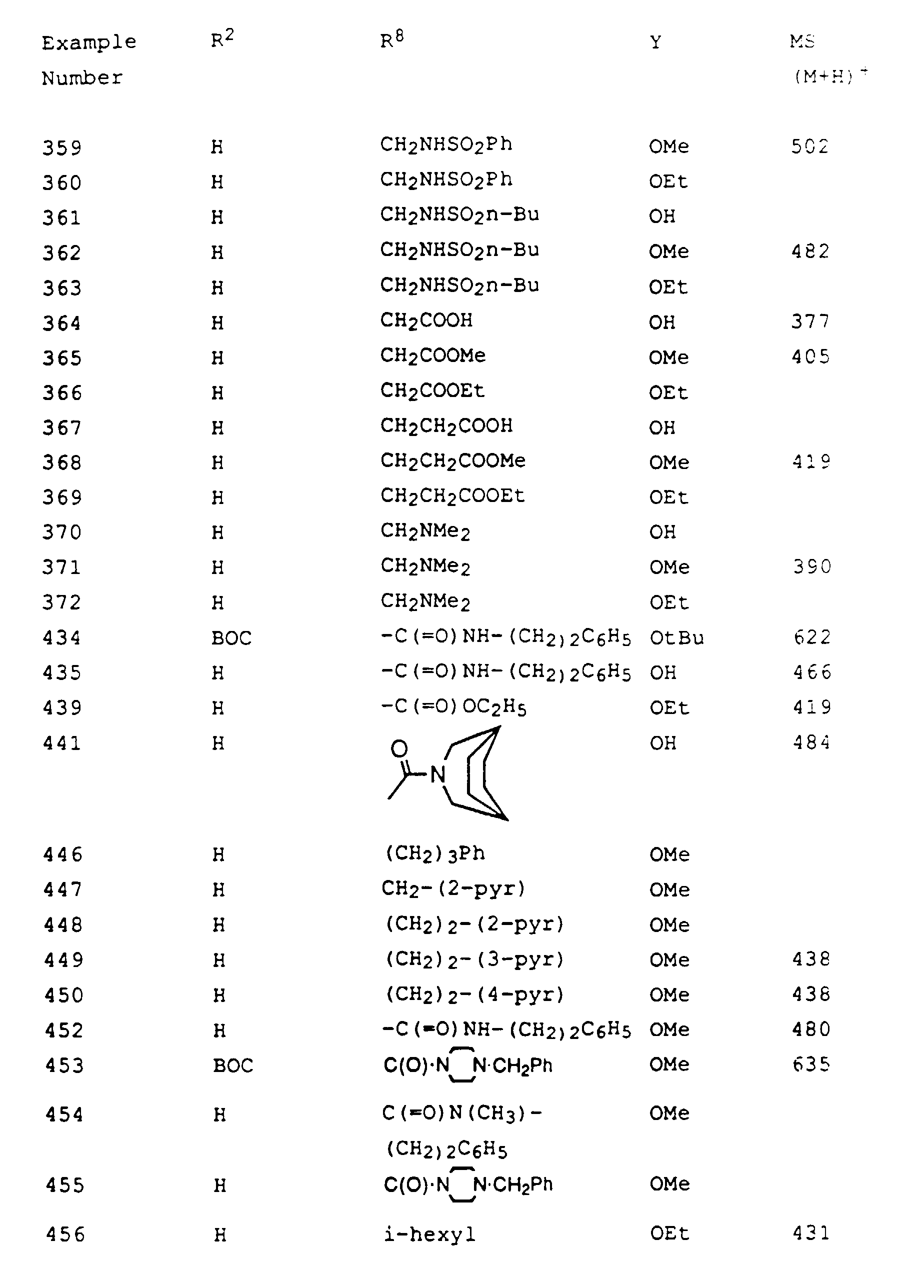

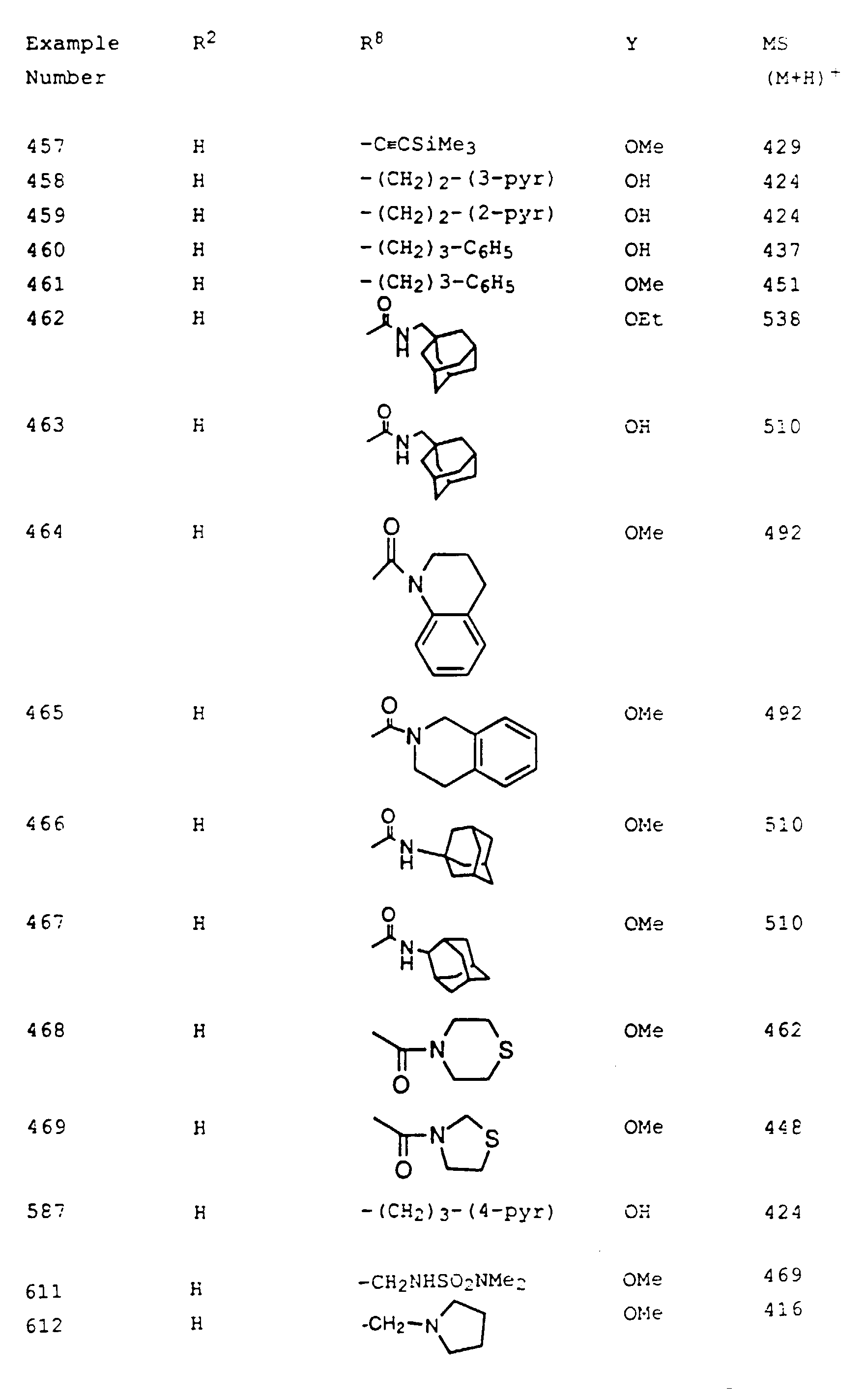

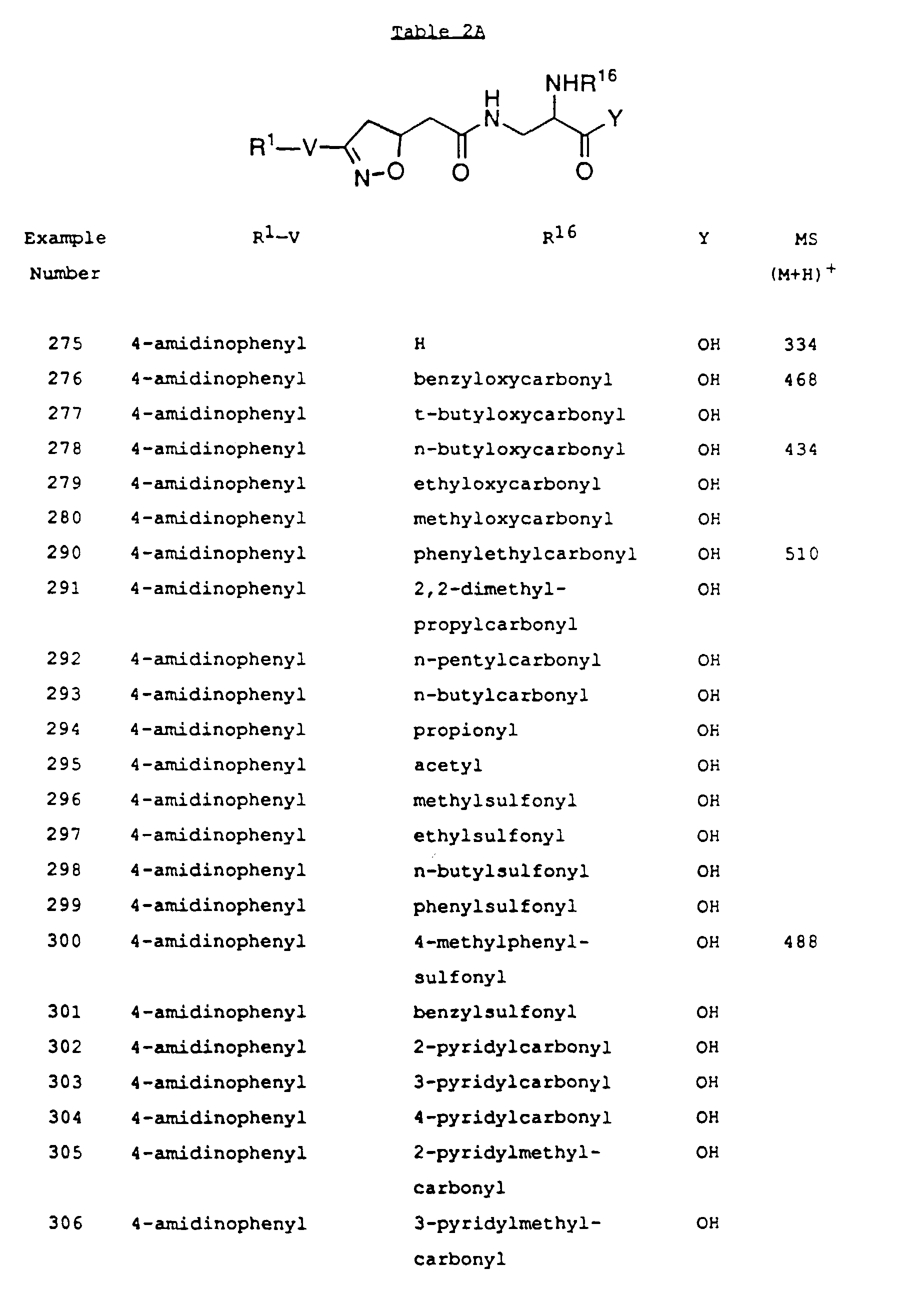

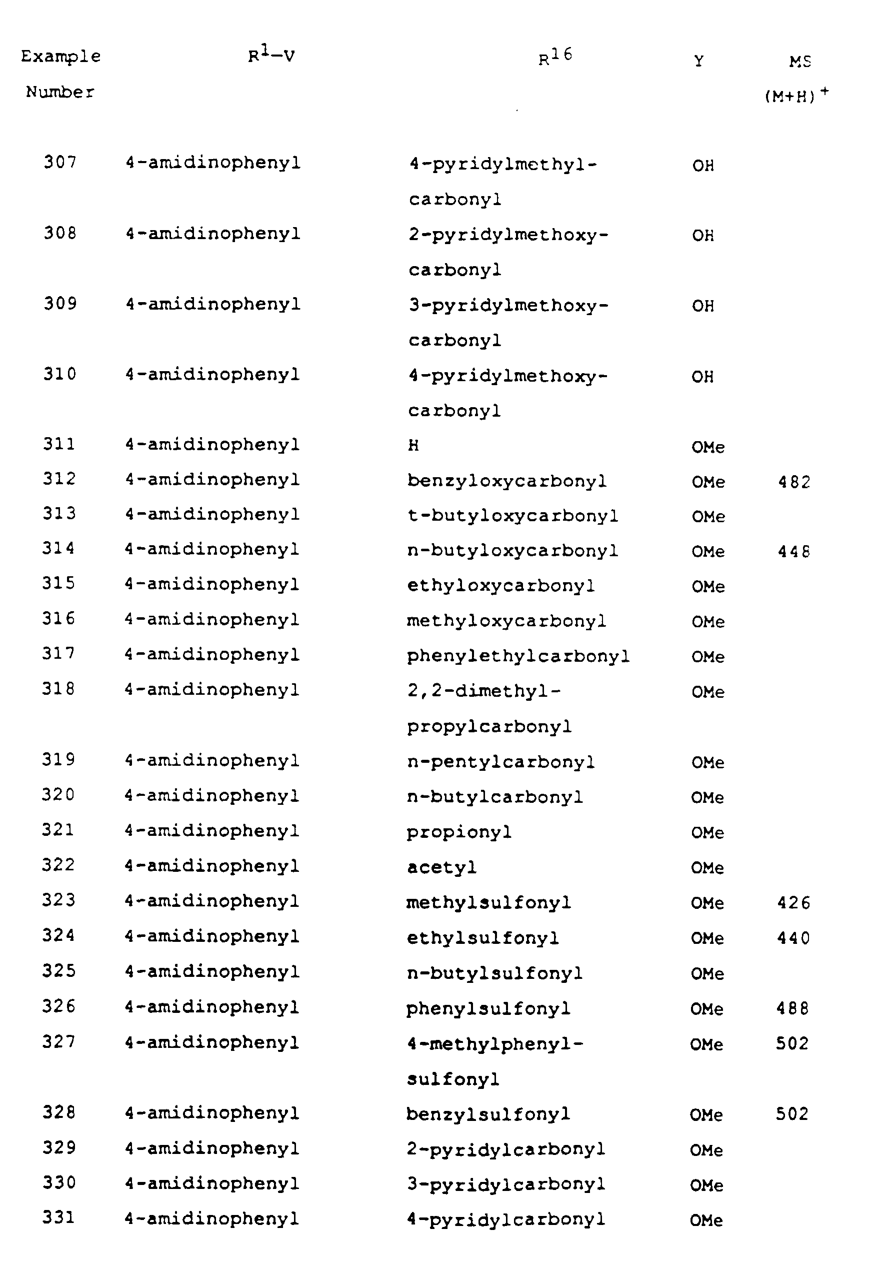

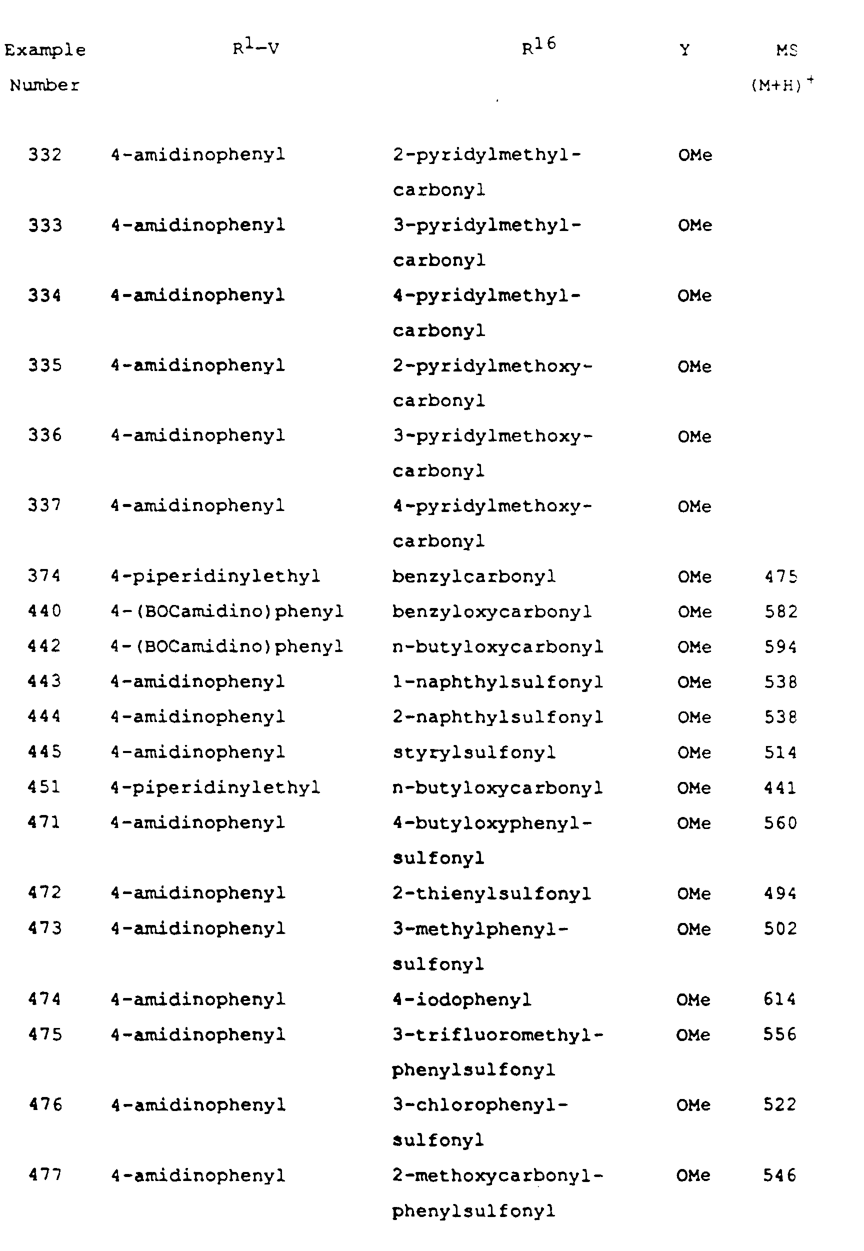

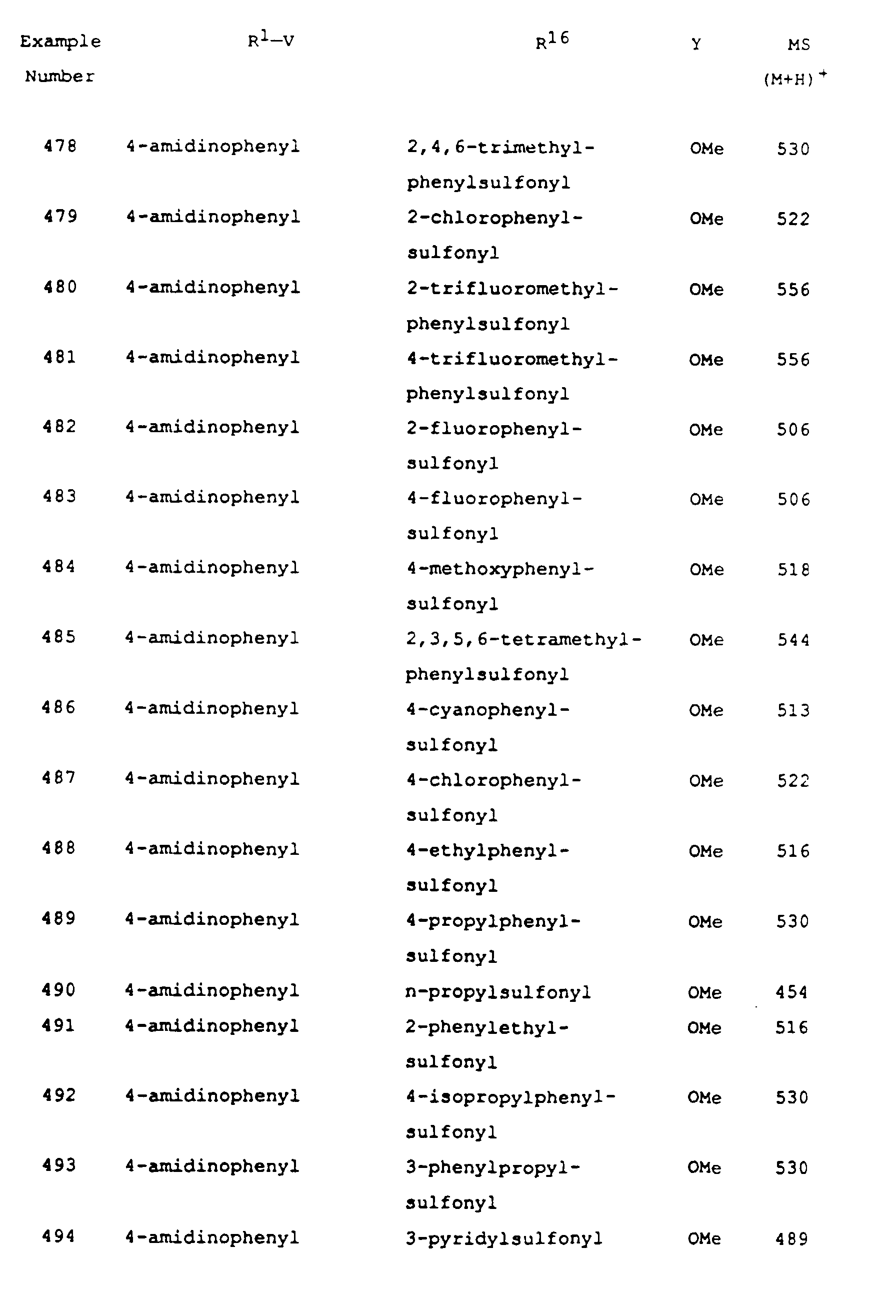

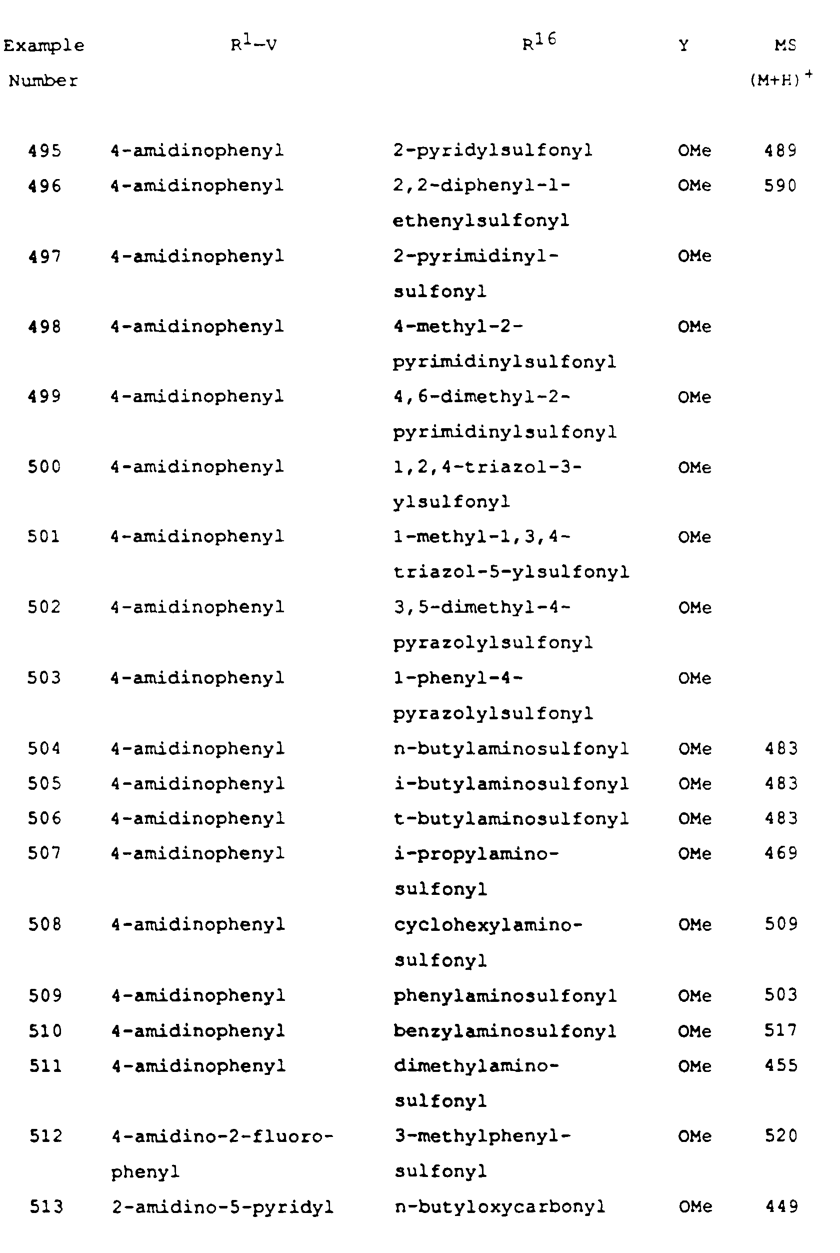

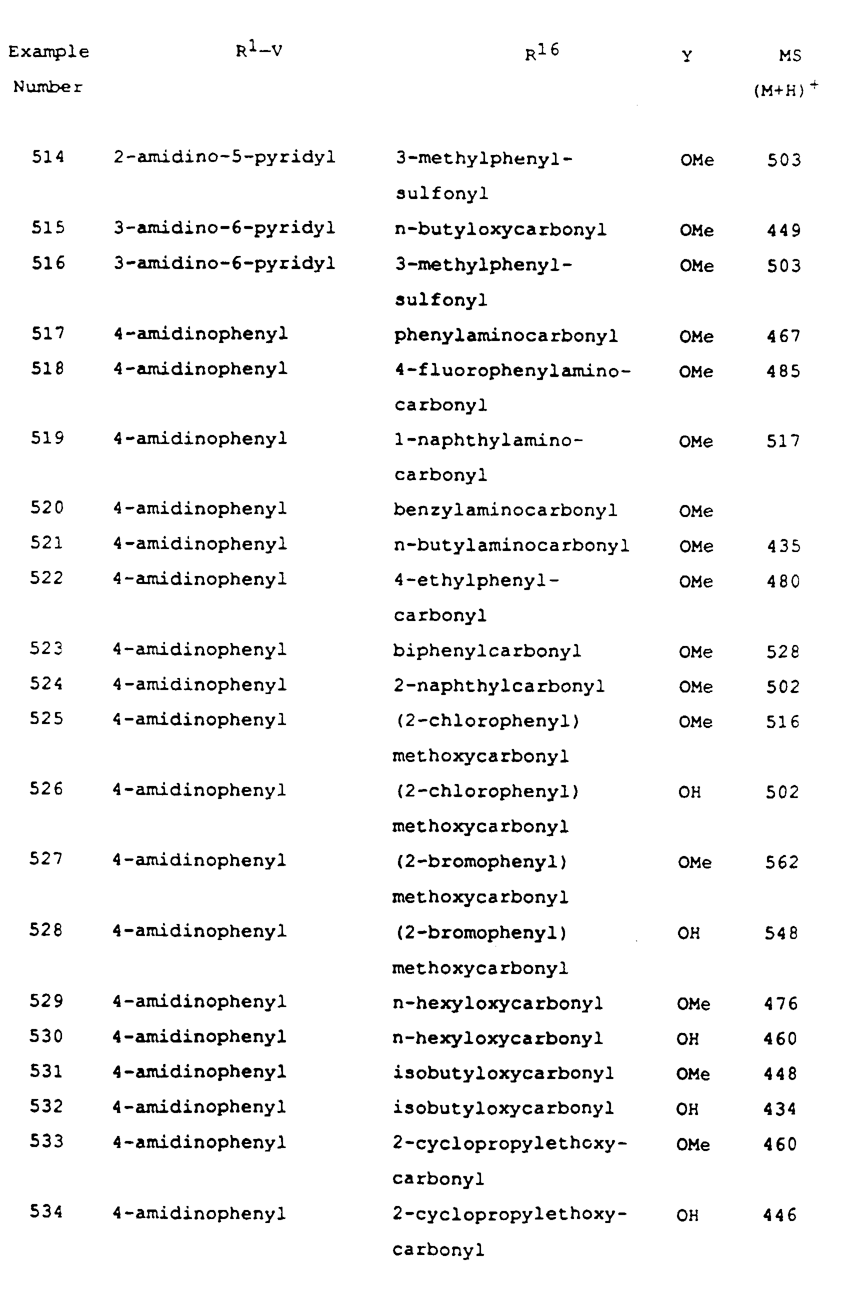

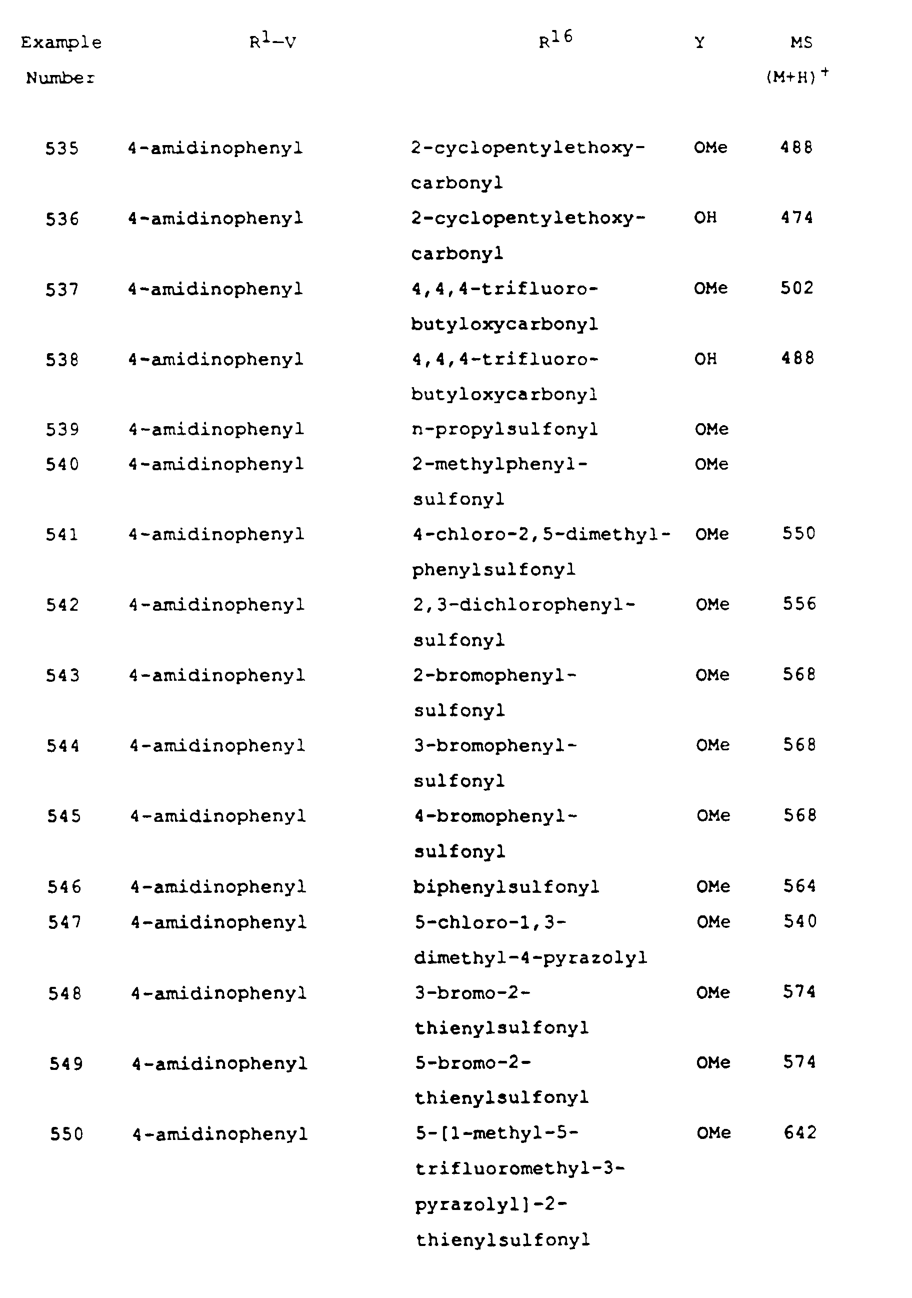

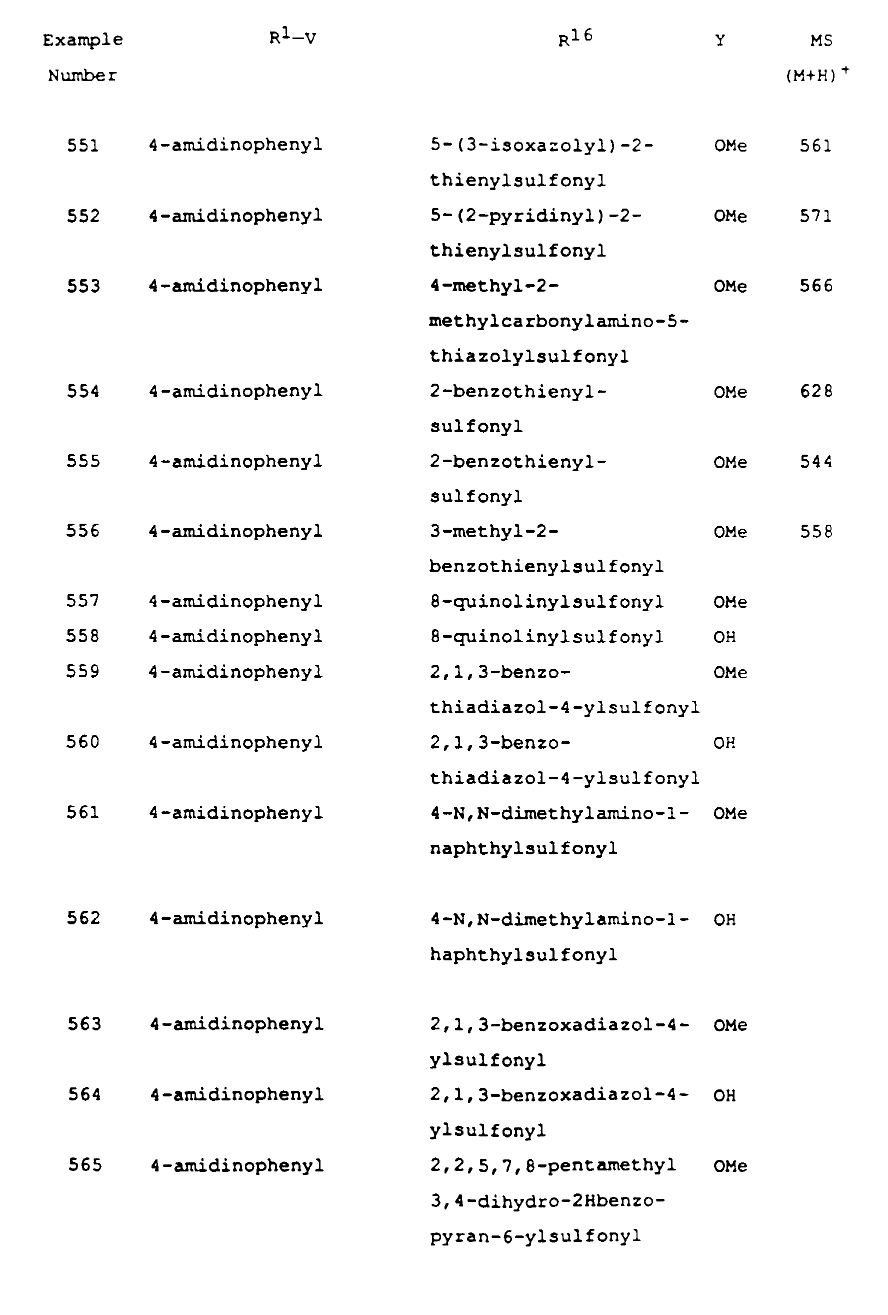

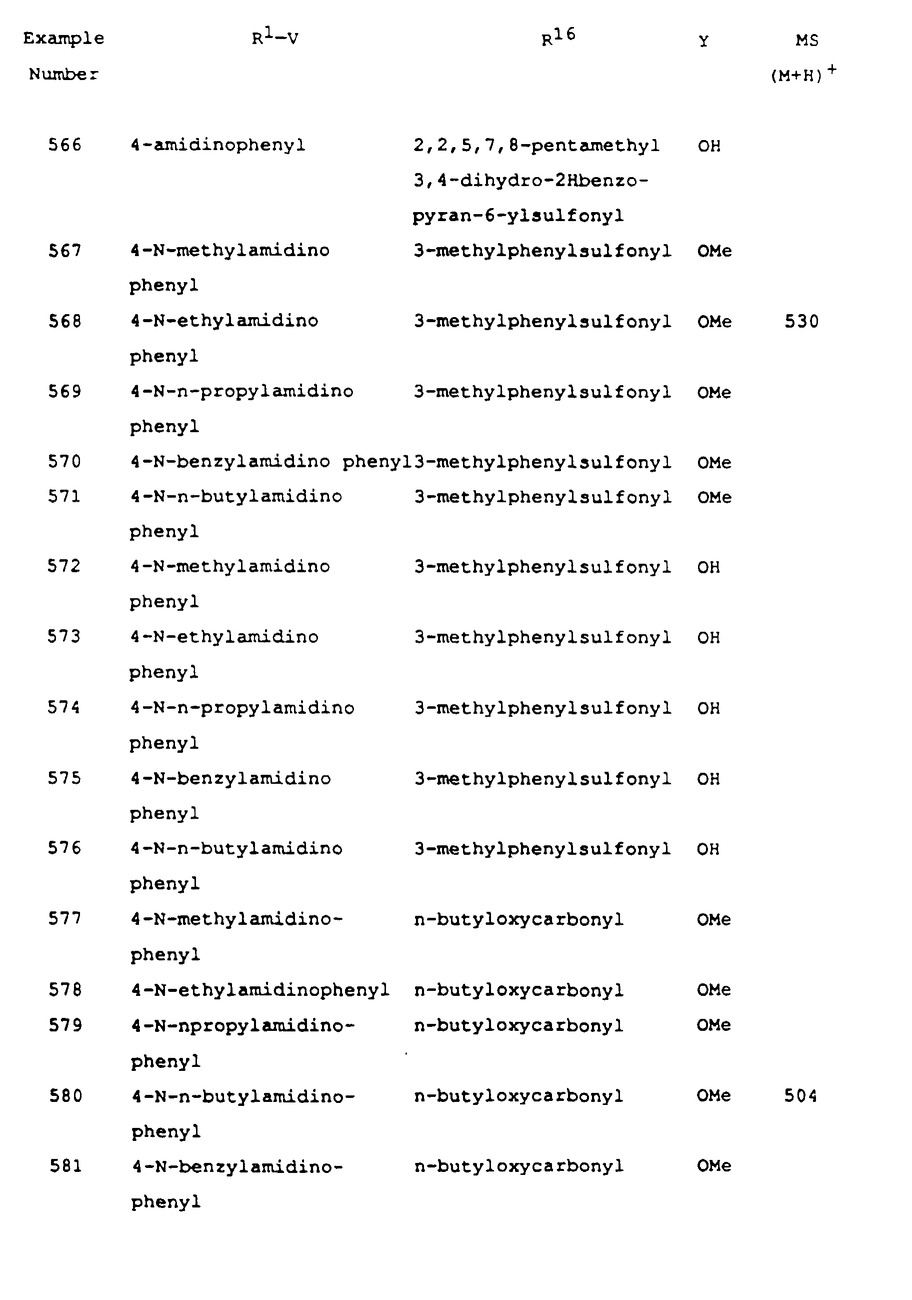

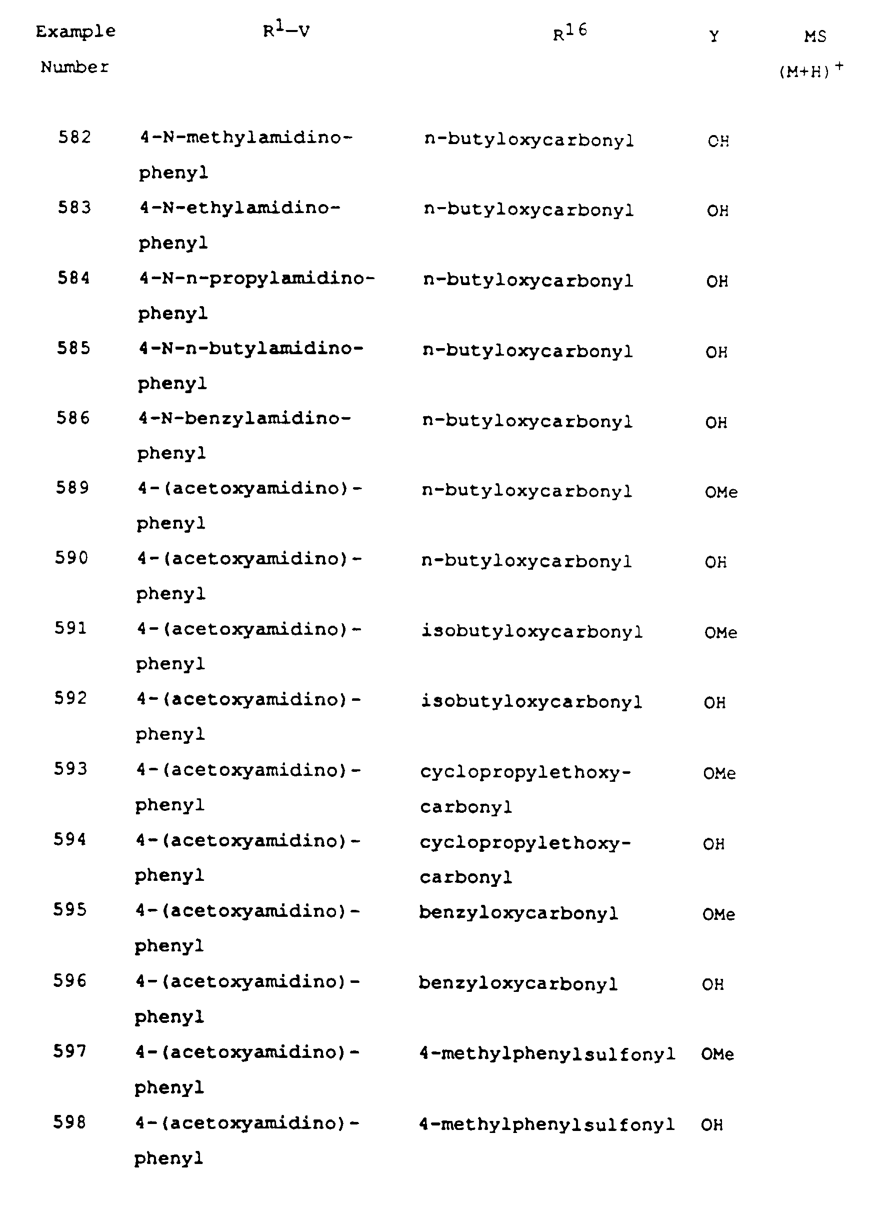

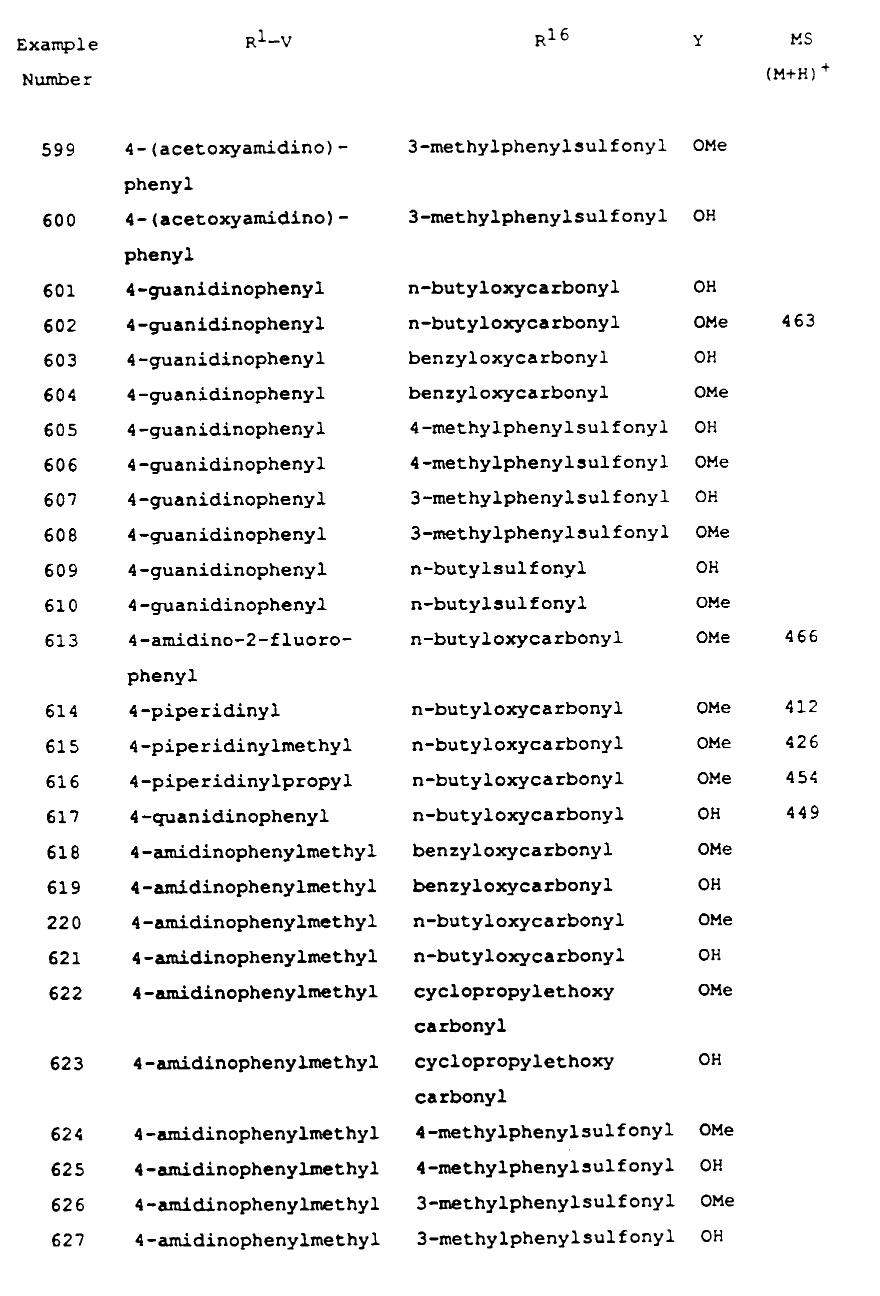

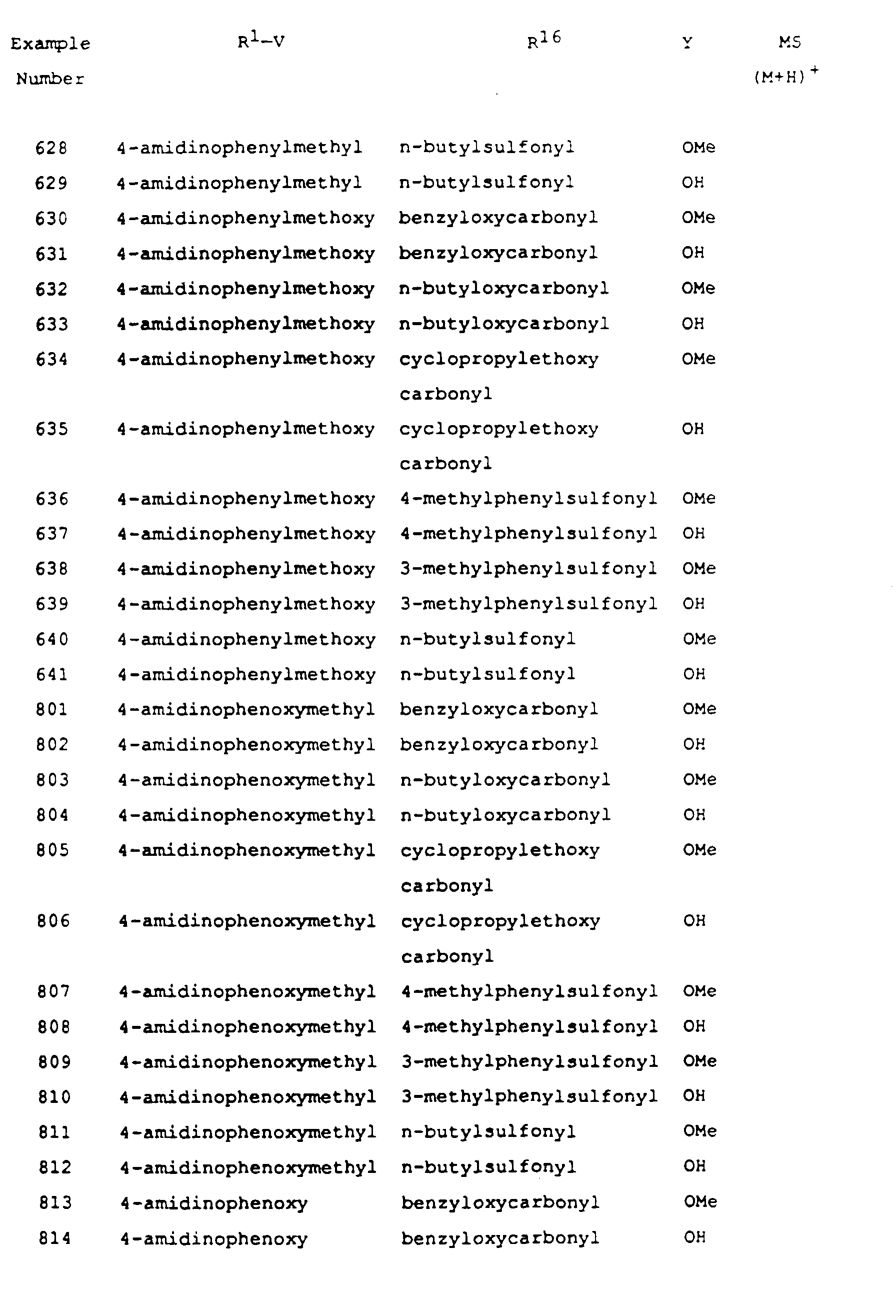

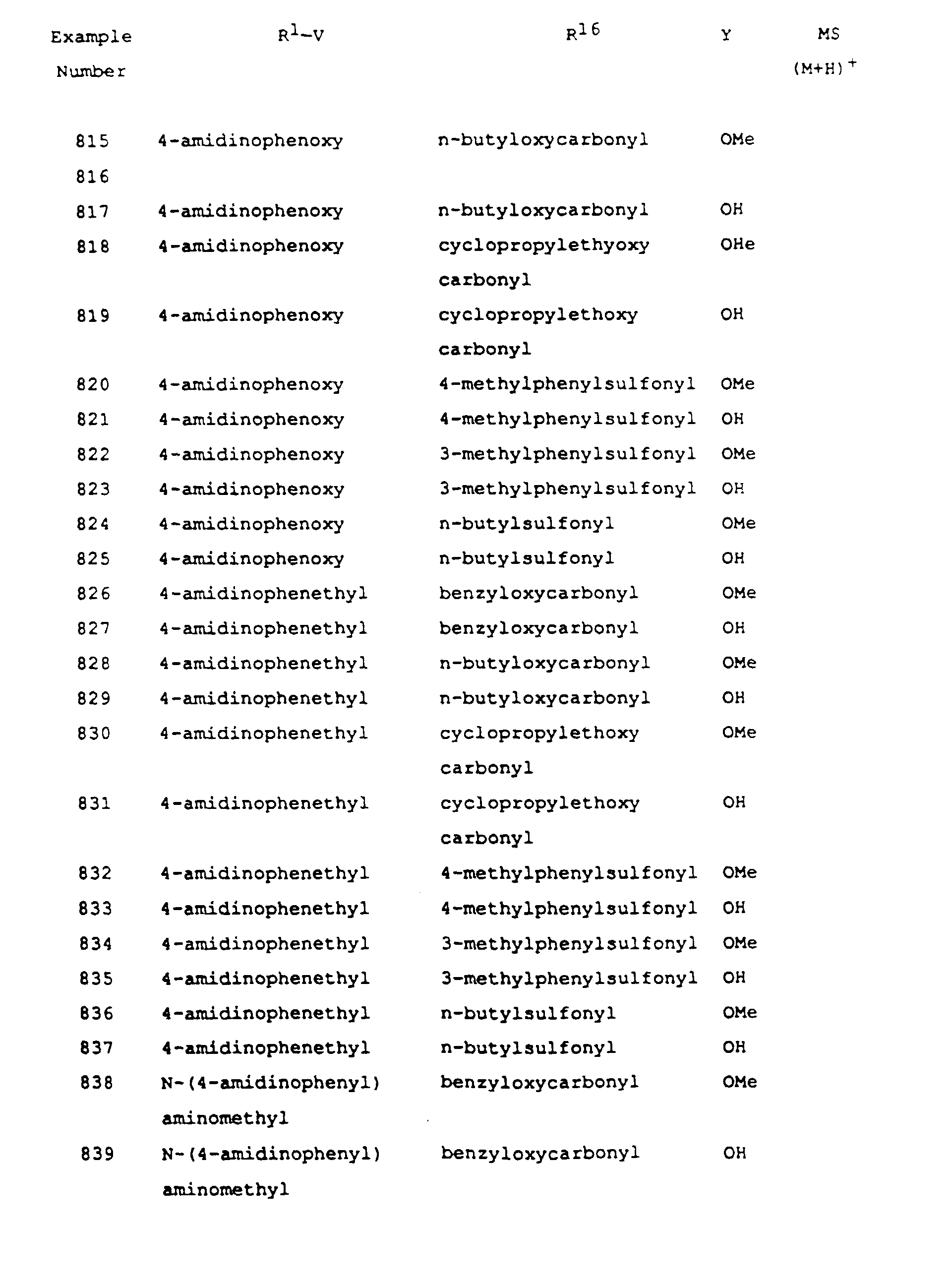

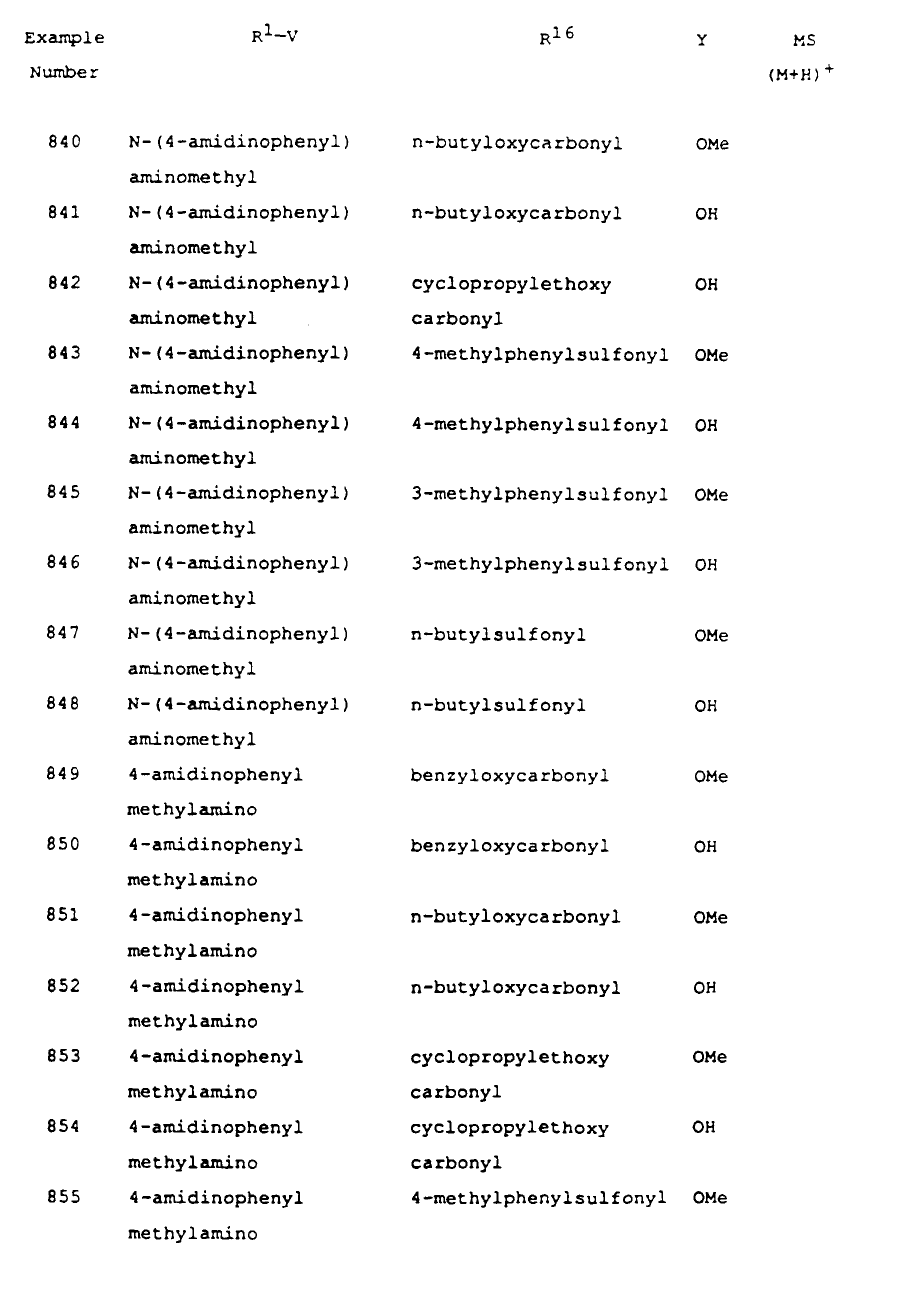

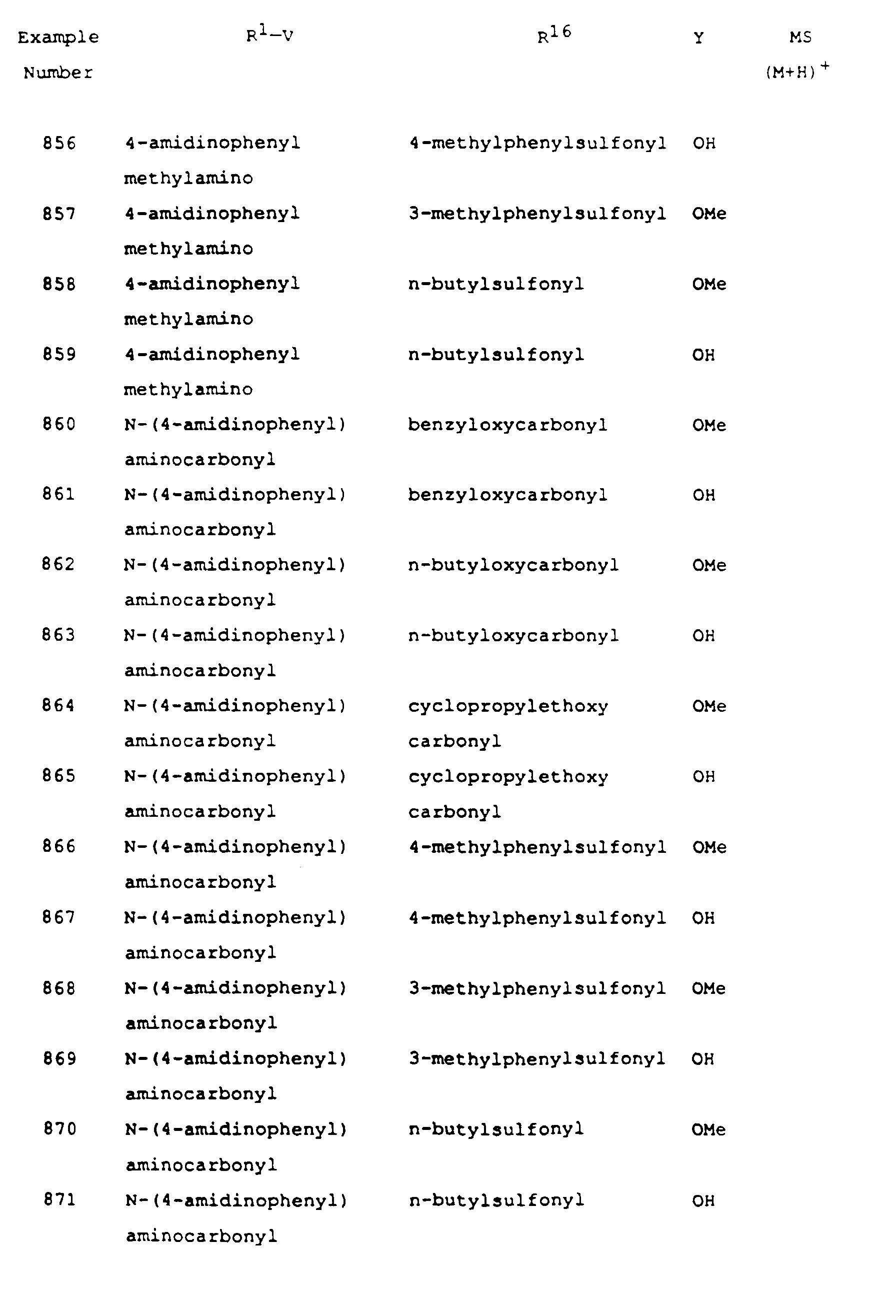

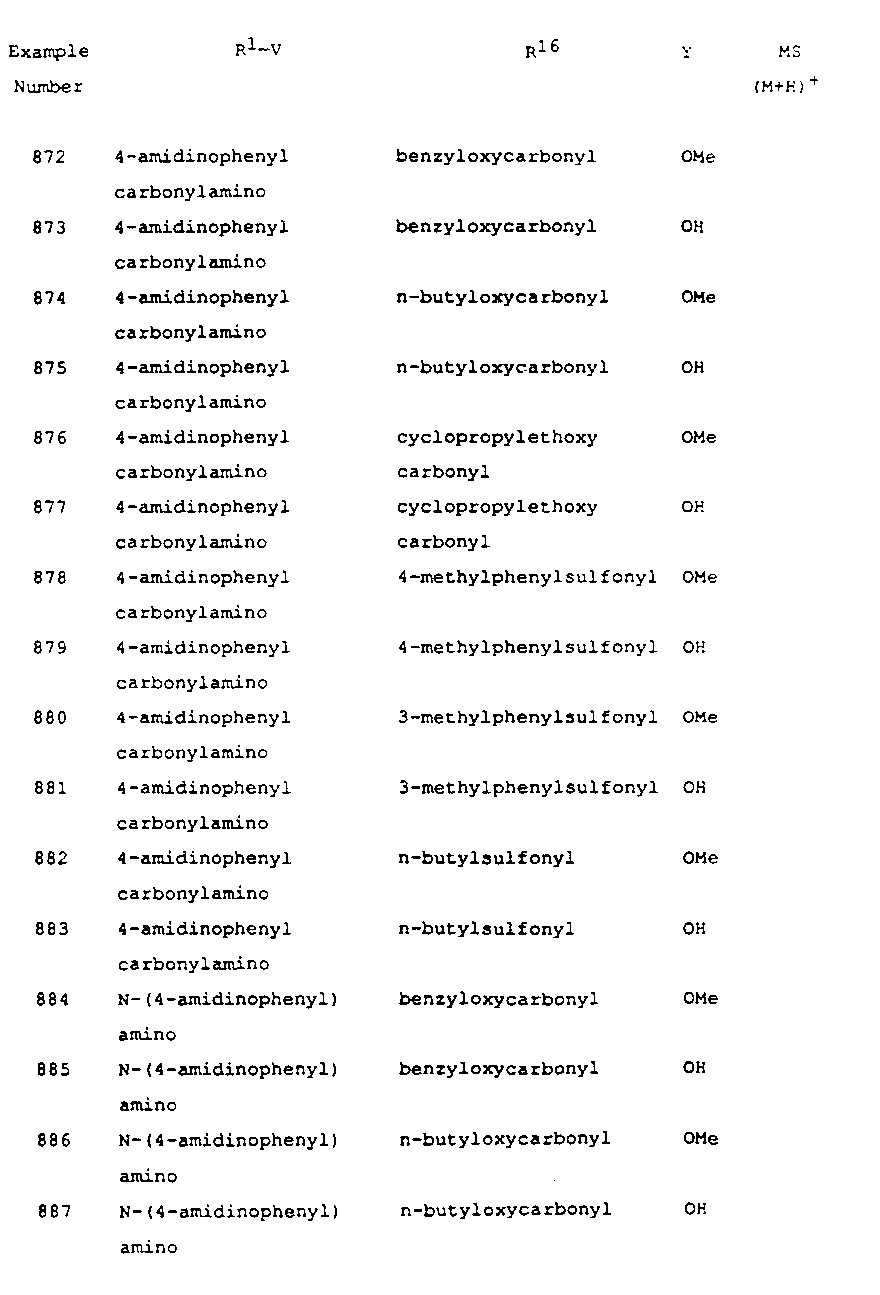

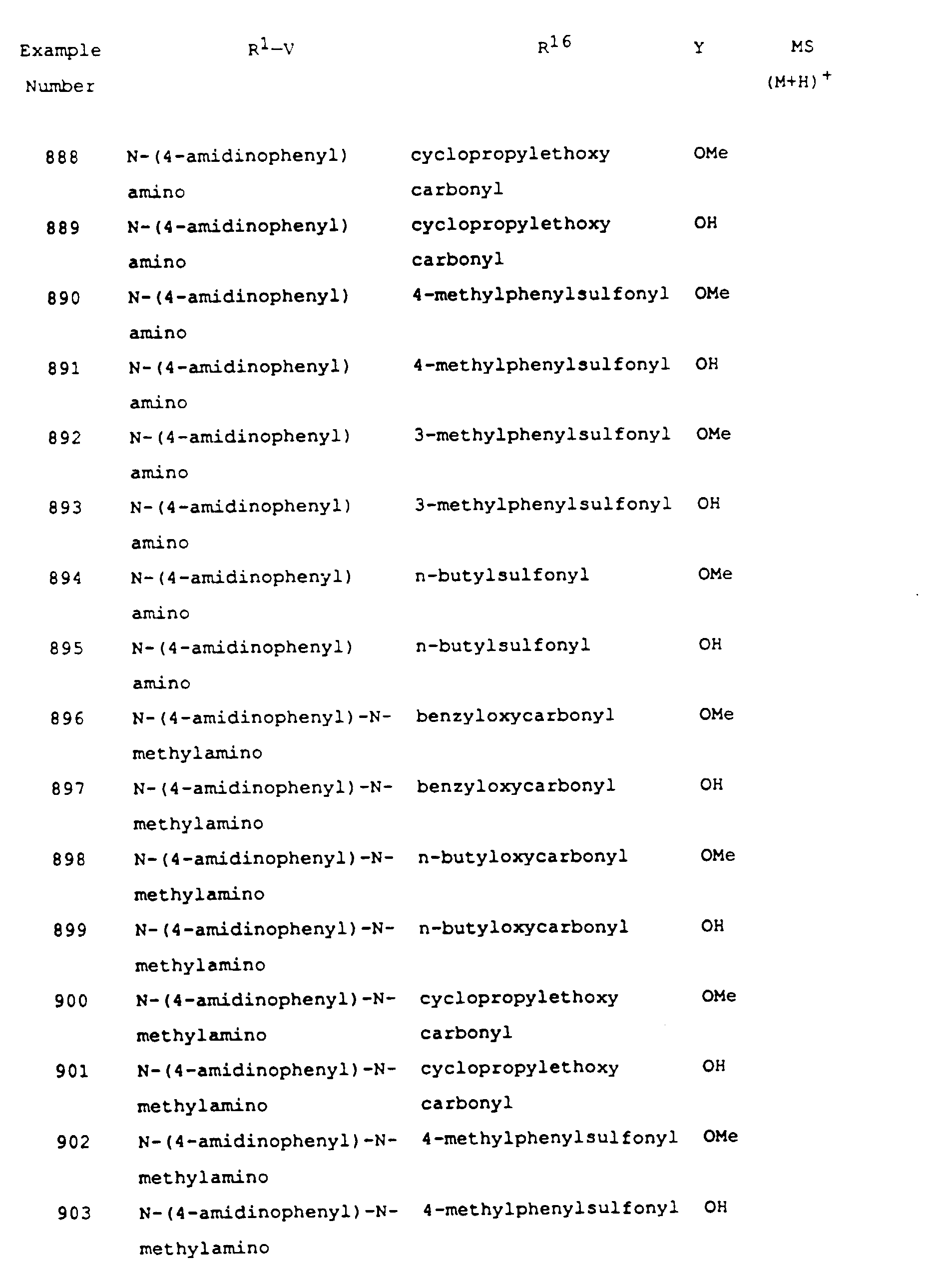

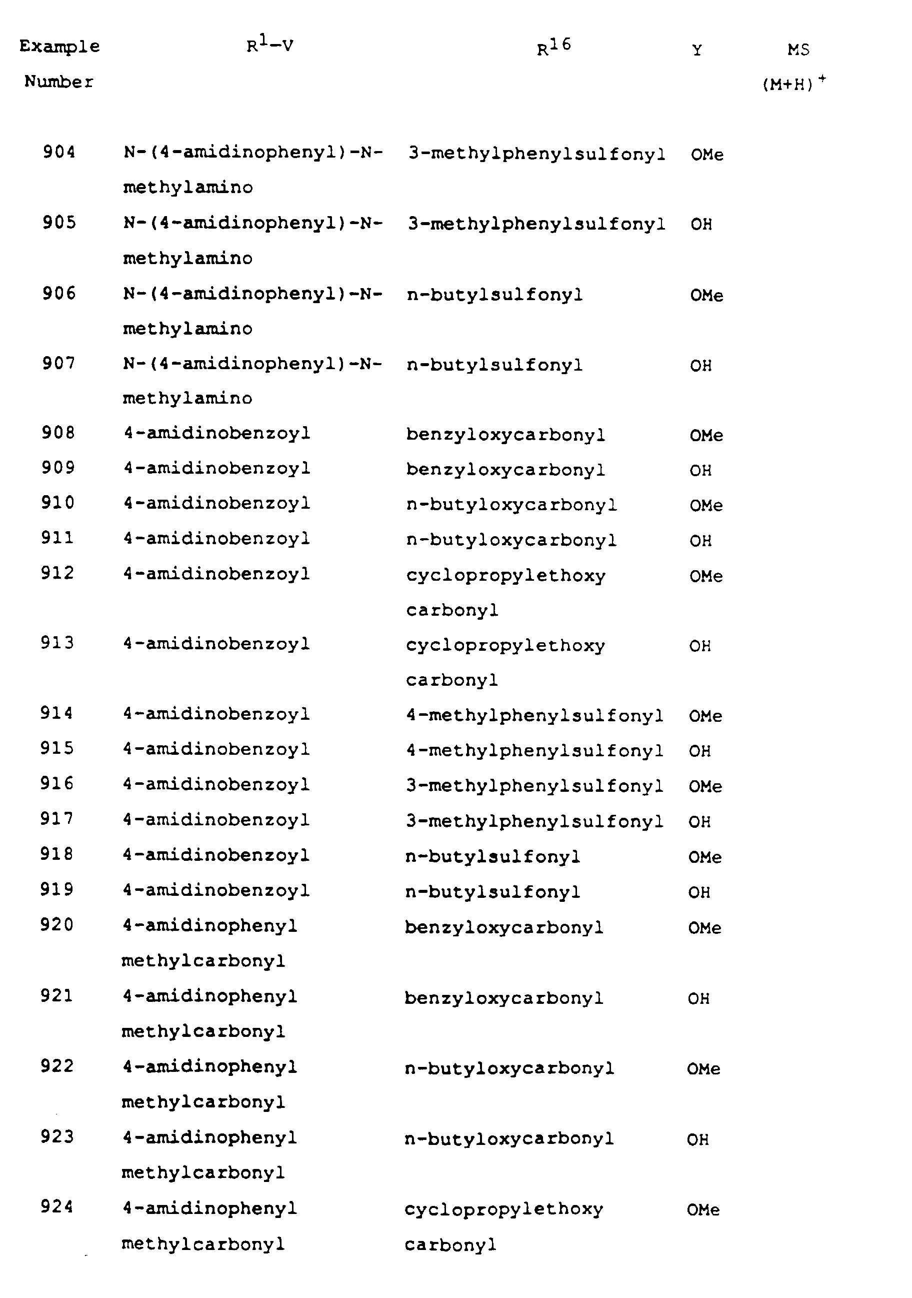

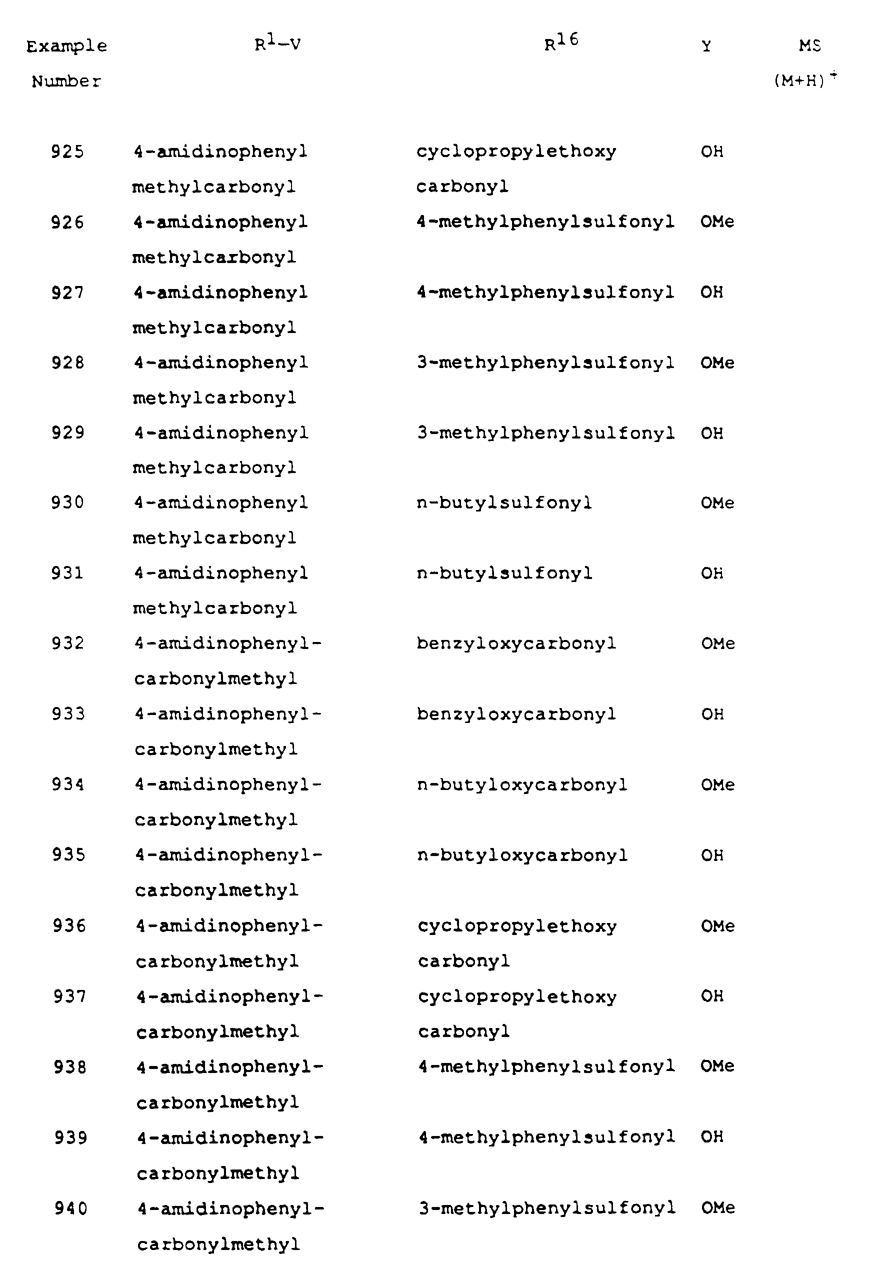

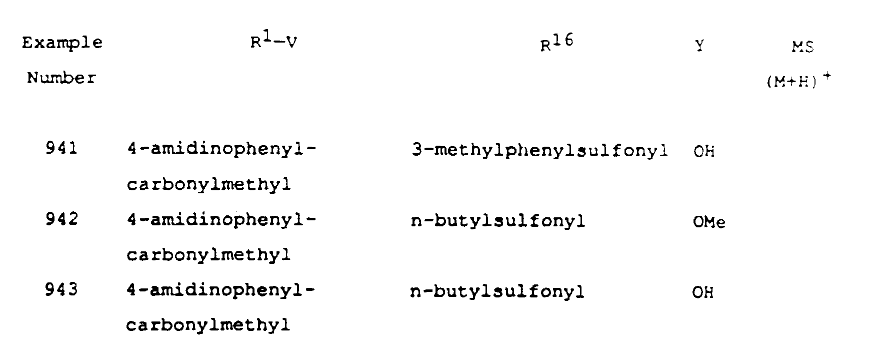

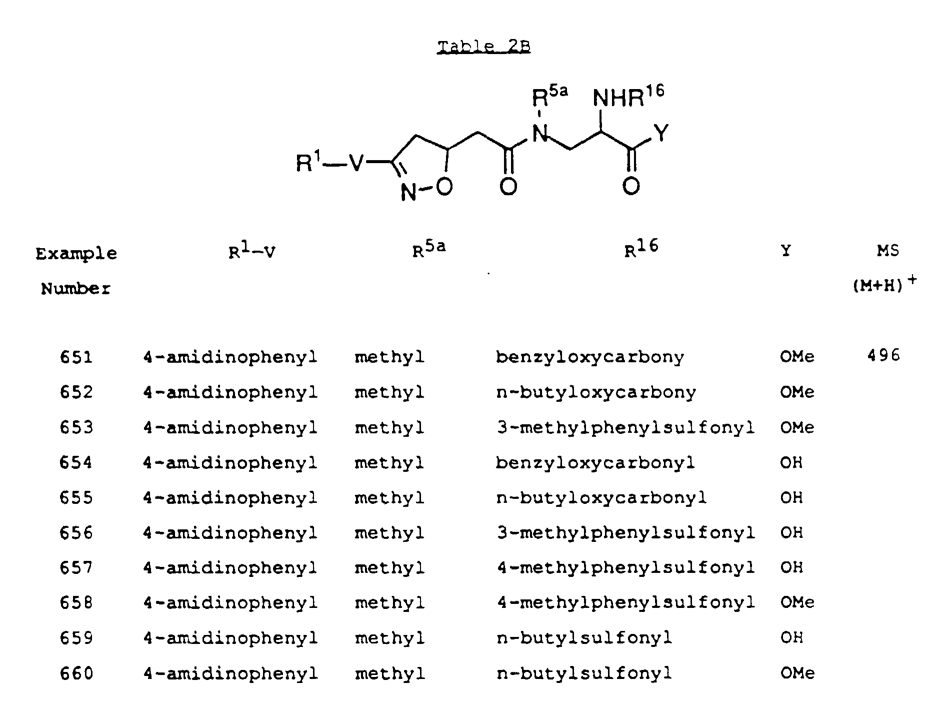

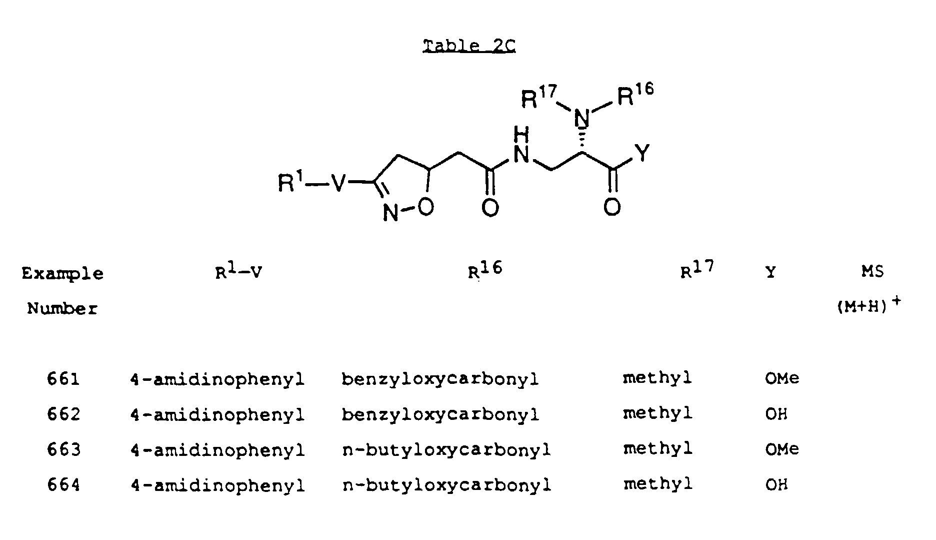

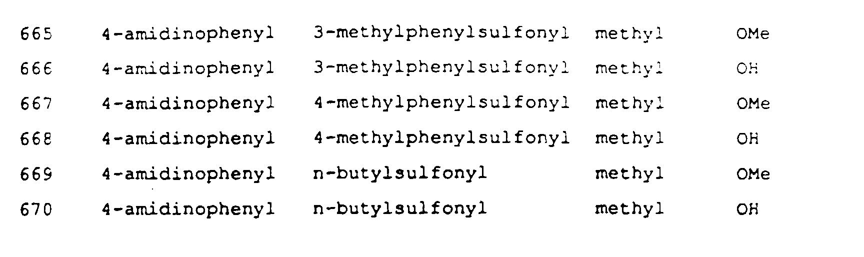

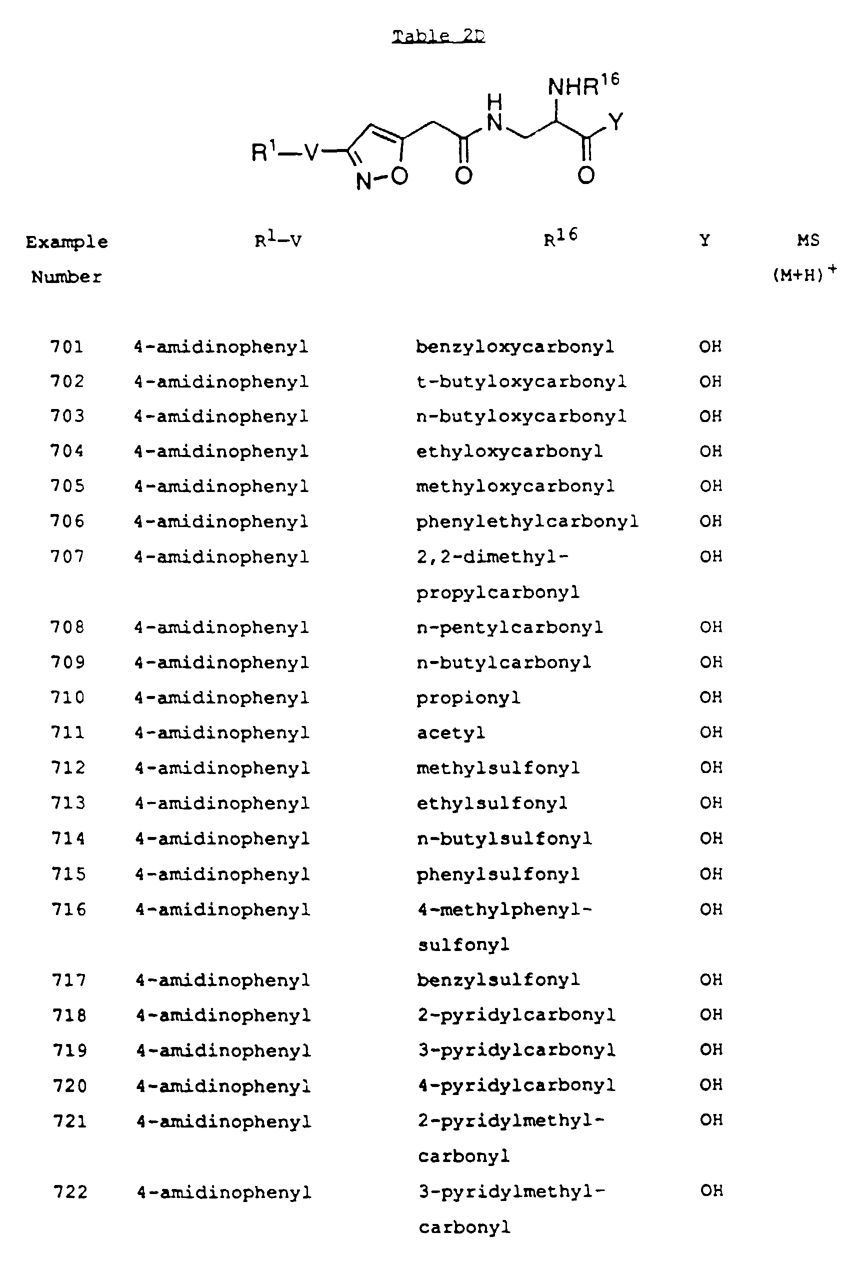

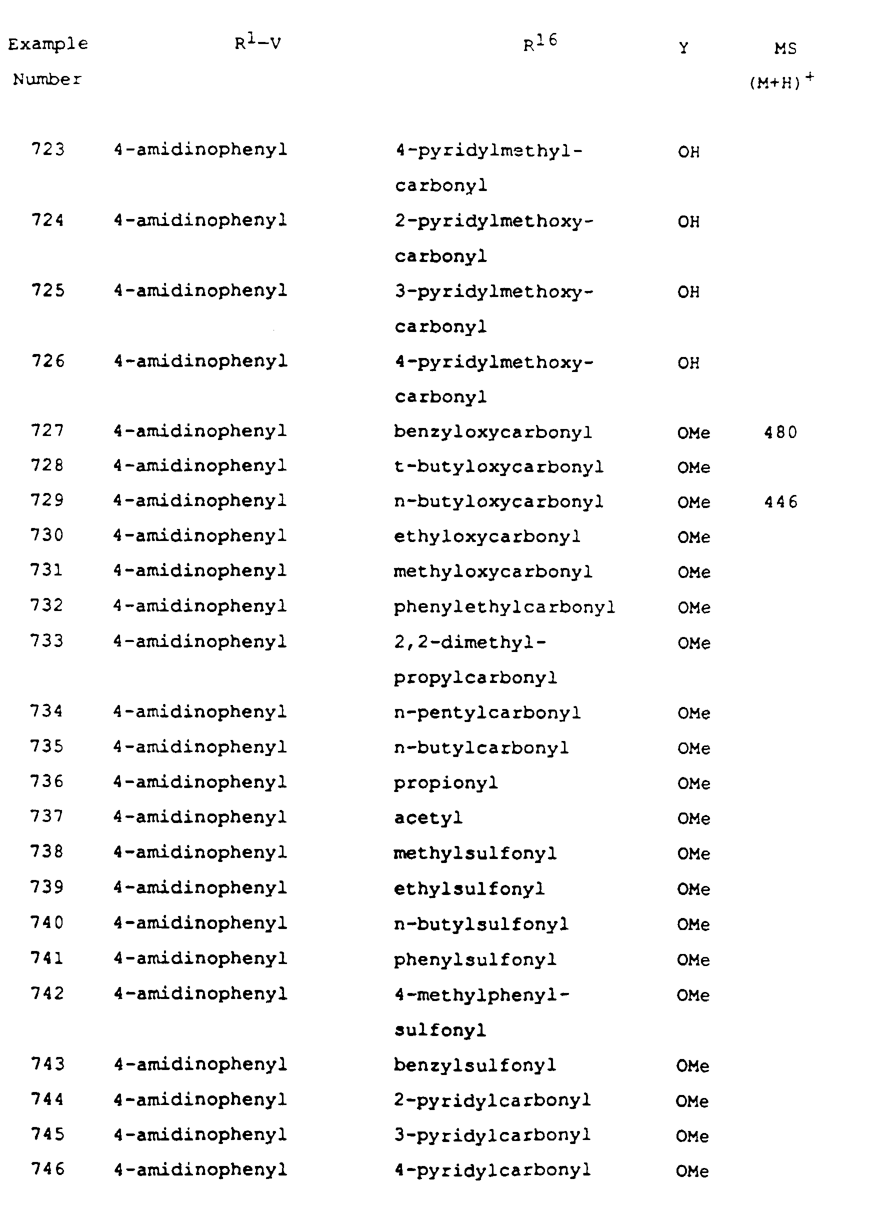

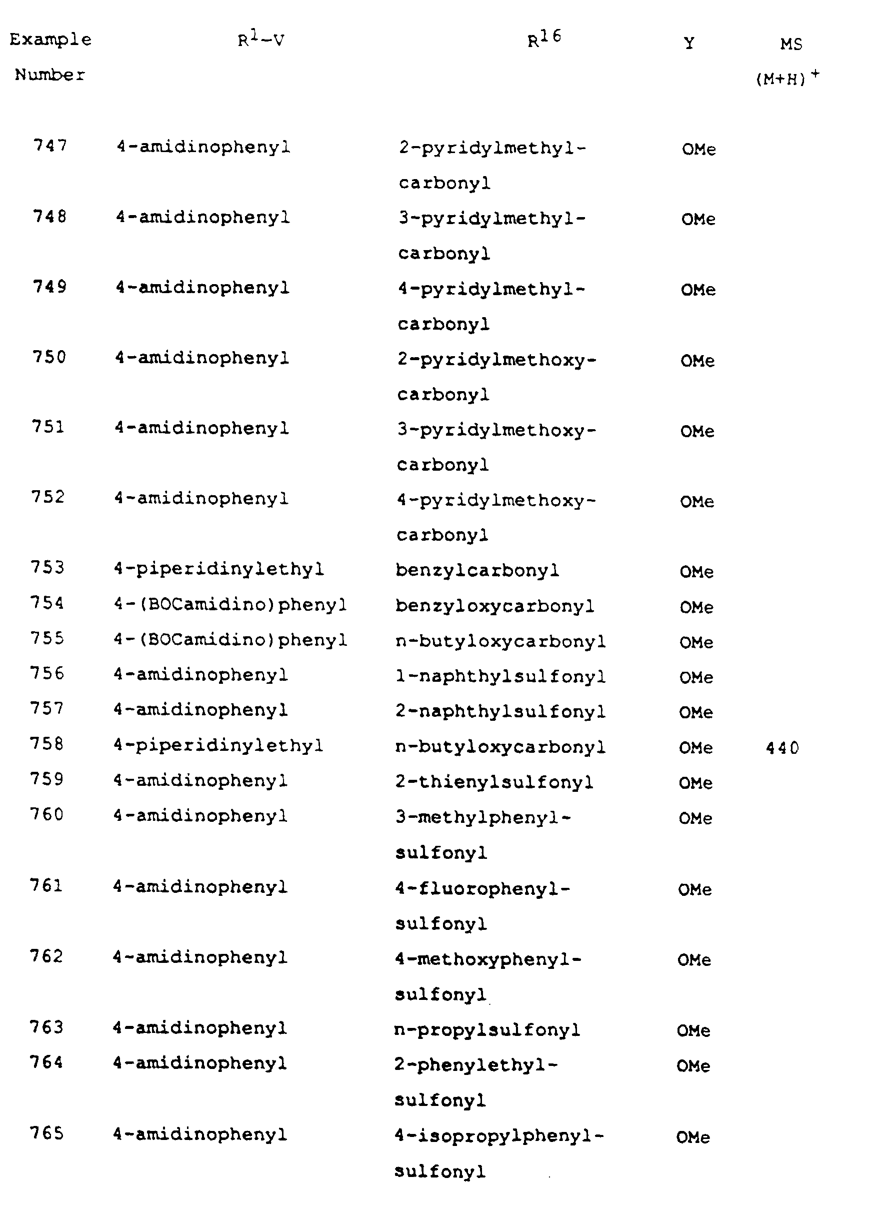

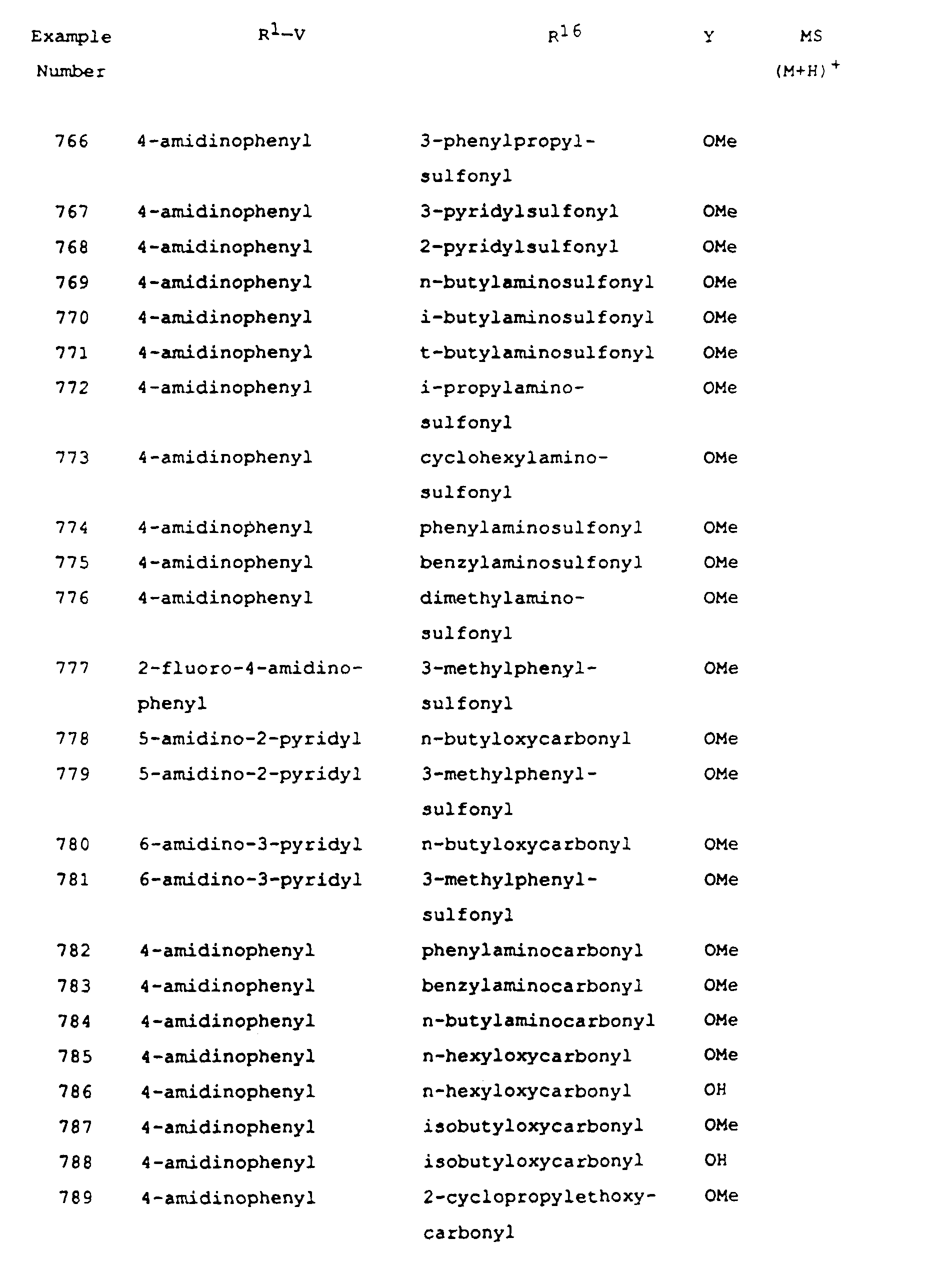

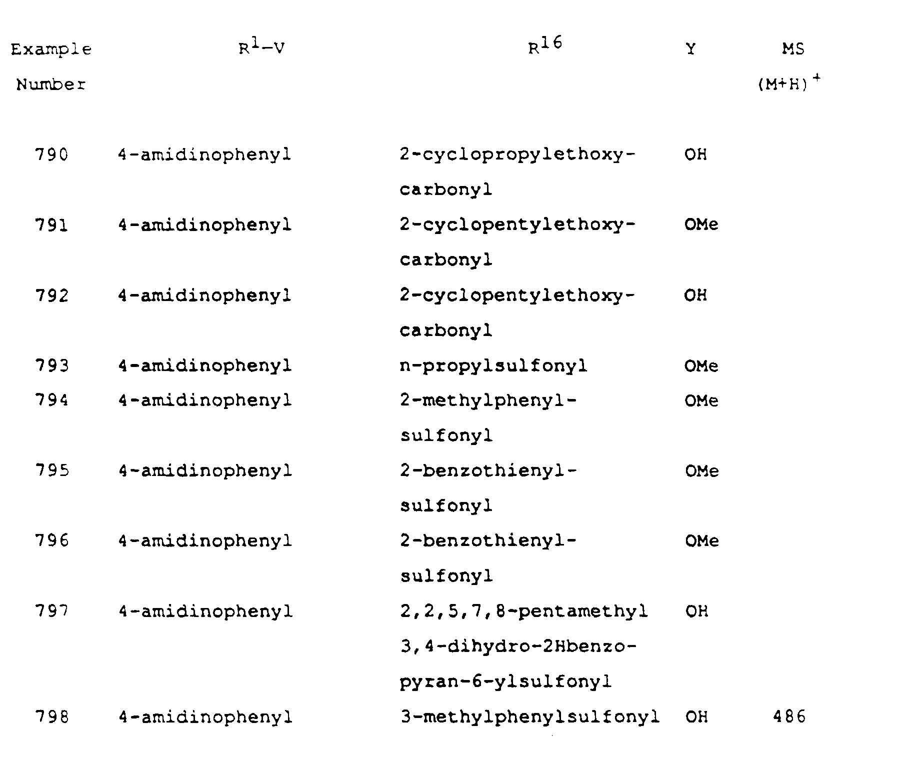

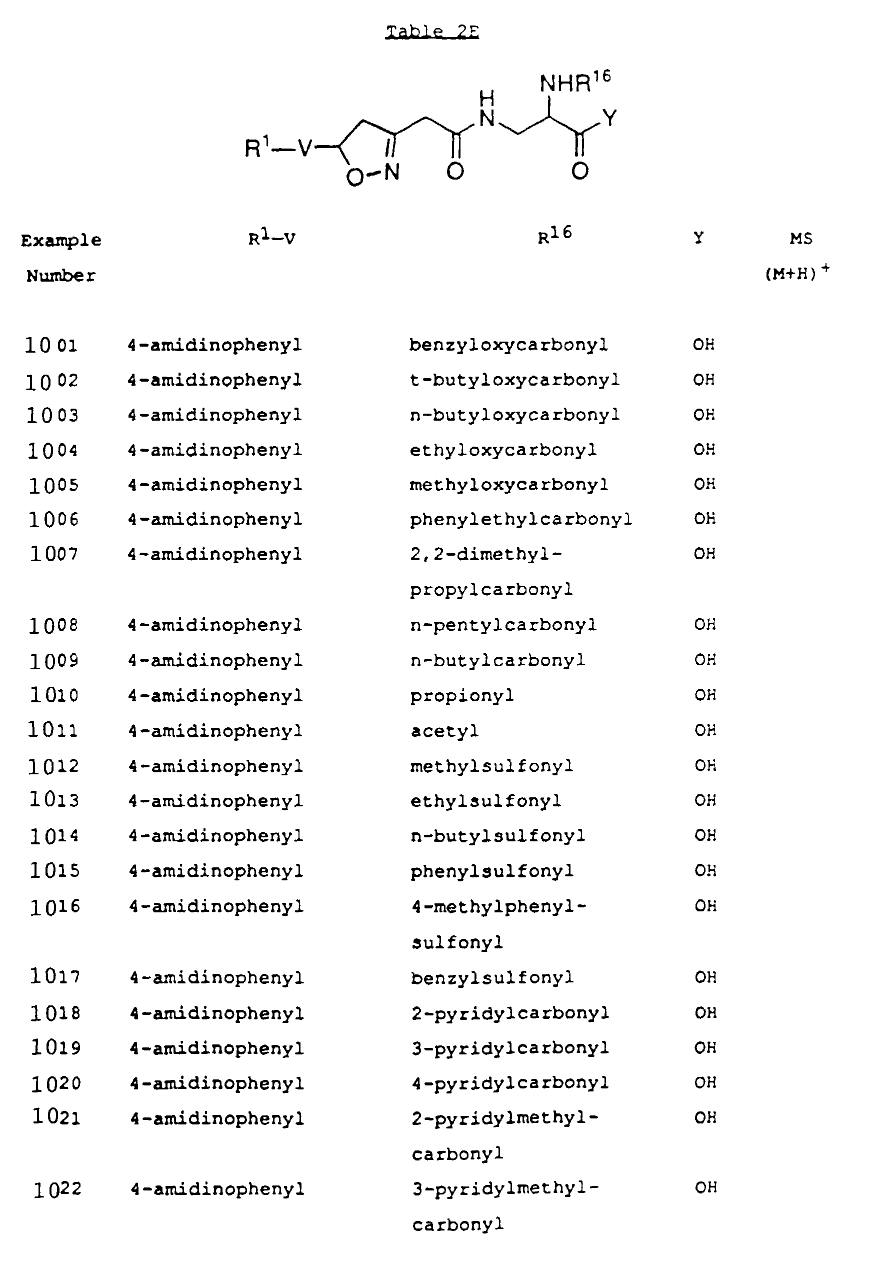

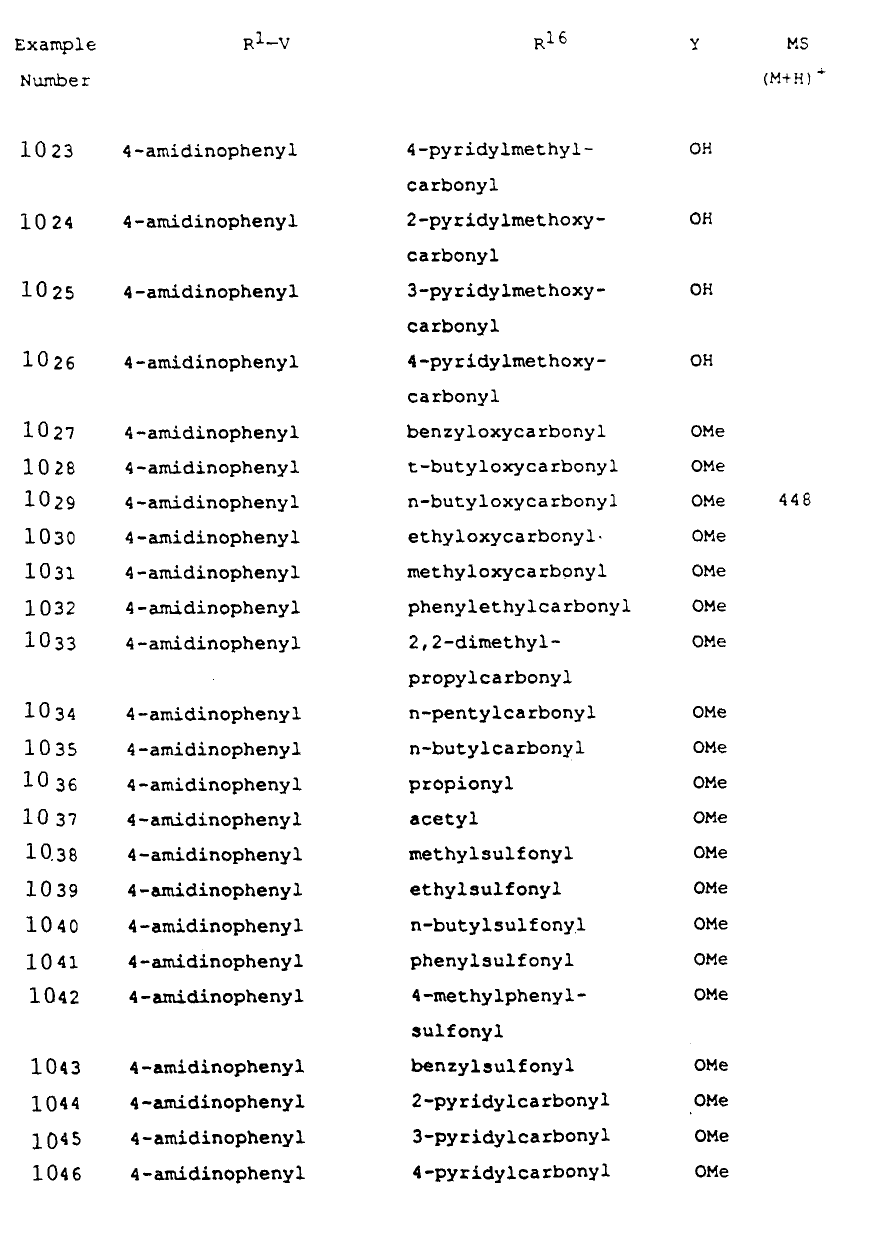

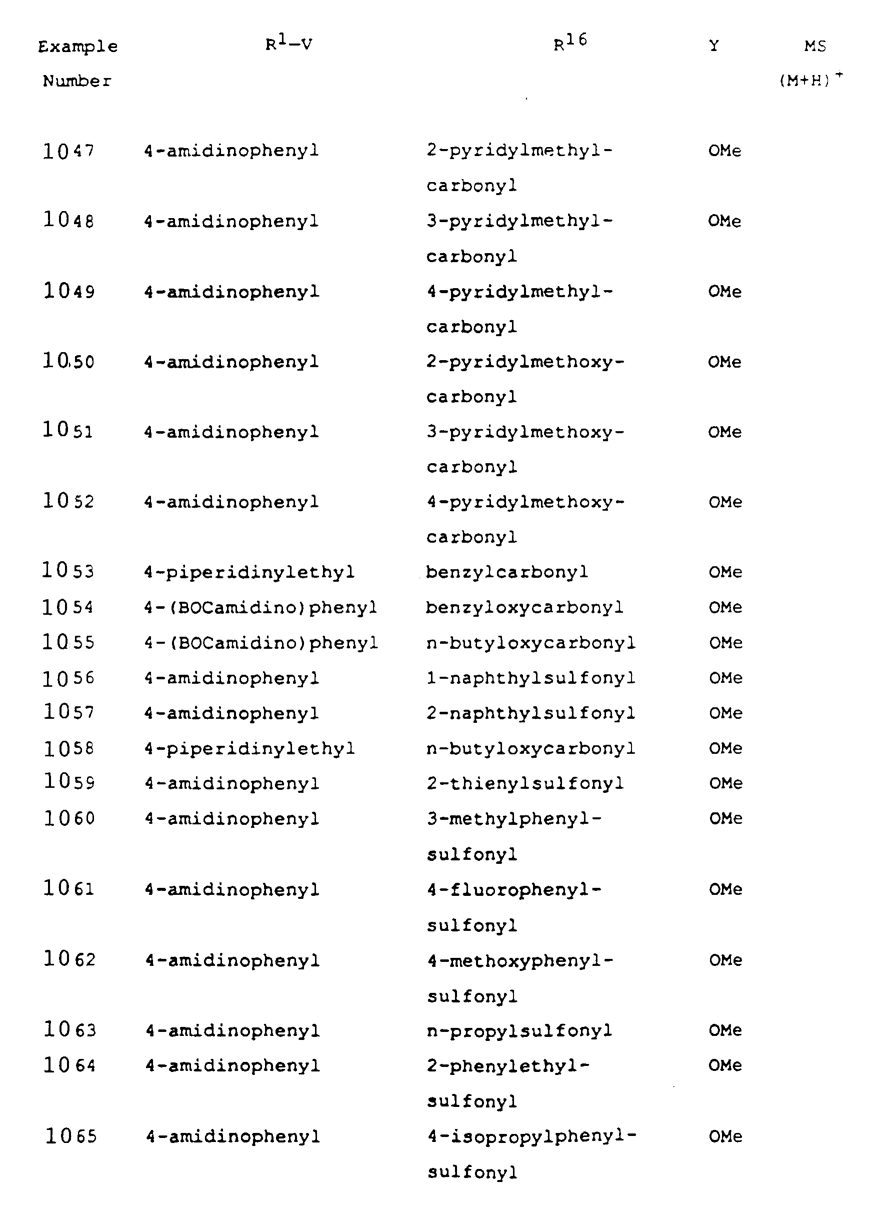

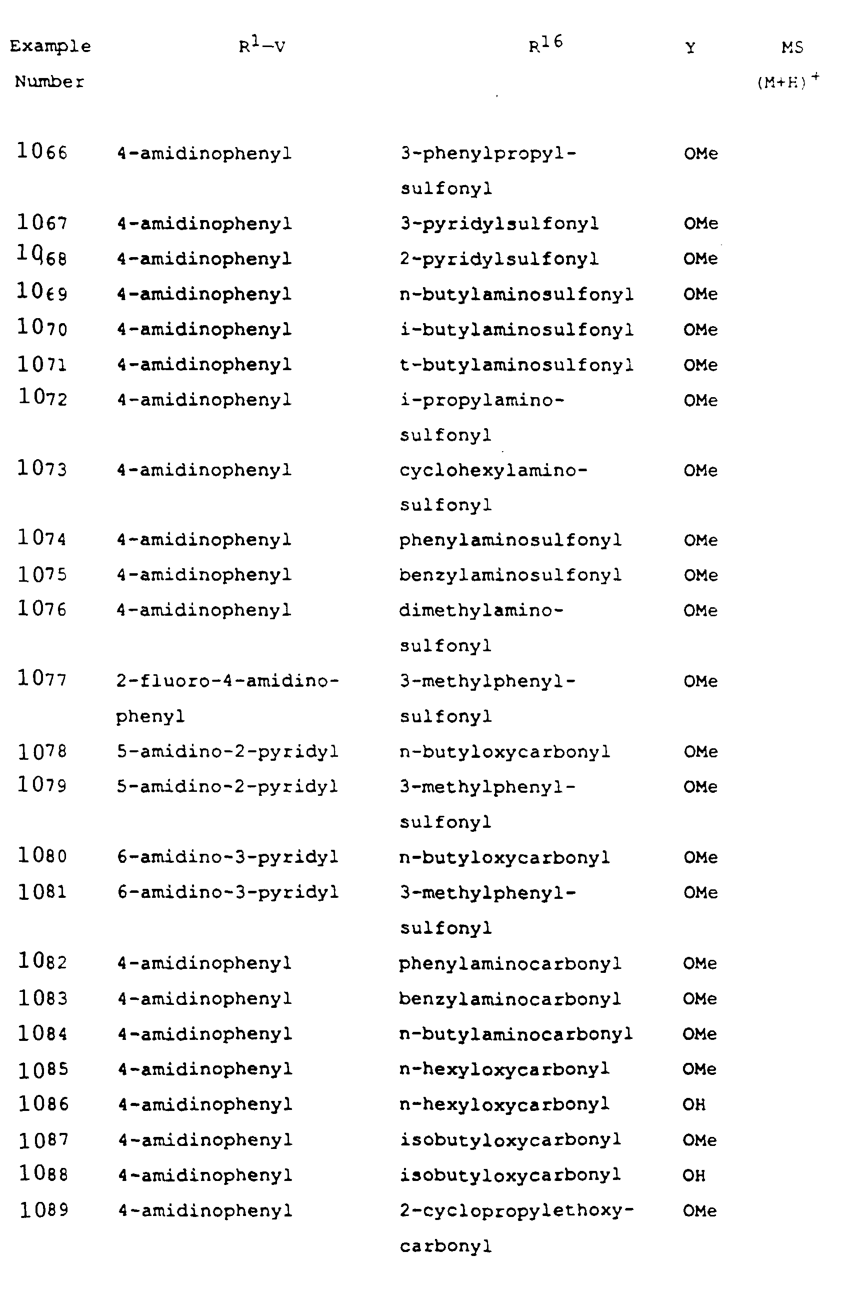

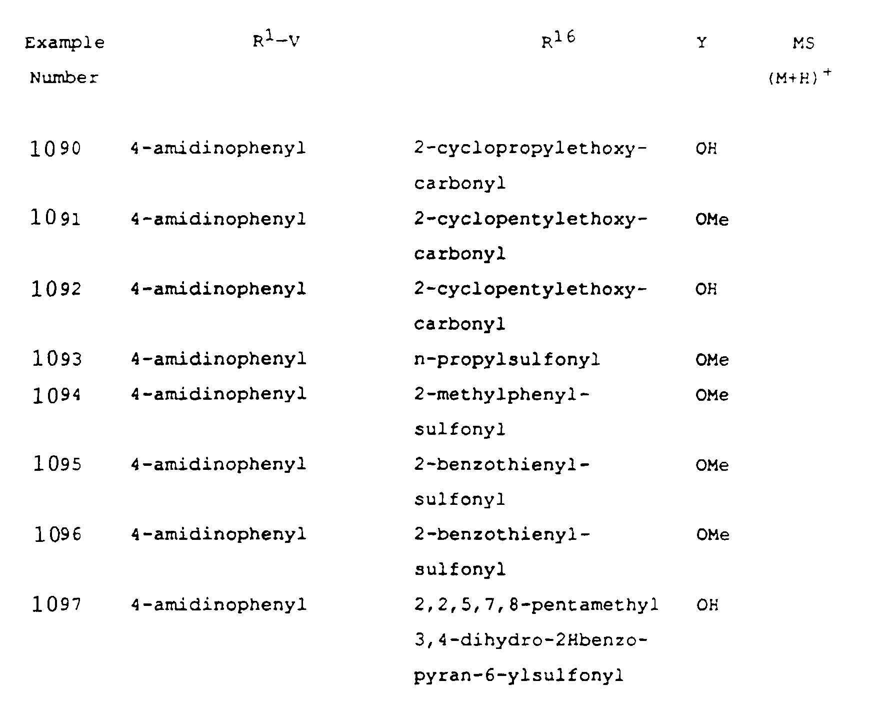

### Utility

The compounds of this invention possess antiplatelet efficacy, as evidenced by their activity in standard platelet aggregation assays or platelet fibrinogen binding assays, as described below. A compound is considered to be active in these assays if it has an IC₅₀ value of less than about 1 mM. Platelet aggregation and fibrinogen binding assays which may be used to demonstrate the antiplatelet activity of the compounds of the invention are described below.

Platelet Aggregation Assay: Venous blood was obtained from the arm of a healthy human donor who was drug-free and aspirin-free for at least two weeks prior to blood collection. Blood was collected into 10 ml citrated Vacutainer tubes. The blood was centrifuged for 15 minutes at 150 x g at room temperature, and platelet-rich plasma (PRP) was removed. The remaining blood was centrifuged for 15 minutes at 1500 x g at room temperature, and platelet-poor plasma (PPP) was removed. Samples were assayed on a aggregometer (PAP-4 Platelet Aggregation Profiler), using PPP as the blank (100% transmittance). 200 µl of PRP was added to each micro test tube, and transmittance was set to 0%. 20 µ of various agonists (ADP, collagen, arachidonate, epinephrine, thrombin) were added to each tube, and the aggregation profiles were plotted (% transmittance versus time). The results are expressed as % inhibition of agonist-induced platelet aggregation. For the IC₅₀ evaluation, the test compounds were added at various concentrations prior to the activation of the platelets.

Ester prodrugs were preincubated (10⁻³ M F.C.) with 100 IU/ml Porcine liver esterase (Sigma Chemical Co., St. Louis, MO, #E-3128) for 2 hours at 37 °C. Aliquots are then diluted in 0.1 M Tris, pH 7.4, to the desired concentrations. Aliquots of 20 µl of the esterase pretreated prodrugs are added to 200 µl of human platelet rich plasma. Samples were placed in platelet profiler (aggregometer) for 8 minutes at 37 °C, followed by the addition of 100 µM Adenosine Diphosphate, (Sigma Chemical Co., St. Louis, MO, #A-6521), to induce platelet aggregation. Platelet aggregation was allowed to proceed for 5 minutes. Percent inhibition is calculated using percent aggregation in the presence of the test compound divided by percent aggregation of control, times 100. This value is subtracted from 100, yielding percent inhibition. Calculation of IC50 is performed on a Texas Instruments TI59 with an IC50 program.

### Purified GPIIb/IIIa-Fibrinogen Binding ELISA

The following reagents are used in the GPIIb/IIIa-fibrinogen binding ELISA:
purified GPIIb/IIIa (148.8 µg/ml);
biotinylated fibrinogen (- 1 mg/ml or 3000 nM);
anti-biotin alkaline phosphatase conjugate (Sigma no. A7418);
flat-bottom, high binding, 96-well plates (Costar Cat. no. 3590);
phosphatase substrate (Sigma 104) (40 mg capsules);
bovine serum albumin (BSA) (Sigma no. A3294);
Alkaline Phosphatase buffer - 0.1 M glycine-HCl, 1 mM MgCl₂.6H₂O, 1 mM ZnCl₂, pH 10.4;
Binding buffer - 20 mM Tris-HCl, 150 mM NaCl, 1 mM CaCl₂.2H₂O, 0.02% NaN₃, pH 7.0;
Buffer A - 50 mM Tris-HCl, 100 mM NaCl, 2 mM CaCl₂.2H₂O, 0.02% NaN₃, pH 7.4;
Buffer A + 3.5% BSA (Blocking buffer);
Buffer A + 0.1% BSA (Dilution buffer); 2N NaOH.

The following method steps are used in the GPIIb/IIIa-fibrinogen binding ELISA:
Coat plates with GPIIb/IIIa in Binding buffer (125 ng/100 µl/well) overnight at 4 °C (Leave first column uncoated for non-specific binding). Cover and freeze plates at -70 °C until used. Thaw plate 1 hour at room temperature or overnight at 4 °C. Discard coating solution and wash once with 200 µl Binding buffer per well. Block plate 2 hours at room temperature on shaker with 200 µl Buffer A + 3.5% BSA (Blocking buffer) per well. Discard Blocking buffer and wash once with 200 µl Buffer A + 0.1% BSA (Dilution buffer) per well. Pipet 11 µl of test compound (10X the concentration to be tested in Dilution buffer) into duplicate wells. Pipet 11 µl Dilution buffer into non-specific and total binding wells. Add 100 µl Biotinylated fibrinogen (1/133 in Dilution buffer, final concentration = 20 nM) to each well. Incubate plates for 3 hours at room temperature on a plate shaker. Discard assay solution and wash twice with 300 µl Binding buffer per well. Add 100 µl Anti-biotin alkaline phosphatase conjugate (1/1500 in Dilution buffer) to each well. Incubate plates for 1 hour at room temperature on plate shaker. Discard conjugate and wash twice with 300 51 Binding buffer per well. Add 100 µl Phosphatase substrate (1.5 mg/ml in Alkaline phosphatase buffer) to each well. Incubate plate at room temperature on shaker until color develops. Stop color development by adding 25 µl 2N NaOH per well. Read plate at 405 nm. Blank against non-specific binding (NSB) well. % Inhibition is calculated as
100 - (Test Compound Abs/Total Abs)x100.

Platelet-Fibrinogen Binding Assay: Binding of ¹²⁵I-fibrinogen to platelets was performed as described by Bennett et al. (1983) Proc. Natl. Acad. Sci. USA 80: 2417-2422, with some modifications as described below. Human PRP (h-PRP) was applied to a Sepharose column for the purification of platelet fractions. Aliquots of platelets (5 X 10⁸ cells) along with 1 m*M* calcium chloride were added to removable 96 well plates prior to the activation of the human gel purified platelets (h-GPP). Activation of the human gel purified platelets was achieved using ADP, collagen, arachidonate, epinephrine, and/or thrombin in the presence of the ligand, ¹²⁵I-fibrinogen. The ¹²⁵I-fibrinogen bound to the activated platelets was separated from the free form by centrifugation and then counted on a gamma counter. For an IC₅₀ evaluation, the test compounds were added at various concentrations prior to the activation of the platelets.

The compounds of Formula I of the present invention may also possess thrombolytic efficacy, that is, they are capable of lysing (breaking up) already formed platelet-rich fibrin blood clots, and thus are useful in treating a thrombus formation, as evidenced by their activity in the tests described below. Preferred compounds of the present invention for use in thrombolysis include those compounds having an IC₅₀ value (that is, the molar concentration of the compound capable of achieving 50% clot lysis) of less than about 1 µM, more preferably an IC₅₀ value of less than about 0.1 µM.

Thrombolytic Assay: Venous blood was obtained from the arm of a healthy human donor who was drug-free and aspirin free for at least two weeks prior to blood collection, and placed into 10 ml citrated Vacutainer tubes. The blood was centrifuged for 15 minutes at 1500 x g at room temperature, and platelet rich plasma (PRP) was removed. To the PRP was then added 1 x 10⁻³ M of the agonist ADP, epinephrine, collagen, arachidonate, serotonin or thrombin, or a mixture thereof, and the PRP incubated for 30 minutes. The PRP was centrifuged for 12 minutes at 2500 x g at room temperature. The supernatant was then poured off, and the platelets remaining in the test tube were resuspended in platelet poor plasma (PPP), which served as a plasminogen source. The suspension was then assayed on a Coulter Counter (Coulter Electronics, Inc., Hialeah, FL), to determine the platelet count at the zero time point. After obtaining the zero time point, test compounds were added at various concentrations. Test samples were taken at various time points and the platelets were counted using the Coulter Counter. To determine the percent of lysis, the platelet count at a time point subsequent to the addition of the test compound was subtracted from the platelet count at the zero time point, and the resulting number divided by the platelet count at the zero time point. Multiplying this result by 100 yielded the percentage of clot lysis achieved by the test compound. For the IC₅₀ evaluation, the test compounds were added at various concentrations, and the percentage of lysis caused by the test compounds was calculated.

The compounds of Formula I of the present invention are also useful for administration in combination with anti-coagulant agents such as warfarin or heparin, or antiplatelet agents such as aspirin, piroxicam or ticlopidine, or thrombin inhibitors such as boropeptides, hirudin or argatroban, or thrombolytic agents such as tissue plasminogen activator, anistreplase, urokinase or streptokinase, or combinations thereof.

The compounds of Formula I of the present invention may also be useful as antagonists of other integrins such as for example, the αᵥ/β₃ or vitronectin receptor, α₄/β₁ or α₅/β₁ and as such may also have utility in the treatment and diagnosis of osteoporosis, cancer metastasis, diabetic retinopathy, rheumatoid arthritis, inflammation, and autoimmune disorders. The compounds of Formula I of the present invention may be useful for the treatment or prevention of other diseases which involve cell adhesion processes, including, but not limited to, infammation, bone degradation, rheumatoid arthritis, asthma, allergies, adult respiratory distress syndrome, graft versus host disease, organ transplantation, septic shock, psoriasis, eczema, contact dermatitis, osteoporosis, osteoarthritis, atherosclerosis, metastasis, wound healing, diabetic retinopathy, inflammatory bowel disease and other autoimmune diseases.

Table A below sets forth the antiplatelet activity of representative compounds of the present invention. The indicated compounds were tested for their ability to inhibit platelet aggregation (using platelet rich plasma (PRP)). The IC₅₀ value (the concentration of antagonist which inhibits platelet aggregation by 50% relative to a control lacking the antagonist) is shown. In Table 5 the IC₅₀ values are expressed as: +++ = IC₅₀ of <10 µM; ++ = IC₅₀ of 10-50 µM; + = IC₅₀ of 50-100 µM (µM = micromolar).

**Table A**

| Example Number | Platelet Aggregation Assay IC₅₀ (without esterase) | Platelet Aggregation Assay IC₅₀ (with esterase) |
|---|---|---|
| 1 | +++ | |
| 4 (isomer A) | ++ | |
| 4 (isomer B) | ++ | |
| 6 | +++ | |
| 7 | >100 | |
| 8 | + | |
| 9 (isomer A) | +++ | |
| 9 (isomer B) | +++ | |
| 43 | +++ | |
| 89 | | +++ |
| 115 | | +++ |
| 119A (3R) | | +++ |
| 119B (3S) | | +++ |
| 120A (3R) | | +++ |
| 120B (3S) | | +++ |
| 120C (3R)↑↑ | | +++ |
| 166 | | +++ |
| | | |
| 275 | | +++ |
| 276 | | +++ |
| 278 | | +++ |
| 290 | | +++ |
| 300 | | +++ |
| 312 | | +++ |
| 314A (2S)↑ | | +++ |
| 314B (2S)↑↑ | | +++ |
| 323 | | +++ |
| 324 | | +++ |
| 326 | | +++ |
| 327 (2S) | | +++ |
| 328 (2S) | | +++ |
| 338 (3S) | + | +++ |
| 339 (3S) | +++ | |
| 340 (3S) | + | ++ |
| 341 (3S) | +++ | |
| 342 (2S) | +++ | |
| 344 (3R) | | +++ |
| 345 | | +++ |
| 347 (3R)↑↑ | | +++ |
| 348 (3R) | | +++ |
| 350 | | +++ |
| 359 | | +++ |
| 362 | | +++ |
| 365 | | +++ |
| 368 | | +++ |
| 373 | ++ | |
| 371A | | +++ |
| 371B | | +++ |
| 374 (2S) | + | +++ |
| | | |
| 439 | | +++ |
| 440 | | +++ |
| 441 | +++ | |
| 442 | | +++ |
| 443 (2S) | | +++ |
| 444 (2S) | | +++ |
| 445 (2S) | | +++ |
| 446 | | +++ |
| 449A | | +++ |
| 449B | | +++ |
| 450 | | +++ |
| 451 | | +++ |
| 452 | | +++ |
| 453 | | +++ |
| 454 | | +++ |
| 455 | | +++ |
| 456 | | ++ |
| 457 | | +++ |
| 458A | +++ | |
| 458B | +++ | |
| 460A | +++ | |
| 460B | +++ | |
| 462 | | +++ |
| 463 | +++ | |
| 464 | | +++ |
| 465 | | +++ |
| 466 | | +++ |
| 467 | | +++ |
| 468 | | +++ |
| 469 | | +++ |
| 470 | | +++ |
| 471 | | +++ |
| 472 | | +++ |
| 473 | | +++ |
| 473A (2S)↑ | | +++ |
| 473B (2S)↑↑ | | +++ |
| 474 | | +++ |
| 475 | | +++ |
| 476 | | +++ |
| 477 | | +++ |
| 478 | | +++ |
| 479 | | +++ |
| 480 | | +++ |
| 481 | | +++ |
| 482 | | +++ |
| 483 | | +++ |
| 484 | | +++ |
| 485 | | +++ |
| 486 | | +++ |
| 487 | | +++ |
| 488 | | +++ |
| 489 | | +++ |
| 490 | | +++ |
| 491 | | +++ |
| 492 | | +++ |
| 493 | | +++ |
| 494 | | +++ |
| 495 | | +++ |
| 496 | | +++ |
| 504 | | +++ |
| 505 | | +++ |
| 506 | | +++ |
| 507 | | +++ |
| 508 | | +++ |
| 509 | | +++ |
| 510 | | +++ |
| 511 | | +++ |
| 512 | | +++ |
| 513 | | +++ |
| 514 | | +++ |
| 515 | | +++ |
| 516 | | +++ |
| 517 | | +++ |
| 518 | | +++ |
| 519 | | +++ |
| 520 | | +++ |
| 522 | | +++ |
| 523 | | +++ |
| 524 | | +++ |
| 525 | | +++ |
| 526 | +++ | |
| 527 | | +++ |
| 528 | +++ | |
| 529 | | +++ |
| 530 | +++ | |
| 531 | | +++ |
| 532 | +++ | |
| 533 | | +++ |
| 534 | +++ | |
| 535 | | +++ |
| 536 | +++ | |
| 537 | | +++ |
| 538 | +++ | |
| 539 | | +++ |
| 540 | | +++ |
| 541 | | +++ |
| 542 | | +++ |
| 543 | | +++ |
| 544 | | +++ |
| 545 | | +++ |
| 546 | | +++ |
| 547 | | +++ |
| 548 | | +++ |
| 549 | | +++ |
| 550 | | +++ |
| 551 | | +++ |
| 552 | | +++ |
| 553 | | +++ |
| 554 | | +++ |
| 555 | | +++ |
| 556 | | +++ |
| 587A (2S)↑↑ | +++ | |
| 588 | | +++ |
| 602 | | +++ |
| 611 | | +++ |
| 612 | | +++ |
| 613 | | +++ |
| 616 | +++ | |
| 651 | | +++ |
| 727 | | +++ |
| 729 | | +++ |
| 798 | | +++ |

| | | |
|---|---|---|
| * Single diastereomer, racemic ↑ S isomer at C5 of isoxazoline ring | | |
| ↑↑ R isomer at C5 of isoxazoline ring | | |

### Dosage and Formulation

The compounds of the present invention can be administered in such oral dosage forms as tablets, capsules (each of which includes sustained release or timed release formulations), pills, powders, granules, elixirs, tinctures, suspensions, syrups, and emulsions. Likewise, they may also be administered in intravenous (bolus or infusion), intraperitoneal, subcutaneous, or intramuscular form, all using dosage forms well known to those of ordinary skill in the pharmaceutical arts. An effective but non-toxic amount of the compound desired can be employed as an anti-aggregation agent.

Dosage forms (pharmaceutical compositions) suitable for administration may contain from 1 milligram to 100 milligrams of active ingredient per dosage unit. In these pharmaceutical compositions the active ingredient will ordinarily be present in an amount of 0.5-95% by weight based on the total weight of the composition.

The active ingredient can be administered orally in solid dosage forms, such as capsules, tablets, and powders, or in liquid dosage forms, such as elixirs, syrups, and suspensions. It can also be administered parenterally, in sterile liquid dosage forms.

Gelatin capsules may contain the active ingredient and powdered carriers, such as lactose, starch, cellulose derivatives, magnesium stearate and stearic acid. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

Suitable pharmaceutical carriers are described in Remington's Pharmaceutical Sciences, Mack Publishing Company, a standard reference text in this field.

The compound of Formula I may be formulated together with the second therapeutic agent in a single dosage unit (that is, combined together in one capsule, tablet, powder, or liquid). When the compound of Formula I and the second therapeutic agent are not formulated together in a single dosage unit, the compound of Formula I and the second therapeutic agent (anti-coagulant agent, anti-platelet agent, thrombin inhibitor, and/or thrombolytic agent) may be administered essentially at the same time, or in any order; for example the compound of Formula I may be administered first, followed by administration of the second agent (anti-coagulant agent, anti-platelet agent, thrombin inhibitor, and/or thrombolytic agent). When not administered at the same time, preferably the administration of the compound of Formula I and the second therapeutic agent occurs less than about one hour apart.

The present invention also includes pharmaceutical kits useful, for example, in the inhibition of platelet aggregation, the treatment of blood clots, and/or the treatment of thromboembolic disorders, which comprise one or more containers containing a pharmaceutical composition comprising a therapeutically effective amount of a compound of Formula I. Such kits may further include, if desired, one or more of various conventional pharmaceutical kit components, such as, for example, containers with one or more pharmaceutically acceptable carriers, additional containers, as will be readily apparent to those skilled in the art. Instructions, either as inserts or as labels, indicating quantities of the components to be administered, guidelines for administration, and/or guidelines for mixing the components, may also be included in the kit.

## Claims

1. A compound of Formula I: 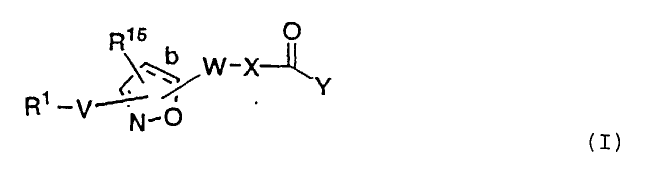 or a pharmaceutically acceptable salt form thereof wherein:
b is a single or double bond;
R¹ is selected from R^{2a}(R³)N- R²(R³)N(R²N=)C-, R^{2a}(R³)N(CH₂)_{p'}Z-, R²(R³)N(R²N=)C(CH₂)_{p"}Z-, R²(R³)N(R²N=)CN(R²)-, 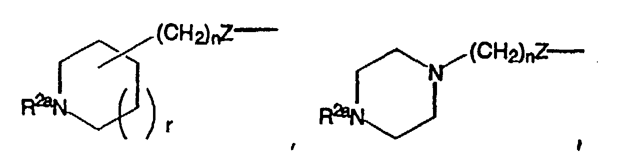 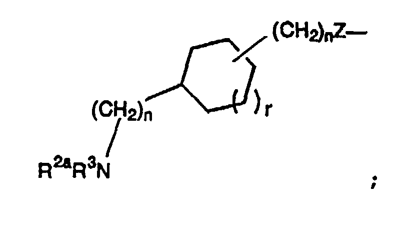
Z is selected from a bond, O, S, S(=O), and S(=O)₂;
R² and R³ are independently selected from H, C₁-C₁₀ alkyl, C₃-C₆ alkenyl, C₃-C₁₁ cycloalkyl, C₄-C₁₁ cycloalkylalkyl, C₆-C₁₀ aryl, C₇-C₁₁ arylalkyl, C₂-C₇ alkylcarbonyl, C₇-C₁₁ arylcarbonyl, C₂-C₁₀ alkoxycarbonyl, C₄-C₁₁ cycloalkoxycarbonyl, C₇-C₁₁ bicycloalkoxycarbonyl, C₇-C₁₁ aryloxycarbonyl, aryl (C₁-C₁₀ alkoxy)carbonyl, (C₁-C₆ alkyl) carbonyloxy (C₁-C₄ alkoxy) carbonyl, arylcarbonyloxy (C₁-C₄ alkoxy)carbonyl, and (C₄-C₁₁ cycloyalkylcarbonyl)oxy (C₁-C₄ alkoxy) carbonyl;
R^{2a} is R² or R²(R³)N(R²N=)C- ;
V is selected from a single bond, -(phenyl)-Q-, -(pyridyl)-Q- and -(pyridazinyl)-Q-, said phenyl, pyridyl and pyridazinyl residues being substituted with 0-2 groups independently selected from H, C₁-C₁₀ alkyl, hydroxy, C₁-C₁₀ alkoxy, nitro, (C₁-C₁₀ alkyl)carbonyl, -N(R¹²)R¹³, cyano, and halo;
Q is selected from a single bond, (CH₂)ₘ-, -CH₂O-, and -OCH₂-, provided that when b is a single bond, and R¹-V- is a substituent on C5 of the central 5-membered ring of Formula I, then Q is not -CH₂O-;
W is -(C(R⁴)₂)ₙ, -C(=O)-N(R^{5a})- or -C(=O)-N(R^{5a})-(C(R⁴)₂)_{n'}- ;
X is - (C(R⁴)₂)ₙ,-C(R⁴) (R⁸)-C(R⁴) (R^{4a})-, with the proviso that when n' is 0 or 1, then at least one of R^{4a} or R⁸ is other than H or methyl;
Y is selected from hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxycarbonyloxyalkyloxy, C₂-C₁₀ alkoxycarbonylalkyloxy, C₅-C₁₀ cycloalkylcarbonyloxyalkyloxy, C₅-C₁₀ cycloalkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyloxyalkyloxy, C₅-C₁₀ alkoxyalkylcarbonyloxyalkyloxy, C₅-C₁₀ (5-alkyl-1,3-dioxa-cyclopenten-2-one-yl)methyloxy, C₁₀-C₁₄ (5-aryl-1,3-dioxa-cyclopenten-2-one-yl)methyloxy, and (R²) (R³)N-(C₁-C₁₀ alkoxy)-;
R⁴ is selected from H, C₁-C₁₀ alkyl, C₁-C₁₀ alkycarbonyl, aryl, arylalkyl, cycloalkyl, and cycloalkylalkyl;
alternatively, two R⁴ groups on adjacent carbon atoms may join to form a bond thereby to form a carbon-carbon double or triple bond between such adjacent carbon atoms;
R^{4a} is selected from H, hydroxy, C₁-C₁₀ alkoxy, nitro, -N(R⁵)R^{5a}, -N(R¹²)R¹³, -N(R¹⁶)R¹⁷, C₁-C₁₀ alkyl substituted with 0-3 R⁶, aryl substituted with 0-3 R⁶, heteroaryl substituted with 0-3 R⁶, and C₁-C₁₀ alkylcarbonyl;
R^{4b} is selected from H, C₁-C₆ alkyl, C₂-C₆ alkenyl, C₂-C₆ alkynyl, C₃-C₇ cycloalkyl, C₇-C₁₄ bicycloalkyl, hydroxy, C₁-C₆ alkoxy, C₁-C₆ alkylthio, C₁-C₆ alkylsulfinyl, C₁-C₆ alkylsulfonyl, nitro, C₁-C₆ alkylcarbonyl, C₆-C₁₀ aryl, -N(R¹²)R¹³, halo, CF₃, CN, (C₁-C₆ alkoxy)carbonyl, carboxy, piperidinyl, morpholinyl, and pyridinyl;
R⁵ is selected from H, C₁-C₈ alkyl, C₃-C₆ alkenyl, C₃-C₁₁ cycloalkyl, C₄-C₁₁ cycloalkylmethyl, C₆-C₁₀ aryl, C₇-C₁₁ arylalkyl, C₁-C₁₀ alkyl substituted with 0-6 R^{4b};
R^{5a} is selected from H, hydroxy, C₁-C₈ alkyl, C₃-C₆ alkenyl, C₃-C₁₁ cycloalkyl, C₄-C₁₁ cycloalkylmethyl, C₁-C₆ alkoxy, benzyloxy, C₆-C₁₀ aryl, heteroaryl, heteroarylalkyl, C₇-C₁₁ arylalkyl, adamantylmethyl, and C₁-C₁₀ alkyl substituted with 0-2 R^{4b};
alternatively, R⁵ and R^{5a} when both are substituents on the same nitrogen atom (as in -N(R⁵)R^{5a}) can be taken together with the nitrogen atom to which they are attached to form 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl or 1-piperazinyl, each being optionally substituted with C₁-C₆ alkyl, C₆-C₁₀ aryl, heteroaryl, C₇-C₁₁ arylalkyl, (C₁-C₆ alkyl) carbonyl, (C₃-C₇ cycloalkyl) carbonyl, (C₁-C₆ alkoxy) carbonyl, (C₇-C₁₁ arylalkoxy) carbonyl, C₁-C₆ alkylsulfonyl or C₆-C₁₀ arylsulfonyl;
R^{5b} is selected from C₁-C₈ alkyl, C₂-C₆ alkenyl, C₃-C₁₁ cycloalkyl, C₄-C₁₁ cycloalkylmethyl, C₆-C₁₀ aryl, C₇-C₁₁ arylalkyl, and C₁-C₁₀ alkyl substituted with 0-2 R^{4b};
R⁶ is selected from H, C₁-C₁₀ alkyl, hydroxy, C₁-C₁₀ alkoxy, nitro, (C₁-C₁₀ alkyl) carbonyl, -N(R¹²)R¹³, cyano, halo, CF₃, -CHO, CO₂R⁵, -C(=O)R^{5a}, -CON(R⁵)R^{5a}, -OC(=O)R^{5a},-OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, -SiMe₃, C₂-C₆ alkenyl, C₃-C₁₁ cycloalkyl , C₄-C₁₁ cycloalkylmethyl, -N(R⁵)R^{5a}, S(O)ₘR⁵, -OR⁵ ;
C₆-C₁₀ aryl optionally substituted with 1-3 groups selected from halogen, C₁-C₆ alkoxy, C₁-C₆ alkyl, CF₃, -S(O)ₘMe, and -NMe₂;
C₇-C₁₁ arylalkyl, said aryl being optionally substituted with 1-3 groups selected from halogen, C₁-C₆ alkoxy, C₁-C₆ alkyl, CF₃, S(O)ₘMe, and -NMe₂ ; methylenedioxy when R⁶ is a substituent on aryl;
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁷;
R⁷ is selected from H, C₁-C₁₀ alkyl, hydroxy, C₁-C₁₀ alkoxy, nitro, (C₁-C₁₀ alkyl) carbonyl, -N(R¹²)R¹³, cyano, halo, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N (R⁵) R^{5a}, -NR^{5a}SO₂R⁵, -S(O)ₘR^{5a}, -SO₂N(R⁵)R^{5a}, C₂-C₆ alkenyl, C₃-C₁₁ cycloalkyl, C₄-C₁₁ cycloalkylmethyl, C₆-C₁₀ aryl, and C₇-C₁₁ arylalkyl;
R⁸ is selected from R⁶, C₁-C₁₀ alkyl substituted with 0-3 R⁶, C₂-C₁₀ alkenyl substituted with 0-3 R⁶, C₂-C₁₀ alkynyl substituted with 0-3 R⁶, C₃-C₈ cycloalkyl substituted with 0-3 R⁶, C₅-C₆ cycloalkenyl substituted with 0-3 R⁶, aryl substituted with 0-3 R⁶;
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁶;
R¹² and R¹³ are independently H, C₁-C₁₀ alkyl, (C₁-C₁₀ alkoxy) carbonyl, (C₁-C₁₀ alkyl) carbonyl, C₁-C₁₀ alkylsulfonyl, aryl (C₁-C₁₀ alkyl) sulfonyl, arylsulfonyl, aryl (C₂-C₁₀ alkenyl) sulfonyl, heteroarylsulfonyl, aryl, C₂-C₆ alkenyl, C₃-C₁₁ cycloalkyl, C₄-C₁₁ cycloalkylalkyl, C₇-C₁₁ arylalkyl, C₇-C₁₁ arylcarbonyl, C₄-C₁₁ cycloalkoxycarbonyl, C₇-C₁₁ bicycloalkoxycarbonyl, C₇-C₁₁ aryloxycarbonyl, heteroarylcarbonyl, heteroarylalkylcarbonyl, or aryl(C₁-C₁₀ alkoxy)carbonyl, wherein said aryls are substituted with 0-3 substituents selected from C₁-C₄ alkyl, C₁-C₄ alkoxy, halo, CF₃, and NO₂;
R¹⁵ is selected from H, hydroxy, C₁-C₁₀ alkyl, C₂-C₁₀ alkenyl, C₂-C₁₀ alkynyl, C₁-C₁₀ alkoxy, (C₁-C₁₀ alkoxy) carbonyl,-CO₂R⁵, and -C(=O)N(R⁵)R^{5a};
R¹⁶ is selected from -C(=O)-O-R^{18a}, -C(=O)-R^{18b}, -C(=O)N(R^{18b})₂, -C(=O)NHSO₂R^{18a}, -C(=O)NHC(=O)R^{18b}, -C(=O)NHC(=O)OR^{18a}, -C(=O)NHSO₂NHR^{18b}, -C(=S)-NH-R^{18b}, -NH-C(=O)-O-R^{18a}, -NH-C(=O)-R^{18b}, -NH-C(=O)-NH-R^{18b}, -SO₂-O-R^{18a}, -SO₂-R^{18a}, -SO₂-N(R^{18b})₂, -SO₂-NHC(=O)OR^{18b}, -P(=S)(OR^{18a})₂, -P(=O)(OR^{18a})₂, -P(=S)(R^{18a})₂, -P(=O)(R^{18a})₂, and 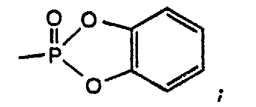
R¹⁷ is selected from H, C₁-C₁₀ alkyl, C₂-C₆ alkenyl, C₃-C₁₁ cycloalkyl, C₄-C₁₅ cycloalkyalkyl, aryl, and aryl(C₁-C₁₀ alkyl)-;
R^{18a} is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹;
a 5-10 membered heterocyclic ring system having 1-3 heteroatoms selected independently from O, S, and N, said heterocyclic ring being substituted with 0-4 R¹⁹;
C₁-C₆ alkyl substituted with a 5-10 membered heterocyclic ring system having 1-3 heteroatoms selected independently from O, S, and N, said heterocyclic ring being substituted with 0-4 R¹⁹;
R^{18b} is R^{18a} or H;
R¹⁹ is selected from H, halogen, CF₃, CN, NO₂, NR¹²R¹³, C₁-C₈ alkyl, C₂-C₆ alkenyl, C₂-C₆ alkynyl, C₃-C₁₁ cycloalkyl, C₄-C₁₁ cycloalkylalkyl, aryl, aryl(C₁-C₆ alkyl)-, C₁-C₆ alkoxy, and C₁-C₄ alkoxycarbonyl;
m is 0-2,
n is 0-4,
n' is 0-4,
p' is 1-7,
p" is 1-7, and
r is 0-3;
provided that n' is chosen such that the number of in-chain atoms connecting R¹ and Y is in the range of 8-18.

2. The compound of claim 1, wherein:
Z is a bond, O, or S;
R² is H, aryl(C₁-C₁₀ alkoxy)carbonyl or C₂-C₁₀ alkoxycarbonyl;
W is -(CH₂)_{n'}-C(=O)-N(R^{5a})-;
X is -(C(R⁴)₂)_{n'}-C(R⁴)(R⁸)-CH(R⁴)-, with the proviso that when n' is 0 or 1, then R⁸ is other than H or methyl;
R⁵ is H or C₁-C₁₀ alkyl substituted with 0-6 R^{4b};
R⁶ is selected from H, C₁-C₁₀ alkyl, hydroxy, C₁-C₁₀ alkoxy, nitro, (C₁-C₁₀ alkyl) carbonyl, -N(R¹²)R¹³, -N(R⁵)R^{5a}, -CO₂R⁵, -S(O)ₘR⁵, -OR⁵, cyano, halo;
C₆-C₁₀ aryl optionally substituted with 1-3 groups selected from halogen, C₁-C₆ alkoxy, C₁-C₆ alkyl, CF₃, -S(O)ₘMe, and -NMe₂;
C₇-C₁₁ arylalkyl, said aryl being optionally substituted with 1-3 groups selected from halogen, C₁-C₆ alkoxy, C₁-C₆ alkyl, CF₃, -S(O)ₘMe, and -NMe₂ ;
methylenedioxy when R⁶ is a substituent on aryl;
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁷;
R⁷ is selected from H, C₁-C₁₀ alkyl, hydroxy, C₁-C₁₀ alkoxy, nitro, (C₁-C₁₀ alkyl) carbonyl, -N(R¹²)R¹³, cyano, and halo;
R⁸ is selected from -CON(R⁵)NR^{5a}, -CO₂R⁵, C₁-C₁₀ alkyl substituted with 0-3 R⁶, C₂-C₁₀ alkenyl substituted with 0-3 R⁶, C₂-C₁₀ alkynyl substituted with 0-3 R⁶, C₃-C₈ cycloalkyl substituted with 0-3 R⁶, C₅-C₆ cycloalkenyl substituted with 0-3 R⁶, aryl substituted with 0-2 R⁶;
a 5-10 membered heterocyclic ring containing 1-3 N, O, or S heteroatoms, wherein said heterocyclic ring may be saturated, partially saturated, or fully unsaturated, said heterocyclic ring being substituted with 0-2 R⁶;
R¹² and R¹³ are each independently selected from: H, C₁-C₁₀ alkyl, (C₁-C₁₀ alkoxy) carbonyl, (C₁-C₁₀ alkyl)carbonyl, C₁-C₁₀ alkylsulfonyl, aryl (C₁-C₁₀ alkyl) sulfonyl, arylsulfonyl, aryl, heteroarylcarbonyl and heteroarylalkylcarbonyl, wherein said aryls are substituted with 0-3 substituents selected from: C₁-C₄ alkyl, C₁-C₄ alkoxy, halo, CF₃, and NO₂; and
R¹⁵ is selected from H, C₁-C₁₀ alkyl, C₂-C₁₀ alkenyl, C₂-C₁₀ alkynyl, C₁-C₁₀ alkoxy, (C₁-C₁₀ alkoxy)carbonyl, -CO₂R⁵, and -C(=O)N(R⁵)R^{5a}.

3. The compound of Claim 2 wherein:
Z is a bond or O;
W is -(CH₂)_{n'}-C(=O)-N(R^{5a})- ; and
X is -C(R⁴)(R⁸)-CH(R⁴)-.

4. The compound of Claim 2 wherein:
R¹ is R²NHC(=NR²)- or R²NHC(=NR²)NH-, and V is phenylene or pyridylene; or
R¹ is 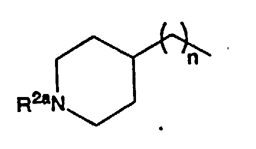
V is a single bond, and n is 1 or 2;
X is -CHR⁸CH₂- ;
Y is selected from hydroxy, C₁-C₁₀ alkoxy, methylcarbonyloxymethoxy-, ethylcarbonyloxymethoxy-, t-butylcarbonyloxymethoxy-, cyclohexylcarbonyloxymethoxy-, 1-(methylcarbonyloxy)ethoxy-, 1-(ethylcarbonyloxy)ethoxy-, 1-(t-butylcarbonyloxy)ethoxy-, 1-(cyclohexylcarbonyloxy)ethoxy-, i-propyloxycarbonyloxymethoxy-, t-butyloxycarbonyloxymethoxy-, 1-(i-propyloxycarbonyloxy)ethoxy-, 1-(cyclohexyloxycarbonyloxy)ethoxy-, 1-(t-butyloxycarbonyloxy)ethoxy-, dimethylaminoethoxy-, diethylaminoethoxy-, (5-methyl-1,3-dioxacyclopenten-2-on-4-yl)methoxy-, (5-(t-butyl)-1,3-dioxacyclopenten-2-on-4-yl)methoxy-, (1,3-dioxa-5-phenylcyclopenten-2-on-4-yl)methoxy-, and 1-(2-(2-methoxypropyl)carbonyloxy)ethoxy-;
R⁶ is selected from H, C₁-C₄ alkyl, hydroxy, C₁-C₄ alkoxy, nitro, C₁-C₁₀ alkylcarbonyl, -N(R¹²)R¹³, -N(R⁵)R^{5a}, -CO₂R⁵, -S(O)ₘR⁵, -OR⁵, cyano, halo;
C₆-C₁₀ aryl optionally substituted with 1-3 groups selected from halogen, C₁-C₆ alkoxy, C₁-C₆ alkyl, CF₃, -S(O)ₘMe, and -NMe₂;
methylenedioxy when R⁶ is a substituent on aryl;
a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, benzimidazolyl, piperidinyl, tetrahydrofuranyl, pyranyl, pyridinyl, 3H-indolyl, pyrrolidinyl, piperidinyl, indolinyl, isoxazolinyl and morpholinyl;
R⁸ is selected from -CON(R⁵)NR^{5a}, -CO₂R⁵, C₁-C₁₀ alkyl substituted with 0-3 R⁶, C₂-C₁₀ alkenyl, substituted with 0-3 R⁶, C₂-C₁₀ alkynyl substituted with 0-3 R⁶; C₃-C₈ cycloalkyl substituted with 0-3 R⁶, aryl substituted with 0-2 R⁶;
a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyridinyl, 3H-indolyl, pyrrolidinyl, piperidinyl, indolinyl and morpholinyl, said heterocyclic ring being substituted with 0-2 R⁶;
R¹² is selected from H, C₁-C₆ alkyl, (C₁-C₄ alkoxy)carbonyl, (C₁-C₆ alkyl)carbonyl, C₁-C₆ alkylsulfonyl, aryl (C₁-C₄ alkyl) sulfonyl, arylsulfonyl, aryl, pyridylcarbonyl and pyridylmethylcarbonyl, wherein said aryls are substituted with 0-3 substituents selected from C₁-C₄ alkyl, C₁-C₄ alkoxy, halo, CF₃, and NO₂; and
R¹³ is H.

5. The compound of Claim 1, or a pharmaceutically acceptable salt form thereof, selected from:
3(*R,S*)-{5(*R*,*S*)-N-[3-(4-amidinophenyl)isoxazolin-5-ylacetyl]amino}-3-phenylpropanoic acid;
3(*R,S*)-{5(*R*,*S*)-N-[3-(4-amidinophenyl)isoxazolin-5-ylacetyl]amino}-pentanoic acid;
3(*R*)-{5(*R*,*S*)-N-[3-(4-amidinophenyl)isoxazolin-5-ylacetyl]amino}heptanoic acid;
3(*R,S*)-{5(*R*,*S*)-N-[3-(4-amidinophenyl)isoxazolin-5-ylacetyl]amino}-4-(phenylthio)butanoic acid;
3(*R,S*)-{5(*R*,*S*)-N-[3-(4-amidinophenyl)isoxazolin-5-ylacetyl]amino}-4-(phenylsulfonamido)butanoic acid;
3(*R,S*)-{5*(R,S)*-N-[3-(4-amidinophenyl)isoxazolin-5-ylacetyl]amino}-4-(n-butylsulfonamido)butanoic acid;
3(*S*)-{5(*R,S*)-N-[3-(4-amidinophenyl)isoxazolin-5-ylacetyl]amino}-3-(adamantylmethylaminocarbonyl)propanoic acid;
3(*S*)-{5(*R,S*)-N-[3-(4-amidinophenyl)isoxazolin-5-ylacetyl]amino}-3-(1-azabicyclo[3.2.2]nonylcarbonyl)propanoic acid;
3(*S*)-{5(*R*,*S*)-N-[3-(4-amidinophenyl)isoxazolin-5-ylacetyl]amino}-3-(phenethylaminocarbonyl)propanoic acid;
3(*R*)-{5(*R,S*)-N-[3-(4-amidinophenyl)isoxazolin-5-ylacetyl]amino}-3-(3-pyridylethyl)propanoic acid;
3(*R*)-{5(*R,S*)-N-[3-(4-amidinophenyl)isoxazolin-5-ylacetyl]amino}-3-(2-pyridylethyl)propanoic acid; and
3(*R*)-{5(*R*,*S*)-N-[3-(4-amidinophenyl)isoxazolin-5-ylacetyl]amino}-3-(phenylpropyl)propanoic acid.

6. The compound of claim 1, wherein
Z is a bond, O, or S;
R² and R³ are independently selected from H, aryl(C₁-C₁₀ alkoxy)carbonyl, and (C₁-C₁₀ alkoxy)carbonyl;
R^{2a} is R² or R²(R³)N(R²N=)C;
W is -C(R⁴)₂-C(=O)-N(R^{5a})- or -C(=O)N(R^{5a})-C(R⁴)₂-;
X is -C(R⁴) (R⁸) -CHR^{4a}-;
R⁴ is selected from H, C₁-C₁₀ alkyl, C₁-C₁₀ alkycarbonyl, aryl, arylalkyl, cycloalkyl, and cycloalkylalkyl;
R^{4a} is selected from hydroxy, C₁-C₁₀ alkoxy, nitro, -N(R⁵)R^{5a}, -N(R¹²)R¹³, -N(R¹⁶)R¹⁷, C₁-C₁₀ alkyl substituted with 0-3 R⁶, aryl substituted with 0-3 R⁶, and C₁-C₁₀ alkylcarbonyl;
R^{4b} is selected from H, C₁-C₆ alkyl, C₂-C₆ alkenyl, C₂-C₆ alkynyl, hydroxy, C₁-C₆ alkoxy, C₁-C₆ alkylthio, C₁-C₆ alkylsulfinyl, C₁-C₆ alkylsulfonyl, nitro, (C₁-C₆ alkyl)carbonyl, C₆-C₁₀ aryl, -N(R¹²)R¹³, halo, CF₃, CN, (C₁-C₆ alkoxy)carbonyl, carboxy, piperidinyl, morpholinyl, and pyridinyl;
R⁵ is C₁-C₁₀ alkyl substituted with 0-2 R^{4b};
R^{5a} is selected from H, hydroxy, C₁-C₈ alkyl, C₃-C₆ alkenyl, C₃-C₁₁ cycloalkyl, C₄-C₁₁ cycloalkylmethyl, C₁-C₆ alkoxy, benzyloxy, C₆-C₁₀ aryl, heteroaryl, heteroarylalkyl, C₇-C₁₁ arylalkyl, adamantylmethyl, and C₁-C₁₀ alkyl substituted with 0-2 R^{4b};
alternatively, R⁵ and R^{5a} can be taken together to be 3-azabicyclononyl, 1,2,3,4-tetrahydro-1-quinolinyl, 1,2,3,4-tetrahydro-2-isoquinolinyl, 1-piperidinyl, 1-morpholinyl, 1-pyrrolidinyl, thiamorpholinyl, thiazolidinyl or 1-piperazinyl, each being optionally substituted with C₁-C₆ alkyl, C₆-C₁₀ aryl, heteroaryl, C₇-C₁₁ arylalkyl, (C₁-C₆ alkyl)carbonyl, (C₃-C₇ cycloalkyl) carbonyl, (C₁-C₆ alkoxy)carbonyl, or (C₇-C₁₁ arylalkoxy) carbonyl;
Y is selected from hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxycarbonyloxyalkyloxy, C₂-C₁₀ alkoxycarbonylalkyloxy, C₅-C₁₀ cycloalkylcarbonyloxyalkyloxy, C₅-C₁₀ cycloalkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyloxyalkyloxy, C₅-C₁₀ alkoxyalkylcarbonyloxyalkyloxy, C₅-C₁₀ (5-alkyl-1,3-dioxa-cyclopenten-2-one-yl)methyloxy, and C₁₀-C₁₄ (5-aryl-1,3-dioxacyclopenten-2-one-yl)methyloxy;
R⁶ and R⁷ are each independently selected from H, C₁-C₁₀ alkyl, hydroxy, C₁-C₁₀ alkoxy, nitro, (C₁-C₁₀ alkyl)carbonyl, -N(R¹²)R¹³, cyano, and halo;
R¹² and R¹³ are each independently selected from H, C₁-C₁₀ alkyl, (C₁-C₁₀ alkoxy)carbonyl, (C₁-C₁₀ alkyl) carbonyl, C₁-C₁₀ alkylsulfonyl, aryl (C₁-C₁₀ alkyl) sulfonyl, arylsulfonyl, heteroarylcarbonyl, heteroarylalkyl-carbonyl and aryl, wherein said aryls are substituted with 0-3 substituents selected from C₁-C₄ alkyl, C₁-C₄ alkoxy, halo, CF₃, and NO₂;
R¹⁵ is selected from: H, C₁-C₁₀ alkyl, C₂-C₁₀ alkenyl, C₂-C₁₀ alkynyl, C₁-C₁₀ alkoxy, (C₁-C₁₀ alkoxy)carbonyl, -CO₂R⁵, and -C(=O)N(R⁵)R^{5a};
R¹⁶ is selected from -C(=O)-O-R^{18a}, -C(=O)-R^{18b}, -C(=O)N(R^{18b})₂, -SO₂-R^{18a}, and -SO₂-N(R^{18b})₂;
R¹⁷ is H or C₁-C₄ alkyl;
R^{18a} is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹;
a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyrimidinyl, 3H-indolyl, pyrrolidinyl, piperidinyl, indolinyl and morpholinyl, said heterocyclic ring being substituted with 0-4 R¹⁹;
C₁-C₆ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, or morpholinyl, said heterocyclic ring being substituted with 0-4 R¹⁹; and
the variables V, Q, R¹, R^{5b}, R^{18b}, R¹⁹, b, n, p', p" and r are as defined in claim 1.

7. The compound of Claim 6 of Formula Ib: 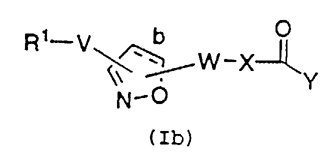 wherein:
R¹ is selected from R^{2a}(R³)N-, R²NH(R²N=)C-, R²NH(R²N=)CNH-, R^{2a}(R³)N(CH₂)_{p'}Z-, R²NH(R²N=)C(CH₂)_{p"}Z-, 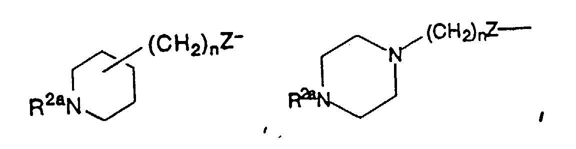 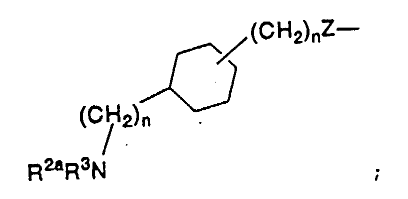
n is 0-1;
p' is 4-6;
p" is 2-4;
Z is a bond or O;
V is a single bond, -(phenyl)- or -(pyridinyl)-;
W is -C(R⁴)₂-C(=O)-N(R^{5a})- or -C(=O)-N(R^{5a})-CH₂- ;
X is-CH₂-CH(N(R¹⁶)R¹⁷)-, or -CH₂-CH(N(R⁵)R^{5a})- ;
Y is selected from hydroxy, C₁-C₁₀ alkoxy, methylcarbonyloxymethoxy-, t-butylcarbonyloxymethoxy-, cyclohexylcarbonyloxymethoxy-, 1-(methylcarbonyloxy)ethoxy-, 1-(ethyl-carbonyloxy)ethoxy-, 1-(t-butylcarbonyloxy)ethoxy-, 1-(cyclohexylcarbonyloxy)ethoxy-, i-propyloxycarbonyloxymethoxy-, t-butyloxycarbonyloxymethoxy-, 1-(i-propyloxycarbonyloxy)ethoxy-, 1-(cyclohexyloxycarbonyloxy)ethoxy-, 1-(t-butyloxycarbonyloxy)ethoxy-, dimethylaminoethoxy-, diethylaminoethoxy-, (5-methyl-1,3-dioxacyclopenten-2-on-4-yl)methoxy-, (5-(t-butyl)-1,3-dioxacyclopenten-2-on-4-yl)methoxy-, (1,3-dioxa-5-phenylcyclopenten-2-on-4-yl)methoxy-, and 1-(2-(2-methoxypropyl)carbonyloxy)ethoxy-;
R¹⁶ is selected from -C(=O)-O-R^{18a}, -C(=O)-R^{18b}, -S(=O)₂-R^{18a}, and SO₂-N(R^{18b})₂ ;
R¹⁷ is H or C₁-C₅ alkyl;
R^{18a} is selected from C₁-C₈ alkyl substituted with 0-2 R¹⁹, C₂-C₈ alkenyl substituted with 0-2 R¹⁹, C₂-C₈ alkynyl substituted with 0-2 R¹⁹, C₃-C₈ cycloalkyl substituted with 0-2 R¹⁹, aryl substituted with 0-4 R¹⁹, aryl(C₁-C₆ alkyl)- substituted with 0-4 R¹⁹,
a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyridinyl, 3H-indolyl, carbazole, pyrrolidinyl, piperidinyl, indolinyl and morpholinyl, said heterocyclic ring being substituted with 0-4 R¹⁹;
C₁-C₆ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, and morpholinyl, said heterocyclic ring being substituted with 0-4 R¹⁹;

8. The compound of Claim 6 wherein:
R¹ is R²NH(R²N=)C- or R²HN(R²N=)CNH-, and V is phenylene or pyridylene; or
R¹ is 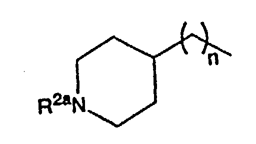
V is a single bond, and n is 1 or 2; and
R^{18a} is selected from C₁-C₄ alkyl substituted with 0-2 R¹⁹, C₂-C₄ alkenyl substituted with 0-2 R¹⁹, C₂-C₄ alkynyl substituted with 0-2 R¹⁹, C₃-C₇ cycloalkyl substituted with 0-2 R¹⁹, aryl substituted with 0-4 R¹⁹, aryl(C₁-C₄ alkyl)- substituted with 0-4 R¹⁹,
a heterocyclic ring system selected from pyridinyl, furanyl, thiazolyl, thienyl, pyrrolyl, pyrazolyl, triazolyl, imidazolyl, benzofuranyl, indolyl, indolinyl, quinolinyl, isoquinolinyl, isoxazolinyl, benzimidazolyl, piperidinyl, tetrahydrofuranyl, pyranyl, pyrimidinyl, 3H-indolyl, pyrrolidinyl, piperidinyl, indolinyl, isoxazolinyl and morpholinyl, said heterocyclic ring being substituted with 0-4 R¹⁹;
C₁-C₄ alkyl substituted with a heterocyclic ring system selected from pyridinyl, furanyl, thiazolyl, thienyl, pyrrolyl, pyrazolyl, imidazolyl, isoxazolinyl, benzofuranyl, indolyl, indolenyl, quinolinyl, isoquinolinyl, benzimidazolyl, piperidinyl, tetrahydrofuranyl, pyranyl, pyridinyl, 3H-indolyl, indolyl, pyrrolidinyl, piperidinyl, indolinyl, isoxazolinyl and morpholinyl, said heterocyclic ring being substituted with 0-4 R¹⁹.

9. The compound of Claim 1 or pharmaceutically acceptable salt forms thereof, which is selected from:
N³-[2-{3-(4-formamidinophenyl)-isoxazolin-5(*R*,*S*)-yl}-acetyl]-N2-(phenylsulfonyl)-2,3-(S)-diaminopropanoic acid;
N³-[2-{3-(4-formamidinophenyl)-isoxazolin-5 (*R,S*)-yl}-acetyl]-N2-(4-methyl-phenyl-sulfonyl)-2,3-(S)-diaminopropanoic acid;
N³-[2-{3-(4-formamidinophenyl)-isoxazolin-5(*R,S*)-yl}-acetyl]-N2- (butanesulfonyl)-2,3-(S)-diaminopropanoic acid;
N³-[2-{3-(4-formamidinophenyl)-isoxazolin-5(*R,S*)-yl}-acetyl]-N2-(propanesulfonyl)-2,3-(S)-diaminopropanoic acid;
N³-[2-{3-(4-formamidinophenyl)-isoxazolin-5(*R,S*)-yl}-acetyl]-N2-(ethanesulfonyl)-2,3-(S)-diaminopropanoic acid;
N³-[2-{3-(4-formamidinophenyl)-isoxazolin-5(*R,S*)-yl}-acetyl]-N2-(methyloxycarbonyl)-2,3-(S)-diaminopropanoic acid;
N³-[2-{3-(4-formamidinophenyl)-isoxazolin-5(*R,S*)-yl}-acetyl]-N2-(ethyloxycarbonyl)-2,3-(S)-diaminopropanoic acid;
N³-[2-{3-(4-formamidinophenyl)-isoxazolin-5(*R,S*)-yl}-acetyl]-N2-(1-propyloxycarbonyl)-2,3-(S)-diaminopropanoic acid;
N³-[2-{3-(4-formamidinophenyl) -isoxazolin-5(*R,S*)-yl}-acetyl]-N2-(2-propyloxycarbonyl)-2,3-(S)-diaminopropanoic acid;
N³-[2-{3-(4-formamidinophenyl)-isoxazolin-5(*R,S*)-yl}-acetyl]-N2-(n-butyloxycarbonyl)-2,3-(S)-diaminopropanoic acid;
N³-[2-{3-(4-formamidinophenyl)-isoxazolin-5(*R*)-yl}-acetyl]-N2-(n-butyloxycarbonyl)-2,3-(S)-diaminopropanoic acid;
N³-[2-{3-(4-formamidinophenyl)-isoxazolin-5(*S*)-yl}-acetyl]-N2-(n-butyloxycarbonyl)-2,3-(S)-diaminopropanoic acid;
N³-[2-{3-(4-formamidinophenyl)-isoxazolin-5(*R*)-yl}-acetyl]-N2-(n-butyloxycarbonyl)-2,3-(R)-diaminopropanoic acid;
N³-[2-{3-(4-formamidinophenyl)-isoxazolin-5(*S*)-yl}-acetyl]-N2-(n-butyloxycarbonyl)-2,3-(R)-diaminopropanoic acid;
N³-[2-{3-(4-formamidinophenyl)-isoxazolin-5(*R,S*)-yl}-acetyl]-N2-(2-butyloxycarbonyl)-2,3-(S)-diaminopropanoic acid;
N³-[2-{3-(4-formamidinophenyl)-isoxazolin-5(*R,S*)-yl}-acetyl]-N2-(1-(2-methyl)-propyloxycarbonyl)-2,3-(S)-diaminopropanoic acid;
N³-[2-{3-(4-formamidinophenyl)-isoxazolin-5(*R,S*)-yl}-acetyl]-N2-(2-(2-methyl)-propyloxycarbonyl)-2,3-(S)-diaminopropanoic acid;
N³-[2-{3-(4-formamidinophenyl)-isoxazolin-5(*R,S*)-yl}-acetyl]-N2-(benzyloxycarbonyl)-2,3-(S)-diaminopropanoic acid;
N³-[2-{3-(4-formamidinophenyl)-isoxazolin-5(*R*)-yl}-acetyl]-N2-(benzyloxycarbonyl)-2,3-(S)-diaminopropanoic acid;
N³-[2-{3-(4-formamidinophenyl)-isoxazolin-5(*S*)-yl}-acetyl]-N2-(benzyloxycarbonyl)-2,3-(S)-diaminopropanoic acid;
N³-[2-{3-(4-formamidinophenyl)-isoxazolin-5(*R,S*)-yl}-acetyl]-N2-(4-methylbenzyloxycarbonyl)-2,3-(S)-diaminopropanoic acid;
N³-[2-{3-(4-formamidinophenyl)-isoxazolin-5(*R,S*)-yl}-acetyl]-N2-(4-methoxybenzyloxycarbonyl)-2,3-(S)-diaminopropanoic acid;
N³-[2-{3-(4-formamidinophenyl)-isoxazolin-5(*R,S*)-yl}-acetyl]-N2-(4-chlorobenzyloxycarbonyl)-2,3-(S)-diaminopropanoic acid;
N³-[2-{3-(4-formamidinophenyl)-isoxazolin-5(*R*,*S*)-yl}-acetyl]-N2-(4-bromobenzyloxycarbonyl)-2,3-(S)-diaminopropanoic acid;
N³-[2-{3-(4-formamidinophenyl)-isoxazolin-5(*R,S*)-yl}-acetyl]-N2-(4-fluorobenzyloxycarbonyl)-2,3-(S)-diaminopropanoic acid;
N³-[2-{3-(4-formamidinophenyl)-isoxazolin-5(*R,S*)-yl}-acetyl]-N2-(4-phenoxybenzyloxycarbonyl)-2,3-(S)-diaminopropanoic acid;
N³-[2-{3-(4-formamidinophenyl)-isoxazolin-5(*R,S*)-yl}-acetyl]-N2-(2-(methyloxyethyl)-oxycarbonyl)-2,3-(S)-diaminopropanoic acid;
N³-[2-{3-(4-formamidinophenyl)-isoxazolin-5(*R,S*)-yl}-acetyl]-N2-(2-pyridinylcarbonyl)-2,3-(S)-diaminopropanoic acid;
N³-[2-{3-(4-formamidinophenyl)-isoxazolin-5(*R,S*)-yl}-acetyl]-N2-(3-pyridinylcarbonyl)-2,3-(S)-diaminopropanoic acid;
N³-[2-{3-(4-formamidinophenyl)-isoxazolin-5(*R,S*)-yl}-acetyl]-N2-(4-pyridinyl-carbonyl)-2,3-(S)-diaminopropanoic acid;
N³-[2-{3-(4-formamidinophenyl)-isoxazolin-5(*R,S*)-yl}-acetyl]-N2-(2-(2-pyridinyl)-acetyl)-2,3-(S)-diaminopropanoic acid;
N³-[2-{3-(4-formamidinophenyl)-isoxazolin-5(*R,S*)-yl}-acetyl]-N2-(2-(3-pyridinyl)-acetyl)-2,3-(S)-diaminopropanoic acid;
N³-[2-{3-(4-formamidinophenyl)-isoxazolin-5(*R,S*)-yl}-acetyl]-N2-(2-(4-pyridinyl)-acetyl)-2,3-(S)-diaminopropanoic acid;
N³-[2-{3-(4-formamidinophenyl)-isoxazolin-5(*R,S*)-yl}-acetyl]-N2-(2-pyridyl-methyloxycarbonyl)-2,3-(S)-diaminopropanoic acid;
N³-[2-{3-(4-formamidinophenyl)-isoxazolin-5(*R,S*)-yl}-acetyl]-N2-(3-pyridyl-methyloxycarbonyl)-2,3-(S)-diaminopropanoic acid;
N³-[2-{3-(4-formamidinophenyl)-isoxazolin-5(*R,S*)-yl}-acetyl]-N2-(4-pyridyl-methyloxycarbonyl)-2,3-(S)-diaminopropanoic acid.
N³-[2-{3-(4-formamidinophenyl)-isoxazolin-5(*R,S*)-yl}-acetyl]-N2-(4-butyloxyphenylsulfonyl)-2,3-(S)-diaminopropanoic acid;
N³-[2-{3-(4-formamidinophenyl)-isoxazolin-5(*R,S*)-yl}-acetyl]-N2-(2-thienylsulfonyl)-2,3-(S)-diaminopropanoic acid;
N³-[2-{3-(4-formamidinophenyl)-isoxazolin-5(*R,S*)-yl}-acetyl]-N2-(3-methylphenylsulfonyl)-2,3-(*R,S*)-diaminopropanoic acid;
N³-[2-{3-(4-formamidinophenyl)-isoxazolin-5(*R,S*)-yl}-acetyl]-N2-(3-methylphenylsulfonyl)-2,3-(S)-diaminopropanoic acid;
N³-[2-{3-(4-formamidinophenyl)-isoxazolin-5(*R,S*)-yl}-acetyl]-N2-(3-methylphenylsulfonyl)-2,3-(R)-diaminopropanoic acid;
N³-[2-{3-(4-formamidinophenyl)-isoxazolin-5(*R*)-yl}-acetyl]-N2-(3-methylphenylsulfonyl)-2,3-(S)-diaminopropanoic acid;
N³-[2-{3-(4-formamidinophenyl)-isoxazolin-5(*S*)-yl}-acetyl]-N2-(3-methylphenylsulfonyl)-2,3-(S)-diaminopropanoic acid;
N³-[2-{3-(4-formamidinophenyl)-isoxazolin-5(*S*)-yl}-acetyl]-N2-(3-methylphenylsulfonyl)-2,3-(R)-diaminopropanoic acid;
N³-[2-{3-(4-formamidinophenyl)-isoxazolin-5(*R*)-yl}-acetyl]-N2-(3-methylphenylsulfonyl)-2,3-(R)-diaminopropanoic acid;
N³- [2-{3-(4-formamidinophenyl)-isoxazolin-5(*R,S*)-yl}-acetyl]-N2-(4-iodophenylsulfonyl)-2,3-(S)-diaminopropanoic acid;
N³-[2-{3-(4-formamidinophenyl)-isoxazolin-5(*R,S*)-yl}-acetyl]-N2-(3-trifluoromethylphenylsulfonyl)-2,3-(S)-diaminopropanoic acid;
N³-[2-{3-(4-formamidinophenyl)-isoxazolin-5(*R,S*)-yl}-acetyl]-N2-(3-chlorophenylsulfonyl)-2,3-(S)-diaminopropanoic acid;
N³-[2-{3-(4-formamidinophenyl)-isoxazolin-5(*R*,*S*)-yl}-acetyl]-N2-(3-2-methoxycarbonylphenylsulfonyl)-2,3-(S)-diaminopropanoic acid;
N³-[2-{3-(4-formamidinophenyl)-isoxazolin-5(*R,S*)-yl}-acetyl]-N2-(2,4,6-trimethylphenylsulfonyl)-2,3-(S)-diaminopropanoic acid;
N³-[2-{3-(4-formamidinophenyl)-isoxazolin-5(*R*,*S*)-yl}-acetyl]-N2-(2-chlorophenylsulfonyl)-2,3-(S)-diaminopropanoic acid;
N³-[2-{3-(4-formamidinophenyl)-isoxazolin-5(*R,S*)-yl}-acetyl]-N2-(4-trifluoromethylphenylsulfonyl)-2,3-(S)-diaminopropanoic acid;
N³-[2-{3-(4-formamidinophenyl)-isoxazolin-5(*R,S*)-yl}-acetyl]-N2-(2-trifluoromethylphenylsulfonyl)-2,3-(S)-diaminopropanoic acid;
N³-[2-{3-(4-formamidinophenyl) -isoxazolin-5(*R,S*)-yl}-acetyl]-N2-(2-fluorophenylsulfonyl)-2,3-(S)-diaminopropanoic acid;
N³-[2-{3-(4-formamidinophenyl)-isoxazolin-5(*R*,*S*)-yl}-acetyl]-N2- (4-fluorophenylsulfonyl)-2,3-(S)-diaminopropanoic acid;
N³-[2-{3-(4-formamidinophenyl)-isoxazolin-5(*R*,*S*)-yl}-acetyl]-N2-(4-methoxyphenylsulfonyl)-2,3-(S)-diaminopropanoic acid;
N³-[2-{3-(4-formamidinophenyl)-isoxazolin-5(*R*,*S*)-yl}-acetyl]-N2-(2,3,5,6-tetramethylphenylsulfonyl)-2,3-(S)-diaminopropanoic acid;
N³-[2-{3-(4-formamidinophenyl)-isoxazolin-5(*R*,*S*)-yl}-acetyl]-N2-(4-cyanophenylsulfonyl)-2,3-(S)-diaminopropanoic acid;
N³-[2-{3-(4-formamidinophenyl)-isoxazolin-5(*R,S*)-yl}-acetyl]-N2-(4-chlorophenylsulfonyl)-2,3-(S)-diaminopropanoic acid;
N³-[2-{3-(4-formamidinophenyl)-isoxazolin-5(*R,S*)-yl}-acetyl]-N2-(4-propylphenylsulfonyl)-2,3-(S)-diaminopropanoic acid;
N³-[2-{3-(4-formamidinophenyl)-isoxazolin-5(*R,S*)-yl}-acetyl]-N2-(2-phenylethylsulfonyl)-2,3(S)-diaminopropanoic acid;
N³-[2-{3-(4-formamidinophenyl)-isoxazolin-5(*R,S*)-yl}-acetyl]-N2-(4-isopropylphenylsulfonyl)-2,3-(S)-diaminopropanoic acid;
N³-[2-{3-(4-formamidinophenyl)-isoxazolin-5(*R,S*)-yl}-acetyl]-N2-(3-phenylpropylsulfonyl)-2,3-(S)-diaminopropanoic acid;
N³-[2-{3-(4-formamidinophenyl)-isoxazolin-5(*R*,*S*)-yl}-acetyl]-N2-(3-pyridylsulfonyl)-2,3-(S)-diaminopropanoic acid;
N³-[2-{3-(4-formamidinophenyl)-isoxazolin-5(*R,S*)-yl}-acetyl]-N2-(phenylaminosulfonyl)-2,3-(S)-diaminopropanoic acid;
N³-[2-{3-(4-formamidinophenyl)-isoxazolin-5(*R,S*)-yl}-acetyl]-N2-(benzylaminosulfonyl)-2,3-(S)-diaminopropanoic acid;
N³-[2-{3-(4-formamidinophenyl)-isoxazolin-5(*R*,*S*)-yl}-acetyl]-N2-(dimethylaminosulfonyl)-2,3-(S)-diaminopropanoic acid,
N³-[2-{3-(2-fluoro-4-formamidinophenyl)-isoxazolin-5(*R,S*)-yl}-acetyl]-N2-(3-methylphenylsulfonyl)-2,3-(S)-diaminopropanoic acid,
N³-[2-{3-(2-formamidino-5-pyridinyl)-isoxazolin-5(*R,S*)-yl}-acetyl]-N2-(n-butyloxycarbonyl)-2,3-(S)-diaminopropanoic acid,
N³-[2-{3-(2-formamidino-5-pyridinyl)-isoxazolin-5(*R,S*)-yl}-acetyl]-N2-(3-methylphenylsulfonyl)-2,3-(S)-diaminopropanoic acid,
N³-[2-{3-(3-formamidino-6-pyridinyl)-isoxazolin-5(*R,S*)-yl}-acetyl]-N2-(n-butyloxycarbonyl)-2,3-(S)-diaminopropanoic acid,
N³-[2-{3-(3-formamidino-6-pyridinyl)-isoxazolin-5(*R,S*)-yl}-acetyl]-N2-(3-methylphenylsulfonyl)-2,3-(S)-diaminopropanoic acid,
N³-[2-{3-(4-formamidinophenyl)-isoxazolin-5(*R,S*)-yl}-acetyl]-N2-(phenylaminocarbonyl)-2,3-(S)-diaminopropanoic acid;
N³-[2-{3-(4-formamidinophenyl)-isoxazolin-5(*R*,*S*)-yl}-acetyl]-N2-(4-fluorophenylaminocarbonyl)-2,3-(S)-diaminopropanoic acid;
N³-[2-{3-(4-formamidinophenyl)-isoxazolin-5(*R,S*)-yl}-acetyl]-N2-(1-naphthylaminocarbonyl)-2,3-(S)-diaminopropanoic acid;
N³-[2-{3-(4-formamidinophenyl)-isoxazolin-5(*R,S*)-yl}-acetyl]-N2-(benzylaminocarbonyl)-2,3-(S)-diaminopropanoic acid;
N³-[2-{3-(4-formamidinophenyl)-isoxazolin-5(*R,S*)-yl}-acetyl]-N2-(3-bromo-2-thienylsulfonyl)-2,3-(S)-diaminopropanoic acid;
N³-[2-{3-(4-formamidinophenyl)-isoxazolin-5 (*R,S*)-yl}-acetyl]-N2- (3-methyl-2-benzothienylsulfonyl)-2,3-(S)-diaminopropanoic acid;
N³-[2-{3-(4-formamidinophenyl)-isoxazolin-5(*R,S*)-yl}-acetyl]-N2-(isobutyloxycarbonyl)-2,3-(S)-diaminopropanoic acid;
N³-[2-{3-(4-formamidinophenyl)-isoxazolin-5(*R*)-yl}-acetyl]-N2-(isobutyloxycarbonyl)-2,3-(S)-diaminopropanoic acid;
N³-[2-{3-(4-formamidinophenyl)-isoxazolin-5(*S*)-yl}-acetyl]-N2-(isobutyloxycarbonyl)-2,3-(S)-diaminopropanoic acid;
N³-[2-{3-(4-formamidinophenyl)-isoxazolin-5(*R,S*)-yl}-acetyl]-N2-(2-cyclopropylethoxycarbonyl)-2,3-(S)-diaminopropanoic acid;
N³-[2-{3-(4-formamidinophenyl)-isoxazolin-5(*R*)-yl}-acetyl]-N2-(2-cyclopropylethoxycarbonyl)-2,3-(S)-diaminopropanoic acid;
N³-[2-{3-(4-formamidinophenyl)-isoxazolin-5(*S*)-yl}-acetyl]-N2-(2-cyclopropylethoxycarbonyl)-2,3-(S)-diaminopropanoic acid;
N³-[2-{3-(4-guanidinophenyl)-isoxazolin-5(*R,S*)-yl}-acetyl]-N2-(n-butyloxycarbonyl)-2,3-(S)-diaminopropanoic acid;
N³-[2-{3-(4-guanidinophenyl)-isoxazolin-5(*R*)-yl}-acetyl]-N2-(n-butyloxycarbonyl)-2,3-(S)-diaminopropanoic acid;
N³-[2-{3-(4-guanidinophenyl)-isoxazolin-5(*R*)-yl}-acetyl]-N2-(3-methylphenylsulfonyl)-2,3-(S)-diaminopropanoic acid; and
N³-[2-{5-(4-formamidinophenyl)-isoxazolin-3(*R,S*)-yl}-acetyl]-N2-(n-butyloxycarbonyl)-2,3-(S)-diaminopropanoic acid.

10. A prodrug ester of a compound of Claim 9, said ester being selected from:
methyl, ethyl, isopropyl, methylcarbonyloxymethyl-, ethylcarbonyloxymethyl-, t-butylcarbonyloxymethyl-, cyclohexylcarbonyloxymethyl-, 1-(methylcarbonyloxy)ethyl-, 1-(ethylcarbonyloxy)ethyl-, 1-(t-butylcarbonyloxy)ethyl-, 1-(cyclohexylcarbonyloxy)ethyl-, i-propyloxycarbonyloxymethyl-, cyclohexylcarbonyloxymethyl-, t-butyloxycarbonyloxymethyl-, 1-(i-propyloxycarbonyloxy)ethyl-, 1-(cyclohexyloxycarbonyloxy)ethyl, 1-(t-butyloxycarbonyloxy)ethyl-, dimethylaminoethyl-, diethylaminoethyl-, (5-methyl-1,3-dioxacyclopenten-2-on-4-yl)methyl-, (5-(t-butyl)-1,3-dioxacyclopenten-2-on-4-yl)methyl-, (1,3-dioxa-5-phenyl-cyclopenten-2-on-4-yl)methyl-, and 1-(2-(2-methoxypropyl) carbonyloxy) ethyl-.

11. The compound of Claim 1, or pharmaceutically acceptable salt forms thereof, which is:
methyl-N³-[2{3-(4-formamidinophenyl)-isoxazolin-5(R)-yl}-acetyl] -N²(n-butyloxycarbonyl)-2,3-(S)-diaminopropionate.

12. A pharmaceutical composition comprising a therapeutically effective amount of a compound as defined in any one of claims 1 to 11 and a pharmaceutically acceptable carrier.

13. Use of a compound as defined in any one of claims 1 to 11 for preparing a medicament for the prevention or treatment of thrombosis.

14. Use of a compound as defined in any one of claims 1 to 11 for preparing a medicament for inhibiting the aggregation of blood platelets .

15. Use of a compound of Claim 1 for preparing a medicament for treating thromboembolic disorders selected from thrombus or embolus formation, harmful platelet aggregation, reocclusion following thrombolysis, reperfusion injury, restenosis, atherosclerosis, stroke, myocardial infarction, and unstable angina.

16. Use of a compound of Claim 1 for preparing a medicament for treating rheumatoid arthritis, asthma, allergies, adult respritatory syndrome, organ transplantation rejection, septic shock, psoriasis, contact dermatitis, osteoporosis, osteoarthritis, tumor metastasis, diabetic retinopathy, inflammatory conditions and inflammatory bowel disease.

## Patentansprüche

1. Verbindung der Formel I: 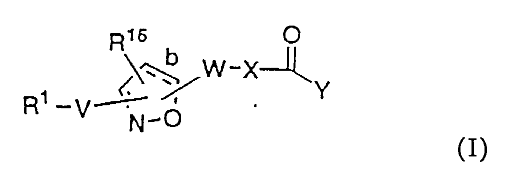 oder ein pharmazeutisch annehmbares Salz davon, wobei:
b eine Einfach- oder Doppelbindung ist;
R¹ aus R^{2a}(R³)N-, R²(R³)N(R²N=)C-, R^{2a}(R³)N(CH₂)ₚ,Z-, R²(R³)N(R²N=)C(CH₂)_{p"}Z-, R₂(R₃)N(R₂N=)CN(R₂)-, 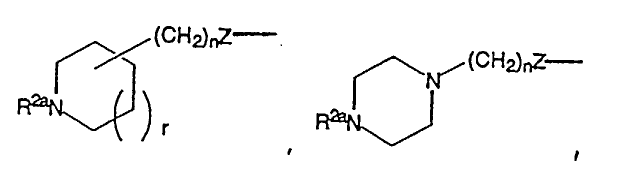 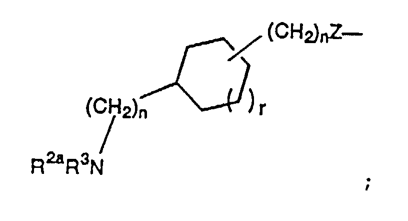 ausgewählt ist
Z aus einer Bindung, O, S, S(=O) und S(=O)₂ ausgewählt ist;
R² und R³ unabhängig aus H, C₁-C₁₀-Alkyl, C₃-C₆-Alkenyl, C₃-C₁₁-Cycloalkyl, C₄-C₁₁-Cycloalkylalkyl, C₆-C₁₀-Aryl, C₇-C₁₁-Arylalkyl, C₂-C₇-Alkylcarbonyl, C₇-C₁₁-Arylcarbonyl, C₂-C₁₀-Alkoxycarbonyl, C₄-C₁₁-Cycloalkoxycarbonyl, C₇-C₁₁-Bicycloalkoxycarbonyl, C₇-C₁₁-Aryloxycarbonyl, Aryl(C₁-C₁₀-alkoxy)carbonyl, (C₁-C₆-Alkyl)carbonyloxy(C₁-C₄-alkoxy)carbonyl, Arylcarbonyloxy(C₁-C₄-alkoxy)carbonyl und (C₄-C₁₁-Cycloalkylcarbonyl)oxy(C₁-C₄-alkoxy)carbonyl ausgewählt sind;
R^{2a} R² oder R²(R³)N(R²N=)C- ist;
V aus einer Einfachbindung, -(Phenyl)-Q-, -(Pyridyl)-Q- und -(Pyridazinyl)-Q- ausgewählt ist, wobei die Phenyl-, Pyridyl- und Pyridazinylreste mit 0-2 Gruppen substituiert sind, die unabhängig aus H, C₁-C₁₀-Alkyl, Hydroxy, C₁-C₁₀-Alkoxy, Nitro, (C₁-C₁₀-Alkyl)carbonyl, -N(R¹²)R¹³, Cyan und Halogen ausgewählt sind;
Q aus einer Einfachbindung, -(CH₂)ₘ-/ -CH₂O- und -OCH₂- ausgewählt ist, mit der Maßgabe, dass Q nicht -CH₂O- ist, wenn b eine Einfachbindung ist und R¹-V- ein Substituent von C5 des zentralen fünfgliedrigen Rings von Formel I ist;
W -(C(R⁴)₂)_{n'}-C(=O)-N(R^{5a})- oder -C(=O)-N(R^{5a})-(C(R⁴)₂)_{n'}- ist;
X -(C(R⁴)₂)_{n'}-C(R⁴)(R⁸)-C(R⁴)(R^{4a})- ist, mit der Maßgabe, dass wenigstens einer der Reste R^{4a} oder R⁸ kein H oder Methyl ist, wenn n' 0 oder 1 ist;
Y aus Hydroxy, C₁-C₁₀-Alkyloxy, C₃-C₁₁-Cycloalkyloxy, C₆-C₁₀-Aryloxy, C₇-C₁₁-Aralkyloxy, C₃-C₁₀-Alkylcarbonyloxyalkyloxy, C₃-C₁₀-Alkoxycarbonyloxyalkyloxy, C₂-C₁₀-Alkoxycarbonylalkyloxy, C₃-C₁₀-Cycloalkylcarbonyloxyalkyloxy, C₅-C₁₀-Cycloalkoxycarbonyloxyalkyloxy, C₅-C₁₀-Cycloalkoxycarbonylalkyloxy, C₇-C₁₁-Aryloxycarbonylalkyloxy, C₈-C₁₂-Aryloxycarbonyloxyalkyloxy, C₈-C₁₂-Arylcarbonyloxyalkyloxy, C₅-C₁₀-Alkoxyalkylcarbonyloxyalkyloxy, C₅-C₁₀-(5-Alkyl-1,3-dioxacyclopenten-2-onyl)methyloxy, C₁₀-C₁₄-(5-Aryl-1,3-dioxacyclopenten-2-onyl)methyloxy und (R²)(R³)N-(C₁-C₁₀-Alkoxy)- ausgewählt ist;
R⁴ aus H, C₁-C₁₀-Alkyl, C₁-C₁₀-Alkylcarbonyl, Aryl, Arylalkyl, Cycloalkyl und Cycloalkylalkyl ausgewählt ist;
alternativ dazu zwei R⁴-Gruppen an benachbarten Kohlenstoffatomen unter Bildung einer Bindung zusammentreten können, so dass eine Kohlenstoff-Kohlenstoff-Doppel- oder Dreifachbindung zwischen diesen benachbarten Kohlenstoffatomen entsteht;
R^{4a} aus H, Hydroxy, C₁-C₁₀-Alkoxy, Nitro, -N(R⁵)R^{5a}, -N(R¹²)R¹³, -N(R¹⁶)R¹⁷, mit 0-3 R⁶ substituiertem C₁-C₁₀-Alkyl, mit 0-3 R⁶ substituiertem Aryl, mit 0-3 R⁶ substituiertem Heteroaryl und C₁-C₁₀-Alkylcarbonyl ausgewählt ist;
R^{4b} aus H, C₁-C₆-Alkyl, C₂-C₆-Alkenyl, C₂-C₆-Alkinyl, C₃-C₇-Cycloalkyl, C₇-C₁₄-Bicycloalkyl, Hydroxy, C₁-C₆-Alkoxy, C₁-C₆-Alkylthio, C₁-C₆-Alkylsulfinyl, C₁-C₆-Alkylsulfonyl, Nitro, C₁-C₆-Alkylcarbonyl, C₆-C₁₀-Aryl, -N(R¹²)R¹³, Halogen, CF₃, CN, (C₁-C₆-Alkoxy)carbonyl, Carboxy, Piperidinyl, Morpholinyl und Pyridinyl ausgewählt ist;
R⁵ aus H, C₁-C₈-Alkyl, C₃-C₆-Alkenyl, C₃-C₁₁-Cycloalkyl, C₄-C₁₁-Cycloalkylmethyl, C₆-C₁₀-Aryl, C₇-C₁₁-Arylalkyl und mit 0-6 R^{4b} substituiertem C₁-C₁₀-Alkyl ausgewählt ist;
R^{5a} aus H, Hydroxy, C₁-C₈-Alkyl, C₃-C₆-Alkenyl, C₃-C₁₁-Cycloalkyl, C₄-C₁₁-Cycloalkylmethyl, C₁-C₆-Alkoxy, Benzyloxy, C₆-C₁₁-Aryl, Heteroaryl, Heteroarylalkyl, C₇-C₁₁-Arylalkyl, Adamantylmethyl und mit 0-2 R^{4b} substituiertem C₁-C₁₀-Alkyl ausgewählt ist;
alternativ dazu R⁵ und R^{5a}, wenn beide Substituenten an demselben Stickstoffatom sind (wie in -N(R⁵)R^{5a}), mit dem Stickstoffatom, an das sie gebunden sind, zusammengenommen werden können unter Bildung von 3-Azabicyclononyl, 1,2,3,4-Tetrahydro-1-chinolinyl, 1,2,3,4-Tetrahydro-2-isochinolinyl, 1-Piperidinyl, 1-Morpholinyl, 1-Pyrrolidinyl, Thiamorpholinyl, Thiazolidinyl oder 1-Piperazinyl, wobei jedes gegebenenfalls mit C₁-C₆-Alkyl, C₆-C₁₀-Aryl, Heteroaryl, C₇-C₁₁-Arylalkyl, (C₁-C₆-Alkyl)carbonyl, (C₃-C₇-Cycloalkyl)carbonyl, (C₁-C₆-Alkoxy)carbonyl, (C₇-C₁₁-Arylalkoxy)carbonyl, C₁-C₆-Alkylsulfonyl oder C₆-C₁₀-Arylsulfonyl substituiert ist;
R^{5b} aus C₁-C₈-Alkyl, C₂-C₆-Alkenyl, C₃-C₁₁-Cycloalkyl, C₄-C₁₁-Cycloalkylmethyl, C₆-C₁₀-Aryl, C₇-C₁₁-Arylalkyl und mit 0-2 R^{4b} substituiertem C₁-C₁₀-Alkyl ausgewählt ist;
R⁶ ausgewählt ist aus H, C₁-C₁₀-Alkyl, Hydroxy, C₁-C₁₀-Alkoxy, Nitro, (C₁-C₁₀-Alkyl)carbonyl, -N(R¹²)R¹³, Cyan, Halogen, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, -SiMe₃, C₂-C₆-Alkenyl, C₃-C₁₁-Cycloalkyl, C₄-C₁₁-Cycloalkylmethyl, -N(R⁵)R^{5a}, -S(O)ₘR⁵, -OR⁵;
C₆-C₁₀-Aryl, das gegebenenfalls mit 1-3 Gruppen substituiert ist, die aus Halogen, C₁-C₆-Alkoxy, C₁-C₆-Alkyl, CF₃, -S(O)ₘMe und -NMe₂ ausgewählt sind;
C₇-C₁₁-Arylalkyl, wobei das Aryl gegebenenfalls mit 1-3 Gruppen substituiert ist, die aus Halogen, C₁-C₆-Alkoxy, C₁-C₆-Alkyl, CF₃, S(O)ₘMe und -NMe₂ ausgewählt sind;
Methylendioxy, wenn R⁶ ein Substituent an Aryl ist;
einem 5-10-gliedrigen heterocyclischen Ring, der 1-3 N-, O-oder S-Heteroatome enthält, wobei der heterocyclische Ring gesättigt, partiell gesättigt oder vollständig ungesättigt sein kann, wobei der heterocyclische Ring mit 0-2 R⁷ substituiert ist;
R⁷ aus H, C₁-C₁₀-Alkyl, Hydroxy, C₁-C₁₀-Alkoxy, Nitro, (C₁-C₁₀-Alkyl)carbonyl, -N(R¹²)R¹³, Cyan, Halogen, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, C₂-C₆-Alkenyl, C₃-C₁₁-Cycloalkyl, C₄-C₁₁-Cycloalkylmethyl, C₆-C₁₁-Aryl und C₇-C₁₁-Arylalkyl ausgewählt ist;
R⁸ ausgewählt ist aus R⁶, mit 0-3 R⁶ substituiertem C₁-C₁₀-Alkyl, mit 0-3 R⁶ substituiertem C₂-C₁₀-Alkenyl, mit 0-3 R⁶ substituiertem C₂-C₁₀-Alkinyl, mit 0-3 R⁶ substituiertem C₃-C₈-Cycloalkyl, mit 0-3 R⁶ substituiertem C₅-C₆-Cycloalkenyl, mit 0-3 R⁶ substituiertem Aryl;
einem 5-10-gliedrigen heterocyclischen Ring, der 1-3 N-, O- oder S-Heteroatome enthält, wobei der heterocyclische Ring gesättigt, partiell gesättigt oder vollständig ungesättigt sein kann, wobei der heterocyclische Ring mit 0-2 R⁶ substituiert ist;
R¹² und R¹³ unabhängig voneinander H, C₁-C₁₀-Alkyl, (C₁-C₁₀-Alkoxy)carbonyl, (C₁-C₁₀-Alkyl)carbonyl, C₁-C₁₀-Alkylsulfonyl, Aryl(C₁-C₁₀-alkyl)sulfonyl, Arylsulfonyl, Aryl(C₂-C₁₀-alkenyl)sulfonyl, Heteroarylsulfonyl, Aryl, C₂-C₆-Alkenyl, C₃-C₁₁-Cycloalkyl, C₄-C₁₁-Cycloalkylalkyl, C₇-C₁₁-Arylalkyl, C₇-C₁₁-Arylcarbonyl, C₄-C₁₁-Cycloalkoxycarbonyl, C₇-C₁₁-Bicycloalkoxycarbonyl, C₇-C₁₁-Aryloxycarbonyl, Heteroarylcarbonyl, Heteroarylalkylcarbonyl oder Aryl(C₁-C₁₀-alkoxy)carbonyl sind, wobei die Arylgruppen mit 0-3 Substituenten substituiert sind, die aus C₁-C₄-Alkyl, C₁-C₄-Alkoxy, Halogen, CF₃ und NO₂ ausgewählt sind;
R¹⁵ aus H, Hydroxy, C₁-C₁₀-Alkyl, C₂-C₁₀-Alkenyl, C₂-C₁₀-Alkinyl, C₁-C₁₀-Alkoxy, (C₁-C₁₀-Alkoxy)carbonyl, -CO₂R⁵ und -C(=O)N(R⁵)R^{5a} ausgewählt ist;
R¹⁶ aus -C(=O)-O-R^{18a}, -C(=O)-R^{18b}, -C(=O)N(R^{18b})₂, -C(=O)NHSO₂R^{18a}, -C(=O)NHC(=O)R^{18b}, -C(=O)NHC(=O)OR^{18a}, -C(=O)NHSO₂NHR^{18b}, -C(=S)-NH-R^{18b}, -NH-C(=O)-O-R^{18a}, -NH-C(=O)-R^{18b}, -NH-C(=O)-NH-R^{18b}, -SO₂-O-R^{18a}, -SO₂-R^{18a}, -SO₂-N(R^{18b})₂, -SO₂-NHC(=O)OR^{18b}, -P(=S)(OR^{18a})₂, -P(=O)(OR^{18a})₂, -P(=S)(R^{18a})₂, -P(=O)(R^{18a})₂ und 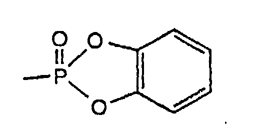 ausgewählt ist;
R¹⁷ aus H, C₁-C₁₀-Alkyl, C₂-C₆-Alkenyl, C₃-C₁₁-Cycloalkyl, C₄-C₁₅-Cycloalkyalkyl, Aryl und Aryl(C₁-C₁₀-alkyl)- ausgewählt ist;
R^{18a} ausgewählt ist aus mit 0-2 R¹⁹ substituiertem C₁-C₈-Alkyl, mit 0-2 R¹⁹ substituiertem C₂-C₈-Alkenyl, mit 0-2 R¹⁹ substituiertem C₂-C₈-Alkinyl, mit 0-2 R¹⁹ substituiertem C₃-C₈-Cycloalkyl, mit 0-4 R¹⁹ substituiertem Aryl, mit 0-4 R¹⁹ substituiertem Aryl(C₁-C₆-alkyl)-;
einem 5-10-gliedrigen heterocyclischen Ring mit 1-3 Heteroatomen, die unabhängig aus 0, S und N ausgewählt sind, wobei der heterocyclische Ring mit 0-4 R¹⁹ substituiert ist;
C₁-C₆-Alkyl, das mit einem 5-10-gliedrigen heterocyclischen Ring mit 1-3 Heteroatomen, die unabhängig aus O, S und N ausgewählt sind, substituiert ist, wobei der heterocyclische Ring mit 0-4 R¹⁹ substituiert ist;
R^{18b} R^{18a} oder H ist;
R¹⁹ aus H, Halogen, CF₃, CN, NO₂, NR¹²R¹³, C₁-C₈-Alkyl, C₂-C₆-Alkenyl, C₂-C₆-Alkinyl, C₃-C₁₁-Cycloalkyl, C₄-C₁₁-Cycloalkylalkyl, Aryl, Aryl(C₁-C₆-alkyl)-, C₁-C₆-Alkoxy und C₁-C₄-Alkoxycarbonyl ausgewählt ist;
m 0-2 ist,
n 0-4 ist,
n' 0-4 ist,
p' 1-7 ist,
p" 1-7 ist und
r 0-3 ist;
mit der Maßgabe, dass n' so gewählt wird, dass die Anzahl von Kettenatomen, die R¹ und Y miteinander verbinden, im Bereich von 8-18 liegt.

2. Verbindung gemäß Anspruch 1, wobei:
Z eine Bindung, O oder S ist;
R² H, Aryl(C₁-C₁₀-alkoxy)carbonyl oder C₂-C₁₀-Alkoxycarbonyl ist;
W -(CH₂)_{n'}-C(=O)-N(R^{5a})- ist;
X -(C(R⁴)₂)_{n'}-C(R⁴)(R⁸)-CH(R⁴)- ist, mit der Maßgabe, dass R⁸ kein H oder Methyl ist, wenn n' 0 oder 1 ist;
R⁵ H oder 0-6 R^{4b} substituiertes C₁-C₁₀-Alkyl ist;
R⁶ ausgewählt ist aus H, C₁-C₁₀-Alkyl, Hydroxy, C₁-C₁₀-Alkoxy, Nitro, (C₁-C₁₀-Alkyl)carbonyl, -N(R¹²)R¹³, -N(R⁵)R^{5a}, -CO₂R⁵, -S(O)ₘR⁵, -OR⁵, Cyan, Halogen;
C₆-C₁₀-Aryl, das gegebenenfalls mit 1-3 Gruppen substituiert ist, die aus Halogen, C₁-C₆-Alkoxy, C₁-C₆-Alkyl, CF₃, -S(O)ₘMe und -NMe₂ ausgewählt sind;
C₇-C₁₁-Arylalkyl, wobei das Aryl gegebenenfalls mit 1-3 Gruppen substituiert ist, die aus Halogen, C₁-C₆-Alkoxy, C₁-C₆-Alkyl, CF₃, S(O)ₘMe und -NMe₂ ausgewählt sind;
Methylendioxy, wenn R⁶ ein Substituent an Aryl ist;
einem 5-10-gliedrigen heterocyclischen Ring, der 1-3 N-, O- oder S-Heteroatome enthält, wobei der heterocyclische Ring gesättigt, partiell gesättigt oder vollständig ungesättigt sein kann, wobei der heterocyclische Ring mit 0-2 R⁷ substituiert ist;
R⁷ aus H, C₁-C₁₀-Alkyl, Hydroxy, C₁-C₁₀-Alkoxy, Nitro, (C₁-C₁₀-Alkyl)carbonyl, -N(R¹²)R¹³, Cyan und Halogen ausgewählt ist;
R⁸ ausgewählt ist aus -CON(R⁵)NR^{5a}, -CO₂R⁵, mit 0-3 R⁶ substituiertem C₁-C₁₀-Alkyl, mit 0-3 R⁶ substituiertem C₂-C₁₀-Alkenyl, mit 0-3 R⁶ substituiertem C₂-C₁₀-Alkinyl, mit 0-3 R⁶ substituiertem C₃-C₈-Cycloalkyl, mit 0-3 R⁶ substituiertem C₅-C₆-Cycloalkenyl, mit 0-2 R⁶ substituiertem Aryl;
einem 5-10-gliedrigen heterocyclischen Ring, der 1-3 N-, O- oder S-Heteroatome enthält, wobei der heterocyclische Ring gesättigt, partiell gesättigt oder vollständig ungesättigt sein kann, wobei der heterocyclische Ring mit 0-2 R⁶ substituiert ist;
R¹² und R¹³ jeweils unabhängig aus H, C₁-C₁₀-Alkyl, (C₁-C₁₀-Alkoxy)carbonyl, (C₁-C₁₀-Alkyl)carbonyl, C₁-C₁₀-Alkylsulfonyl, Aryl(C₁-C₁₀-alkyl)sulfonyl, Arylsulfonyl, Aryl, Heteroarylcarbonyl und Heteroarylalkylcarbonyl ausgewählt sind, wobei die Arylgruppen mit 0-3 Substituenten substituiert sind, die aus C₁-C₄-Alkyl, C₁-C₄-Alkoxy, Halogen, CF₃ und NO₂ ausgewählt sind; und
R¹⁵ aus H, C₁-C₁₀-Alkyl, C₂-C₁₀-Alkenyl, C₂-C₁₀-Alkinyl, C₁-C₁₀-Alkoxy, (C₁-C₁₀-Alkoxy)carbonyl, -CO₂R⁵ und -C(=O)N(R⁵)R^{5a} ausgewählt ist.

3. Verbindung gemäß Anspruch 2, wobei:
Z eine Bindung oder O ist;
W -(CH₂)_{n'}-C(=O)-N(R^{5a})- ist; und
X -C(R⁴)(R⁸)-CH(R⁴)- ist.

4. Verbindung gemäß Anspruch 2, wobei:
R¹ R²NHC(=NR²)- oder R²NHC(=NR²)NH- ist und V Phenylen oder Pyridylen ist; oder
R¹ 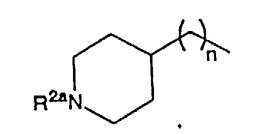 ist, V eine Einfachbindung ist und n 1 oder 2 ist;
X -CHR⁸CH₂- ist;
Y aus Hydroxy, C₁-C₁₀-Alkoxy, Methylcarbonyloxymethoxy-, Ethylcarbonyloxymethoxy-, t-Butylcarbonyloxymethoxy-, Cyclohexylcarbonyloxymethoxy-, 1-(Methylcarbonyloxy)ethoxy-, 1-(Ethylcarbonyloxy)ethoxy-, 1-(t-Butylcarbonyloxy)ethoxy-, 1-(Cyclohexylcarbonyloxy)ethoxy-, i-Propyloxycarbonyloxymethoxy-, t-Butyloxycarbonyloxymethoxy-, 1-(i-Propyloxycarbonyloxy)ethoxy-, 1-(Cyclohexyloxycarbonyloxy)ethoxy-, 1-(t-Butyloxycarbonyloxy)ethoxy-, Dimethylaminoethoxy-, Diethylaminoethoxy-, (5-Methyl-1,3-dioxacyclopenten-2-on-4-yl)methoxy-, (5-(t-Butyl)-1,3-dioxacyclopenten-2-on-4-yl)methoxy-, (1,3-Dioxa-5-phenylcyclopenten-2-on-4-yl)methoxy- und 1-(2-(2-Methoxypropyl)carbonyloxy)ethoxy- ausgewählt ist;
R⁶ ausgewählt ist aus H, C₁-C₄-Alkyl, Hydroxy, C₁-C₄-Alkoxy, Nitro, C₁-C₁₀-Alkylcarbonyl, -N(R¹²)R¹³, -N(R⁵)R^{5a}, -CO₂R⁵, -S(O)ₘR⁵, -OR⁵, Cyan, Halogen;
C₆-C₁₀-Aryl, das gegebenenfalls mit 1-3 Gruppen substituiert ist, die aus Halogen, C₁-C₆-Alkoxy, C₁-C₆-Alkyl, CF₃, -S(O)ₘMe und -NMe₂ ausgewählt sind;
Methylendioxy, wenn R⁶ ein Substituent an Aryl ist;
einem heterocyclischen Ringsystem, das aus Pyridinyl, Furanyl, Thiazolyl, Thienyl, Pyrrolyl, Pyrazolyl, Triazolyl, Imidazolyl, Benzofuranyl, Indolyl, Indolinyl, Chinolinyl, Isochinolinyl, Benzimidazolyl, Piperidinyl, Tetrahydrofuranyl, Pyranyl, Pyridinyl, 3H-Indolyl, Pyrrolidinyl, Piperidinyl, Indolinyl, Isoxazolinyl und Morpholinyl ausgewählt ist;
R⁸ ausgewählt ist aus -CON(R⁵)NR^{5a}, -CO₂R⁵, mit 0-3 R⁶ substituiertem C₁-C₁₀-Alkyl, mit 0-3 R⁶ substituiertem C₂-C₁₀-Alkenyl, mit 0-3 R⁶ substituiertem C₂-C₁₀-Alkinyl, mit 0-3 R⁶ substituiertem C₃-C₈-Cycloalkyl, mit 0-2 R⁶ substituiertem Aryl;
einem heterocyclischen Ringsystem, das aus Pyridinyl, Furanyl, Thiazolyl, Thienyl, Pyrrolyl, Pyrazolyl, Triazolyl, Imidazolyl, Benzofuranyl, Indolyl, Indolinyl, Chinolinyl, Isochinolinyl, Isoxazolinyl, Benzimidazolyl, Piperidinyl, Tetrahydrofuranyl, Pyranyl, Pyridinyl, 3H-Indolyl, Pyrrolidinyl, Piperidinyl, Indolinyl und Morpholinyl ausgewählt ist, wobei der heterocyclische Ring mit 0-2 R⁶ substituiert ist;
R¹² aus H, C₁-C₆-Alkyl, (C₁-C₄-Alkoxy)carbonyl, (C₁-C₆-Alkyl)carbonyl, C₁-C₆-Alkylsulfonyl, Aryl(C₁-C₄-alkyl)sulfonyl, Arylsulfonyl, Aryl, Pyridylcarbonyl und Pyridylmethylcarbonyl ausgewählt ist, wobei die Arylgruppen mit 0-3 Substituenten substituiert sind, die aus C₁-C₄-Alkyl, C₁-C₄-Alkoxy, Halogen, CF₃ und NO₂ ausgewählt sind; und
R¹³ H ist.

5. Verbindung gemäß Anspruch 1 oder eine pharmazeutisch annehmbare Salzform davon, ausgewählt aus:
3(R,S)-{5(R,S)-N-[3-(4-Amidinophenyl)isoxazolin-5-ylacetyl)amino)-3-phenylpropansäure;
3(R,S)-{5(R,S)-N-[3-(4-Amidinophenyl)isoxazolin-5-ylacetyl]amino}pentansäure;
3(R)-{5(R,S)-N-[3-(4-Amidinophenyl)isoxazolin-5-ylacetyl]amino}heptansäure;
3(R,S)-{5(R,S)-N-[3-(4-Amidinophenyl)isoxazolin-5-ylacetyl]amino}-4-(phenylthio)butansäure;
3(R,S)-{5(R,S)-N-[3-(4-Amidinophenyl)isoxazolin-5-ylacetyl]amino}-4-(phenylsulfonamido)butansäure;
3(R,S)-{5(R,S)-N-[3-(4-Amidinophenyl)isoxazolin-5-ylacetyl]amino}-4-(n-butylsulfonamido)butansäure;
3(S)-{5(R,S)-N-[3-(4-Amidinophenyl)isoxazolin-5-ylacetyl]amino}-3-(adamantylmethylaminocarbonyl)propansäure;
3(S)-{5(R,S)-N-[3-(4-Amidinophenyl)isoxazolin-5-ylacetyl]amino}-3-(1-azabicyclo[3.2.2]nonylcarbonyl)propansäure;
3(S)-{5(R,S)-N-[3-(4-Amidinophenyl)isoxazolin-5-ylacetyl]amino}-3-(phenethylaminocarbonyl)propansäure;
3(R)-{5(R,S)-N-[3-(4-Amidinophenyl)isoxazolin-5-ylacetyl]amino}-3-(3-pyridylethyl)propansäure;
3(R)-{5(R,S)-N-[3-(4-Amidinophenyl)isoxazolin-5-ylacetyl]amino}-3-(2-pyridylethyl)propansäure; und
3(R)-{5(R,S)-N-[3-(4-Amidinophenyl)isoxazolin-5-ylacetyl]amino}-3-(phenylpropyl)propansäure.

6. Verbindung gemäß Anspruch 1, wobei
Z eine Bindung, O oder S ist;
R² und R³ unabhängig aus H, Aryl(C₁-C₁₀-alkoxy)carbonyl und (C₁-C₁₀-Alkoxy)carbonyl ausgewählt sind;
R^{2a} R² oder R²(R³)N(R²N=)C- ist;
W -C(R⁴)₂-C(=O)-N(R^{5a})- oder -C(=O)-N(R^{5a})-C(R⁴)₂- ist;
X -C(R⁴)(R⁸)-CHR^{4a}- ist;
R⁴ aus H, C₁-C₁₀-Alkyl, C₁-C₁₀-Alkylcarbonyl, Aryl, Arylalkyl, Cycloalkyl und Cycloalkylalkyl ausgewählt ist;
R^{4a} aus Hydroxy, C₁-C₁₀-Alkoxy, Nitro, -N(R⁵)R^{5a}, -N(R¹²)R¹³, -N(R¹⁶)R¹⁷, mit 0-3 R⁶ substituiertem C₁-C₁₀-Alkyl, mit 0-3 R⁶ substituiertem Aryl und C₁-C₁₀-Alkylcarbonyl ausgewählt ist;
R^{4b} aus H, C₁-C₆-Alkyl, C₂-C₆-Alkenyl, C₂-C₆-Alkinyl, Hydroxy, C₁-C₆-Alkoxy, C₁-C₆-Alkylthio, C₁-C₆-Alkylsulfinyl, C₁-C₆-Alkylsulfonyl, Nitro, (C₁-C₆-Alkyl)carbonyl, C₆-C₁₀-Aryl, -N(R¹²)R¹³, Halogen, CF₃, CN, (C₁-C₆-Alkoxy)carbonyl, Carboxy, Piperidinyl, Morpholinyl und Pyridinyl ausgewählt ist;
R⁵ mit 0-2 R^{4b} substituiertes C₁-C₁₀-Alkyl ist;
R^{5a} aus H, Hydroxy, C₁-C₈-Alkyl, C₃-C₆-Alkenyl, C₃-C₁₁-Cycloalkyl, C₄-C₁₁-Cycloalkylmethyl, C₁-C₆-Alkoxy, Benzyloxy, C₆-C₁₀-Aryl, Heteroaryl, Heteroarylalkyl, C₇-C₁₁-Arylalkyl, Adamantylmethyl und mit 0-2 R^{4b} substituiertem C₁-C₁₀-Alkyl ausgewählt ist;
alternativ dazu R⁵ und R^{5a} zusammengenommen werden können unter Bildung von 3-Azabicyclononyl, 1,2,3,4-Tetrahydro-1-chinolinyl, 1,2,3,4-Tetrahydro-2-isochinolinyl, 1-Piperidinyl, 1-Morpholinyl, 1-Pyrrolidinyl, Thiamorpholinyl, Thiazolidinyl oder 1-Piperazinyl, wobei jedes gegebenenfalls mit C₁-C₆-Alkyl, C₆-C₁₀-Aryl, Heteroaryl, C₇-C₁₁-Arylalkyl, (C₁-C₆-Alkyl)carbonyl, (C₃-C₇-Cycloalkyl)carbonyl, (C₁-C₆-Alkoxy)carbonyl oder (C₇-C₁₁-Arylalkoxy)carbonyl substituiert ist;
Y aus Hydroxy, C₁-C₁₀-Alkyloxy, C₃-C₁₁-Cycloalkyloxy, C₆-C₁₀-Aryloxy, C₇-C₁₁-Aralkyloxy, C₃-C₁₀-Alkylcarbonyloxyalkyloxy, C₃-C₁₀-Alkoxycarbonyloxyalkyloxy, C₂-C₁₀-Alkoxycarbonylalkyloxy, C₅-C₁₀-Cycloalkylcarbonyloxyalkyloxy, C₅-C₁₀-Cycloalkoxycarbonyloxyalkyloxy, C₅-C₁₀-Cycloalkoxycarbonylalkyloxy, C₇-C₁₁-Aryloxycarbonylalkyloxy, C₈-C₁₂-Aryloxycarbonyloxyalkyloxy, C₈-C₁₂-Arylcarbonyloxyalkyloxy, C₅-C₁₀-Alkoxyalkylcarbonyloxyalkyloxy, C₅-C₁₀-(5-Alkyl-1,3-dioxacyclopenten-2-onyl)methyloxy und C₁₀-C₁₄-(5-Aryl-1,3-dioxacyclopenten-2-onyl)methyloxy ausgewählt ist;
R⁶ und R⁷ jeweils unabhängig aus H, C₁-C₁₀-Alkyl, Hydroxy, C₁-C₁₀-Alkoxy, Nitro, (C₁-C₁₀-Alkyl)carbonyl, -N(R¹²)R¹³, Cyan und Halogen ausgewählt sind;
R¹² und R¹³ jeweils unabhängig aus H, C₁-C₁₀-Alkyl, (C₁-C₁₀-Alkoxy)carbonyl, (C₁-C₁₀-Alkyl)carbonyl, C₁-C₁₀-Alkylsulfonyl, Aryl(C₁-C₁₀-alkyl)sulfonyl, Arylsulfonyl, Heteroarylcarbonyl, Heteroarylalkylcarbonyl und Aryl ausgewählt sind, wobei die Arylgruppen mit 0-3 Substituenten substituiert sind, die aus C₁-C₄-Alkyl, C₁-C₄-Alkoxy, Halogen, CF₃ und NO₂ ausgewählt sind;
R¹⁵ aus H, C₁-C₁₀-Alkyl, C₂-C₁₀-Alkenyl, C₂-C₁₀-Alkinyl, C₁-C₁₀-Alkoxy, (C₁-C₁₀-Alkoxy)carbonyl, -CO₂R⁵ und -C(=O)N(R⁵)R^{5a} ausgewählt ist;
R¹⁶ aus -C(=O)-O-R^{18a}, -C(=O)-R^{18b}, -C(=O)N(R^{18b})₂, -SO₂-R^{18a} und -SO₂-N(R^{18b})₂ ausgewählt ist;
R¹⁷ H oder C₁-C₄-Alkyl ist;
R^{18a} ausgewählt ist aus mit 0-2 R¹⁹ substituiertem C₁-C₈-Alkyl, mit 0-2 R¹⁹ substituiertem C₂-C₈-Alkenyl, mit 0-2 R¹⁹ substituiertem C₂-C₈-Alkinyl, mit 0-2 R¹⁹ substituiertem C₃-C₈-Cycloalkyl, mit 0-4 R¹⁹ substituiertem Aryl, mit 0-4 R¹⁹ substituiertem Aryl(C₁-C₆-alkyl)-;
einem heterocyclischen Ringsystem, das aus Pyridinyl, Furanyl, Thiazolyl, Thienyl, Pyrrolyl, Pyrazolyl, Triazolyl, Imidazolyl, Benzofuranyl, Indolyl, Indolinyl, Chinolinyl, Isochinolinyl, Isoxazolinyl, Benzimidazolyl, Piperidinyl, Tetrahydrofuranyl, Pyranyl, Pyrimidinyl, 3H-Indolyl, Pyrrolidinyl, Piperidinyl, Indolinyl und Morpholinyl ausgewählt ist, wobei der heterocyclische Ring mit 0-4 R¹⁹ substituiert ist;
C₁-C₆-Alkyl, das mit einem heterocyclischen Ringsystem substituiert ist, das aus Pyridinyl, Furanyl, Thiazolyl, Thienyl, Pyrrolyl, Pyrazolyl, Imidazolyl, Isoxazolinyl, Benzofuranyl, Indolyl, Indolinyl, Chinolinyl, Isochinolinyl, Benzimidazolyl, Piperidinyl, Tetrahydrofuranyl, Pyranyl, Pyridinyl, 3H-Indolyl, Indolyl, Pyrrolidinyl, Piperidinyl, Indolinyl und Morpholinyl ausgewählt ist, wobei der heterocyclische Ring mit 0-4 R¹⁹ substituiert ist; und
die Variablen V, Q, R¹, R^{5b}, R^{18b}, R¹⁹, b, n, p', p" und r wie in Anspruch 1 definiert sind.

7. Verbindung gemäß Anspruch 6 mit der Formel Ib: 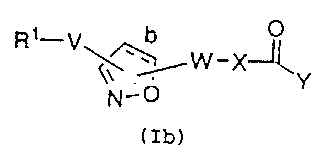 wobei:
R¹ aus R^{2a}(R³)N-, R²NH(R²N=)C-, R²NH(R²N=)CNH-, R^{2a}(R³)N(CH₂)_{p'}Z-, R²NH(R²N=)C(CH₂)_{p"}Z-, 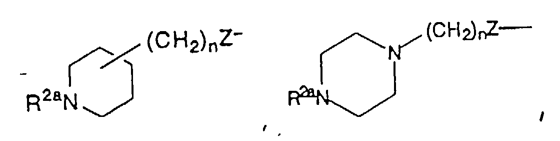 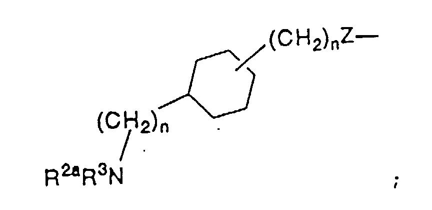 ausgewählt ist;
n 0-1 ist;
p' 4-6 ist;
p" 2-4 ist;
Z eine Bindung oder O ist;
V eine Einfachbindung, -(Phenyl)- oder -(Pyridinyl)- ist;
W -C(R⁴)₂-C(=O)-N(R^{5a})- oder -C(=O)-N(R^{5a})-CH₂- ist;
X -CH₂-CH(N(R¹⁶)R¹⁷)- oder -CH₂-CH(N(R⁵)R^{5a})- ist;
Y aus Hydroxy, C₁-C₁₀-Alkoxy, Methylcarbonyloxymethoxy-, t-Butylcarbonyloxymethoxy-, Cyclohexylcarbonyloxymethoxy-, 1-(Methylcarbonyloxy)ethoxy-, 1-(Ethylcarbonyloxy)ethoxy-, 1-(t-Butylcarbonyloxy)ethoxy-, 1-(Cyclohexylcarbonyloxy)ethoxy-, i-Propyloxycarbonyloxymethoxy-, t-Butyloxycarbonyloxymethoxy-, 1-(i-Propyloxycarbonyloxy)ethoxy-, 1-(Cyclohexyloxycarbonyloxy)ethoxy-, 1-(t-Butyloxycarbonyloxy)ethoxy-, Dimethylaminoethoxy-, Diethylaminoethoxy-, (5-Methyl-1,3-dioxacyclopenten-2-on-4-yl)methoxy-, (5-(t-Butyl)-1,3-dioxacyclopenten-2-on-4-yl)methoxy-, (1,3-Dioxa-5-phenylcyclopenten-2-on-4-yl)methoxy- und 1-(2-(2-Methoxypropyl)carbonyloxy)ethoxy- ausgewählt ist;
R¹⁶ aus -C(=O)-O-R^{18a}, -C(=O)-R^{18b}, -S(=O)₂-R^{18a} und -SO₂-N(R^{18b})₂ ausgewählt ist;
R¹⁷ H oder C₁-C₅-Alkyl ist;
R^{18a} ausgewählt ist aus mit 0-2 R¹⁹ substituiertem C₁-C₈-Alkyl, mit 0-2 R¹⁹ substituiertem C₂-C₈-Alkenyl, mit 0-2 R¹⁹ substituiertem C₂-C₈-Alkinyl, mit 0-2 R¹⁹ substituiertem C₃-C₈-Cycloalkyl, mit 0-4 R¹⁹ substituiertem Aryl, mit 0-4 R¹⁹ substituiertem Aryl(C₁-C₆-alkyl)-;
einem heterocyclischen Ringsystem, das aus Pyridinyl, Furanyl, Thiazolyl, Thienyl, Pyrrolyl, Pyrazolyl, Triazolyl, Imidazolyl, Benzofuranyl, Indolyl, Indolinyl, Chinolinyl, Isochinolinyl, Isoxazolinyl, Benzimidazolyl, Piperidinyl, Tetrahydrofuranyl, Pyranyl, Pyridinyl, 3H-Indolyl, Carbazol, Pyrrolidinyl, Piperidinyl, Indolinyl und Morpholinyl ausgewählt ist, wobei der heterocyclische Ring mit 0-4 R¹⁹ substituiert ist;
C₁-C₆-Alkyl, das mit einem heterocyclischen Ringsystem substituiert ist, das aus Pyridinyl, Furanyl, Thiazolyl, Thienyl, Pyrrolyl, Pyrazolyl, Imidazolyl, Isoxazolinyl, Benzofuranyl, Indolyl, Indolinyl, Chinolinyl, Isochinolinyl, Benzimidazolyl, Piperidinyl, Tetrahydrofuranyl, Pyranyl, Pyridinyl, 3H-Indolyl, Indolyl, Pyrrolidinyl, Piperidinyl, Indolinyl und Morpholinyl ausgewählt ist, wobei der heterocyclische Ring mit 0-4 R¹⁹ substituiert ist.

8. Verbindung gemäß Anspruch 6, wobei:
R¹ R²NH(R²N=)C- oder R²HN(R²N=)CNH- ist und V Phenylen oder Pyridylen ist; oder
R¹ 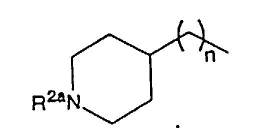 ist, V eine Einfachbindung ist und n 1 oder 2 ist; und
R^{18a} ausgewählt ist aus mit 0-2 R¹⁹ substituiertem C₁-C₈-Alkyl, mit 0-2 R¹⁹ substituiertem C₂-C₈-Alkenyl, mit 0-2 R¹⁹ substituiertem C₂-C₈-Alkinyl, mit 0-2 R¹⁹ substituiertem C₃-C₈-Cycloalkyl, mit 0-4 R¹⁹ substituiertem Aryl, mit 0-4 R¹⁹ substituiertem Aryl(C₁-C₆-alkyl)-;
einem heterocyclischen Ringsystem, das aus Pyridinyl, Furanyl, Thiazolyl, Thienyl, Pyrrolyl, Pyrazolyl, Triazolyl, Imidazolyl, Benzofuranyl, Indolyl, Indolinyl, Chinolinyl, Isochinolinyl, Isoxazolinyl, Benzimidazolyl, Piperidinyl, Tetrahydrofuranyl, Pyranyl, Pyrimidinyl, 3H-Indolyl, Pyrrolidinyl, Piperidinyl, Indolinyl, Isoxazolinyl und Morpholinyl ausgewählt ist, wobei der heterocyclische Ring mit 0-4 R¹⁹ substituiert ist;
C₁-C₄-Alkyl, das mit einem heterocyclischen Ringsystem substituiert ist, das aus Pyridinyl, Furanyl, Thiazolyl, Thienyl, Pyrrolyl, Pyrazolyl, Imidazolyl, Isoxazolinyl, Benzofuranyl, Indolyl, Indolinyl, Chinolinyl, Isochinolinyl, Benzimidazolyl, Piperidinyl, Tetrahydrofuranyl, Pyranyl, Pyridinyl, 3H-Indolyl, Indolyl, Pyrrolidinyl, Piperidinyl, Indolinyl, Isoxazolinyl und
Morpholinyl ausgewählt ist, wobei der heterocyclische Ring mit 0-4 R¹⁹ substituiert ist.

9. Verbindung gemäß Anspruch 1 oder pharmazeutisch annehmbare Salzformen davon, ausgewählt aus:
N³-[2-{3-(4-Formamidinophenyl)isoxazolin-5(R,S)-yl)acetyl]-N²-(phenylsulfonyl)-2,3(S)-diaminopropansäure;
N³-[2-{3-(4-Formamidinophenyl)isoxazolin-5(R,S)-yl}acetyl]-N²-(4-methylphenylsulfonyl)-2,3(S)-diaminopropansäure;
N³-[2-{3-(4-Formamidinophenyl)isoxazolin-5(R,S)-yl}acetyl]-N²-(butansulfonyl)-2,3(S)-diaminopropansäure;
N³-[2-{3-(4-Formamidinophenyl)isoxazolin-5(R,S)-yl}acetyl]-N²-(propansulfonyl)-2,3(S)-diaminopropansäure;
N³-[2-[3-(4-Formamidinophenyl)isoxazolin-5(R,S)-yl}acetyl]-N²-(ethansulfonyl)-2,3(S)-diaminopropansäure;
N³-[2-{3-(4-Formamidinophenyl)isoxazolin-5(R,S)-yl}acetyl]-N²-(methyloxycarbonyl)-2,3(S)-diaminopropansäure;
N³-[2-{3-(4-Formamidinophenyl)isoxazolin-5(R,S)-yl}acetyl]-N²-(ethyloxycarbonyl)-2,3(S)-diaminopropansäure;
N³-[2-{3-(4-Formamidinophenyl)isoxazolin-5(R,S)-yl}acetyl]-N²-(1-propyloxycarbonyl)-2,3(S)-diaminopropansäure;
N³-[2-{3-(4-Formamidinophenyl)isoxazolin-5(R,S)-yl}acetyl]-N²-(2-propyloxycarbonyl)-2,3(S)-diaminopropansäure;
N³-[2-{3-(4-Formamidinophenyl)isoxazolin-5(R,S)-yl}acetyl]-N²-(n-butyloxycarbonyl)-2,3(S)-diaminopropansäure;
N³-[2-{3-(4-Formamidinophenyl)isoxazolin-5(R)-yl}acetyl]-N²-(n-butyloxycarbonyl)-2,3(S)-diaminopropansäure;
N³-[2-{3-(4-Formamidinophenyl)isoxazolin-5(S)-yl}acetyl]-N²-(n-butyloxycarbonyl)-2,3(S)-diaminopropansäure;
N³-[2-{3-(4-Formamidinophenyl)isoxazolin-5(R)-yl}acetyl]-N²-(n-butyloxycarbonyl)-2,3(R)-diaminopropansäure;
N³-[2-{3-(4-Formamidinophenyl)isoxazolin-5(S)-yl}acetyl]-N²-(n-butyloxycarbonyl)-2,3(R)-diaminopropansäure;
N³-[2-{3-(4-Formamidinophenyl)isoxazolin-5(R,S)-yl}acetyl]-N²-(2-butyloxycarbonyl)-2,3(S)-diaminopropansäure;
N³-[2-{3-(4-Formamidinophenyl)isoxazolin-5(R,S)-yl}acetyl)-N²-(1-(2-methyl)propyloxycarbonyl)-2,3(S)-diaminopropansäure;
N³-[2-{3-(4-Formamidinophenyl)isoxazolin-5(R,S)-yl}acetyl]-N²-(2-(2-methyl)propyloxycarbonyl)-2,3(S)-diaminopropansäure;
N³-[2-{3-(4-Formamidinophenyl)isoxazolin-5(R,S)-yl}acetyl]-N²-(benzyloxycarbonyl)-2,3(S)-diaminopropansäure;
N³-[2-{3-(4-Formamidinophenyl)isoxazolin-5(R)-yl}acetyl]-N²-(benzyloxycarbonyl)-2,3(S)-diaminopropansäure;
N³-[2-{3-(4-Formamidinophenyl)isoxazolin-5(S)-yl}acetyl]-N²-(benzyloxycarbonyl)-2,3(S)-diaminopropansäure;
N³-[2-{3-(4-Formamidinophenyl)isoxazolin-5(R,S)-yl}acetyl]-N²-(4-methylbenzyloxycarbonyl)-2,3(S)-diaminopropansäure;
N³-[2-{3-(4-Formamidinophenyl)isoxazolin-5(R,S)-yl}acetyl)-N²-(4-methoxybenzyloxycarbonyl)-2,3(S)-diaminopropansäure;
N³-[2-{3-(4-Formamidinophenyl)isoxazolin-5(R,S)-yl}acetyl)-N²-(4-chlorbenzyloxycarbonyl)-2,3(S)-diaminopropansäure;
N³-[2-{3-(4-Formamidinophenyl)isoxazolin-5(R,S)-yl}acetyl]-N²-(4-brombenzyloxycarbonyl)-2,3(S)-diaminopropansäure;
N³-[2-{3-(4-Formamidinophenyl)isoxazolin-5(R,S)-yl}acetyl)-N²-(4-fluorbenzyloxycarbonyl)-2,3(S)-diaminopropansäure;
N³-(2-{3-(4-Formamidinophenyl)isoxazolin-5(R,S)-yl}acetyl]-N²-(4-phenoxybenzyloxycarbonyl)-2,3(S)-diaminopropansäure;
N³-[2-{3-(4-Formamidinophenyl)isoxazolin-5(R,S)-yl}acetyl]-N²-(2-(methyloxyethyl)oxycarbonyl)-2,3(S)-diaminopropansäure;
N³-(2-{3-(4-Formamidinophenyl)isoxazolin-5(R,S)-yl}acetyl]-N²-(2-pyridinylcarbonyl)-2,3(S)-diaminopropansäure;
N³-[2-{3-(4-Formamidinophenyl)isoxazolin-5(R,S)-yl}acetyl]-N²-(3-pyridinylcarbonyl)-2,3(S)-diaminopropansäure;
N³-[2-{3-(4-Formamidinophenyl)isoxazolin-5(R,S)-yl}acetyl]-N²-(4-pyridinyl-carbonyl)-2,3(S)-diaminopropansäure;
N³-[2-{3-(4-Formamidinophenyl)isoxazolin-5(R,S)-yl}acetyl]-N²-(2-(2-pyridinyl)acetyl)-2,3(S)-diaminopropansäure;
N³-(2-{3-(4-Formamidinophenyl)isoxazolin-5(R,S)-yl}acetyl]-N²-(2-(3-pyridinyl)acetyl)-2,3(S)-diaminopropansäure;
N³-[2-{3-(4-Formamidinophenyl)isoxazolin-5(R,S)-yl}acetyl]-N²-(2-(4-pyridinyl)acetyl)-2,3(S)-diaminopropansäure;
N³-[2-{3-(4-Formamidinophenyl)isoxazolin-5(R,S)-yl}acetyl]-N²-(2-pyridylmethyloxycarbonyl)-2,3(S)-diaminopropansäure;
N³-[2-{3-(4-Formamidinophenyl)isoxazolin-5(R,S)-yl}acetyl]-N²-(3-pyridylmethyloxycarbonyl)-2,3(S)-diaminopropansäure;
N³-[2-{3-(4-Formamidinophenyl)isoxazolin-5(R,S)-yl}acetyl]-N²-(4-pyridylmethyloxycarbonyl)-2,3(S)-diaminopropansäure;
N³-(2-{3-(4-Formamidinophenyl)isoxazolin-5(R,S)-yl}acetyl]-N²-(4-butyloxyphenylsulfonyl)-2,3(S)-diaminopropansäure;
N³-[2-{3-(4-Formamidinophenyl)isoxazolin-5(R,S)-yl}acetyl]-N²-(2-thienylsulfonyl)-2,3(S)-diaminopropansäure;
N³-[2-{3-(4-Formamidinophenyl)isoxazolin-5(R,S)-yl}acetyl]-N²-(3-methylphenylsulfonyl)-2,3(R,S)-diaminopropansäure;
N³-[2-{3-(4-Formamidinophenyl)isoxazolin-5(R,S)-yl}acetyl]-N²-(3-methylphenylsulfonyl)-2,3(S)-diaminopropansäure;
N³-[2-{3-(4-Formamidinophenyl)isoxazolin-5(R,S)-yl}acetyl]-N²-(3-methylphenylsulfonyl)-2,3(R)-diaminopropansäure;
N³-[2-{3-(4-Formamidinophenyl)isoxazolin-5(R)-yl}acetyl]-N²-(3-methylphenylsulfonyl)-2,3(S)-diaminopropansäure;
N³-[2-{3-(4-Formamidinophenyl)isoxazolin-5(S)-yl}acetyl]-N²-(3-methylphenylsulfonyl)-2,3(S)-diaminopropansäure;
N³-[2-{3-(4-Formamidinophenyl)isoxazolin-5(S)-yl}acetyl]-N²-(3-methylphenylsulfonyl)-2,3(R)-diaminopropansäure;
N³-[2-{3-(4-Formamidinophenyl)isoxazolin-5(R)-yl}acetyl]-N²-(3-methylphenylsulfonyl)-2,3(R)-diaminopropansäure;
N³-[2-{3-(4-Formamidinophenyl)isoxazolin-5(R,S)-yl}acetyl]-N²-(4-iodphenylsulfonyl)-2,3(S)-diaminopropansäure;
N³-(2-{3-(4-Formamidinophenyl)isoxazolin-5(R,S)-yl}acetyl]-N²-(3-trifluormethylphenylsulfonyl)-2,3(S)-diaminopropansäure;
N³-[2-{3-(4-Formamidinophenyl)isoxazolin-5(R,S)-yl}acetyl]-N²-(3-chlorphenylsulfonyl)-2,3(S)-diaminopropansäure;
N³-[2-{3-(4-Formamidinophenyl)isoxazolin-5(R,S)-yl}acetyl]-N²-(3-2-methoxycarbonylphenylsulfonyl)-2,3(S)-diaminopropansäure;
N³-[2-{3-(4-Formamidinophenyl)isoxazolin-5(R,S)-yl}acetyl]-N²-(2,4,6-trimethylphenylsulfonyl)-2,3(S)-diaminopropansäure;
N³-(2-{3-(4-Formamidinophenyl)isoxazolin-5(R,S)-yl}acetyl]-N²-(2-chlorphenylsulfonyl)-2,3(S)-diaminopropansäure;
N³-(2-{3-(4-Formamidinophenyl)isoxazolin-5(R,S)-yl}acetyl]-N²-(4-trifluormethylphenylsulfonyl)-2,3(S)-diaminopropansäure;
N³-[2-{3-(4-Formamidinophenyl)isoxazolin-5(R,S)-yl}acetyl]-N²-(2-trifluormethylphenylsulfonyl)-2,3(S)-diaminopropansäure;
N³-[2-{3-(4-Formamidinophenyl)isoxazolin-5(R,S)-yl}acetyl]-N²-(2-fluorphenylsulfonyl)-2,3(S)-diaminopropansäure;
N³-[2-{3-(4-Formamidinophenyl)isoxazolin-5(R,S)-yl}acetyl]-N²-(4-fluorphenylsulfonyl)-2,3(S)-diaminopropansäure;
N³-(2-{3-(4-Formamidinophenyl)isoxazolin-5(R,S)-yl}acetyl]-N²-(4-methoxyphenylsulfonyl)-2,3(S)-diaminopropansäure;
N³-[2-{3-(4-Formamidinophenyl)isoxazolin-5(R,S)-yl}acetyl]-N²-(2,3,5,6-tetramethylphenylsulfonyl)-2,3(S)-diaminopropansäure;
N³-(2-{3-(4-Formamidinophenyl)isoxazolin-5(R,S)-yl}acetyl]-N²-(4-cyanphenylsulfonyl)-2,3(S)-diaminopropansäure;
N³-[2-{3-(4-Formamidinophenyl)isoxazolin-5(R,S)-yl}acetyl]-N²-(4-chlorphenylsulfonyl)-2,3(S)-diaminopropansäure;
N³-(2-{3-(4-Formamidinophenyl)isoxazolin-5(R,S)-yl}acetyl]-N²-(4-propylphenylsulfonyl)-2,3(S)-diaminopropansäure;
N³-[2-{3-(4-Formamidinophenyl)isoxazolin-5(R,S)-yl}acetyl]-N²-(2-phenylethylsulfonyl)-2,3(S)-diaminopropansäure;
N³-[2-{3-(4-Formamidinophenyl)isoxazolin-5(R,S)-yl}acetyl]-N²-(4-isopropylphenylsulfonyl)-2,3(S)-diaminopropansäure;
N³-[2-{3-(4-Formamidinophenyl)isoxazolin-5(R,S)-yl}acetyl]-N²-(3-phenylpropylsulfonyl)-2,3(S)-diaminopropansäure;
N³-[2-{3-(4-Formamidinophenyl)isoxazolin-5(R,S)-yl}acetyl]-N²-(3-pyridylsulfonyl)-2,3(S)-diaminopropansäure;
N³-[2-{3-(4-Formamidinophenyl)isoxazolin-5(R,S)-yl}acetyl]-N²-(phenylaminosulfonyl)-2,3(S)-diaminopropansäure;
N³-[2-{3-(4-Formamidinophenyl)isoxazolin-5(R,S)-yl}acetyl)-N²-(benzylaminosulfonyl)-2,3(S)-diaminopropansäure;
N³-[2-{3-(4-Formamidinophenyl)isoxazolin-5(R,S)-yl}acetyl]-N²-(dimethylaminosulfonyl)-2,3(S)-diaminopropansäure;
N³-[2-{3-(2-Fluor-4-formamidinophenyl)isoxazolin-5(R,S)-yl}acetyl]-N²-(3-methylphenylsulfonyl)-2,3(S)-diaminopropansäure;
N³-[2-{3-(2-Formamidino-5-pyridinyl)isoxazolin-5(R,S)-yl}acetyl]-N²-(n-butyloxycarbonyl)-2,3(S)-diaminopropansäure;
N³-[2-{3-(2-Formamidino-5-pyridinyl)isoxazolin-5(R,S)-yl}acetyl]-N²-(3-methylphenylsulfonyl)-2,3(S)-diaminopropansäure;
N³-[2-{3-(3-Formamidino-6-pyridinyl)isoxazolin-5(R,S)-yl}acetyl]-N²-(n-butyloxycarbonyl)-2,3(S)-diaminopropansäure;
N³-[2-{3-(3-Formamidino-6-pyridinyl)isoxazolin-5(R,S)-yl}acetyl]-N²-(3-methylphenylsulfonyl)-2,3(S)-diaminopropansäure;
N³-[2-(3-(4-Formamidinophenyl)isoxazolin-5(R,S)-yl}acetyl}-N²-(phenylaminocarbonyl)-2,3(S)-diaminopropansäure;
N³-[2-{3-(4-Formamidinophenyl)isoxazolin-5(R,S)-yl}acetyl}-N²-(4-fluorphenylaminocarbonyl)-2,3(S)-diaminopropansäure;
N³-[2-{3-(4-Formamidinophenyl)isoxazolin-5(R,S)-yl}acetyl]-N²-(1-naphthylaminocarbonyl)-2,3(S)-diaminopropansäure;
N³-[2-{3-(4-Formamidinophenyl)isoxazolin-5(R,S)-yl}acetyl)-N²-(benzylaminocarbonyl)-2,3(S)-diaminopropansäure;
N³-[2-{3-(4-Formamidinophenyl)isoxazolin-5(R,S)-yl}acetyl]-N²-(3-brom-2-thienylsulfonyl)-2,3(S)-diaminopropansäure;
N³-[2-{3-(4-Formamidinophenyl)isoxazolin-5(R,S)-yl}acetyl]-N²-(3-methyl-2-benzothienylsulfonyl)-2,3(S)-diaminopropansäure;
N³-[2-{3-(4-Formamidinophenyl)isoxazolin-5(R,S)-yl}acetyl]-N²-(isobutyloxycarbonyl)-2,3(S)-diaminopropansäure;
N³-[2-{3-(4-Formamidinophenyl)isoxazolin-5(R)-yl}acetyl]-N²-(isobutyloxycarbonyl)-2,3(S)-diaminopropansäure;
N³-[2-{3-(4-Formamidinophenyl)isoxazolin-5(S)-yl}acetyl]-N²-(isobutyloxycarbonyl)-2,3(S)-diaminopropansäure;
N³-[2-{3-(4-Formamidinophenyl)isoxazolin-5(R,S)-yl}acetyl]-N²-(2-cyclopropylethoxycarbonyl)-2,3(S)-diaminopropansäure;
N³-[2-{3-(4-Formamidinophenyl)isoxazolin-5(R)-yl}acetyl]-N²-(2-cyclopropylethoxycarbonyl)-2,3(S)-diaminopropansäure;
N³-[2-{3-(4-Formamidinophenyl)isoxazolin-5(S)-yl}acetyl]-N²-(2-cyclopropylethoxycarbonyl)-2,3(S)-diaminopropansäure;
N³-[2-{3-(4-Guanidinophenyl)isoxazolin-5(R,S)-yl}acetyl]-N²-(n-butyloxycarbonyl)-2,3(S)-diaminopropansäure;
N³-[2-(3-(4-Guanidinophenyl)isoxazolin-5(R)-yl}acetyl]-N²-(n-butyloxycarbonyl)-2,3(S)-diaminopropansäure;
N³-[2-{3-(4-Guanidinophenyl)isoxazolin-5(R)-yl}acetyl]-N²-(3-methylphenylsulfonyl)-2,3(S)-diaminopropansäure; und
N³-[2-{5-(4-Formamidinophenyl)isoxazolin-3(R,S)-yl}acetyl]-N²-(n-butyloxycarbonyl)-2,3(S)-diaminopropansäure.

10. Medikamentenvorstufe in Form eines Esters einer Verbindung gemäß Anspruch 9, wobei der Ester ausgewählt ist aus:
Methyl, Ethyl, Isopropyl, Methylcarbonyloxymethyl-, Ethylcarbonyloxymethyl-, t-Butylcarbonyloxymethyl-, Cyclohexylcarbonyloxymethyl-, 1-(Methylcarbonyloxy)ethyl-, 1-(Ethylcarbonyloxy)ethyl-, 1-(t-Butylcarbonyloxy)ethyl-, 1-(Cyclohexylcarbonyloxy)ethyl-, i-Propyloxycarbonyloxymethyl-, Cyclohexylcarbonyloxymethyl-, t-Butyloxycarbonyloxymethyl-, 1-(i-Propyloxycarbonyloxy)ethyl-, 1-(Cyclohexyloxycarbonyloxy)ethyl, 1-(t-Butyloxycarbonyloxy)ethyl-, Dimethylaminoethyl-, Diethylaminoethyl-, (5-Methyl-1,3-dioxacyclopenten-2-on-4-yl)methyl-, (5-(t-Butyl)-1,3-dioxacyclopenten-2-on-4-yl)methyl-, (1,3-Dioxa-5-phenylcyclopenten-2-on-4-yl)methyl- und 1-(2-(2-Methoxypropyl)carbonyloxy)ethyl-.

11. Verbindung gemäß Anspruch 1 oder pharmazeutisch annehmbare Salzformen davon, und zwar:
Methyl-N³-[2(3-(4-formamidinophenyl)isoxazolin-5(R)-yl)acetyl]-N²-(n-butyloxycarbonyl)-2,3(S)-diaminopropionat.

12. Pharmazeutische Zusammensetzung, die eine therapeutisch wirksame Menge einer Verbindung gemäß einem der Ansprüche 1 bis 11 sowie einen pharmazeutisch annehmbaren Träger umfasst.

13. Verwendung einer Verbindung gemäß einem der Ansprüche 1 bis 11 zur Herstellung eines Medikaments zur Prävention oder Behandlung von Thrombose.

14. Verwendung einer Verbindung gemäß einem der Ansprüche 1 bis 11 zur Herstellung eines Medikaments zur Hemmung der Aggregation von Blutplättchen.

15. Verwendung einer Verbindung gemäß Anspruch 1 zur Herstellung eines Medikaments zur Behandlung von thromboembolischen Störungen, die aus Thrombus- oder Emboliebildung, schädlicher Blutplättchenaggregation, Reokklusion nach einer Thrombolyse, Reperfusionsschädigung, Restenose, Atherosklerose, Schlaganfall, Myokardinfarkt und instabiler Angina ausgewählt sind.

16. Verwendung einer Verbindung gemäß Anspruch 1 zur Herstellung eines Medikaments zur Behandlung von rheumatoider Arthritis, Asthma, Allergien, posttraumatischer Lungeninsuffizienz, Organtransplantatabstoßung, septischem Schock, Psoriasis, Kontaktdermatitis, Osteoporose, Osteoarthritis, Tumormetastasen, diabetischer Retinopathie, Entzündungszuständen und Morbus Crohn.

## Revendications

1. Composé de Formule I: 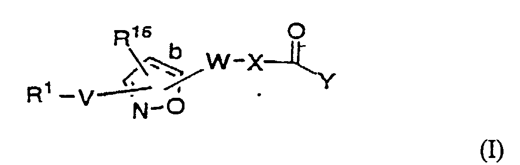 ou forme de sel pharmaceutiquement acceptable de cette dernière, dans laquelle:
b est une liaison simple ou double;
R¹ est choisi parmi R^{2a}(R³)N-, R²(R³)N(R²N=)C-, R^{2a}(R³)N(CH₂)_{p'}Z-, R²(R³)N(R²N=)C(CH₂)_{p"}Z-, R²(R³)N(R²N=)CN(R²)-, 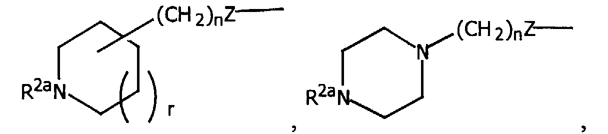 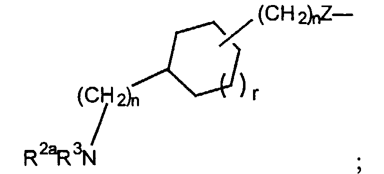
Z est choisi parmi une liaison, O, S, S(=O) et S(=O)₂;
R² et R³ sont indépendamment choisis parmi H, les groupes C₁-C₁₀ alkyle, C₃-C₆ alkényle, C₃-C₁₁ cycloalkyle, C₄-C₁₁ cycloalkylalkyle, C₆-C₁₀ aryle, C₇-C₁₁ arylalkyle, C₂-C₇ alkylcarbonyle, C₇-C₁₁ arylcarbonyle, C₂-C₁₀ alkoxycarbonyle, C₄-C₁₁ cycloalkoxycarbonyle, C₇-C₁₁ bicycloalkoxycarbonyle, C₇-C₁₁ aryloxycarbonyle, aryl(C₁-C₁₀ alkoxy)carbonyle, (C₁-C₆ alkyl)carbonyloxy(C₁-C₄ alkoxy)carbonyle, arylcarbonyloxy(C₁-C₄ alkoxy)carbonyle et (C₄-C₁₁ cycloalkylcarbonyl)oxy(C₁-C₄ alkoxy)carbonyle;
R^{2a} est R² ou R²(R³)N(R²N=)C- ;
V est choisi parmi une liaison simple, -(phényl)-Q-, -(pyridyl)-Q- et -(pyridazinyl)-Q-, lesdits résidus de phényle, de pyridyle et pyridazinyle étant substitués avec O à 2 groupes indépendamment choisis parmi H et les groupes C₁-C₁₀ alkyle, hydroxy, C₁-C₁₀ alkoxy, nitro, (C₁-C₁₀ alkyl)carbonyle, -N(R¹²)R¹³, cyano et halogène;
Q est choisi parmi une liaison simple, -(CH₂)ₘ-, -CH₂O- et -OCH₂-, pourvu que lorsque b est une liaison simple et R¹-V- est un substituant sur C5 de l'anneau central avec 5 membres de la Formule I, alors Q ne soit pas -CH₂O-;
W est -(C(R⁴)₂)_{n'}-C(=O)-N(R^{5a})- ou -C(=O)-N(R^{5a})-(C(R⁴)₂)_{n'}-;
X est -(C(R⁴)₂)_{n'}-C(R⁴)(R⁸)-C(R⁴)(R^{4a})-, avec la condition que lorsque n' est égal à 0 ou 1, alors au moins l'un des R^{4a} ou R⁸ soit différent de H ou du groupe méthyle;
Y est choisi parmi les groupes hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxycarbonyloxyalkyloxy, C₂-C₁₀ alkoxycarbonylalkyloxy, C₅-C₁₀ cycloalkylcarbonyloxyalkyloxy, C₅-C₁₀ cycloalkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyloxyalkyloxy, C₅-C₁₀ alkoxyalkylcarbonyloxyalkyloxy, C₅-C₁₀ (5-alkyl-1,3-dioxa-cyclopentène-2-on-yl)méthyloxy, C₁₀-C₁₄ (5-aryl-1,3-dioxa-cyclopentène-2-on-yl)méthyloxy; et (R²)(R³)N-(C₁-C₁₀ alkoxy)-;
R⁴ est choisi parmi H, les groupes C₁-C₁₀ alkyle, C₁-C₁₀ alkylcarbonyle, aryle, arylalkyle, cycloalkyle et cycloalkylalkyle;
alternativement deux groupes R⁴ sur des atomes de carbone adjacents peuvent se joindre pour former une liaison et constituer de cette façon une liaison double ou triple carbone-carbone entre les tels atomes de carbone adjacents;
R^{4a} est choisi parmi H, les groupes hydroxy, C₁-C₁₀ alkoxy, nitro, -N(R⁵)R^{5a}, -N(R¹²)R¹³, -N(R¹⁶)R¹⁷, C₁-C₁₀ alkyle substitué avec 0-3 R⁶, aryle substitué avec 0-3 R⁶, hétéroaryle substitué avec 0-3 R⁶ et C₁-C₁₀ alkylcarbonyle;
R^{4b} est choisi parmi H, les groupes C₁-C₆ alkyle, C₂-C₆ alkényle, C₂-C₆ alkynyle, C₃-C₇ cycloalkyle, C₇-C₁₄ bicycloalkyle, hydroxy, C₁-C₆ alkoxy, C₁-C₆ alkylthio, C₁-C₆ alkylsulfinyle, C₁-C₆ alkylsulfonyle, nitro, C₁-C₆ alkylcarbonyle, C₆-C₁₀ aryle, -N(R¹²)R¹³, halogène, CF₃, CN, (C₁-C₆ alkoxy)carbonyle, carboxy, pipéridinyle, morpholinyle et pyridinyle;
R⁵ est choisi parmi H, les groupes C₁-C₈ alkyle, C₃-C₆ alkényle, C₃-C₁₁ cycloalkyle, C₄-C₁₁ cycloalkylméthyle, C₆-C₁₀ aryle, C₇-C₁₁ arylalkyle, C₁-C₁₀ alkyle substitué avec 0-6 R^{4b};
R^{5a} est choisi parmi H, les groupes hydroxy, C₁-C₈ alkyle, C₃-C₆ alkényle, C₃-C₁₁ cycloalkyle, C₄-C₁₁ cycloalkylméthyle, C₁-C₆ alkoxy, benzyloxy, C₆-C₁₀ aryle, hétéroaryle, hétéroarylalkyle, C₇-C₁₁ arylalkyle, adamantylméthyle et C₁-C₁₀ alkyle substitué avec 0-2 R^{4b};
alternativement, R⁵ et R^{5a}, lorsque tous les deux sont des substituants sur le même atome d'azote (comme dans -N(R⁵)R^{5a}), peuvent être pris ensemble avec l'atome d'azote auquel ils sont attachés pour former du 3-azabicyclononyle, 1,2,3,4-tétrahydro-1-quinolinyle, 1,2,3,4-tétrahydro-2-isoquinolinyle, 1-pipéridinyle, 1-morpholinyle, 1-pyrrolidinyle, thiamorpholinyle, thiazolidinyle ou 1-pipérazinyle, chacun étant facultativement substitué avec les groupes C₁-C₆ alkyle, C₆-C₁₀ aryle, hétéroaryle, C₇-C₁₁ arylalkyle, (C₁-C₆ alkyl)carbonyle, (C₃-C₇ cycloalkyl)carbonyle, (C₁-C₆ alkoxy)carbonyle, (C₇-C₁₁ arylalkoxy)carbonyle, C₁-C₆ alkylsulfonyle ou C₆-C₁₀ arylsulfonyle;
R^{5b} est choisi parmi les groupes C₁-C₈ alkyle, C₂-C₆ alkényle, C₃-C₁₁ cycloalkyle, C₄-C₁₁ cycloalkylméthyle, C₆-C₁₀ aryle, C₇-C₁₁ arylalkyle et C₁-C₁₀ alkyle substitué avec 0-2 R^{4b};
R⁶ est choisi parmi H, les groupes C₁-C₁₀ alkyle, hydroxy, C₁-C₁₀ alkoxy, nitro, (C₁-C₁₀ alkyl)carbonyle, -N(R¹²)R¹³, cyano, halogène, CF₃, -CHO, -CO₂R⁵, -C(=O)R^{5a}, -CONR⁵R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, -SiMe₃, C₂-C₆ alkényle, C₃-C₁₁ cycloalkyle, C₄-C₁₁ cycloalkylméthyle, N(R⁵)R^{5a}, -S(O)ₘR⁵, -OR⁵;
le groupe C₆-C₁₀ aryle facultativement substitué avec 1-3 groupes choisis parmi un halogène, C₁-C₆ alkoxy, C₁-C₆ alkyle, CF₃, S(O)ₘMe et -NMe₂;
le groupe C₇-C₁₁ arylalkyle, ledit groupe aryle étant facultativement substitué avec 1-3 groupes choisis parmi un halogène, les groupes C₁-C₆ alkoxy, C₁-C₆ alkyle, CF₃, S(O)ₘMe et -NMe₂;
le groupe méthylènedioxy, lorsque R⁶ est un substituant sur le groupe aryle;
un anneau hétérocyclique avec 5-10 membres contenant les hétéroatomes 1-3 N, O ou S, où ledit anneau hétérocyclique peut être saturé, être partiellement saturé ou être complètement insaturé, ledit anneau hétérocyclique étant substitué avec 0-2 R⁷;
R⁷ est choisi parmi H, les groupes C₁-C₁₀ alkyle, hydroxy, C₁-C₁₀ alkoxy, nitro, (C₁-C₁₀ alkyl)carbonyle, -N(R¹²)R¹³, cyano, halogène, CF₃, -CHO, -CO₂R⁵, -C(=O)R^{5a}, -CON(R⁵)R^{5a}, -OC(=O)R^{5a}, -OC(=O)OR^{5b}, -OR^{5a}, -OC(=O)N(R⁵)R^{5a}, -OCH₂CO₂R⁵, -CO₂CH₂CO₂R⁵, NO₂, -NR^{5a}C(=O)R^{5a}, -NR^{5a}C(=O)OR^{5b}, -NR^{5a}C(=O)N(R⁵)R^{5a}, -NR^{5a}SO₂N(R⁵)R^{5a}, -NR^{5a}SO₂R⁵, -S(O)ₘR^{5a}, -SO₂N(R⁵)R^{5a}, C₂-C₆ alkényle, C₃-C₁₁ cycloalkyle, C₄-C₁₁ cycloalkylméthyle, C₆-C₁₀ aryle et C₇-C₁₁ arylalkyle;
R⁸ est choisi parmi les groupes R⁶; C₁-C₁₀ alkyle substitué avec 0-3 R⁶; C₂-C₁₀ alkényle substitué avec 0-3 R⁶, C₂-C₁₀ alkynyle substitué avec 0-3 R⁶, C₃-C₈ cycloalkyle substitué avec 0-3 R⁶, C₅-C₆ cycloalkényle substitué avec 0-3 R⁶, aryle substitué avec 0-3 R⁶;
un anneau hétérocyclique avec 5-10 membres contenant les hétéroatomes 1-3 N, O ou S, où ledit anneau hétérocyclique peut être saturé, être partiellement saturé ou être complètement insaturé, ledit anneau hétérocyclique étant substitué avec 0-2 R⁶;
R¹² et R¹³ sont indépendamment H, les groupes C₁-C₁₀ alkyle, (C₁-C₁₀ alkoxy)carbonyle, (C₁-C₁₀ alkyl)carbonyle, C₁-C₁₀ alkylsulfonyle, aryle(C₁-C₁₀ alkyl)sulfonyle, arylsulfonyle, aryl(C₂-C₁₀ alkényl)sulfonyle, hétéroarylsulfonyle, aryle, C₂-C₆ alkényle, C₃-C₁₁ cycloalkyle, C₄-C₁₁ cycloalkylalkyle, C₇-C₁₁ arylalkyle, C₇-C₁₁ arylcarbonyle, C₄-C₁₁ cycloalkoxycarbonyle, C₇-C₁₁ bicycloalkoxycarbonyle, C₇-C₁₁ aryloxycarbonyle, hétéroarylcarbonyle, hétéroarylalkylcarbonyle ou aryl(C₁-C₁₀ alkoxy)carbonyle où lesdits groupes aryles sont substitués avec 0-3 substituants choisis parmi les groupes C₁-C₄ alkyle, C₁-C₄ alkoxy, halogène, CF₃ et NO₂;
R¹⁵ est choisi parmi H, les groupes hydroxy, C₁-C₁₀ alkyle, C₂-C₁₀ alkényle, C₂-C₁₀ alkynyle, C₁-C₁₀ alkoxy, (C₁-C₁₀ alkoxy)carbonyle, -CO₂R⁵ et -C(=O)N(R⁵)R^{5a};
R¹⁶ est choisi parmi -C(=O)-O-R^{18a}, -C(=O)-R^{18b}, -C(=O)N(R^{18b})₂, -C(=O)NHSO₂R^{18a}, -C(=O)NHC(=O)R^{18b}, -C(=O)NHC(=O)OR^{18a}, -C(=O)NHSO₂NHR^{18b}, -C(=S)-NH-R^{18b}, -NH-C(=O)-O-R^{18a}, -NH-C(=O)-R^{18b}, -NH-C(=O)-NH-R^{18b}, -SO₂-O-R^{18a}, -SO₂-R^{18a}, -SO₂-N(R^{18b})₂, -SO₂-NHC(=O)OR^{18b}, -P(=S)(OR^{18a})₂, -P(=O)(OR^{18a})₂, -P(=S)(R^{18a})₂, -P(=O)(R^{18a})₂, et 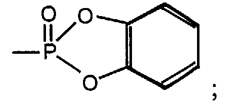
R¹⁷ est choisi parmi H, les groupes C₁-C₁₀ alkyle, C₂-C₆ alkényle, C₃-C₁₁ cycloalkyle, C₄-C₁₅ cycloalkylalkyle, aryle et aryl(C₁-C₁₀ alkyl)-;
R^{18a} est choisi parmi les groupes C₁-C₈ alkyle substitué avec 0-2 R¹⁹, C₂-C₈ alkényle substitué avec 0-2 R¹⁹, C₂-C₈ alkynyle substitué avec 0-2 R¹⁹, C₃-C₈ cycloalkyle substitué avec 0-2 R¹⁹, aryle substitué avec 0-4 R¹⁹, aryl(C₁-C₆ alkyl)- substitué avec 0-4 R¹⁹,
un système d'anneau hétérocyclique avec 5-10 membres ayant 1-3 hétéroatomes choisis indépendamment parmi O, S et N, ledit anneau hétérocyclique étant substitué avec 0-4 R¹⁹,
le groupe C₁-C₆ alkyle substitué avec un système d'anneau hétérocyclique avec 5-10 membres ayant 1-3 hétéroatomes choisis indépendamment parmi O, S et N, ledit anneau hétérocyclique étant substitué avec 0-4 R¹⁹,
R^{18b} est R^{18a} ou H;
R¹⁹ est choisi parmi H, un halogène, CF₃, CN, NO₂, NR¹²R¹³, les groupes C₁-C₈ alkyle, C₂-C₆ alkényle, C₂-C₆ alkynyle, C₃-C₁₁ cycloalkyle, C₄-C₁₁ cycloalkylalkyle, aryle, aryl(C₁-C₆ alkyl)-, C₁-C₆ alkoxy et C₁-C₄ alkoxycarbonyle;
m vaut de 0-2,
n vaut de 0-4,
n' vaut de 0-4,
p' vaut de 1-7,
p" vaut de 1-7, et
r vaut de 0-3;
à condition que n' soit choisi de telle sorte que le nombre d'atomes en chaîne liant R¹ et Y se situe dans le domaine de 8 à 18.

2. Composé de la revendication 1, dans lequel:
Z est une liaison, O ou S;
R² est H, un groupe aryl(C₁-C₁₀ alkoxy)carbonyle ou C₂-C₁₀ alkoxycarbonyle;
W est -(CH₂)_{n'}-C(=O)-N(R^{5a})-;
X est -C(R⁴)₂)_{n'}-C(R⁴)(R⁸)-CH(R⁴)-, avec la condition que lorsque n' est égal à 0 ou 1, alors R⁸ soit différent de H ou du groupe méthyle;
R⁵ est H ou un groupe C₁-C₁₀ alkyle substitué avec 0-6 R^{4b};
R⁶ est choisi parmi H, les groupes C₁-C₁₀ alkyle, hydroxy, C₁-C₁₀ alkoxy, nitro, (C₁-C₁₀ alkyl)carbonyle, -N(R¹²)R¹³, -N(R⁵)R^{5a}, -CO₂R⁵, -S(O)ₘR⁵, -OR⁵, cyano, halogène;
le groupe C₆-C₁₀ aryle facultativement substitué avec 1-3 groupes choisis parmi un halogène, les groupes C₁-C₆ alkoxy, C₁-C₆ alkyle, CF₃, -S(O)ₘMe et -NMe₂;
le groupe C₇-C₁₁ arylalkyle, ledit groupe aryle étant facultativement substitué avec 1-3 groupes choisis parmi un halogène, les groupes C₁-C₆ alkoxy, C₁-C₆ alkyle, CF₃, -S(O)ₘMe et -NMe₂;
le groupe méthylènedioxy, lorsque R⁶ est un substituant sur l'aryle;
un anneau hétérocyclique avec 5-10 membres contenant les hétéroatomes 1-3 N, O ou S, où ledit anneau hétérocyclique peut être saturé, être partiellement saturé ou être complètement insaturé, ledit anneau hétérocyclique étant substitué avec 0-2 R⁷;
R⁷ est choisi parmi H, les groupes C₁-C₁₀ alkyle, hydroxy, C₁-C₁₀ alkoxy, nitro, (C₁-C₁₀ alkyl)carbonyle, -N(R¹²)R¹³, cyano et halogène;
R⁸ est choisi parmi les groupes -CON(R⁵)NR^{5a}, -CO₂R⁵, C₁-C₁₀ alkyle substitué avec 0-3 R⁶, C₂-C₁₀ alkényle substitué avec 0-3 R⁶, C₂-C₁₀ alkynyle substitué avec 0-3 R⁶, C₃-C₈ cycloalkyle substitué avec 0-3 R⁶, C₅-C₆ cycloalkényle substitué avec 0-3 R⁶, aryle substitué avec 0-2 R⁶;
un anneau hétérocyclique avec 5-10 membres contenant les hétéroatomes 1-3 N, O ou S, où ledit anneau hétérocyclique peut être saturé, être partiellement saturé ou être complètement insaturé, ledit anneau hétérocyclique étant substitué avec 0-2 R⁶;
R¹² et R¹³ sont chacun indépendamment choisis parmi: H, les groupes C₁-C₁₀ alkyle, (C₁-C₁₀ alkoxy)carbonyle, (C₁-C₁₀ alkyl)carbonyle, C₁-C₁₀ alkylsulfonyle, aryl(C₁-C₁₀ alkyl)sulfonyle, arylsulfonyle, aryle, hétéroarylcarbonyle et hétéroarylalkylcarbonyle, où lesdits groupes aryles sont substitués avec 0-3 substituants choisis parmi: les groupes C₁-C₄ alkyle, C₁-C₄ alkoxy, halogène, CF₃ et NO₂; et
R¹⁵ est choisi parmi H, les groupes C₁-C₁₀ alkyle, C₂-C₁₀ alkényle, C₂-C₁₀ alkynyle, C₁-C₁₀ alkoxy, (C₁-C₁₀ alkoxy)carbonyle, -CO₂R⁵ et -C(=O)N(R⁵)R^{5a}.

3. Composé de la revendication 2, dans lequel:
Z est une liaison ou bien O;
W est -(CH₂)_{n'}-C(=O)-N(R^{5a})-; et
X est -C(R⁴)(R⁸)-CH(R⁴)-.

4. Composé de la revendication 2, dans lequel:
R¹ est R²NHC(=NR²)- ou R²NHC(=NR²)NH- et V est du phénylène ou du pyridylène; ou
R¹ est 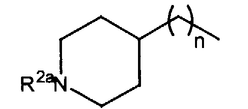
V est une liaison simple et n vaut 1 ou 2;
X est -CHR⁸CH₂ -;
Y est choisi parmi les groupes hydroxy, C₁-C₁₀ alkoxy, méthylcarbonyloxyméthoxy-, éthylcarbonyloxyméthoxy-, t-butylcarbonyloxyméthoxy-, cyclohexylcarbonyle, oxyméthoxy-, 1-(méthylcarbonyloxy)éthoxy-, 1-(éthylcarbonyloxy)éthoxy-, 1-(t-butylcarbonyloxy)éthoxy-, 1-(cyclohexylcarbonyloxy)éthoxy-, i-propyloxycarbonyloxyméthoxy-, t-butyloxycarbonyloxyméthoxy-, 1-(i-propyloxycarbonyloxy)éthoxy-, 1-(cyclohexyloxycarbonyloxy)éthoxy, 1-(t-butyloxycarbonyloxy)éthoxy-, diméthylaminoéthoxy-, diéthylaminoéthoxy-, (5-méthyl-1,3-dioxacyclopentène-2-on-4-yl)méthoxy-, (5-(t-butyl)-1,3-dioxacyclopentène-2-on-4-yl)méthoxy-, (1,3-dioxa-5-phényl-cyclopentène-2-on-4-yl)méthoxy- et 1-(2-(2-méthoxypropyl)carbonyloxy)éthoxy-;
R⁶ est choisi parmi H, les groupes C₁-C₄ alkyle, hydroxy, C₁-C₄ alkoxy, nitro, C₁-C₁₀ alkylcarbonyle, -N(R¹²)R¹³, -N(R⁵)R^{5a}, -CO₂R⁵, -S(O)ₘR⁵, -OR⁵, cyano, halogène;
le groupe C₆-C₁₀ aryle facultativement substitué avec 1-3 groupes choisis parmi un halogènegène, les groupes C₁-C₆ alkoxy, C₁-C₆ alkyle, CF₃, -S(O)ₘMe et -NMe₂;
le groupe méthylènedioxy, lorsque R⁶ est un substituant sur le groupe aryle;
un système d'anneau hétérocyclique choisi parmi le pyridinyle, furanyle, thiazolyle, thiényle, pyrrolyle, pyrazolyle, triazolyle, imidazolyle, benzofuranyle, indolyle, indolinyle, quinolinyle, isoquinolinyle, benzimidazolyle, pipéridinyle, tétrahydrofuranyle, pyranyle, pyridinyle, 3H-indolyle, pyrrolidinyle, pipéridinyle, indolinyle, isoxazolinyle et morpholinyle;
R⁸ est choisi parmi les groupes -CON(R⁵)NR^{5a}, -CO₂R⁵, C₁-C₁₀ alkyle substitué avec 0-3 R⁶, C₂-C₁₀ alkényle substitué avec 0-3 R⁶, C₂-C₁₀ alkynyle substitué avec 0-3 R⁶; C₃-C₈ cycloalkyle substitué avec 0-3 R⁶, aryle substitué avec 0-2 R⁶;
un système d'anneau hétérocyclique choisi parmi le pyridinyle, furanyle, thiazolyle, thiényle, pyrrolyle, pyrazolyle, triazolyle, imidazolyle, benzofuranyle, indolyle, indolinyle, quinolinyle, isoquinolinyle, isoxazolinyle, benzimidazolyle, pipéridinyle, tétrahydrofuranyle, pyranyle, pyridinyle, 3H-indolyle, pyrrolidinyle, pipéridinyle, indolinyle et morpholinyle, ledit anneau hétérocyclique étant substitué avec 0-2 R⁶;
R¹² est choisi parmi H, les groupes C₁-C₆ alkyle, (C₁-C₄ alkoxy)carbonyle, (C₁-C₆ alkyl)carbonyle, C₁-C₆ alkylsulfonyle, aryl(C₁-C₄ alkyl)sulfonyle, arylsulfonyle, aryle, pyridylcarbonyle et pyridylméthylcarbonyle, où lesdits groupes aryles sont substitués avec 0-3 substituants choisis parmi les groupes C₁-C₄ alkyle, C₁-C₄ alkoxy, halogène, CF₃ et NO₂; et
R¹³ est H

5. Composé de la revendication 1 ou forme de sel pharmaceutiquement acceptable de ce dernier, choisi parmi:
acide 3*(R,S)*-{5*(R,S)*-N-[3-(4-amidinophényl)isoxazolin-5-ylacétyl]amino}-3-phénylpropanoïque;
acide 3*(R,S)*-{5*(R,S)*-N-[3-(4-amidinophényl)isoxazolin-5-ylacétyl]amino}pentanoïque;
acide 3*(R)*-{5*(R,S)*-N-[3-(4-amidinophényl)isoxazolin-5-ylacétyl]amino}heptanoïque;
acide 3(*R,S*)-{5*(R,S)*-N-[3-(4-amidinophényl)isoxazolin-5-ylacétyl]amino}-4-(phénylthio)butanoïque;
acide 3*(R,S)*-{5*(R,S)*-N-[3-(4-amidinophényl)isoxazolin-5-ylacétyl]amino}-4-(phénylsulfonamido)butanoïque;.
acide 3*(R,S)*-{5(R,S)-N-[3-(4-amidinophényl)isoxazolin-5-ylacétyl]amino}-4-(n-butylsulfonamido)butanoïque;
acide 3*(S)*-(5*(R,S)*-N-[3-(4-amidinophényl)isoxazolin-5-ylacétyl]amino}-3-(adamantylméthylaminocarbonyl)propanoïque;
acide 3*(S)*-{5*(R,S)*-N-[3-(4-amidinophényl)isoxazolin-5-ylacétyl]amino}-3-(1-azabicyclo[3.2.2]-nonylcarbonyl)propanoïque;
acide 3(*S*)-{5*(R,S)*-N-[3-(4-amidinophényl)isoxazolin-5-ylacétyl]amino}-3-(phénéthylaminocarbonyl)propanoïque;
acide 3*(R)*-{5*(R,S)*-N-[3-(4-amidinophényl)isoxazolin-5-ylacétyl]amino}-3-(3-pyridyléthyl)propanoïque;
acide 3*(R)*-{5(R,S)-N-[3-(4-amidinophényl)isoxazolin-5-ylacétyl]amino}-3-(2-pyridyléthyl)propanoïque; et
acide 3*(R)*-{5*(R,S)*-N-[3-(4-amidinophényl)isoxazolin-5-ylacétyl]amino}-3-(phénylpropyl)propanoïque.

6. Composé de la revendication 1, dans lequel:
Z est une liaison, O ou S:
R² et R³ sont indépendamment choisis parmi H, les groupes aryl(C₁-C₁₀ alkoxy)carbonyle et (C₁-C₁₀ alkoxy)carbonyle;
R^{2a} est R² ou R²(R³)N(R²N=)C;
W est -C(R⁴)₂-C(=O)-N(R^{5a})- ou -C(=O)-N(R^{5a})-C(R⁴)₂-;
X est -C(R⁴)(R⁸)-CHR^{4a}-;
R⁴ est choisi parmi H, les groupes C₁-C₁₀ alkyle, C₁-C₁₀ alkylcarbonyle, aryle, arylalkyle, cycloalkyle et cycloalkylalkyle;
R^{4a} est choisi parmi les groupes hydroxy, C₁-C₁₀ alkoxy, nitro, -N(R⁵)R^{5a}, -N(R¹²)R¹³, -N(R¹⁶)R¹⁷, C₁-C₁₀ alkyle substitué avec 0-3 R⁶, aryle substitué avec 0-3 R⁶ et C₁-C₁₀ alkylcarbonyle;
R^{4b} est choisi parmi H, les groupes C₁-C₆ alkyle, C₂-C₆ alkényle, C₂-C₆ alkynyle, hydroxy, C₁-C₆ alkoxy, C₁-C₆ alkylthio, C₁-C₆ alkylsulfinyle, C₁-C₆ alkylsulfonyle, nitro, (C₁-C₆ alkyl)carbonyle, C₆-C₁₀ aryle, -N(R¹²)R¹³, halogène, CF₃, CN, (C₁-C₆ alkoxy)carbonyle, carboxy, pipéridinyle, morpholinyle et pyridinyle;
R⁵ est un groupe C₁-C₁₀ alkyle substitué avec 0-2 R^{4b};
R^{5a} est choisi parmi H, les groupes hydroxy, C₁-C₈ alkyle, C₃-C₆ alkényle, C₃-C₁₁ cycloalkyle, C₄-C₁₁ cycloalkylméthyle, C₁-C₆ alkoxy, benzyloxy, C₆-C₁₀ aryle, hétéroaryle, hétéroarylalkyle, C₇-C₁₁ arylalkyle, adamantylméthyle et C₁-C₁₀ alkyle substitué avec 0-2 R^{4b};
alternativement, R⁵ et R^{5a} peuvent être pris ensemble pour être du 3-azabicyclononyle, 1,2,3,4-tétrahydro-1-quinolinyle, 1,2,3,4-tétrahydro-2-isoquinolinyle, 1-pipéridinyle, 1-morpholinyle, 1-pyrrolidinyle, thiamorpholinyle, thiazolidinyle ou 1-pipérazinyle, chacun étant facultativement substitué avec les groupes C₁-C₆ alkyle, C₆-C₁₀ aryle, hétéroaryle, C₇-C₁₁ arylalkyle, (C₁-C₆ alkyl)carbonyle, (C₃-C₇ cycloalkyl)carbonyle, (C₁-C₆ alkoxy)carbonyle ou (C₇-C₁₁ arylalkoxy)carbonyle;
Y est choisi parmi les groupes hydroxy, C₁-C₁₀ alkyloxy, C₃-C₁₁ cycloalkyloxy, C₆-C₁₀ aryloxy, C₇-C₁₁ aralkyloxy, C₃-C₁₀ alkylcarbonyloxyalkyloxy, C₃-C₁₀ alkoxycarbonyloxyalkyloxy, C₂-C₁₀ alkoxycarbonylalkyloxy, C₅-C₁₀ cycloalkylcarbonyloxyalkyloxy, C₅-C₁₀ cycloalkoxycarbonyloxyalkyloxy, C₅-C₁₀ cycloalkoxycarbonylalkyloxy, C₇-C₁₁ aryloxycarbonylalkyloxy, C₈-C₁₂ aryloxycarbonyloxyalkyloxy, C₈-C₁₂ arylcarbonyloxyalkyloxy, C₅-C₁₀ alkoxyalkylcarbonyloxyalkyloxy, C₅-C₁₀ (5-alkyl-1,3-dioxa-cyclopentène-2-on-yl)méthyloxy et C₁₀-C₁₄ (5-aryl-1,3-dioxa-cyclopentène-2-on-yl)méthyloxy;
R⁶ et R⁷ sont choisis chacun indépendamment parmi H, les groupes C₁-C₁₀ alkyle, hydroxy, C₁-C₁₀ alkoxy, nitro, (C₁-C₁₀ alkyl)carbonyle, -N(R¹²)R¹³, cyano et halogène;
R¹² et R¹³ sont choisis chacun indépendamment parmi H, les groupes C₁-C₁₀ alkyle, (C₁-C₁₀ alkoxy)carbonyle, (C₁-C₁₀ alkyl)carbonyle, C₁-C₁₀ alkylsulfonyle, aryl(C₁-C₁₀ alkyl)sulfonyle, arylsulfonyle, hétéroarylcarbonyle, hétéroarylalkylcarbonyle et aryle, où lesdits groupes aryles sont substitués avec 0-3 substituants choisis parmi les groupes C₁-C₄ alkyle, C₁-C₄ alkoxy, halogène, CF₃ et NO₂;
R¹⁵ est choisi parmi: H, les groupes C₁-C₁₀ alkyle, C₂-C₁₀ alkényle, C₂-C₁₀ alkynyle, C₁-C₁₀ alkoxy, (C₁-C₁₀ alkoxy)carbonyle, -CO₂R⁵ et -C(=O)N(R⁵)R^{5a};
R¹⁶ est choisi parmi -C(=O)-O-R^{18a}, -C(=O)-R^{18b}, -C(=O)N(R^{18b})₂, -SO₂-R^{18a} et -SO₂-N(R^{18b})₂;
R¹⁷ est H ou un groupe C₁-C₄ alkyle;
R^{18a} est choisi parmi un groupe C₁-C₈ alkyle substitué avec 0-2 R¹⁹, C₂-C₈ alkényle substitué avec 0-2 R¹⁹, C₂-C₈ alkynyle substitué avec 0-2 R¹⁹, C₃-C₈ cycloalkyle substitué avec 0-2 R¹⁹, aryle substitué avec 0-4 R¹⁹, aryl(C₁-C₆ alkyl)- substitué avec 0-4 R¹⁹,
un système d'anneau hétérocyclique choisi parmi le pyridinyle, furanyle, thiazolyle, thiényle, pyrrolyle, pyrazolyle, triazolyle, imidazolyle, benzofuranyle, indolyle, indolinyle, quinolinyle, isoquinolinyle, isoxazolinyle, benzimidazolyle, pipéridinyle, tétrahydrofuranyle, pyranyle, pyrimidinyle, 3*H*-indolyle, pyrrolidinyle, pipéridinyle, indolinyle et morpholinyle, ledit anneau hétérocyclique étant substitué avec 0-4 R¹⁹;
un groupe C₁-C₆ alkyle substitué avec un système d'anneau hétérocyclique choisi parmi le pyridinyle, furanyle, thiazolyle, thiényle, pyrrolyle, pyrazolyle, imidazolyle, isoxazolinyle, benzofuranyle, indolyle, indolényle, quinolinyle, isoquinolinyle, benzimidazolyle, pipéridinyle, tétrahydrofuranyle, pyranyle, pyridinyle, 3*H*-indolyle, indolyle, pyrrolidinyle, pipéridinyle, indolinyle ou morpholinyle, ledit anneau hétérocyclique étant substitué avec 0-4 R¹⁹; et
les variables V, Q, R¹, R^{5b}, R^{18b}, R¹⁹, b, n, p', p" et r sont telles que définies dans la revendication 1.

7. Composé de la revendication 6 de Formule Ib: 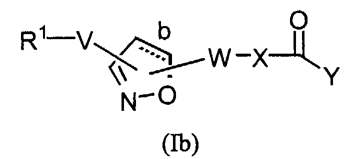 dans laquelle
R¹ est choisi parmi R^{2a}(R³)N-, -R²NH(R²N=)C-, R²NH(R²N=)CNH-, R^{2a}(R³)N(CH₂)_{p'}Z-, R²NH(R²N=)C(CH₂)_{p"}Z-, 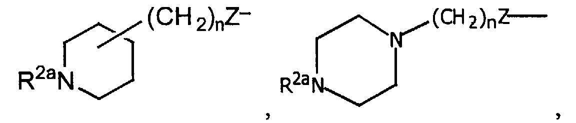 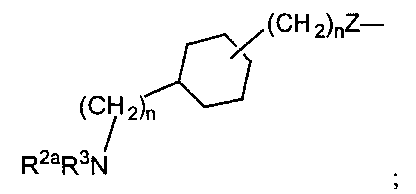
n vaut 0-1;
p' vaut 4-6;
p" vaut 2-4;
Z est une liaison ou O;
V est une liaison simple, -(phényl)- ou -(pyridinyl)-;
W est -C(R⁴)₂-C(=O)-N(R^{5a})- ou -C(=O)-N(R^{5a})-CH₂-;
X est -CH₂-CH(N(R¹⁶)R¹⁷)- ou -CH₂-CH(N(R⁵)R^{5a})-;
Y est choisi parmi les groupes hydroxy, C₁-C₁₀ alkoxy, méthylcarbonyloxyméthoxy-, t-butylcarbonyloxyméthoxy-, cyclohexylcarbonyloxyméthoxy-, 1-(méthylcarbonyloxy)éthoxy-, 1-(éthylcarbonyloxy)éthoxy-, 1-(t-butylcarbonyloxy)éthoxy-, 1-(cyclohexylcarbonyloxy)éthoxy-, *i*-propyloxycarbonyloxyméthoxy-, *t*-butyloxycarbonyloxyméthoxy-, 1-(i-propyloxycarbonyloxy)éthoxy-, 1-(cyclohexyloxycarbonyloxy)éthoxy-, 1-(*t*-butyloxycarbonyloxy)éthoxy-, diméthylaminoéthoxy-, diéthylaminoéthoxy-, (5-méthyl-1,3-dioxacyclopentène-2-on-4-yl)méthoxy-, (5-(*t*-butyl)-1,3-dioxacyclopentène-2-on-4-yl)méthoxy-, (1,3-dioxa-5-phényl-cyclopentène-2-on-4-yl)méthoxy- et 1-(2-(2-méthoxypropyl)carbonyloxy)éthoxy-,
R¹⁶ est choisi parmi -C(=O)-O-R^{18a}, -C(=O)-R^{18b}, -S(=O)₂-R^{18a} et -SO₂-N(R^{18b})₂;
R¹⁷ est H ou un groupe C₁-C₅ alkyle;
R^{18a} est choisi parmi les groupes C₁-C₈ alkyle substitué avec 0-2 R¹⁹, C₂-C₈ alkényle substitué avec 0-2 R¹⁹, C₂-C₈ alkynyle substitué avec 0-2 R¹⁹, C₃-C₈ cycloalkyle substitué avec 0-2 R¹⁹, aryle substitué avec 0-4 R¹⁹, aryl(C₁-C₆ alkyl)- substitué avec 0-4 R¹⁹,
un système d'anneau hétérocyclique choisi parmi le pyridinyle, furanyle, thiazolyle, thiényle, pyrrolyle, pyrazolyle, triazolyle, imidazolyle, benzofuranyle, indolyle, indolinyle, quinolinyle, isoquinolinyle, isoxazolinyle, benzimidazolyle, pipéridinyle, tétrahydrofuranyle, pyranyle, pyridinyle, 3*H*-indolyle, carbazole, pyrrolidinyle, pipéridinyle, indolinyle et morpholinyle, ledit anneau hétérocyclique étant substitué avec 0-4 R¹⁹;
un groupe C₁-C₆ alkyle substitué avec un système d'anneau hétérocyclique choisi parmi le pyridinyle, furanyle, thiazolyle, thiényle, pyrrolyle, pyrazolyle, imidazolyle, isoxazolinyle, benzofuranyle, indolyle, indolényle, quinolinyle, isoquinolinyle, benzimidazolyle, pipéridinyle, tétrahydrofuranyle, pyranyle, pyridinyle, 3*H*-indolyle, indolyle, pyrrolidinyle, pipéridinyle, indolinyle et morpholinyle, ledit anneau hétérocyclique étant substitué avec 0-4 R¹⁹;

8. Composé de la revendication 6 dans lequel:
R¹ est R²NH(R²N=)C- ou R²HN(R²N=)CNH- et V est du phénylène ou du pyridylène; ou bien
R¹ est 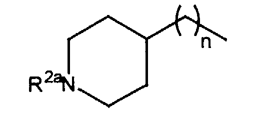
V est une liaison simple et n vaut 1 ou 2; et
R^{18a} est choisi parmi le groupe C₁-C₄ alkyle substitué avec 0-2 R¹⁹, C₂-C₄ alkényle substitué avec 0-2 R¹⁹, C₂-C₄ alkynyle substitué avec 0-2 R¹⁹, C₃-C₇ cycloalkyle substitué avec 0-2 R¹⁹, aryle substitué avec 0-4 R¹⁹, aryl(C₁-C₄ alkyl)- substitué avec 0-4 R¹⁹,
un système d'anneau hétérocyclique choisi parmi le pyridinyle, furanyle, thiazolyle, thiényle, pyrrolyle, pyrazolyle, triazolyle, imidazolyle, benzofuranyle, indolyle, indolinyle, quinolinyle, isoquinolinyle, isoxazolinyle, benzimidazolyle, pipéridinyle, tétrahydrofuranyle, pyranyle, pyrimidinyle, 3*H*-indolyle, pyrrolidinyle, pipéridinyle, indolinyle, isoxazolinyle et morpholinyle, ledit anneau hétérocyclique étant substitué avec 0-4 R¹⁹;
un groupe C₁-C₄ alkyle substitué avec un système d'anneau hétérocyclique choisi parmi le pyridinyle, furanyle, thiazolyle, thiényle, pyrrolyle, pyrazolyle, imidazolyle, isoxazolinyle, benzofuranyle, indolyle, indolényle, quinolinyle, isoquinolinyle, benzimidazolyle, pipéridinyle, tétrahydrofuranyle, pyranyle, pyridinyle, 3*H*-indolyle, indolyle, pyrrolidinyle, pipéridinyle, indolinyle, isoxazolinyle et morpholinyle, ledit anneau hétérocyclique étant substitué avec 0-4 R¹⁹.

9. Composé de la revendicationl ou formes de sel pharmaceutiquement acceptable de ce dernier qui est choisi parmi:
acide N³-[2-{3-(4-formamidinophényl)-isoxazolin-5(*R,S*)-yl}-acétyl]-N2-(phénylsulfonyl)-2,3 -(S)-diaminopropanoïque;
acide N³-[2-{3-(4-formamidinophényl)-isoxazolin-5(*R,S*)-yl}-acétyl]-N2-(4-méthyl-phényl-sulfonyl)-2,3-(S)-diaminopropanoïque;
acide N³-[2-{3-(4-formamidinophényl)-isoxazolin-5(*R,S*)-yl}-acétyl]-N2-(butanesulfonyl)-2,3 -(S)-diaminopropanoïque;
acide N³-[2-{3-(4-formamidinophényl)-isoxazolin-5(*R,S*)-yl}-acétyl]-N2-(propanesulfonyl)-2,3 -(S)-diaminopropanoïque;
acide N³-[2-{3-(4-formamidinophényl)-isoxazolin-5(*R,S*)-yl}-acétyl]-N2-(éthanesulfonyl)-2,3 -(S)-diaminopropanoïque;
acide N³-[2-{3-(4-formamidinophényl)-isoxazolin-5(*R,S*)-yl}-acétyl]-N2-(méthyloxycarbonyl)-2,3-(S)-diaminopropanoïque;
acide N³-[2-{3-(4-formamidinophényl)-isoxazolin-5(*R,S*)-yl}-acétyl]-N2-(éthyloxycarbonyl)-2,3-(S)-diaminopropanoïque;
acide N³-[2-{3-(4-formamidinophényl)-isoxazolin-5(*R,S*)-yl}-acétyl]-N2-(1-propyloxycarbonyl)-2,3 -(S)-diaminopropanoïque;
acide N³-[2-{3-(4-formamidinophényl)-isoxazolin-5(*R,S*)-yl}-acétyl]-N2-(2-propyloxycarbonyl)-2,3-(S)-diaminopropanoïque;
acide N³-[2-{3-(4-formamidinophényl)-isoxazolin-5(*R,S*)-yl}-acétyl]-N2-(n-butyloxycarbonyl)-2,3-(S)-diaminopropanoïque;
acide N³-[2-{3-(4-formamidinophényl)-isoxazolin-5(*R*)-yl}-acétyl]-N2-(n-butyloxycarbonyl)-2,3-(S)-diaminopropanoïque;
acide N³-[2-{3-(4-formamidinophényl)-isoxazolin-5(*S*)-yl}-acétyl]-N2-(n-butyloxycarbonyl)-2,3-(S)-diaminopropanoïque;
acide N³-[2-{3-(4-formamidinophényl)-isoxazolin-5(*R*)-yl}-acétyl]-N2-(n-butyloxycarbonyl)-2,3-(R)-diaminopropanoïque;
acide N³-[2-{3-(4-formamidinophényl)-isoxazolin-5(*S*)-yl}-acétyl]-N2-(n-butyloxycarbonyl)-2,3-(R)-diaminopropanoïque;
acide N³-[2-{3-(4-formamidinophényl)-isoxazolin-5(*R,S*)-yl}-acétyl]-N2-(2-butyloxycarbonyl)-2,3-(S)-diaminopropanoïque;
acide N³-[2-{3-(4-formamidinophényl)-isoxazolin-5(*R,S*)-yl}-acétyl]-N2-(1-(2-méthyl)-propyloxycarbonyl)-2,3-(S)-diaminopropanoïque;
acide N³-[2-{3-(4-formamidinophényl)-isoxazolin-5(*R,S*)-yl}-acétyl]-N2-(2-(2-méthyl)-propyloxycarbonyl)-2,3-(S)-diaminopropanoïque;
acide N³-[2-{3-(4-formamidinophényl)-isoxazolin-5(*R,S*)-yl}-acétyl]-N2-(benzyloxycarbonyl)-2,3-(S)-diaminopropanoïque;
acide N³-[2-{3-(4-formamidinophényl)-isoxazolin-5(*R*)-yl}-acétyl]-N2-(benzyloxycarbonyl)-2,3-(S)-diaminopropanoïque;
acide N³-[2-{3-(4-formamidinophényl)-isoxazolin-5(*S*)-yl}-acétyl]-N2-(benzyloxycarbonyl)-2,3-(S)-diaminopropanoïque;
acide N³-[2-{3-(4-formamidinophényl)-isoxazolin-5(*R,S*)-yl}-acétyl]-N2-(4-méthylbenzyloxycarbonyl)-2,3-(S)-diaminopropanoïque;
acide N³-[2-{3-(4-formamidinophényl)-isoxazolin-5(*R,S*)-yl}-acétyl]-N2-(4-méthoxybenzyloxycarbonyl)-2,3-(S)-diaminopropanoïque;
acide N³-[2-{3-(4-formamidinophényl)-isoxazolin-5(*R,S*)-yl}-acétyl]-N2-(4-chlorobenzyloxycarbonyl)-2,3-(S)-diaminopropanoïque;
acide N³-[2-{3-(4-formamidinophényl)-isoxazolin-5(*R,S*)-yl}-acétyl]-N2-(4-bromobenzyloxycarbonyl)-2,3-(S)-diaminopropanoïque;
acide N³-[2-{3-(4-formamidinophényl)-isoxazolin-5(*R,S*)-yl}-acétyl]-N2-(4-fluorobenzyloxycarbonyl)-2,3-(S)-diaminopropanoïque;
acide N³-[2-{3-(4-formamidinophényl)-isoxazolin-5(*R,S*)-yl}-acétyl]-N2-(4-phénoxybenzyloxycarbonyl)-2,3-(S)-diaminopropanoïque;
acide N³-[2-{3-(4-formamidinophényl)-isoxazolin-5(*R,S*)-yl}-acétyl]-N2-(2-(méthyloxyéthyl)-oxycarbonyl)-2,3-(S)-diaminopropanoïque;
acide N³-[2-{3-(4-formamidinophényl)-isoxazolin-5(*R,S*)-yl}-acétyl]-N2-(2-pyridinylcarbonyl)-2,3-(S)-diaminopropanoïque;
acide N³-[2-{3-(4-formamidinophényl)-isoxazolin-5(*R,S*)-yl}-acétyl]-N2-(3-pyridinylcarbonyl)-2,3-(S)-diaminopropanoïque;
acide N³-[2-{3-(4-formamidinophényl)-isoxazolin-5(*R,S*)-yl}-acétyl]-N2-(4-pyridinyl-carbonyl)-2,3-(S)-diaminopropanoïque;
acide N³-[2-{3-(4-formamidinophényl)-isoxazolin-5(*R,S*)-yl}-acétyl]-N2-(2-(2-pyridinyl)-acétyl)-2,3-(S)-diaminopropanoïque;
acide N³-[2-{3-(4-formamidinophényl)-isoxazolin-5(*R,S*)-yl}-acétyl]-N2-(2-(3-pyridinyl)-acétyl)-2,3-(S)-diaminopropanoïque;
acide N³-[2-{3-(4-formamidinophényl)-isoxazolin-5(*R,S*)-yl}-acétyl]-N2-(2-(4-pyridinyl)-acétyl)-2,3-(S)-diaminopropanoïque;
acide N³-[2-{3-(4-formamidinophényl)-isoxazolin-5(*R,S*)-yl}-acétyl]-N2-(2-pyridyl-méthyloxycarbonyl)-2,3-(S)-diaminopropanoïque;
acide N³-[2-{3-(4-formamidinophényl)-isoxazolin-5(*R,S*)-yl}-acétyl]-N2-(3-pyridyl-méthyloxycarbonyl)-2,3-(S)-diaminopropanoïque;
acide N³-[2-{3-(4-formamidinophényl)-isoxazolin-5(*R,S*)-yl}-acétyl]-N2-(4-pyridyl-méthyloxycarbonyl)-2,3-(S)-diaminopropanoïque;
acide N³-[2-{3-(4-formamidinophényl)-isoxazolin-5(*R,S*)-yl}-acétyl]-N2-(4-butyloxyphénylsulfonyl)-2,3-(S)-diaminopropanoïque;
acide N³-[2-{3-(4-formamidinophényl)-isoxazolin-5(*R,S*)-yl}-acétyl]-N2-(2-thiénylsulfonyl)-2,3-(S)-diaminopropanoïque;
acide N³-[2-{3-(4-formamidinophényl)-isoxazolin-5(*R,S*)-yl}-acétyl]-N2-(3-méthylphénylsulfonyl)-2,3-(R, S)-diaminopropanoïque;
acide N³-[2-{3-(4-formamidinophényl)-isoxazolin-5(*R,S*)-yl}-acétyl]-N2-(3-méthylphénylsulfonyl)-2,3-(S)-diaminopropanoïque;
acide N³-[2-{3-(4-formamidinophényl)-isoxazolin-5(*R,S*)-yl}-acétyl]-N2-(3-méthylphénylsulfonyl)-2,3-(R)-diaminopropanoïque;
acide N³-[2-{3-(4-formamidinophényl)-isoxazolin-5(*R*)-yl}-acétyl]-N2-(3-méthylphénylsulfonyl)-2,3-(S)-diaminopropanoïque;
acide N³-[2-{3-(4-formamidinophényl)-isoxazolin-5(*S*)-yl}-acétyl]-N2-(3-méthylphénylsulfonyl)-2,3-(S)-diaminopropanoïque;
acide N³-[2-{3-(4-formamidinophényl)-isoxazolin-5(*S*)-yl}-acétyl]-N2-(3-méthylphénylsulfonyl)-2,3-(R)-diaminopropanoïque;
acide N³-[2-{3-(4-formamidinophényl)-isoxazolin-5(*R*)-yl}-acétyl]-N2-(3-méthylphénylsulfonyl)-2,3-(R)-diaminopropanoïque;
acide N³-[2-{3-(4-formamidinophényl)-isoxazolin-5*(R,S*)-yl}-acétyl]-N2-(4-iodophénylsulfonyl)-2,3-(S)-diaminopropanoïque;
acide N³-[2-{3-(4-formamidinophényl)-isoxazolin-5(*R,S*)-yl}-acétyl]-N2-(3-trifluorométhylphénylsulfonyl)-2,3-(S)-diaminopropanoïque;
acide N³-[2-{3-(4-formamidinophényl)-isoxazolin-5(*R,S*)-yl}-acétyl]-N2-(3-chlorophénylsulfonyl)-2,3-(S )-diaminopropanoïque;
acide N³-[2-{3-(4-formamidinophényl)-isoxazolin-5(*R,S*)-yl}-acétyl]-N2-(3-2-méthoxycarbonylphénylsulfonyl)-2,3-(S)-diaminopropanoïque;
acide N³-[2-{3-(4-formamidinophényl)-isoxazolin-5(*R,S*)-yl}-acétyl]-N2-(2,4,6-triméthylphénylsulfonyl)-2,3-(S)-diaminopropanoïque;
acide N³-[2-{3-(4-formamidinophényl)-isoxazolin-5(*R,S*)-yl}-acétyl]-N2-(2-chlorophénylsulfonyl)-2,3-(S)-diaminopropanoïque;
acide N³-[2-{3-(4-formamidinophényl)-isoxazolin-5*(R,S*)-yl}-acétyl]-N2-(4-trifluorométhylphénylsulfonyl)-2,3-(S)-diaminopropanoïque;
acide N³-[2-{3-(4-formamidinophényl)-isoxazolin-5(*R,S*)-yl}-acétyl]-N2-(2-trifluorométhylphénylsulfonyl)-2,3-(S)-diaminopropanoïque;
acide N³-[2-{3-(4-formamidinophényl)-isoxazolin-5(*R,S*)-yl}-acétyl]-N2-(2-fluorophénylsulfonyl)-2,3-(S)-diaminopropanoïque;
acide N³-[2-{3-(4-formamidinophényl)-isoxazolin-5*(R,S*)-yl}-acétyl]-N2-(4-fluorophénylsulfonyl)-2,3-(S)-diaminopropanoïque;
acide N³-[2-{3-(4-formamidinophényl)-isoxazolin-5*(R,S*)-yl}-acétyl]-N2-(4-méthoxyphénylsulfonyl)-2,3-(S)-diaminopropanoïque;
acide N³-[2-{3-(4-formamidinophényl)-isoxazolin-5(*R,S*)-yl}-acétyl]-N2-(2,3,5,6-tétraméthylphénylsulfonyl)-2,3-(S)-diaminopropanoïque;
acide N³-[2-{3-(4-formamidinophényl)-isoxazolin-5(*R,S*)-yl}-acétyl]-N2-(4-cyanophénylsulfonyl)-2,3-(S)-diaminopropanoïque;
acide N³-[2-{3-(4-formamidinophényl)-isoxazolin-5(*R,S*)-yl}-acétyl]-N2-(4-chlorophénylsulfonyl)-2,3-(S)-diaminopropanoïque;
acide N³-[2-{3-(4-formamidinophényl)-isoxazolin-5(*R,S*)-yl}-acétyl]-N2-(4-propylphénylsulfonyl)-2,3-(S)-diaminopropanoïque;
acide N³-[2-{3-(4-formamidinophényl)-isoxazolin-5(*R,S*)-yl}-acétyl]-N2-(2-phényléthylsulfonyl)-2,3-(S)-diaminopropanoïque;
acide N³-[2-{3-(4-formamidinophényl)-isoxazolin-5*(R,S*)-yl}-acétyl]-N2-(4-isopropylphénylsulfonyl)-2,3-(S)-diaminopropanoïque;
acide N³-[2-{3-(4-formamidinophényl)-isoxazolin-5(*R,S*)-yl}-acétyl]-N2-(3-phénylpropylsulfonyl)-2,3-(S)-diaminopropanoïque;
acide N³-[2-{3-(4-formamidinophényl)-isoxazolin-5(*R,S*)-yl}-acétyl]-N2-(3-pyridylsulfonyl)-2,3-(S)-diaminopropanoïque;
acide N³-[2-{3-(4-formamidinophényl)-isoxazolin-5(*R,S*)-yl}-acétyl]-N2-(phénylaminosulfonyl)-2,3-(S)-diaminopropanoïque;
acide N³-[2-{3-(4-formamidinophényl)-isoxazolin-5(*R,S*)-yl}-acétyl]-N2-(benzylaminosulfonyl)-2,3-(S)-diaminopropanoïque;
acide N³-[2-{3-(4-formamidinophényl)-isoxazolin-5(*R,S*)-yl}-acétyl]-N2-(diméthylaminosulfonyl)-2,3-(S)-diaminopropanoïque,
acide N³-[2-{3-(2-fluoro-4-formamidinophényl)-isoxazolin-5(*R,S*)-yl}-acétyl]-N2-(3-méthylphénylsulfonyl)-2,3-(S)-diaminopropanoïque,
acide N³-[2-{3-(2-formamidino-5-pyridinyl)-isoxazolin-5(*R,S*)-yl}-acétyl]-N2-(n-butyloxycarbonyl)-2,3-(S)-diaminopropanoïque,
acide N³-[2-{3-(2-formamidino-5-pyridinyl)-isoxazolin-5(*R,S*)-yl}-acétyl]-N2-{3-méthylphénylsulfonyl)-2,3-(S)-diaminopropanoïque,
acide N³-[2-{3-(3-formamidino-6-pyridinyl)-isoxazolin-5(*R,S*)-yl}-acétyl]-N2-(n-butyloxycarbonyl)-2,3-(S)-diaminopropanoïque,
acide N³-[2-{3-(3-formamidino-6-pyridinyl)-isoxazolin-5(*R,S*)-yl}-acétyl]-N2-(3-méthylphénylsulfonyl)-2,3-(S)-diaminopropanoïque,
acide N³-[2-{3-(4-formamidinophényl)-isoxazolin-5(*R,S*)-yl}-acétyl]-N2-(phénylaminocarbonyl)-2,3-(S)-diaminopropanoïque;
acide N³-[2-{3-(4-formamidinophényl)-isoxazolin-5(*R,S*)-yl}-acétyl]-N2-(4-fluorophénylaminocarbonyl)-2,3-(S)-diaminopropanoïque;
acide N³-[2-{3-(4-formamidinophényl)-isoxazolin-5(*R,S*)-yl}-acétyl]-N2-(1-naphthylaminocarbonyl)-2,3-(S)-diaminopropanoïque;
acide N³-[2-{3-(4-formamidinophényl)-isoxazolin-5(*R,S*)-yl}-acétyl]-N2-(benzylaminocarbonyl)-2,3-(S)-diaminopropanoïque;
acide N³-[2-{3-(4-formamidinophényl)-isoxazolin-5(*R,S*)-yl}-acétyl]-N2-(3-bromo-2-thiénylsulfonyl)-2,3-(S)-diaminopropanoïque;
acide N³-[2-{3-(4-formamidinophényl)-isoxazolin-5(*R,S*)-yl}-acétyl]-N2-(3-méthyl-2-benzothiénylsulfonyl)-2,3-(S)-diaminopropanoïque,
acide N³-[2-{3-(4-formamidinophényl)-isoxazolin-5(*R,S*)-yl}-acétyl]-N2-(isobutyloxycarbonyl)-2,3-(S)-diaminopropanoïque,
acide N³-[2-{3-(4-formamidinophényl)-isoxazolin-5(*R*)-yl}-acétyl]-N2-(isobutyloxycarbonyl)-2,3-(S)-diaminopropanoïque,
acide N³-[2-{3-(4-formamidinophényl)-isoxazolin-5(*S*)-yl}-acétyl]-N2-(isobutyloxycarbonyl)-2,3-(S)-diaminopropanoïque,
acide N³-[2-{3-(4-formamidinophényl)-isoxazolin-5(*R,S*)-yl}-acétyl]-N2-(2-cyclopropyléthoxycarbonyl)-2,3-(S)-diaminopropanoïque,
acide N³-[2-{3-(4-formamidinophényl)-isoxazolin-5(*R*)-yl}-acétyl]-N2-(2-cyclopropyléthoxycarbonyl)-2,3-(S)-diaminopropanoïque;
acide N³-[2-{3-(4-formamidinophényl)-isoxazolin-5(*S*)-yl}-acétyl]-N2-(2-cyclopropyléthoxycarbonyl)-2,3-(S)-diaminopropanoïque;
acide N³-[2-{3-(4-guanidinophényl)-isoxazolin-5(*R,S*)-yl}-acétyl]-N2-(n-butyloxycarbonyl)-2,3-(S)-diaminopropanoïque;
acide N³-[2-{3-(4-guanidinophényl)-isoxazolin-5(*R*)-yl}-acétyl]-N2-(n-butyloxycarbonyl)-2,3-(S)-diaminopropanoïque;
acide N³-[2-{3-(4-guanidinophényl)-isoxazolin-5(*R*)-yl}-acétyl]-N2-(3-méthylphénylsulfonyl)-2,3-(S)-diaminopropanoïque;
acide N³-[2-{5-(4-formamidinophényl)-isoxazolin-3(*R,S*)-yl}-acétyl]-N2-(n-butyloxycarbonyl)-2,3-(S)-diaminopropanoïque.

10. Ester d'un promédicament d'un composé de la revendication 9, ledit ester étant choisi parmi du:
méthyle, éthyle, isopropyle, méthylcarbonyloxyméthyl-, éthylcarbonyloxyméthyl-, t-butylcarbonyloxyméthyl-, cyclohexylcarbonyloxyméthyl-, 1-(méthylcarbonyloxy)éthyl-, 1-(éthylcarbonyloxy)éthyl-, 1-(t-butylcarbonyloxy)éthyl-, 1-(cyclohexylcarbonyloxy)éthyl-, i-propyloxycarbonyloxyméthyl-, cyclohexylcarbonyloxyméthyl-, t-butyloxycarbonyloxyméthyl-, 1-(i-propyloxycarbonyloxy)éthyl-, 1-(cyclohexyloxycarbonyloxy)éthyl-, 1-(t-butyloxycarbonyloxy)éthyl-, diméthylaminoéthyl-, diéthylaminoéthyl-, (5-méthyl-1,3-dioxacyclopentène-2-on-4-yl)méthyl-, (5-(t-butyl)-1,3-dioxacyclopentène-2-on-4-yl)méthyl-, (1,3-dioxa-5-phényl-cyclopentène-2-on-4-yl)méthyl- et 1-(2-(2-méthoxypropyl)carbonyloxy)éthyl-.

11. Composé de la revendication 1 ou forme de sel pharmaceutiquement acceptable de ce dernier, qui est du
méthyl-N³-[2{3-(4-formamidinophényl)-isoxazolin-5(*R*)-yl}-acétyl]-N2(n-butyloxycarbonyl)-2,3-(S)-diaminopropionate.

12. Composition pharmaceutique comprenant une quantité thérapeutiquement efficace d'un composé, tel que défini dans n'importe laquelle des revendications 1 à 11, ainsi qu'un support pharmaceutiquement acceptable.

13. Utilisation d'un composé tel que défini dans n'importe laquelle des revendications 1 à 11 pour la préparation d'un médicament pour la prévention ou le traitement de la thrombose.

14. Utilisation d'un composé tel que défini dans n'importe laquelle des revendications 1 à 11 pour la préparation d'un médicament pour inhiber l'agrégation de plaquettes sanguines.

15. Utilisation d'un composé de la revendication 1 pour la préparation d'un médicament pour le traitement de troubles thromboemboliques pris parmi une formation de thrombus ou d'embolus, une agrégation nocive de plaquettes, une réocclusion à la suite d'une thrombolyse, des lésions dues à des perfusions répétées, la resténose, l'athérosclérose, une attaque, un infarctus du myocarde et un angor instable.

16. Utilisation d'un composé de la revendication 1 pour la préparation d'un médicament pour le traitement de polyarthrite rhumatoïde, d'asthme, d'allergies, d'insuffisance respiratoire chez l'adulte, de rejet de greffes d'organes, de choc septique, de psoriasis, de dermatite de contact, d'ostéoporose, d'ostéoarthrite, de métastases tumorales, de rétinopathie diabétique, d'état inflammatoire et d'affection intestinale inflammatoire.
